(19) Europäisches Patentamt / European Patent Office / Office européen des brevets

(11) **EP 2 949 661 A1**

(12) **EUROPEAN PATENT APPLICATION**

(43) Date of publication:
**02.12.2015 Bulletin 2015/49**

(51) Int Cl.:
***C07K 14/415*** (2006.01)     ***C12N 15/82*** (2006.01)

(21) Application number: **15162086.1**

(22) Date of filing: **11.08.2010**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO SE SI SK SM TR**<br><br>(30) Priority: **19.08.2009 EP 09168166**<br>**19.08.2009 EP 09168159**<br>**19.08.2009 EP 09168213**<br>**21.08.2009 EP 09168392**<br>**21.08.2009 EP 09168433**<br>**21.08.2009 EP 09168402**<br>**14.10.2009 US 251309 P**<br>**14.10.2009 US 251307 P**<br>**14.10.2009 US 251308 P**<br>**14.10.2009 US 251312 P**<br>**14.10.2009 US 251306 P**<br>**14.10.2009 US 251311 P**<br><br>(62) Document number(s) of the earlier application(s) in accordance with Art. 76 EPC:<br>**10740676.1 / 2 467 394** | (71) Applicant: **BASF Plant Science Company GmbH**<br>**67056 Ludwigshafen (DE)**<br><br>(72) Inventor: **Sanz Molinero, Ana Isabel**<br>**28035 Madrid (ES)**<br><br>(74) Representative: **Hennin, Caroline Marie Odile et al**<br>**BASF SE**<br>**Global Intellectual Property**<br>**Carl-Bosch-Strasse 38**<br>**67056 Ludwigshafen (DE)**<br><br>Remarks:<br>•The complete document including Reference Tables and the Sequence Listing can be downloaded from the EPO website<br>•This application was filed on 31-03-2015 as a divisional application to the application mentioned under INID code 62. |

(54)     **PLANTS HAVING ENHANCED YIELD-RELATED TRAITS AND A METHOD FOR MAKING THE SAME**

(57)     The present invention relates generally to the field of molecular biology and concerns a method for enhancing yield-related traits in plants by modulating expression in a plant of a nucleic acid encoding a FSM1-like (Fruit Sant/Myb) polypeptide, or a PIF3-like (PHYTOCHROME INTERACTING FACTOR) polypeptide, or an Uroporphyrinogen III decarboxylase (UROD) polypeptide, or an AS-MTT (Abiotic Stress Membrane Tethered Transcription factor) polypeptide, or an EXO-1 polypeptide, or a YiAP2 (Yield increasing Apetala 2) polypeptide. The present invention also concerns plants having modulated expression of a nucleic acid encoding a FSM1-like polypeptide, which plants have enhanced yield-related traits relative to corresponding wild type plants or other control plants. The invention also provides constructs useful in the methods of the invention.

EP 2 949 661 A1

**Description**

The present invention relates generally to the field of molecular biology and concerns a method for enhancing yield-related traits in plants by modulating expression in a plant of a nucleic acid encoding a FSM1-like (Fruit Sant/Myb) polypeptide. The present invention also concerns plants having modulated expression of a nucleic acid encoding a FSM1-like polypeptide, which plants have enhanced yield-related traits relative to corresponding wild type plants or other control plants. The invention also provides constructs useful in the methods of the invention.

The present invention relates generally to the field of molecular biology and concerns a method for enhancing yield-related traits in plants by modulating expression in a plant of a nucleic acid encoding a PIF3-like (PHYTOCHROME INTERACTING FACTOR) polypeptide. The present invention also concerns plants having modulated expression of a nucleic acid encoding a PIF3-like polypeptide, which plants have enhanced yield-related traits relative to corresponding wild type plants or other control plants. The invention also provides constructs useful in the methods of the invention.

The present invention relates generally to the field of molecular biology and concerns a method for enhancing yield-related traits in plants by modulating expression in a plant of a nucleic acid encoding an Uroporphyrinogen III decarboxylase (UROD) polypeptide. The present invention also concerns plants having modulated expression of a nucleic acid encoding a UROD polypeptide, which plants have enhanced yield-related traits relative to corresponding wild type plants or other control plants. The invention also provides constructs useful in the methods of the invention.

The present invention relates generally to the field of molecular biology and concerns a method for enhancing various economically important yield-related traits in plants. More specifically, the present invention concerns a method for enhancing yield-related traits in plants by modulating expression in a plant of a nucleic acid encoding an AS-MTT (Abiotic Stress Membrane Tethered Transcription factor) polypeptide. The present invention also concerns plants having modulated expression of a nucleic acid encoding an AS-MTT polypeptide, which plants have enhanced yield-related traits relative to control plants. The invention also provides constructs comprising AS-MTT-encoding nucleic acids, useful in performing the methods of the invention.

The present invention relates generally to the field of molecular biology and concerns a method for enhancing yield-related traits in plants by modulating expression in a plant of a nucleic acid encoding an EXO-1 polypeptide. The present invention also concerns plants having modulated expression of a nucleic acid encoding an EXO-1 polypeptide, which plants have enhanced yield-related traits relative to corresponding wild type plants or other control plants. The invention also provides constructs useful in the methods of the invention.

The present invention relates generally to the field of molecular biology and concerns a method for enhancing yield-related traits in plants by modulating expression in a plant of a nucleic acid encoding a YiAP2 (Yield increasing Apetala 2) polypeptide. The present invention also concerns plants having modulated expression of a nucleic acid encoding a YiAP2 polypeptide, which plants have enhanced yield-related traits relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta 218, 1-14, 2003). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

It has now been found that various yield-related traits may be improved in plants by modulating expression in a plant of a nucleic acid encoding a FSM1-like (Fruit Sant/Myb) polypeptide, or a PIF3-like (Phytochrome Interacting Factor 3) polypeptide, or a UROD (Uroporphyrinogen III decarboxylase) polypeptide, or an AS-MTT (Abiotic Stress Membrane Tethered Transcription factor) polypeptide, or an EXO-1 (Protein Of Interest) polypeptide, or a YiAP2 polypeptide, in a plant.

## Background

### 1. FSM1-like (Fruit Sant/Myb) polypeptides

FSM1-like proteins are transcription factors characterised by the presence of a SANT/MYB domain. They were first described in tomato (Barg et al., Planta 221, 197-211, 2005); and are reportedly involved in the determination of floral symmetry (Corley et al., Proc. Natl. Acad. Sci. 102, 5068-5073, 2005). The RADIALIS protein for example, an FSM1-like protein in Antirrhinium, is expressed in the dorsal region of the floral meristem, where it interacts with CYC and DICH to control the flower asymmetry (Baxter et al. Plant J. 52, 105-113, 2007). Tomato FSM1 is expressed in the fruit during the very early developmental stages. Ectopic expression of tomato fsm1 in Arabidopsis resulted in severe developmental alterations manifested in retarded growth, and reduced apical dominance during tomato and Arabidopsis seedling development (Barg et al., 2005).

### 2. PIF3-like (Phytochrome Interacting Factor 3) polypeptides

Phytochromes are dimeric chromoproteins that regulate plant responses to red (R) and farred (FR) light. The Arabidopsis thaliana genome encodes five phytochrome apoproteins: type I phyA mediates responses to FR, and type II phyB-phyE mediate shade avoidance and classical R/FR-reversible responses. Roles in light regulation of hypocotyl length, leaf area, and flowering time are demonstrated for heterodimeric phytochromes containing phyC or phyE. Following a pulse of red light, phyA, phyB, phyC, and phyD interact in vivo with the PIF3 (PHYTOCHROME INTERACTING FACTOR3) basic helix-loop-helix transcription factor, and this interaction is FR reversible. Therefore, most or all of the type I and type II phytochromes, including heterodimeric forms, appear to function through PIF-mediated pathways (Clack et al., Plant Cell. 2009 Mar;21 (3):786-99).

In Arabidopsis, members of the PIF family have been shown to control light-regulated gene expression directly and indirectly. PIF1, PIF3, PIF4 and PIF5 are degraded in response to light signals, and physical interaction of PIF3 with phytochromes is necessary for the light-induced phosphorylation and degradation of PIF3. (Castillon et al.,Trands in Plant Science, Volume 12, Issue 11, November 2007, Pages 514-521)

## 3. UROD (Uroporphyrinogen III decarboxylase) polypeptide

Uroporphyrinogen III decarboxylase or UROD is an enzyme involved in the conversion of uroporphyrinogen-III into coporphyrinogen, a precursor in heme or chlorophyll biosynthesis. The enzymatic reaction is as follows:

uroporphyrinogen-III <=> 4 CO2 + coproporphyrinogen III

Martins et al., 2001 (The Journal of Biological Chemistry Vol. 276, No. 47, Issue of November 23, pp. 44108-44116) describe the crystal structure and substrate binding modeling of the Uroporphyrinogen-III Decarboxylase from *Nicotiana tabacum.*

Mohanty et al., 2006 (Planta 224: 692-699) report that the uroporphyrinogen decarboxylase gene (UroD) and gene product abundance was stimulated by light and heat-stress. Also reported is increased enzymatic activity of UroD in heat-stressed cucumber seedlings.

Mock and Grimm 1997 (Plant Physiol. 113: 1101-1112) report introducing a full-length cDNA sequence encoding tobacco (Nicotiana *tabacum)* uroporphyrinogen III decarboxylase (UROD; EC 4.1.1.37) in reverse orientation under the control of a cauliflower mosaic virus 35s promoter derivative into the tobacco genome to study the effects of deregulated UROD expression on tetrapyrrole biosynthesis. The authors report the transformants to have reduced UROD activity and stunted plant growth and necrotic leaf lesions. They further report that antisense RNA expression caused reduced UROD protein levels and reduced activity to 45% of wild type, which was correlated with the accumulation of uroporphyrin(ogen) and with the intensity of necrotic damage. Also mentioned is that chlorophyll levels were only slightly reduced (up to 15%), indicating that the plants sustained cellular damage from accumulating photosensitive porphyrins rather than from chlorophyll deficiency.

## 4. AS-MTT (Abiotic Stress Membrane Tethered Transcription factor) polypeptides

Membrane-tethered transcription factors (MTTFs) differ from cytosolic transcription factors (TF) in that they are innately membrane-bound. To attain TF activity, MTTFs are released from the membrane anchor as a result of proteolytic cleavage. This enables MTTFs to travel to the nucleus and modulate gene expression. A genome-scale analysis showed that over 10% of all transcription factors are membrane bound, indicating that activation of MTFs occurs at the genomic level, allowing transcription to be regulated rapidly under stressful conditions. Seo et al. (2008). Trends Plant Sci. 13(10):550-556. Arabidopsis MTTFs characterized to date belong to either the bZIP or the NAC family. Chen et al. (2008). Curr Opin Plant Biol. 11(6):695-701. The current application characterizes a novel class of MTTFs, the AS-MTT (Abiotic Stress-MT), which comprise a DUF 1664 domain. DUF 1664 refers to a protein domain of previously unknown function which is highly conserved amongst plant MTTFs.

## 5. EXO-1 polypeptide

RAD2 nucleases are involved in DNA repair processes. They exist in all eukaryotic organisms. There is at least 4-5 classes based on sequence and activity. XPG (Class I): incises the target strand 3' to the bubble-like, damage-containing structure. FEN-1 (Class II) exhibits a flap endonuclease activity for bifurcated DNA structures. Exo-1 (Class III) operates as a 5'-nuclease (i.e. either a 5'-flap endonuclease or a 5'-3' exonuclease). SEND/GEN (Class IV) is still yet unclear in the opinion of scientific community.

## 6. YiAP2 polypeptides

The APETALA2-like genes form a large multi-gene family of transcription factors which play an important role during the plant life cycle, being key regulators of many developmental processes. Many studies in Arabidopsis have revealed that the APETALA2 (AP2) gene is implicated in the establishment of floral meristem and floral organ identity as well as temporal and spatial regulation of flower homeotic gene expression (Gil-Humanes et al. 2009 BMC Plant Biol. 29;9(1):66). The APETALA2-like (AP2-like) gene family exhibits patterns of both gene and domain duplication, coupled with changes in sequence, exon arrangement, and expression. Genetic evolution studies analyses of AP2-like genes from green plants support the presence of the two major lineages in the AP2-like genes, the euAP2 and the AINTEGUMENTA (ANT). Furthermore a duplication of the AP2 domain occurred prior to the separation of the two lineages. The ANT lineage is supported by a 10-aa insertion in the AP2-R1 domain and a 1-aa insertion in the AP2-R2 domain, relative to all other members of the AP2-like family. MicroRNA172-binding sequences, the function of which has been studied in some of the AP2-like genes in Arabidopsis, are restricted to the euAP2 lineage and therefore absent from AP2-like proteins of the ANT lineage (Kim et al; Mol Biol Evol. 2006 Jan;23(1):107-20). Protein encoded by members of the euAP2 gene family, which posses only one AP2 domain, more specifically of the ERF type have been previously described as useful to use in methods to enhance yield-related traits in plants (WO 2007/144190). Surprisingly, it has now been found that modulating expression of a nucleic acid encoding an AP2-like polypeptide of the ANT linage, the YiAP2 polypeptide, gives plants having enhanced yield-related traits relative to control plants.

According one embodiment, there is provided a method for improving yield-related traits in plants relative to control plants, comprising modulating expression in a plant of a nucleic acid encoding a YiAP2 polypeptide.

## Summary

### 1. FSM1-like (Fruit Sant/Myb) polypeptides

Surprisingly, it has now been found that modulating expression of a nucleic acid encoding a FSM1-like polypeptide gives plants having enhanced yield-related traits, in particular increased yield relative to control plants.

According one embodiment, there is provided a method for improving yield-related traits in plants relative to control plants, comprising modulating expression in a plant of a nucleic acid encoding a FSM1-like polypeptide.

### 2. PIF3-like (Phytochrome Interacting Factor 3) polypeptides

Surprisingly, it has now been found that modulating expression of a nucleic acid encoding a PIF3-like polypeptide gives plants having enhanced yield-related traits, in particular increased seed yield and/or early vigour, relative to control plants.

According one embodiment, there is provided a method for improving yield-related traits in plants relative to control plants, comprising modulating expression in a plant of a nucleic acid encoding a PIF3-like polypeptide.

### 3. UROD (Uroporphyrinogen III decarboxylase) polypeptide

Surprisingly, it has now been found that modulating expression of a nucleic acid encoding a UROD polypeptide gives plants having enhanced yield-related traits, in particular increased yield relative to control plants.

According one embodiment, there is provided a method for improving yield-related traits in plants relative to control plants, comprising modulating expression in a plant of a nucleic acid encoding a UROD polypeptide.

### 4. AS-MTT (Abiotic Stress Membrane Tethered Transcription factor) polypeptides

Surprisingly, it has now been found that modulating expression of a nucleic acid encoding an AS-MTT polypeptide gives plants having enhanced yield-related traits, relative to control plants.

According one embodiment, there is provided a method for enhancing yield related traits of a plant relative to control plants, comprising modulating expression of a nucleic acid encoding an AS-MTT polypeptide in a plant.

### 5. EXO-1 polypeptide

Surprisingly, it has now been found that modulating expression of a nucleic acid encoding an EXO-1 polypeptide gives plants having enhanced yield-related traits, in particular increased yield relative to control plants.

According one embodiment, there is provided a method for improving yield-related traits in plants relative to control plants, comprising modulating expression in a plant of a nucleic acid encoding an EXO-1 polypeptide.

Definitions

The following definitions will be used throughout the present specification.

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide and may range from 1 to 10 amino acids; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---------|----------------------------|---------|----------------------------|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain, Motif/Consensus sequence/Signature

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Reciprocal BLAST

Typically, this involves a first BLAST involving BLASTing a query sequence (for example using any of the sequences listed in Table A of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived. The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

<u>Hybridisation</u>

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point $(T_m)$ for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The $T_m$ is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The $T_m$ may be calculated using the following equations, depending on the types of hybrids:

1) DNA-DNA hybrids (Meinkoth and Wahl, Anal. Biochem., 138: 267-284, 1984):

$$T_m = 81.5°C + 16.6 \mathrm{x} \log_{10}[Na^+]^a + 0.41 \mathrm{x}\%[G/C^b] - 500 \mathrm{x}[L^c]^{-1} - 0.61 \mathrm{x}\%\ \text{formamide}$$

2) DNA-RNA or RNA-RNA hybrids:

$$T_m = 79.8°C + 18.5\ (\log_{10}[Na^+]^a) + 0.58\ (\%G/C^b) + 11.8\ (\%G/C^b)^2 - 820/L^c$$

3) oligo-DNA or oligo-RNA[d] hybrids:

For <20 nucleotides: $T_m = 2\ (I_n)$

For 20-35 nucleotides: $T_m = 22 + 1.46 (I_n)$

[a] or for other monovalent cation, but only accurate in the 0.01-0.4 M range.

[b] only accurate for %GC in the 30% to 75% range.

[c] L = length of duplex in base pairs.

[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Construct

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
| --- | --- |
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

Table 2b: Examples of root-specific promoters

| Gene Source | Reference |
|---|---|
| RCc3 | Plant Mol Biol. 1995 Jan;27(2):237-48 |
| Arabidopsis PHT1 | Koyama et al. J Biosci Bioeng. 2005 Jan;99(1):38-42.; Mudge et al. (2002, Plant J. 31:341) |
| Medicago phosphate transporter | Xiao et al., 2006, Plant Biol (Stuttg). 2006 Jul;8(4):439-49 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 17 (6): 1139-1154 |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below. Further examples of seed-specific promoters are given in Qing Qu and Takaiwa (Plant Biotechnol. J. 2, 113-125, 2004), which disclosure is incorporated by reference herein as if fully set forth.

Table 2c: Examples of seed-specific promoters

| Gene source | Reference |
| --- | --- |
| seed-specific genes | Simon et al., Plant Mol. Biol. 5: 191, 1985; |
|  | Scofield et al., J. Biol. Chem. 262: 12202, 1987.; |
|  | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
|  | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat $\alpha$, $\beta$, $\gamma$-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice $\alpha$-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice $\alpha$-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum $\alpha$-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |

(continued)

| Gene source | Reference |
|---|---|
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley ltr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |

(continued)

| Gene source | Reference |
|---|---|
| PRO0095 | WO 2004/070039 |

**Table 2f:** Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g:** Examples of green tissue-specific promoters

| Gene | Expression | Reference |
|---|---|---|
| Maize Orthophosphate dikinase | Leaf specific | Fukavama et al., Plant Physiol. 2001 Nov;127(3):1136-46 |
| Maize Phosphoenolpyruvate carboxylase | Leaf specific | Kausch et al., Plant Mol Biol. 2001 Jan;45(1):1-15 |
| Rice Phosphoenolpyruvate carboxylase | Leaf specific | Lin et al., 2004 DNA Seq. 2004 Aug;15(4):269-76 |
| Rice small subunit Rubisco | Leaf specific | Nomura et al., Plant Mol Biol. 2000 Sep;44(1):99-106 |
| rice beta expansin EXBP9 | Shoot specific | WO 2004/070039 |
| Pigeonpea small subunit Rubisco | Leaf specific | Panguluri et al., Indian J Exp Biol. 2005 Apr;43(4):369-72 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h:** Examples of meristem-specific promoters

| Gene source | Expression pattern | Reference |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |

(continued)

| Gene source | Expression pattern | Reference |
|---|---|---|
| WAK1 & WAK 2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not present in, or originating from, the genome of said plant, or are present in the genome of said plant but not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

It shall further be noted that in the context of the present invention, the term "isolated nucleic acid" or "isolated polypeptide" may in some instances be considered as a synonym for a "recombinant nucleic acid" or a "recombinant polypeptide", respectively and refers to a nucleic acid or polypeptide that is not located in its natural genetic environment and/or that has been modified by recombinant methods.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. For the purposes of this invention, the original unmodulated expression may also be absence of any expression. The term "modulating the activity" shall mean any change of the expression of the inventive nucleic acid sequences or encoded proteins, which leads to increased yield and/or increased growth of the plants. The expression can increase from zero (absence of or immeasurable expression) to a certain amount, or can decrease from a certain amount to immeasurable small amounts or zero.

<u>Expression</u>

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

<u>Increased expression/overexpression</u>

The term "increased expression" or "overexpression" as used herein means any form of expression that is additional to the original wild-type expression level. For the purposes of this invention, the original wild-type expression level might also be zero ((absence of or immeasurable expression)).

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

<u>Decreased expression</u>

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050.

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium-mediated* transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium-mediated* transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:1-9; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield related Traits

Yield related traits comprise one or more of early flowering time, yield, biomass, seed yield, early vigour, greenness index, increased growth rate, improved agronomic traits (such as improved Water Use Efficiency (WUE), Nitrogen Use Efficiency (NUE), etc.).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The terms "yield" of a plant and "plant yield" are used interchangeably herein and are meant to refer to vegetative biomass such as root and/or shoot biomass, to reproductive organs, and/or to propagules such as seeds of that plant.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, panicle length, number of spikelets per panicle, number of flowers (florets) per panicle, increase in the seed filling rate (which is the number of filled seeds divided by the total number of seeds and multiplied by 100), increase in thousand kernel weight, among others. In rice, submergence tolerance may also result in increased yield.

Early flowering time

Plants having an "early flowering time" as used herein are plants which start to flower earlier than control plants. Hence this term refers to plants that show an earlier start of flowering. Flowering time of plants can be assessed by counting the number of days ("time to flower") between sowing and the emergence of a first inflorescence. The "flowering time" of a plant can for instance be determined using the method as described in WO 2007/093444.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increased growth rate

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Stress resistance

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35%, 30% or 25%, more preferably less than 20% or 15% in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. "Mild stresses" are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures.
"Biotic stresses" are typically those stresses caused by pathogens, such as bacteria, viruses, fungi, nematodes and insects.

The "abiotic stress" may be an osmotic stress caused by a water stress, e.g. due to drought, salt stress, or freezing stress. Abiotic stress may also be an oxidative stress or a cold stress. "Freezing stress" is intended to refer to stress due to freezing temperatures, i.e. temperatures at which available water molecules freeze and turn into ice. "Cold stress", also called "chilling stress", is intended to refer to cold temperatures, e.g. temperatures below 10°, or preferably below 5°C, but at which water molecules do not freeze. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

In particular, the methods of the present invention may be performed under non-stress conditions. In an example, the methods of the present invention may be performed under non-stress conditions such as mild drought to give plants having increased yield relative to control plants.

In another embodiment, the methods of the present invention may be performed under stress conditions.

In an example, the methods of the present invention may be performed under stress conditions such as drought to give plants having increased yield relative to control plants. In another example, the methods of the present invention may be performed under stress conditions such as nutrient deficiency to give plants having increased yield relative to control plants.

Nutrient deficiency may result from a lack of nutrients such as nitrogen, phosphates and other phosphorous-containing compounds, potassium, calcium, magnesium, manganese, iron and boron, amongst others.

In yet another example, the methods of the present invention may be performed under stress conditions such as salt stress to give plants having increased yield relative to control plants. The term salt stress is not restricted to common salt (NaCl), but may be any one or more of: NaCl, KCl, LiCl, $MgCl_2$, $CaCl_2$, amongst others.

In yet another example, the methods of the present invention may be performed under stress conditions such as cold stress or freezing stress to give plants having increased yield relative to control plants.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following:

(a) an increase in seed biomass (total seed weight) which may be on an individual seed basis and/or per plant and/or per square meter;
(b) increased number of flowers per plant;
(c) increased number of seeds and/or increased number of filled seeds;
(d) increased seed filling rate (which is expressed as the ratio between the number of filled seeds divided by the total number of seeds);
(e) increased harvest index, which is expressed as a ratio of the yield of harvestable parts, such as seeds, divided by the biomass of aboveground plant parts; and
(f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Biomass

The term "biomass" as used herein is intended to refer to the total weight of a plant. Within the definition of biomass, a distinction may be made between the biomass of one or more parts of a plant, which may include:

- aboveground (harvestable) parts such as but not limited to shoot biomass, seed biomass, leaf biomass, etc. and/or
- (harvestable) parts below ground, such as but not limited to root biomass, etc., and/or
- vegetative biomass such as root biomass, shoot biomass, etc., and/or
- reproductive organs, and/or
- propagules such as seed.

Marker assisted breeding

Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Use as probes in (gene mapping)

Use of nucleic acids encoding the protein of interest for genetically and physically mapping the genes requires only a nucleic acid sequence of at least 15 nucleotides in length. These nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids encoding the protein of interest. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding the protein of interest in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Detailed description of the invention

Surprisingly, it has now been found that modulating expression in a plant of a nucleic acid encoding a FSM1-like polypeptide gives plants having enhanced yield-related traits relative to control plants. According to a first embodiment, the present invention provides a method for enhancing yield-related traits in plants relative to control plants, comprising modulating expression in a plant of a nucleic acid encoding a FSM1-like polypeptide and optionally selecting for plants having enhanced yield-related traits.

The invention also provides hitherto unknown FSM1-like-encoding nucleic acids and FSM1-like polypeptides useful for conferring enhanced yield-related traits in plants relative to control plants.

According to a further embodiment of the present invention, there is therefore provided an isolated nucleic acid molecule selected from:

(i) a nucleic acid represented by any of SEQ ID NO: 17, SEQ ID NO: 239, SEQ ID NO: 259, SEQ ID NO: 261, SEQ ID NO: 265, or SEQ ID NO: 277;

(ii) the complement of a nucleic acid represented by any of SEQ ID NO: 17, SEQ ID NO: 239, SEQ ID NO: 259, SEQ ID NO: 261, SEQ ID NO: 265, or SEQ ID NO: 277;

(iii) a nucleic acid encoding a FSM1-like polypeptide, comprising a MYB/SANT domain and having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 18, SEQ ID NO: 240, SEQ ID NO: 260, SEQ ID NO: 262, SEQ ID NO: 266, or SEQ ID NO: 278, and additionally or alternatively comprising one or more motifs having, in increasing order of preference, 4, or less than 4, less than 3, less than 2, or no substitutions (sequence mismatches) to any one or more of the motifs given in SEQ ID NO: 283 to SEQ ID NO: 288, and further preferably conferring enhanced yield-related traits relative to control plants.

(iv) a nucleic acid molecule which hybridizes with a nucleic acid molecule of (i) to (iii) under high stringency hybridization conditions and preferably confers enhanced yield-related traits relative to control plants.

According to a further embodiment of the present invention, there is also provided an isolated polypeptide selected from:

(i) an amino acid sequence represented by SEQ ID NO: 18, SEQ ID NO: 240, SEQ ID NO: 260, SEQ ID NO: 262, SEQ ID NO: 266, or SEQ ID NO: 278;

(ii) an amino acid sequence, comprising a MYB/SANT domain and having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 18, SEQ ID NO: 240, SEQ ID NO: 260, SEQ ID NO: 262, SEQ ID NO: 266, or SEQ ID NO: 278, and additionally or alternatively comprising one or more motifs having, in increasing order of preference, 4 or less than 4, less than 3, less than 2, or no substitutions (sequence mismatches) to any of the motifs given in SEQ ID NO: 283 to SEQ ID NO: 288, and further preferably conferring enhanced yield-related traits relative to control plants;

(iii) derivatives of any of the amino acid sequences given in (i) or (ii) above.

Furthermore, it has now surprisingly been found that modulating expression in a plant of a nucleic acid encoding a PIF3-like polypeptide gives plants having enhanced yield-related traits relative to control plants. According to a first embodiment, the present invention provides a method for enhancing yield-related traits in plants relative to control plants, comprising modulating expression in a plant of a nucleic acid encoding a PIF3-like polypeptide and optionally selecting for plants having enhanced yield-related traits.

The invention also provides hitherto unknown PIF3-like-encoding nucleic acids and PIF3-like polypeptides useful for conferring enhanced yield-related traits in plants relative to control plants.

According to a further embodiment of the present invention, there is therefore provided an isolated nucleic acid molecule comprising any one of the following features selected from:

(i) a nucleic acid represented by SEQ ID NO: 356;

(ii) the complement of a nucleic acid represented by SEQ ID NO: 356;

(iii) a nucleic acid encoding a PIF3-like polypeptide having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 357, and additionally or alternatively comprising one or more motifs having in increasing order of preference less than 5, less than 4, less than 3, less than 2, or less than 1 substitutions (sequence mismatches) compared to any one or more of the motifs given in SEQ ID NO: 358 to SEQ ID NO: 363, and further preferably conferring enhanced yield-related traits relative to control plants.

(iv) a nucleic acid molecule which hybridizes with a nucleic acid molecule of (i) to (iii) under high stringency hybridization conditions and preferably confers enhanced yield-related traits relative to control plants.

According to a further embodiment of the present invention, there is also provided an isolated polypeptide selected from:

(i) an amino acid sequence represented by SEQ ID NO: 357;
(ii) an amino acid sequence having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 357, and additionally or alternatively comprising one or more motifs having in increasing order of preference less than 5, less than 4, less than 3, less than 2, or less than 1 substitutions (sequence mismatches) compared to any one or more of the motifs given in SEQ ID NO: 358 to SEQ ID NO: 363, and further preferably conferring enhanced yield-related traits relative to control plants;
(iii) derivatives of any of the amino acid sequences given in (i) or (ii) above.

Furthermore, it has now surprisingly been found that modulating expression in a plant of a nucleic acid encoding a UROD polypeptide gives plants having enhanced yield-related traits relative to control plants. According to a first embodiment, the present invention provides a method for enhancing yield-related traits in plants relative to control plants, comprising modulating expression in a plant of a nucleic acid encoding a UROD polypeptide and optionally selecting for plants having enhanced yield-related traits.

Furthermore, it has now surprisingly been found that modulating expression in a plant of a nucleic acid encoding an AS-MTT polypeptide gives plants having enhanced yield-related traits relative to control plants. According to a first embodiment, the present invention provides a method for enhancing yield-related traits in plants relative to control plants, comprising modulating expression in a plant of a nucleic acid encoding an AS-MTT polypeptide and optionally selecting for plants having enhanced yield-related traits.

Furthermore, it has now surprisingly been found that modulating expression in a plant of a nucleic acid encoding an EXO-1 polypeptide gives plants having enhanced yield-related traits relative to control plants. According to a first embodiment, the present invention provides a method for enhancing yield-related traits in plants relative to control plants, comprising modulating expression in a plant of a nucleic acid encoding an EXO-1 polypeptide and optionally selecting for plants having enhanced yield-related traits.

The invention also provides hitherto unknown EXO-1-encoding nucleic acids and EXO-1 polypeptides useful for conferring enhanced yield-related traits in plants relative to control plants.

Furthermore, it has now surprisingly been found that modulating expression in a plant of a nucleic acid encoding a YiAP2 polypeptide gives plants having enhanced yield-related traits relative to control plants. According to a first embodiment, the present invention provides a method for enhancing yield-related traits in plants relative to control plants, comprising modulating expression in a plant of a nucleic acid encoding a YiAP2 polypeptide and optionally selecting for plants having enhanced yield-related traits.

A preferred method for modulating (preferably, increasing) expression of a nucleic acid encoding a FSM1-like (Fruit Sant/Myb) polypeptide, or a PIF3-like (Phytochrome Interacting Factor 3) polypeptide, or a UROD polypeptide, or an AS-MTT (Abiotic Stress Membrane Tethered Transcription factor) polypeptide, or an EXO-1 polypeptide, or a YiAP2 polypeptide, is by introducing and expressing in a plant a nucleic acid encoding a FSM1-like (Fruit Sant/Myb) polypeptide, or a PIF3-like (Phytochrome Interacting Factor 3) polypeptide, or a UROD polypeptide, or an AS-MTT (Abiotic Stress Membrane Tethered Transcription factor) polypeptide, or an EXO-1 polypeptide, or a YiAP2 polypeptide.

In one embodiment, a "protein useful in the methods of the invention" is taken to mean a FSM1-like polypeptide as defined herein. In another embodiment, "nucleic acid useful in the methods of the invention" is taken to mean a nucleic acid capable of encoding such a FSM1-like polypeptide. The nucleic acid to be introduced into a plant (and therefore useful in performing the methods of the invention) is any nucleic acid encoding the type of protein which will now be described, hereafter also named *"FSM1-like* nucleic acid" or *"FSM1-like* gene".

A "FSM1-like polypeptide" as defined herein refers to any polypeptide comprising a MYB/SANT domain (SMART accession SM00717, Pfam accession PF00249).

Additionally or alternatively, the FSM1-like protein comprises one or more of the following motifs:

Motif 1 (SEQ ID NO: 283): W[TS][PA]K[QE]NK[LA]FE[RK]ALAVYD[KR][DE]TPDR W[HSQ]N[VI]A[RK]A
Motif 2 (SEQ ID NO: 284): GGK[ST][AV][ED]EV[KR]RHYE[IL]L
Motif 3 (SEQ ID NO: 285): D[VL][KF][HF]I[ED][SN]G[RM]VPFP[NK]Y.

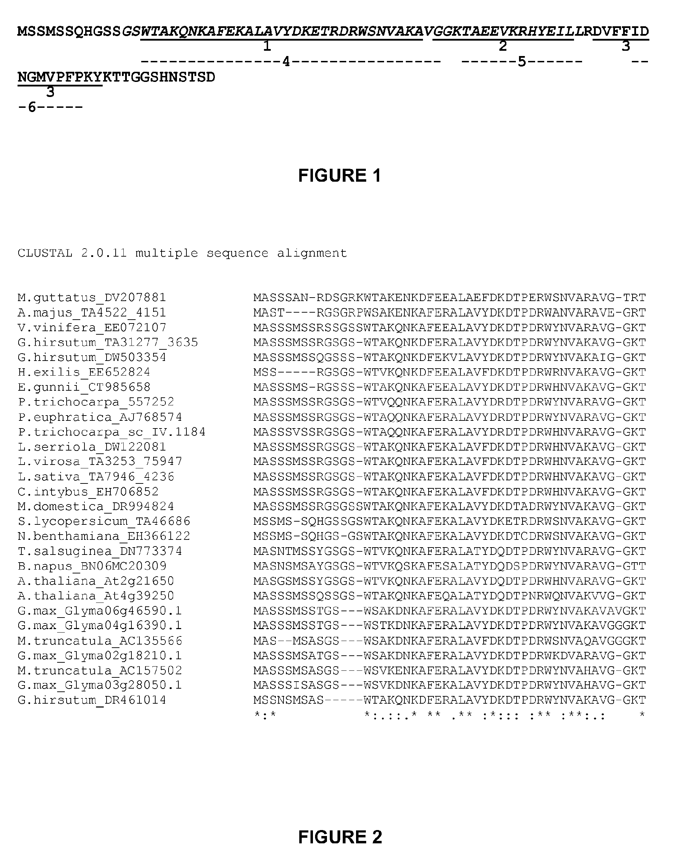

These motifs were derived using the MEME algorithm (Bailey and Elkan, Proceedings of the Second International Conference on Intelligent Systems for Molecular Biology, pp. 28-36, AAAI Press, Menlo Park, California, 1994.), MEME motifs represent the amino acid residues that are present in at least 80% of the FSM1-like proteins given in the sequence listing. The location of the motifs is shown in Fig 1 (representing the protein sequence of SEQ ID NO: 2).
Alternatively or additionally, the FSM1-like polypeptide also comprises one or more of the following motifs:

Motif    4    (SEQ    ID    NO:    286):    W(T/S)(A/V/T)(K/Q)(E/Q/D)(N/S)K(D/A)FEX(A/V)LA(E/V/T)(F/Y)D(K/R/Q)(D/E)(T/S)(P/A/R/C)(E/D/N)RWX(N/D)VA(R/K/Q/H)(A/V)(V/I)(G/E/A)
wherein X on position 11 can be any amino acid, preferably one of E, R, K, S, Q, and
wherein X on position 25 can be any amino acid, preferably one of S, A, Y, H, Q, K
Motif 5 (SEQ ID NO: 287): T(V/P/A/T)(E/D)(E/D)(V/A)KX(H/Q)Y(E/D)(V/I/L/H)L(L/V)
wherein X on position 7 can be any amino acid, preferably one of K, R, Q, H, S
Motif 6 (SEQ ID NO: 288): I(E/D)(S/N)(G/D)XV(P/A)(L/F/Y)P
wherein X on position 5 can be any amino acid, preferably one of K, Q, R, H, M

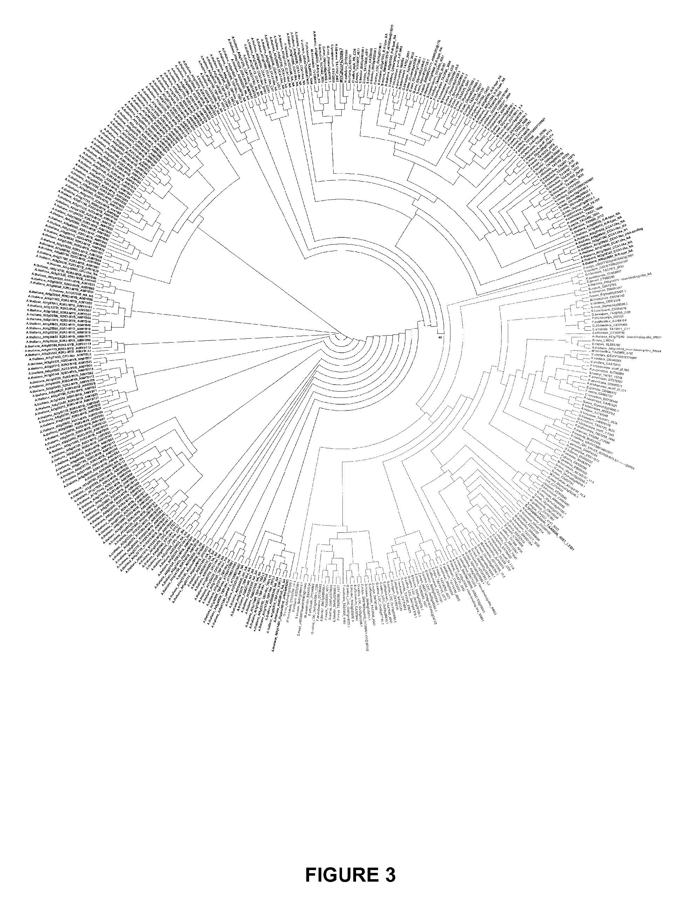

More preferably, the FSM1-like polypeptide comprises in increasing order of preference, at least 2, at least 3, at least 4, at least 5, or all 6 of the above motifs.
Additionally or alternatively, the homologue of a FSM1-like protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 2, provided that the homologous protein comprises a MYB/SANT domain and preferably also any one or more of the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the motifs in a FSM1-like polypeptide have, in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any one or more of the motifs represented by SEQ ID NO: 283 to SEQ ID NO: 288 (Motifs 1 to 6).
Preferably, the polypeptide sequence which when used in the construction of a phylogenetic tree, such as the one depicted in Figure 3, clusters with the group of FSM1-like polypeptides comprising the amino acid sequence represented by SEQ ID NO: 2 rather than with other MYB proteins.
In another embodiment, a "protein useful in the methods of the invention" is taken to mean a PIF3-like polypeptide as defined herein. I another embodiment a "nucleic acid useful in the methods of the invention" is taken to mean a nucleic acid capable of encoding such a PIF3-like polypeptide. The nucleic acid to be introduced into a plant (and therefore useful in performing the methods of the invention) is any nucleic acid encoding the type of protein which will now be described, hereafter also named "PIF3-like nucleic acid" or "PIF3-like gene".
A "PIF3-like polypeptide" as defined herein refers to SEQ ID NO: 293 and any homologous (preferably orthologous) polypeptide comprising a Helix-Loop-Helix domain (Pfam accession PF00010, InterPro IPR001092). Additionally or alternatively, the PIF3-like polypeptide comprises one or more of the following motifs:

Motif    7    (SEQ    ID    NO:    338):    [RKG][STG][TG][TS][TAS][KR]R[RS]RAAEVHNLSERRR RDRINEKM[RK]ALQELIP[HNR]CNK[TS]DKAS[MI]LDEAIEYLKSLQ[LM]Q[VL]Q[IM]M[ WS]MG[SGC]G
Motif 8 (SEQ ID NO: 339): [LI]x[PE][DE][ND][EGD]LVELLW[QCE]NG[QHG]W wherein x represents any amino acid.

These motifs were derived from MEME motifs (Bailey and Elkan, Proceedings of the Second International Conference on Intelligent Systems for Molecular Biology, pp. 28-36, AAAI Press, Menlo Park, California, 1994). MEME motifs represent the amino acid residues that are present in at least 80% of the FSM1-like proteins given in the sequence listing. The location of the motifs is shown in Fig 1 (representing the protein sequence of SEQ ID NO: 293).
Additionally or alternatively, the PIF3-like polypeptide comprises one or more of the following motifs:

Motif 9 (SEQ ID NO: 340): [E/D/N/G]L[V/I]EL[L/Q]W[R/Q/K/C][D/N]G[Q/E/H]W

Motif 10 (SEQ ID NO: 341): RAAEVHN
Motif 11 (SEQ ID NO: 342): SERRRRDRINE
Motif 12 (SEQ ID NO: 343): TDKAS[M/I]L[D/E]EAI[E/D]YLKSLQ[L/M/F]QLQ[M/V/L]M WMG

Preferably, the PIF3-like polypeptide comprises in increasing order of preference, at least 1, at least 2, or at least 3 of the above motifs.

Additionally or alternatively, the homologue of a PIF3-like protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 293, provided that the homologous protein comprises any one or more of the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the motifs in a PIF3-like polypeptide have, in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any one or more of the motifs represented by SEQ ID NO: 338 to SEQ ID NO: 343 (Motifs 7 to 12).

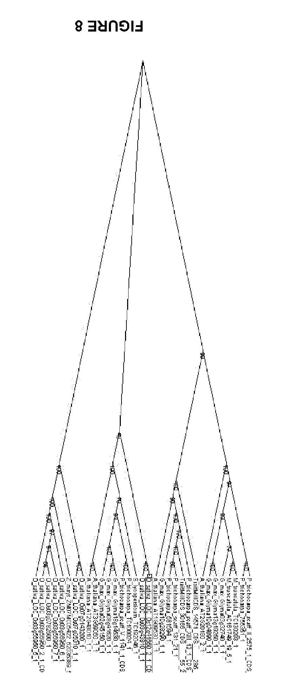

Preferably, the polypeptide sequence which when used in the construction of a phylogenetic tree, such as the one depicted in Figure 8, clusters with the group of PIF3-like polypeptides (eventually refer to a specific group defined in the literature) comprising the amino acid sequence represented by SEQ ID NO: 293 rather than with any other group of bHLH proteins.

In another embodiment, a "protein useful in the methods of the invention" is taken to mean a UROD polypeptide as defined herein. In another embodiment, a "nucleic acid useful in the methods of the invention" is taken to mean a nucleic acid capable of encoding such a UROD polypeptide. The nucleic acid to be introduced into a plant (and therefore useful in performing the methods of the invention) is any nucleic acid encoding the type of protein which will now be described, hereinafter also named "UROD nucleic acid" or "UROD gene". A "UROD polypeptide" as defined herein refers to any polypeptide having uroporphyrinogen III decarboxylase activity, meaning any polypeptide having the capability to convert uroporphyrinogen III into coporphyrinogen.

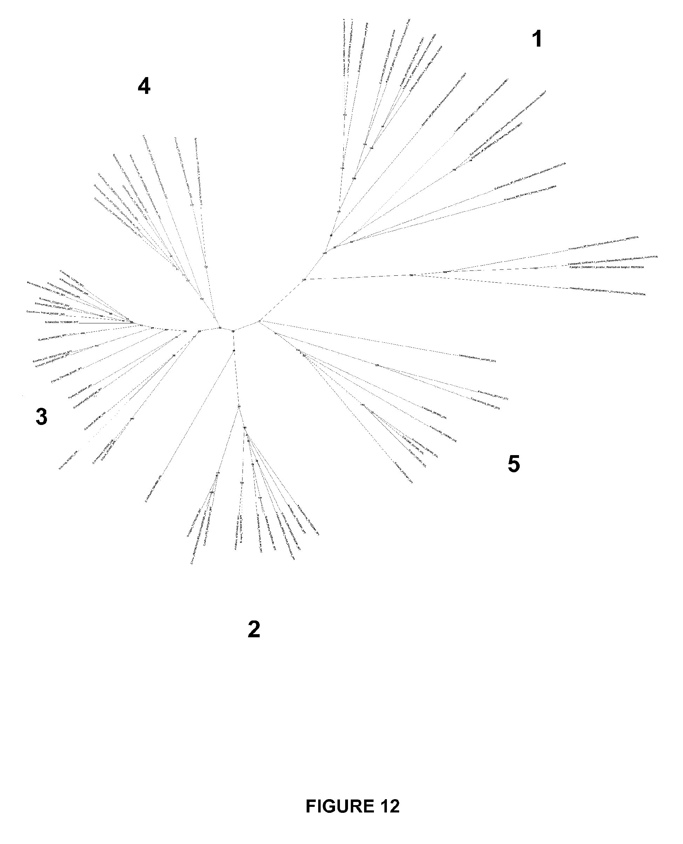

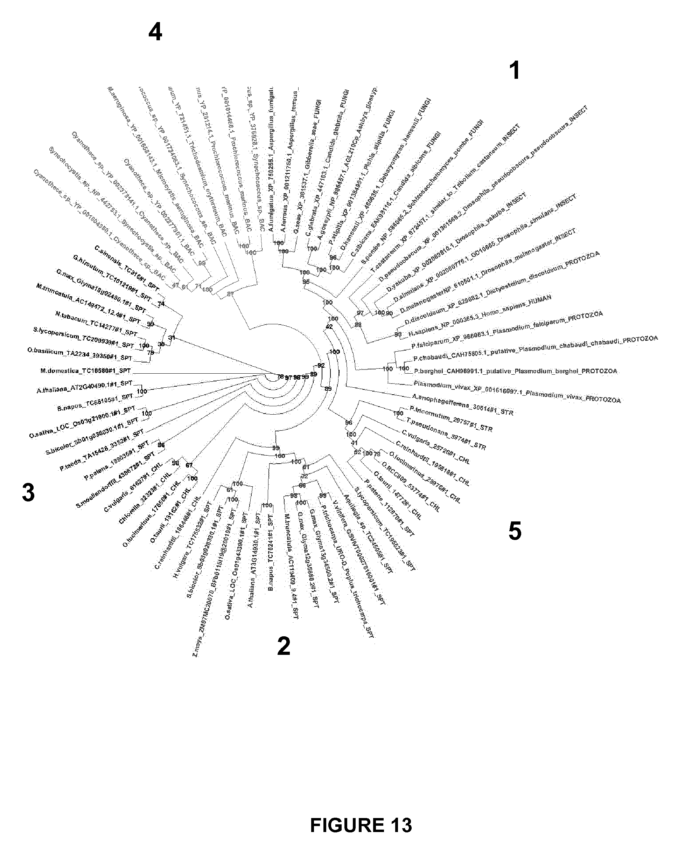

Phylogenetic analysis shows that UROD polypeptides can be separated into two main clades: photosynthetic and non-photosynthetic (see Figures 12 and 13). Preferably, UROD polypeptides useful in the methods of the invention belong to the photosynthetic clade. The photosynthetic clade can be further subdivided into four main sub-clades: HEM1, HEM2, CYANO and others. Most preferably, UROD polypeptides useful in the methods of the invention belong to the HEM1 sub-clade.

UROD polypeptides typically comprise one or more of the following domains:

| Method | | Domain ID and name | Short Name | Location |
|---|---|---|---|---|
| Interpro | IPR000257 | Uroporphyrinogen decarboxylase | URO-D | 4.8e-146 [50-390]T |
| PFAM | PF01208 | Uroporphyrinogen decarboxylase | UROD_1 | NA [68-77] |
| PROSITE | PS00907 | Uroporphyrinogen decarboxylase | UROD_2 | 8e-5[187-203]T |
| Interpro | IPR006361 | Uroporphyrinogen decarboxylase HemE | | |
| PANTHER | PTHR21091: SF2 | Uroporphyrinogen decarboxylase | | 1e-127 [65-390]T |
| TIGRFAMs | TIGR01464 | hemE: uroporphyrinogen decarboxylase | | 3.6e-187 [54-389]T |

UROD polypeptides typically comprise any one or more of Motifs 13 to 15. Motifs 12 to 15 are typically found in UROD polypeptides and can help distinguish UROD polypeptides from other polypeptides.
Motif 13: VERPPVW[ML]MRQAGRY[ML][KPA][EVS][YF][QR][ED]L[RC][EK]K[YH][PD][SF]F
Motif 14: D[GA]VI[LI]FSDIL[TV][PI][LP][PQ][AG]M[GN]IPFD[IM]V[EK][GS]KGP[VI]
Motif 15: LGFVGAP[WF]TLA[TAS]Y[VIM][VI]EGG[SG][ST]K

Preferably, in addition to any one or more of Motifs 13 to 15, a UROD polypeptide comprises any one or more of Motifs 16 to 18. Motifs 16 to 18 are typically found in photosynthetic UROD (PS-UROD) polypeptides.
Motif 16: QPW[RK][AV]F[KQ]PDGVI[LM]FSDILTPLP[GA]M[GN][IV]PFD

Motif 17: RPP[VA]W[ML]MRQAGRYM[KA][VS]Y[RQ]DL[CA][EDK]K[YH]P

Motif 18: Y[IV]RYQ[IAV][DE]SGAQ[VAC][VI]QIFDSW[AG][GT][QE]L[SP]P[QV]D[FWY]EE[FW][SA][LK]PY[LIQ][KQ]Q[IV][VI]D

Further preferably, in addition to any one or more of Motifs 13 to 15, a UROD polypeptide comprises any one or more of Motifs 16 to 18 and/or any one or more of Motifs 19 to 21. Motifs 19 to 21 are typically found in the HEM1 class of photosynthetic UROD (PS-UROD) polypeptides.

Motif 19: MCHTAP[HND]VLR[AGT]LLSHL[TA][QK]AI[SA][DE]Y[IV][IV][YF]QV[EN]SGA[HQ] CIQIFDSWGGQLPP[HED][MV]W

Motif 20: YINGNGGLLERMK[DG]TG[VA]DVIGLDWTVDMADGRRRLG[SN][GD]I[SG][VI]Q GNVDPA[YF]L

Motif 21: A[VL]LGFVGAPWTIATY[IV]VEGG[TM]T[RN]TYT[NT]IK

According to another embodiment, in addition to any one or more of Motifs 13 to 15, a UROD polypeptide comprises any one or more of Motifs 16 to 18 and/or any one or more of Motifs 22 to 24. Motifs 22 to 24 are typically found in the HEM2 class of photosynthetic UROD (PS-UROD) polypeptides.

Motif 22: W[LM]MRQAGRYMK[SV]YQ[DIT][LI]C[EK][KR][YH]P[ST]FRERSEN[VA]DL[VA]V EISLQPW[KR][VA]FKPDGVI

Motif 23: YI[RQ]YQAD[NS]GAQ[AV]VQIFDSWATELSPVDFEEFSLPYLKQI[VI][DAE][AEST] VK[KQ]THP[DN]L

Motif 24: YVG[EQ]AL[TS]ILR[KAE]EV[GND]N[EK]A[AT]VLGFVGAPFTLASY[VI]VEGGSSK[NH][FY][TS]KIK[RK][LM]AF

According to another embodiment, in addition to any one or more of Motifs 13 to 15, a UROD polypeptide comprises any one or more of Motifs 25 to 27. Motifs 25 to 27 are typically found in the CYANO class of photosynthetic UROD (PS-UROD) polypeptides.

Motif 25: PVWMMRQAGRYMK[VI]YRDLR[DE][KN][YH]PSFRERSEN[PA]DL[AS][IY]EIS [LM]QP[WF][RHK]AFQPDG

Motif 26: Y[VL][RS]YQI[DQ][CS]GAQV[VI]Q[ML]FDSWAGQL[ST]PQDY[DE][TVE]FA[LA]P YQ[QK][KQ]W[RDN][LQ]VK[EA][TK]HPDT

Motif 27: [TR]LRQEVGN[QK][AS][TA]VLGFVG[AS]PWTLAAY[AV][IV]EGKSSK[ND]YA[VI]I KAMAFSEP[EA][IL]LH

According to another embodiment, in addition to any one or more of Motifs 13 to 15, a UROD polypeptide comprises any one or more of Motifs 28 to 30. Motifs 28 to 30 are typically found in the CYANO class of photosynthetic UROD (PS-UROD) polypeptides.

Motif 28: FR[EH]RSET[PA][ED]IAIELS[LM]Q[PC][WH]RA[FY][GKR][PM]DG[VI]IMFSDILTP LP[AT][LM]GI[ED]FD[VIM]VKGK

Motif 29: [HS]AFL[DS][KH]L[AT][DE][AM][LI][GI]VY[VA]C[HY]QI[ED]SGAQV[VI]Q[ILV]F[DE] SWA[HG][HQ]LSP[QA]Q[FY][LE]EF[SA][HKL]P[YA][AN][EQ]

Motif 30: QPW[RK][AV]F[KQ]PDGVI[LM]FSDILTPLP[GA]M[GN][IV]PFD

Motifs 13 to 30 were generated using MEME 4.0.0 from public website (http://meme.nbcr.net/meme4/cgi-bin/meme.cgi); The MEME algorithm was as disclosed in Bailey and Elkan, Proceedings of the Second International Conference on Intelligent Systems for Molecular Biology, pp. 28-36, AAAI Press, Menlo Park, California, 1994.

More preferably, the UROD polypeptide comprises in increasing order of preference any 2, 3, 4, 5, 6, 7, 8, 9, 10, 11, 12 , 13, 14, 15, 16, 17 or 18 of the abovementioned Motifs.

Additionally or alternatively, the homologue of a UROD protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 368, provided that the homologous protein comprises any one or more of the conserved motifs as outlined above.

The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the motifs in a UROD polypeptide have, in increasing order of preference, at least 70%, 71 %, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, or 99% sequence identity to any one or more of the motifs represented by SEQ ID NO: 515 to SEQ ID NO: 532 (Motifs 13 to 30).

Preferably, the polypeptide sequence which when used in the construction of a phylogenetic tree, such as the one depicted in Figure 12 or 13, clusters with the group of UROD polypeptides comprising the amino acid sequence represented by SEQ ID NO: 368 rather than with any other group.

In another embodiment a "protein useful in the methods of the invention" is taken to mean an AS-MTT polypeptide as defined herein. In another embodiment a "nucleic acid useful in the methods of the invention" is taken to mean a nucleic acid capable of encoding such an AS-MTT polypeptide. The nucleic acid to be introduced into a plant (and therefore useful in performing the methods of the invention) is any nucleic acid encoding the type of protein which will now be described, hereafter also named "AS-MTT nucleic acid" or "AS-MTT gene".

An "AS-MTT polypeptide" as defined herein refers to any polypeptide comprising a pfam conserved domain having accession number PF07889.

A PF07889 domain is also referred to as a DUF1664 domain in the terminology of the pfam database (Pfam version 23.0 (July 2008, 10340 families) R.D. Finn, J. Tate, J. Mistry, P.C. Coggill, J.S. Sammut, H.R. Hotz, G. Ceric, K. Forslund, S.R. Eddy, E.L. Sonnhammer and A. Bateman Nucleic Acids Research (2008) Database Issue 36:D281-D288). A person skilled in the art could readily determine whether any amino acid sequence in question falls within the definition of an "AS-MTT polypeptide" using known techniques and software to consult or search databases of conserved protein domains such as pfam. For Example Interpro database maybe searched as detailed in the Examples section herein.

PF07889 domain refers to a protein domain of approximately 100 amino acids long which occurs in proteins of plant origin.

Preferably the polypeptide useful in the methods of the invention comprises a protein domain having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to the amino acid sequence located between amino acid coordinates 94 to 126 in SEQ ID NO: 537 or to the amino acid sequence of a PF07889 domain as present in any of the polypeptides of Table A4.

Additionally or alternatively, the "AS-MTT polypeptide" useful in the methods of the invention is located at a membrane, preferably at the endoplamic reticulum. Preferably the "AS-MTT polypeptide" comprises a transmembrane domain, most preferably the transmembrane domain any of the transmembrane domains present in any of the polypeptides of Table A4, most preferably represented by the sequence located between amino acids 1 to 23 or 1 to 25 or 95 to 115 of SEQ ID NO: 537. A person skilled in the art could readily determine whether any amino acid sequence in question falls comprises a transmembrane domain using known techniques and software such as for Example TMHMM or SignalP as described in the Examples section herein.

Additionally or alternatively, preferably an "AS-MTT polypeptide" as defined herein is one comprising any one or more of the Motifs described below:

### Motif 31:

G[WL][SK][FL]SD[LV]M[YF][VA]T[KR]R[NS][ML][AS][ND]AV[SA][SN][VL][TS]K[QH]L[ED][Q N]VS[ESD][AS]LAA[TA]K[RK]HL[TS]QR (wherein amino acids between bracket represent alternative amino acids at that position), or a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to the sequence of Motif 31, preferably to Motif 31 as present in any of the polypeptides of Table A4 and represented by a sequence of 43 amino acid in length starting at the indicated amino acid coordinate in Table 3a;

### Motif 32:

AA[AT][VL]G[AV][VLM]GY[GC]YMWWK (wherein amino acids between bracket represent alternative amino acids at that position), or a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to the sequence of Motif 32, preferably to Motif 32 as present in any of the polypeptides of Table A4 as represented by a sequence of 15 amino acid in length starting at the indicated amino acid coordinate in Table 3a;

### Motif 33:

GAG[LY]TG[ST][IV][LV][LA][KR][NE]G[KR]L (wherein amino acids between bracket represent alternative amino acids at that position), or a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%,72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to the sequence of Motif 33, preferably to Motif 33 as present in any of the polypeptides of Table A4 as represented by a sequence of 15 amino acid in length starting at the indicated amino acid coordinate in Table 3a.

Additionally or alternatively, preferably the polypeptide useful in the methods of the invention comprises a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to any one or more of:

(i) Motif 31; and
(ii) Motif 32; and
(iii) Motif 33.

Amino acids indicated between brackets represent alternative amino acids at such amino acid position.
Motifs 31, 32 and 33 are typically found in AS-MTT polypeptides from any plant origin.
Additionally or alternatively, more preferably an "AS-MTT polypeptide" as defined herein is one comprising any one or more of the Motifs described below:

**Motif 34:**

G[LI][SK]F[SA]DLMYVTKR[NS]MA[NT]AV[SA][NS][LM]TK[HN]L[ED]QV[SQ][ED][AS]L[AS]A [AT]K[KR]HLTQR (wherein amino acids between bracket represent alternative amino acids at that position), or a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to the sequence of Motif 34, preferably to Motif 34 as present in any of the polypeptides of Table A4 as represented by a sequence of 43 amino acid in length starting at the indicated amino acid coordinate in Table 3b;

**Motif 35:**

[NG][GS][GS][SQ][GQ]G[NG]L[TS][SG]L[IV][VM]PAA[TA][LV]GA[LV]GYGYMWWK (wherein amino acids between bracket represent alternative amino acids at that position), or a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to the sequence of Motif 35, preferably to Motif 35 as present in any of the polypeptides of Table A4 as represented by a sequence of 29 amino acid in length starting at the indicated amino acid coordinate in Table 3b;

**Motif 36:**

[MA]AMQ[AS]G[MIV]G[LFV][ST][KR][IV][LV]IL[AV]GAGYTGT[IV][LMV]LKNG (wherein amino acids between bracket represent alternative amino acids at that position), or a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to the sequence of Motif 36, preferably to Motif 36 as present in any of the polypeptides of Table A4 as represented by a sequence of 29 amino acid in length starting at the indicated amino acid coordinate in Table 3b.

Additionally or alternatively, more preferably the polypeptide useful in the methods of the invention comprises a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to any one or more of:

(iv) Motif 34; and
(v) Motif 35; and
(vi) Motif 36.

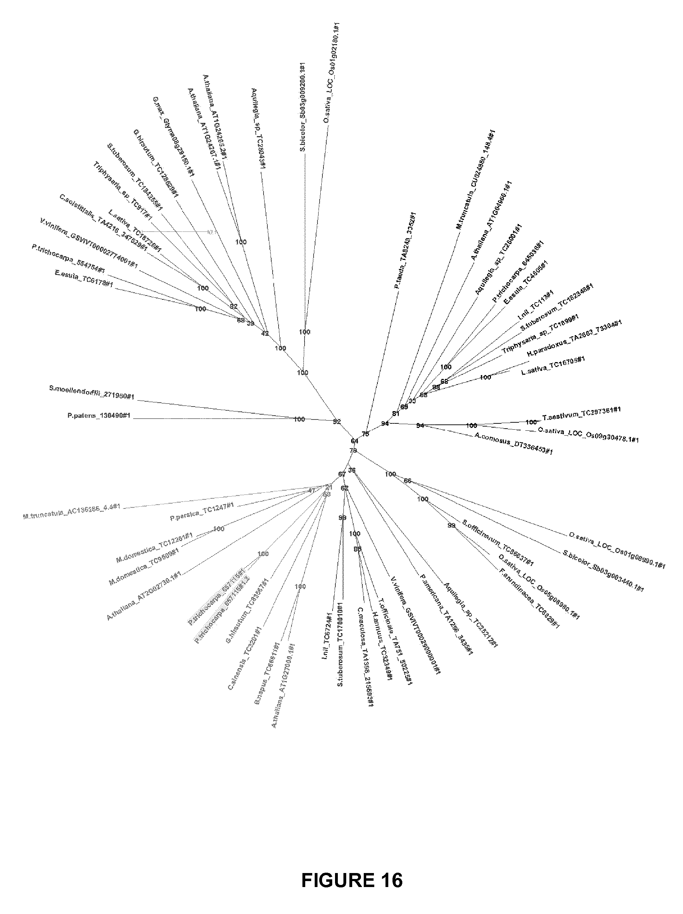

Motifs 34, 35 and 36 are typically found in any AS-MTT polypeptide from clades A and B. Such clades referring to the phylogenetic tree of Figure 16 as described in the Examples section herein.

Additionally or alternatively, even more preferably an "AS-MTT polypeptide" as defined herein is one comprising any one or more of the Motifs described below:

**Motif 37:**

LI[VM]PAATLGA[LV]GYGYMWWKGL[KS]FSDLMYVTKR[NS]MA[TS]AV[SAE]NLTK[HN]L[ ED][QS]VSE (wherein amino acids between bracket represent alternative amino acids at that position), or a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to the sequence of Motif 37, preferably to Motif 37 as present in any of the polypeptides of Table A4 as represented by a sequence of 50 amino acid in length starting at the indicated amino acid coordinate in Table 3c;

**Motif 38:**

MAMQ[AS]G[IV][GS][LVF]S[KR]ILILAGAGYT[GS]TI[LM]LKNGKLS[DE][LI][LI]GELQSL[VL][ KN]G[ML][EG][K E]SG[ED] (wherein amino acids between bracket represent alternative amino acids at that position), or a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to the sequence of Motif 38, preferably to Motif 38 as present in any of the polypeptides of Table A4 as represented by a sequence of 50 amino acid in length starting at the indicated amino acid coordinate in Table 3c;

**Motif 39:**

A[LI][AS][AT]AK[KT]HLTQRIQ[NH][LV]DDK[MV]E[KES]Q[KN][ED][IL]SK[SA]I[QK][NE][DN] VNA[AV][QS]E[ND]L (wherein amino acids between bracket represent alternative amino acids at that position), or a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to the sequence of Motif 39, preferably to Motif 39 as present in any of the polypeptides of Table A4 as represented by a sequence of 41 amino acid in length starting at the indicated amino acid coordinate in Table 3c.

Additionally or alternatively, even more preferably the polypeptide useful in the methods of the invention comprises a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to any one or more of:

(vii) Motif 37; and
(viii) Motif 38; and
(ix) Motif 39.

Motifs 37, 38 and 39 are typically found in any AS-MTT polypeptides from clade A. This clade A is shown in the phylogenetic tree of Figure 16 as defined in the Examples section herein.

Most preferably an "AS-MTT polypeptide" as defined herein is one comprising any one or more of the Motifs as described in Tables 3a, 3b, 3c.

**Tables 3a.** Motifs 31, Motif 32, and Motif 33 as present in the polypeptides of Table A4.

| | ALL AS-MTT | | |
|---|---|---|---|
| | Motif 31 | Motif 32 | Motif 33 |
| Length (in amino acid) | 43 | 15 | 15 |
| Name | Amino acid coordinate Start | Amino acid coordinate Start | Amino acid coordinate Start |
| P.trichocarpa_657115#1_A | 114 | 99 | 16 |
| P.trichocarpa_657115#1.2_A | 111 | 98 | 16 |
| P.persica_TC1247#1_A | 111 | 98 | 16 |
| C.sinensis_TC3201#1_A | 115 | 100 | 16 |
| M.domestica_TC12261#1_A | 115 | 99 | 16 |
| A.thaliana_AT2G02730.1#1_A | 120 | 99 | 16 |
| G.hirsutum_TC83567#1_A | 118 | 99 | 18 |
| B.napus_TC66611#1_A | 114 | 99 | 18 |
| A.thaliana_AT1G27000.1#1_A | 113 | 94 | 16 |
| M.domestica_TC9509#1_A | 116 | 97 | 16 |
| M.truncatula_AC136286_4.4#1_A | 113 | 97 | 16 |
| P.trichocarpa_566293#1_A | 113 | 102 | 16 |
| S.officinarum_TC86637#1_B | 116 | 103 | 16 |
| P.americana_TA1256_3435#1_B | 116 | 103 | 16 |
| C.maculosa_TA1398_215693#1_B | 117 | 98 | 16 |
| O.sativa_LOC_Os01g08990.1#1_B | 114 | 96 | 16 |
| O.sativa_LOC_Os05g08980.1#1_B | 114 | 101 | 16 |
| T.officinale_TA751_50225#1_B | 113 | 101 | 16 |
| F.arundinacea_TC6829#1_B | 113 | 95 | 16 |
| S.bicolor_Sb03g003440.1#1_B | 114 | 94 | 16 |
| I.nil_TC6724#1_B | 110 | 94 | 16 |
| S.tuberosum_TC170010#1_B | 116 | 101 | 16 |
| Aquilegia_sp_TC25212#1_B | 114 | 101 | 17 |
| V.vinifera_GSVIVT00029000001#1_B | 112 | 101 | 17 |
| H.annuus_TC32349#1_B | 114 | 99 | 16 |
| Acomosus_DT336453#1_bZIP17-like | 112 | 97 | 17 |
| Triphysaria_sp_TC1899#1_bZIP17-like | 116 | 99 | 12 |
| M.truncatula_CU024880_148.4#1_bZIP17-like | 112 | 98 | 12 |
| L.sativa_TC16705#1_bZIP17-like | 109 | 99 | 16 |
| P.taeda_TA8248_3352#1_bZIP17-like | 111 | 98 | 16 |
| H.paradoxus_TA2663_73304#1_bZIP17-like | 114 | 100 | 16 |
| E.esula_TC4805#1_bZIP17-like | 115 | 100 | 12 |
| I.nil_TC113#1_bZIP17-like | 110 | 98 | 16 |

(continued)

| | ALL AS-MTT | | |
|---|---|---|---|
| | Motif 31 | Motif 32 | Motif 33 |
| Length (in amino acid) | 43 | 15 | 15 |
| Name | Amino acid coordinate Start | Amino acid coordinate Start | Amino acid coordinate Start |
| A.thaliana_AT1G04960.1#1_bZIP17-like | 109 | 96 | 12 |
| O.sativa_LOC_Os09g30478.1#1_bZIP17-like | 114 | 99 | 12 |
| Aquilegia_sp_TC26001#1_bZIP17-like | 112 | 96 | 12 |
| H.vulgare_TC157185#1_bZIP17-like | 111 | 104 | 12 |
| P.trichocarpa_645036#1_bZIP17-like | 112 | 97 | 16 |
| T.aestivum_TC297361#1_bZIP17-like | 114 | 96 | 16 |
| S.tuberosum_TC182348#1_bZIP17-like | 109 | 97 | 18 |
| E.esula_TC6178#1_Unk | 114 | 97 | 9 |
| L.sativa_TC18726#1_Unk | 112 | 100 | 16 |
| P.patens_136490#1_Unk | 113 | 97 | 16 |
| V.vinifera_GSVIVT00002774001#1_Unk | 112 | 99 | 12 |
| C.solstitialis_TA4216_347529#1_Unk | 112 | 97 | 16 |
| S.moellendorffii_271950#1_Unk | 119 | 97 | 12 |
| A.thaliana_AT1G24267.1#1_Unk | 106 | 98 | 17 |
| G.hirsutum_TC128629#1_Unk | 112 | 91 | 17 |
| A.thaliana_AT1G24265.2#1_Unk | 115 | 95 | 12 |
| G.max_Glyma08g29150.1#1_Unk | 113 | 96 | 12 |
| S.bicolor_Sb03g009200.1#1_Unk | 111 | 96 | 16 |
| 0.sativa_LOC_Os01g02180.1#1_Unk | 109 | 99 | 10 |
| Aquilegia_sp_TC28043#1_Unk | 111 | 97 | 12 |
| S.tuberosum_TC184255#1_Unk | 112 | 94 | 18 |
| P.trichocarpa_554754#1_Unk | 112 | 97 | 10 |
| Triphysaria_sp_TC917#1_Unk | 113 | 98 | 65 |

**Table 3b.** Motifs 34, Motif 35, and Motif 36 as present the polypeptides of Clades A and B of the phylogenetic tree of Figure 16.

| | Clades A and B | | |
|---|---|---|---|
| | Motif 34 | Motif 35 | Motif 36 |
| Length in amino acids | 43 | 29 | 29 |
| Name | Amino acid coordinate Start | Amino acid coordinate End | Amino acid coordinate End |
| I.nil_TC6724#1_B | 111 | 83 | 1 |
| C.maculosa_TA1398-215693#1_B | 113 | 83 | 1 |
| S.tuberosum_TC170010#1_B | 111 | 87 | 1 |

(continued)

| Name | Clades A and B | | |
|---|---|---|---|
| | Motif 34 | Motif 35 | Motif 36 |
| Length in amino acids | 43 | 29 | 29 |
| Name | Amino acid coordinate Start | Amino acid coordinate End | Amino acid coordinate End |
| O.sativa_LOC_Os05g08980.1#1_B | 118 | 87 | 1 |
| S.officinarum_TC86637#1_B | 120 | 87 | 1 |
| H.annuus_TC32349#1_B | 113 | 87 | 1 |
| P.trichocarpa_566293#1_A | 116 | 84 | 1 |
| P.trichocarpa_657115#1_A | 116 | 82 | 1 |
| P.americana_TA1256_3435#1_B | 116 | 81 | 1 |
| A.thaliana_AT1G27000.1#1_A | 114 | 82 | 1 |
| T.officinale_TA751_50225#1_B | 113 | 80 | 1 |
| Aquilegia_sp_TC25212#1_B | 110 | 80 | 1 |
| F.arundinacea_TC6829#1_B | 117 | 85 | 1 |
| P.trichocarpa_657115#1.2_A | 116 | 83 | 1 |
| G.hirsutum_TC83567#1_A | 110 | 84 | 1 |
| B.napus_TC66611#1_A | 112 | 84 | 1 |
| P.persica_TC1247#1_A | 109 | 88 | 1 |
| V.vinifera_GSVIVT00029000001#1_B | 116 | 89 | 1 |
| M.domestica_TC12261#1_A | 112 | 89 | 1 |
| A.thaliana_AT2G02730.1#1_A | 111 | 82 | 2 |
| O.sativa_LOC_Os01g08990.1#1_B | 111 | 90 | 1 |
| C.sinensis_TC3201#1_A | 109 | 87 | 1 |
| S.bicolor_Sb03g003440.1#1_B | 114 | 85 | 1 |
| M.domestica_TC9509#1_A | 112 | 81 | 1 |
| M.truncatula_AC136286_4.4#1_A | 119 | 82 | 1 |

**Table 3c.** Motifs 37, Motif 38, and Motif 39 as present the polypeptides of Clades A of the phylogenetic tree of Figure 16.

| Name | Clade A | | |
|---|---|---|---|
| | Motif 37 | Motif 38 | Motif 39 |
| Length in amino acids | 50 | 50 | 41 |
| Name | Amino acid coordinate Start | Amino acid coordinate End | Amino acid coordinate End |
| P.trichocarpa_566293#1_A | 97 | 1 | 147 |
| P.trichocarpa_657115#1_A | 97 | 1 | 147 |
| P.trichocarpa_657115#1.2_A | 97 | 1 | 147 |
| P.persica_TC1247#1_A | 90 | 1 | 141 |
| M.domestica_TC12261#1_A | 93 | 1 | 143 |

(continued)

| | Clade A | | |
| --- | --- | --- | --- |
| | Motif 37 | Motif 38 | Motif 39 |
| Length in amino acids | 50 | 50 | 41 |
| Name | Amino acid coordinate Start | Amino acid coordinate End | Amino acid coordinate End |
| G.hirsutum_TC83567#1_A | 91 | 1 | 140 |
| A.thaliana_AT2G02730.1#1_A | 92 | 1 | 143 |
| A.thaliana_AT1G27000.1#1_A | 95 | 1 | 145 |
| M.domestica_TC9509#1_A | 93 | 1 | 140 |
| B.napus_TC66611#1_A | 93 | 1 | 143 |
| C.sinensis_TC3201#1_A | 90 | 1 | 150 |
| M.truncatula_AC136286_4.4#1_A | 100 | 2 | 142 |

More preferably, the AS-MTT polypeptide comprises in increasing order of preference, at least 2, at least 3, at least 4, at least 5, at least 6, at least 7, at least 8, or all 12.

Alternatively, preferably, the AS-MTT polypeptide refers to a homologue of any of the polypeptides of Table A4.

Additionally or alternatively, the homologue of an AS-MTT protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100 % overall sequence identity to the amino acid represented by any of the polypeptides of Table A4 and most preferably to SEQ ID NO: 537. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the homologue of an AS-MTT polypeptide has, in increasing order of preference, at least 70%, 71 %, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, or 99% sequence identity to any of the motifs represented by Motifs 37, Motif 38 and Motif 39.

Preferably, the AS-MTT polypeptide sequence when used in the construction of a phylogenetic tree such as the one depicted in Figure 16, clusters within Clade A, preferably with the polypeptide represented by SEQ ID NO: 537.

In another embodiment a "protein useful in the methods of the invention" is taken to mean an EXO-1 polypeptide as defined herein. In another embodiment a "nucleic acid useful in the methods of the invention" is taken to mean a nucleic acid capable of encoding such an EXO-1 polypeptide. The nucleic acid to be introduced into a plant (and therefore useful in performing the methods of the invention) is any nucleic acid encoding the type of protein which will now be described, hereinafter also named "*EXO-1* nucleic acid" or "*EXO-1* gene".

An "EXO-1 polypeptide" as defined herein refers to any polypeptide comprising at least a XPG_N domain in the N-terminal region of the polypeptide with a PFAM accession number PF 00752 followed by a XPG_1 domain having a PFAM accession number PF 00867 and included in the EXONUCLEASE 1 domain (PTHR 11081 :SF8 or PTHR 11081 :SF9).

EXO-1 polypeptides typically belong to the nucleases family and proteins comprising EXO-1 polypeptide are involved in DNA repair process, more specifically having a 5'-nuclease activity (EC 3.1.11.1).

Preferably, the XPG_N domain of an EXO-1 polypeptide has, in increasing order of preference at least, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% or more sequence identity to the sequence located between amino acid 1 and 99 of SEQ ID NO: 763.

Preferably, the XPG_I domain of an EXO-1 polypeptide has, in increasing order of preference at least, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% or more sequence identity to the sequence located between amino acid 138 and 230 of SEQ ID NO: 763.

Preferably, the EXONUCLEASE 1 domain of an EXO-1 polypeptide has, in increasing order of preference at least, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% or more sequence identity to the sequence located between amino acid 17 and 333 of SEQ ID NO: 763.

Additionally or alternatively, the EXO-1 polypeptide useful in the methods of the invention comprises one or more sequence motifs having at least, in increasing order of preference 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, or 99% or more sequence identity to any one or more of motifs 40 to 42:

**Motif 40:** G[QKC][RT]VA[VI]D[TA]YSWLH[KR][GA]A[YL]SC[SA]RELC[KEL]GLPT

**Motif 41:** Y[CF]M[HK]RVN[LM]L[RL]H[YH][GK][VI]KP[IV][LV]VFDGGRLPMK[AS][DE][QTE] ENKR[AR]R[SK]RKENL[EA]RA[KR]E[HL][ELW]

**Motif 42:** V[DQA]A[VI]ITEDSDL[IL][AP][FY]GC[PK]R[IV][IF]FK[ML]D[KR][FYN]GQG

The above mentioned motifs were outlined using MEME algorithm (Bailey and Elkan, Proceedings of the Second International Conference on Intelligent Systems for Molecular Biology, pp. 28-36, AAAI Press, Menlo Park, California, 1994.)

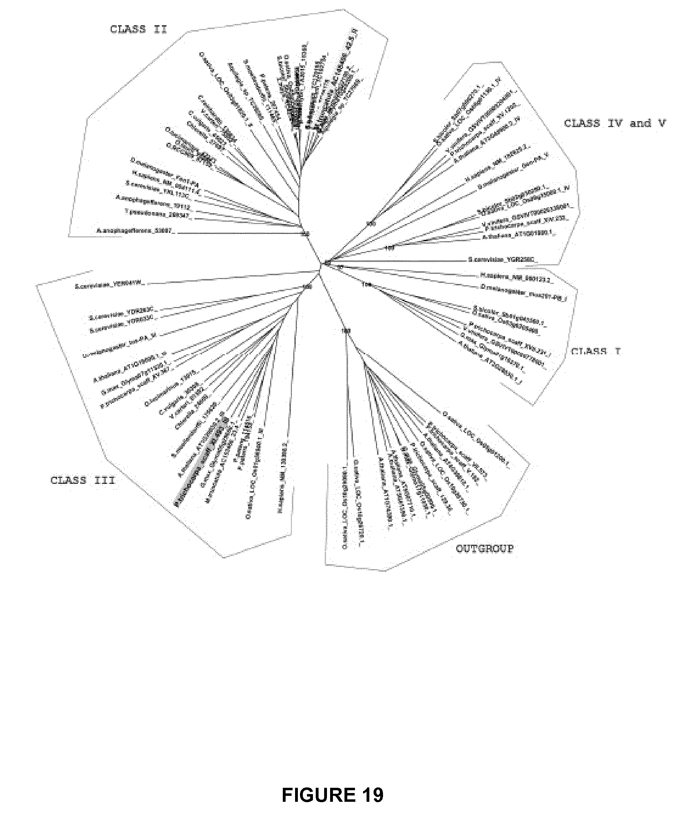

Additionally or alternatively, the homologue of an EXO-1 protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 763, provided that the homologous protein comprises any one or more of the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the motifs in an EXO-1 polypeptide have, in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any one or more of the motifs represented by SEQ ID NO: 828 to SEQ ID NO: 830 (Motifs 40 to 42). Preferably, the polypeptide sequence which when used in the construction of a phylogenetic tree, such as the one depicted in Figure 19, clusters with the group of EXO-1 polypeptides comprising the amino acid sequence represented by SEQ ID NO: 763 rather than with any other group.

In another embodiment a "protein useful in the methods of the invention" is taken to mean a YiAP2 polypeptide as defined herein. In another embodiment a "nucleic acid useful in the methods of the invention" is taken to mean a nucleic acid capable of encoding such a YiAP2 polypeptide. The nucleic acid to be introduced into a plant (and therefore useful in performing the methods of the invention) is any nucleic acid encoding the type of protein which will now be described, hereafter also named "*YiAP2* nucleic acid" or "*YiAP2* gene".

A "YiAP2 polypeptide" as defined herein refers to any polypeptide comprising at least two AP2 domains (Pfam accession number PF00847; InterPro accession IPR001471) and anyone or more of the following motifs:

**Motif 43** as represented by a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to EYIGSLR[R/G]KSSGFSRGVSKYRGVARHHHDGR WEARIGKVFGNKYLYLGT[Y/F] (SEQ ID NO: 890) or to any of the homologous region of Motif 43 as present in any of the polypeptides of Table A6, wherein amino acids between bracket represent alternative amino acids at that position;

**Motif 44** as represented by a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to RKSVPRDSPPQRSSIYRGVTRHRWTGRYEAHL WDKNCWNESQNKKGRQVY (SEQ ID NO: 891)or to any of the homologous region of Motif 44 as present in any of the polypeptides of Table A6.

**Motif 45** as represented by a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to YD[DN]EEAAA[RH]AYDLAALKYWG[QH]DTILNFPLS [NT]YEEEL[KV]EMEG (SEQ ID NO: 892) or to any of the homologous region of Motif 45 as present in any of the polypeptides of Table A6, wherein amino acids between bracket represent alternative amino acids at that position;

**Motif 46** as represented by a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to ATQEEAA[TQ]AYD[MR]AAIEYRGLNAVTNFDLS [RK]YIKWLRPNNNQ (SEQ ID NO: 893) or to any of the homologous region of Motif 46 as present in any of the polypeptides of Table A6, wherein amino acids between bracket represent alternative amino acids at that position;

**Motif 47** as represented by a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to [QE]SQ[KP][PN][RTQ][CTR][ST][FN]P[DNE][DN][IN] [QN]T[YV][FT][ET][CK][TQ][DK] (SEQ ID NO: 894) or to any of the homologous region of Motif 47 as present in any of the polypeptides of Table A6, wherein amino acids between bracket represent alternative amino acids at that position;

**Motif 48** as represented by a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to QS[SP]KFKE[MT][LV]E (SEQ ID NO: 895) or to any of the homologous region of Motif 48 as present in any of the polypeptides of Table A6, wherein amino acids between bracket represent alternative amino acids at that position.

Table 4a localises the homologous region of Motifs 43 to 48 in the polypeptides of Table A6. The length of the each motif as well as the amino acid coordinate labelling the start of motif is given.
Preferably A "YiAP2 polypeptide" comprises at least two AP2 domains (Pfam accession number PF00847; InterPro accession IPR001471) and in increasing order of preference at least 1, 2, 3, 4, 5, or all 6 motifs, more preferably motifs 43, 44 and 46.

**Table 4a.** Motifs 43 to 48 as present in the polypeptides of table A6.

| | YiAP2 | | | | | |
|---|---|---|---|---|---|---|
| Motif Name | Motif 43 | Motif 44 | Motif 45 | Motif 46 | Motif 47 | Motif 48 |
| Motif Length (in amino acid) | 50 | 50 | 41 | 41 | 21 | 10 |
| Name polypeptide | Amino acid coordinate Start | Amino acid coordinate Start | Amino acid coordinate Start | Amino acid coordinate Start | Amino acid coordinate Start | Amino acid coordinate Start |
| S.tuberosum_TC187592#1 | 142 | 44 | 97 | 192 | 369 | 314 |
| A.thaliana_AT1G16060.1#1 | 142 | 44 | 97 | 192 | 350 | 303 |
| O.sativa_LOC_Os08g34360. | 139 | 41 | 94 | 189 | 344 | 295 |
| Z.mays_TC386652#1 | 137 | 39 | 92 | 187 | 325 | 292 |
| G.hirsutum_TC125414#1 | 134 | 36 | 89 | 184 | 313 | 290 |
| R.communis_TA2948_3988#1 | 133 | 36 | 88 | 183 | 310 | 287 |
| R.communis_EG658396#1 | 133 | 36 | 88 | 183 | 310 | 287 |
| S.bicolor_Sb02g025080.1# | 132 | 36 | 87 | 182 | 308 | 287 |
| O.sativa_LOC_Os09g25600. | 131 | 35 | 86 | 181 | 303 | 283 |
| M.truncatula_AC126784_13 | 131 | 35 | 86 | 181 | 301 | 281 |
| G.max_Glyma17g07860.1#1 | 131 | 34 | 86 | 181 | 299 | 279 |
| Medtr_AP2 | 131 | 33 | 86 | 181 | 299 | 278 |
| V.vinifera_GSVIVT0001713 | 129 | 31 | 84 | 179 | 294 | 277 |
| C.sinensis_TC9216#1 | 128 | 30 | 83 | 178 | 275 | 277 |
| C.clementina_TC2567#1 | 128 | 30 | 83 | 178 | 238 | 277 |
| C.clementina_TC1922#1 | 128 | 30 | 83 | 178 | 226 | 277 |
| C.clementina_DY301305#1 | 128 | 30 | 83 | 178 | 10 | 275 |
| C.clementina_DY290073#1 | 128 | 30 | 83 | 178 | 7 | 265 |
| C.clementina_DY288437#1 | 128 | 30 | 83 | 178 | 7 | 232 |
| Aquilegia_sp_TC20979#1 | 128 | 30 | 83 | 178 | 7 | 228 |
| C.sinensis_EY655317#1 | 128 | 30 | 83 | 178 | 7 | 228 |
| C.sinensis_EY726128#1 | 128 | 30 | 83 | 178 | 7 | 6 |

(continued)

| | YiAP2 | | | | | |
|---|---|---|---|---|---|---|
| Motif Name | Motif 43 | Motif 44 | Motif 45 | Motif 46 | Motif 47 | Motif 48 |
| Motif Length (in amino acid) | 50 | 50 | 41 | 41 | 21 | 10 |
| Name polypeptide | Amino acid coordinate Start | Amino acid coordinate Start | Amino acid coordinate Start | Amino acid coordinate Start | Amino acid coordinate Start | Amino acid coordinate Start |
| Triphysaria_sp_TC12598#1 | 128 | 30 | 83 | 178 | 7 | 6 |
| O.basilicum_TA1087_39350 | 126 | 28 | 81 | 176 | 7 | 6 |
| P.trichocarpa_800184#1 | 122 | 27 | 77 | 172 | 7 | 6 |
| G.max_Glyma07g02380.1#1 | 112 | 17 | 67 | 162 | 4 | 6 |

A PF00847 domain is also referred to as AP2 domain in the terminology of the pfam database (Pfam version 23.0 (July 2008, 10340 families) R.D. Finn, J. Tate, J. Mistry, P.C. Coggill, J.S. Sammut, H.R. Hotz, G. Ceric, K. Forslund, S.R. Eddy, E.L. Sonnhammer and A. Bateman Nucleic Acids Research (2008) Database Issue 36:D281-D288). A person skilled in the art could readily determine whether any amino acid sequence in question falls within the definition of a "YiAP2polypeptide" using known techniques and software to consult or search databases of conserved protein domains such as pfam. For Example Interpro database maybe searched as detailed in the Examples section herein.

PF00847 domain refers to a protein domain of approximately 60 amino acids long which typically occurs in proteins of plant origin.

Preferably an AP2 domain in a polypeptide useful in the methods of the invention comprises a protein domain having in increasing order of preference at least 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% sequence identity to any one or more of the following:

(i) the amino acid sequence located between amino acid coordinates 49 to 106 of SEQ ID NO: 836, which represent the N-terminal AP2 domain in SEQ ID NO: 836;
(ii) the sequence located between amino acid coordinates 148 to 200 of SEQ ID NO: 836, which represent the C-terminal AP2 domain in SEQ ID NO: 836;
(iii) the amino acid sequence of an AP2 domain as present in any of the polypeptides of Table A6.

Conserved motifs in YiAP2 polypeptides, such as Motifs 43 to 48 may be defined by using the algorithm MEME; Bailey and Elkan, Proceedings of the Second International Conference on Intelligent Systems for Molecular Biology, pp. 28-36, AAAI Press, Menlo Park, California, 1994, Bailey et al., Nucleic Acids Research, 34, W369-W373, 2006), with the set of sequences of Table A6.

Additionally or alternatively, the "YiAP2 polypeptide" useful in the methods of the invention, and when expressed in plants, is located in the nucleus. Preferably the "YiAP2 polypeptide" comprises a nuclear localisation signal. A person skilled in the art can readily determine whether an amino acid sequence in question comprises a comprises a nuclear localisation signal using known techniques and software such as for example SignalP as described in the Examples section herein.

Alternatively and preferably, the YiAP2 polypeptide refers to a homologue of any of the polypeptides of Table A6.

Preferably the homologue of an YiAP2 polypeptide comprises two or more AP2 domains having in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to any one or more of the following:

(i) the amino acid sequence located between amino acid coordinates 49 to 106 of SEQ ID NO: 836, which represent the N-terminal AP2 domain in SEQ ID NO: 836;
(ii) the sequence located between amino acid coordinates 148 to 200 of SEQ ID NO: 836, which represent the C-terminal AP2 domain in SEQ ID NO: 836;
(iii) the amino acid sequence of an AP2 domain as present in any of the polypeptides of Table A6.

The AP2 domain is well known to a person skill in the art, and described extensively in databases such as pfam, interpro and smart (Bateman et al., Nucleic Acids Research 30(1): 276-280 (2002); Mulder et al., (2003) Nucl. Acids. Res. 31, 315-318; Letunic et al. (2006) Nucleic Acids Res 34, D257-D260). The sequence **"xazGVpxzxhGxzWxucltcsxxxxxxxxxxttc laLGoFsotytAAhAYDxAAhhhhGxxpAhhNFsxxxxtt"** (SEQ ID NO: 897) represents a consensus sequence of an AP2 domain as provided in the SMART database. The accession number for the AP2 domain in the SMART database is SM00380. The non-capital small letters represent a code for groupings of amino acids. Amino acids within the same group share similar chemical properties. The description of the codes used for each amino acid grouping in the consensus sequence is given in table 4b. Any of the amino acids within one given group of Table 4b represents an alternative amino acid at the position where the corresponding groups code is indicated. The AP2 domain is around 60-70 amino acids in length and has DNA binding activity (Ohme-takagi and Shinshi; Plant Cell 1995;7:173-182) and when these sequences are aligned, gaps and insertions, typically up to 5 amino acids, may be allowed. It for example binds to the GCC-box present in promoters of pathogenesis-related proteins (Liu et al. 2006. FEBS Lett. 580(5): 1303-8).

**Table 4b.** Description of the amino acid groups used in the AP2 consensus sequence. "Group" refers to the amino acid classification, "code" denotes the abbreviation code used for a group, "residues" indicate the amino acids falling within in a given class.

| Group | code | Amino Acid Residues |
|---|---|---|
| alcohol | o | S,T |
| aliphatic | l | I,L,V |
| any | x | A,C,D,E,F,G,H,I,K,L,M,N,P,Q,R,S,T,V,W,Y |
| aromatic | a | F,H,W,Y |
| charged | c | D,E,H,K,R |
| hydrophobic | h | A,C,F,G,H,I,K,L,M,R,T,V,W,Y |
| negative | y | D,E |
| polar | p | C,D,E,H,K,N,Q,R,S,T |
| positive | z | H,K,R |
| small | s | A,C,D,G,N,P,S,T,V |
| tiny | u | A,G,S |
| turnlike | t | A,C,D,E,G,H,K,N,Q,R,S,T |

Additionally or alternatively, a YiAP2 protein has in increasing order of preference at least 90%, 91%, 92%, 93%, 94%, 95%, 96%, 97%, 98%, or 99% overall sequence identity to the amino acid represented by the consensus sequence of an AP2 domain as represented above (SEQ ID NO: 897).

Methods to identify an AP2 domain are described herein. Further details are given in the Examples section.

Additionally or alternatively, the homologue of an YiAP2 protein has in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100 % overall sequence identity to the amino acid represented by any of the polypeptides of Table A6 most preferably to SEQ ID NO: 836. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

Preferably the YiAP2 polypeptide useful in the methods of the invention is capable of binding to a DNA fragment of at least 10, 20, 30, 50, 60, 70, 80, 90, 100, 200, 250, 500, 650, 750, 1000, 1500 nucleotides long comprising one or more GCC boxes (SEQ ID NO: 896: TAAGAGCCGCC) preferably to the promoter DNA fragment used by Ohme-takagi 1995. Methods to assay DNA binding of AP2 domains are well known in the art, for example as described by Yoh-Sakumaa 2002, Biochemical and Biophysical Research Communications, 290, 998-1009) and yeast one hybrid assay maybe used such as described in Middleton et al., 2007 (Plant Cell 19: 1221-1234) or in Kizis and Pages (Plant J. 2002;30(6):679-89).

The terms "domain", "signature" and "motif" are defined in the "definitions" section herein.

Furthermore, FSM1-like polypeptides (at least in their native form) typically have DNA-binding activity. Tools and techniques for measuring DNA-binding activity (e.g. gel retardation assays) are well known in the art. Further details are provided in Example 6.

In addition, FSM1-like polypeptides, when expressed in rice according to the methods of the present invention as outlined in Examples 7 and 8, give plants having increased yield related traits, in particular increased seed yield (Example 11).

Furthermore, PIF3-like polypeptides (at least in their native form) typically have protein binding activity. Tools and techniques for measuring protein binding activity are well known in the art, a well known example is the Yeast Two Hybrid assay. Further details are provided in Example 6.

In addition, PIF3-like polypeptides, when expressed in rice according to the methods of the present invention as outlined in Examples 7 and 8, give plants having increased yield related traits, in particular early vigour or increased seed yield.

Furthermore, UROD polypeptides (at least in their native form) typically have uroporphyrinogen II decarboxylase or UROD enzyme activity catalysing the following reaction:

uroporphyrinogen-III <=> 4 CO2 + coproporphyrinogen III.

UROD is classified in Enzyme Classification code: EC.4.1.1.37.

Tools and techniques for measuring porphyrin activity are well known in the art and are mainly performed by chromatographic and spectral methods, such as HPLC and TLC (Jacobs and Jacobs, 1993, Plant Physiol. 101(4): 1181-1187) and Magnetic Circular Dichroism (Ivanetich et al., 1984, Clin Chem. 30(3): 391-4). The crystal structure of UROD in plants has been determined by Martins et al., 2001, J Biol Chem. 276(47): 44108-44116.

In addition, UROD polypeptides, when expressed in rice according to the methods of the present invention as outlined in the Examples section herein, give plants having increased yield related traits.

Furthermore, preferably the AS-MTT polypeptide typically have DNA binding activity. Tools and techniques for measuring DNA binding activity are well known in the art.

In addition, preferably AS-MTT polypeptides, when expressed in rice according to the methods of the present invention as outlined in the Examples section, give plants having increased yield related traits, preferably biomass or seed yield increase preferably when the plant is cultivated under (abiotic) stress conditions.

Furthermore, EXO-1 polypeptides (at least in their native form) typically have nuclease activity. Tools and techniques for measuring nuclease activity are well known in the art. Further details are provided in Example 6.

In addition, EXO-1 polypeptides, when expressed in rice according to the methods of the present invention as outlined in Examples 7 and 8, give plants having increased yield related traits, in particular increased biomass, erectness, height, greenness, root mass.

Furthermore, preferably the YiAP2 polypeptide typically have DNA binding activity. Tools and techniques for measuring DNA binding activity are well known in the art.

In addition, preferably YiAP2 polypeptides, when expressed in rice according to the methods of the present invention as outlined in the Examples section, give plants having increased yield related traits, preferably increased seed yield.

Concerning FSM1-like polypeptides, the present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any FSM1-like-encoding nucleic acid or FSM1-like polypeptide as defined herein.

Examples of nucleic acids encoding FSM1-like polypeptides are given in Table A1 of the Examples section herein. Such nucleic acids are useful in performing the methods of the invention. The amino acid sequences given in Table A1 of the Examples section are example sequences of orthologues and paralogues of the FSM1-like polypeptide represented by SEQ ID NO: 2, the terms "orthologues" and "paralogues" being as defined herein. Further orthologues and paralogues may readily be identified by performing a so-called reciprocal blast search as described in the definitions section; where the query sequence is SEQ ID NO: 1 or SEQ ID NO: 2, the second BLAST (back-BLAST) would be against *Solanum lycopersicum* sequences.

Concerning PIF3-like polypeptides, the present invention is illustrated by transforming plants with the nucleic acid sequence represented by SEQ ID NO: 292, encoding the polypeptide sequence of SEQ ID NO: 293. However, performance of the invention is not restricted to these sequences; the methods of the invention may advantageously be performed using any PIF3-like-encoding nucleic acid or PIF3-like polypeptide as defined herein.

Examples of nucleic acids encoding PIF3-like polypeptides are given in Table A2 of the Examples section herein. Such nucleic acids are useful in performing the methods of the invention. The amino acid sequences given in Table A2 of the Examples section are example sequences of orthologues and paralogues of the PIF3-like polypeptide represented by SEQ ID NO: 293, the terms "orthologues" and "paralogues" being as defined herein. Further orthologues and paralogues may readily be identified by performing a so-called reciprocal blast search as described in the definitions section; where the query sequence is SEQ ID NO: 292 or SEQ ID NO: 293, the second BLAST (back-BLAST) would be against rice sequences.

Concerning UROD polypeptides, the present invention is illustrated by transforming plants with the nucleic acid sequence represented by SEQ ID NO: 367, encoding the polypeptide sequence of SEQ ID NO: 368. However, performance of the invention is not restricted to these sequences; the methods of the invention may advantageously be performed using any UROD-encoding nucleic acid or UROD polypeptide as defined herein.

Examples of nucleic acids encoding UROD polypeptides are given in Table A3 of the Examples section herein. Such nucleic acids are useful in performing the methods of the invention. The amino acid sequences given in Table A3 of the Examples section are example sequences of orthologues and paralogues of the UROD polypeptide represented by SEQ ID NO: 368, the terms "orthologues" and "paralogues" being as defined herein. Further orthologues and paralogues may readily be identified by performing a so-called reciprocal blast search as described in the definitions section; where the query sequence is SEQ ID NO: 367 or SEQ ID NO: 368, the second BLAST (back-BLAST) would be against Poplar sequences.

Concerning AS-MTT polypeptides, the present invention is illustrated by transforming plants with the nucleic acid sequence represented by SEQ ID NO: 536, encoding the polypeptide sequence of SEQ ID NO: 537. However, performance of the invention is not restricted to these sequences; the methods of the invention may advantageously be performed using any AS-MTT-encoding nucleic acid or AS-MTT polypeptide as defined herein.

Examples of nucleic acids encoding AS-MTT polypeptides are given in Table A4 of the Examples section herein. Such nucleic acids are useful in performing the methods of the invention. The amino acid sequences given in Table A4 of the Examples section are example sequences of orthologues and paralogues of the AS-MTT polypeptide represented by SEQ ID NO: 537, the terms "orthologues" and "paralogues" being as defined herein. Further orthologues and paralogues may readily be identified by performing a so-called reciprocal blast search as described in the definitions section; where the query sequence is SEQ ID NO: 536 or SEQ ID NO: 537, the second BLAST (back-BLAST) would be against poplar sequences.

Concerning EXO-1 polypeptides, the present invention is illustrated by transforming plants with the nucleic acid sequence represented by SEQ ID NO: 762, encoding the polypeptide sequence of SEQ ID NO: 763. However, performance of the invention is not restricted to these sequences; the methods of the invention may advantageously be performed using any EXO-1-encoding nucleic acid or EXO-1 polypeptide as defined herein.

Examples of nucleic acids encoding EXO-1 polypeptides are given in Table A5 of the Examples section herein. Such nucleic acids are useful in performing the methods of the invention. The amino acid sequences given in Table A5 of the Examples section are example sequences of orthologues and paralogues of the EXO-1 polypeptide represented by SEQ ID NO: 763, the terms "orthologues" and "paralogues" being as defined herein. Further orthologues and paralogues may readily be identified by performing a so-called reciprocal blast search as described in the definitions section; where the query sequence is SEQ ID NO: 762 or SEQ ID NO: 763, the second BLAST (back-BLAST) would be against Populus trichocarpa sequences.

Concerning YiAP2 polypeptides, the present invention is illustrated by transforming plants with the nucleic acid sequence represented by SEQ ID NO: 835, encoding the polypeptide sequence of SEQ ID NO: 836. However, performance of the invention is not restricted to these sequences; the methods of the invention may advantageously be performed using any YiAP2-encoding nucleic acid or YiAP2 polypeptide as defined herein.

Examples of nucleic acids encoding YiAP2 polypeptides are given in Table A6 of the Examples section herein. Such nucleic acids are useful in performing the methods of the invention. The amino acid sequences given in Table A6 of the Examples section are example sequences of orthologues and paralogues of the YiAP2 polypeptide represented by SEQ ID NO: 836, the terms "orthologues" and "paralogues" being as defined herein. Further orthologues and paralogues may readily be identified by performing a so-called reciprocal blast search as described in the definitions section; where the query sequence is SEQ ID NO: 835 or SEQ ID NO: 836, the second BLAST (back-BLAST) would be against Medicago truncatula sequences.

Nucleic acid variants may also be useful in practising the methods of the invention. Examples of such variants include nucleic acids encoding homologues and derivatives of any one of the amino acid sequences given in Table A1 to A6 of the Examples section, the terms "homologue" and "derivative" being as defined herein. Also useful in the methods of the invention are nucleic acids encoding homologues and derivatives of orthologues or paralogues of any one of the amino acid sequences given in Table A1 to A6 of the Examples section. Homologues and derivatives useful in the methods of the present invention have substantially the same biological and functional activity as the unmodified protein from which they are derived. Further variants useful in practising the methods of the invention are variants in which codon usage is optimised or in which miRNA target sites are removed.

Further nucleic acid variants useful in practising the methods of the invention include portions of nucleic acids encoding FSM1-like polypeptides, or PIF3-like polypeptides, or UROD polypeptides, or AS-MTT polypeptides, or EXO-1 polypeptides, or YiAP2 polypeptides, nucleic acids hybridising to nucleic acids encoding FSM1-like polypeptides, or PIF3-like polypeptides, or UROD polypeptides, or AS-MTT polypeptides, or EXO-1 polypeptides, or YiAP2 polypeptides, splice variants of nucleic acids encoding FSM1-like polypeptides, or PIF3-like polypeptides, or UROD polypeptides, or AS-MTT polypeptides, or EXO-1 polypeptides, or YiAP2 polypeptides, allelic variants of nucleic acids encoding FSM1-like polypeptides, or PIF3-like polypeptides, or UROD polypeptides, or AS-MTT polypeptides, or EXO-1 polypeptides, or YiAP2 polypeptides, and variants of nucleic acids encoding FSM1-like polypeptides, or PIF3-like polypeptides, or UROD polypeptides, or AS-MTT polypeptides, or EXO-1 polypeptides, or YiAP2 polypeptides, obtained by gene shuffling. The terms hybridising sequence, splice variant, allelic variant and gene shuffling are as described herein.

Nucleic acids encoding FSM1-like polypeptides, or PIF3-like polypeptides, or UROD polypeptides, or AS-MTT polypeptides, or EXO-1 polypeptides, or YiAP2 polypeptides, need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A1 to A6 of the Examples section, or a portion of a nucleic acid encoding an orthologue, paralogue or homologue of any of the amino acid sequences given in Table A of the Examples section.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Concerning FSM1-like polypeptides, portions useful in the methods of the invention, encode a FSM1-like polypeptide as defined herein, and have substantially the same biological activity as the amino acid sequences given in Table A1 of the Examples section. Preferably, the portion is a portion of any one of the nucleic acids given in Table A1 of the Examples section, or is a portion of a nucleic acid encoding an orthologue or paralogue of any one of the amino acid sequences given in Table A1 of the Examples section. Preferably the portion is at least 150, 200, 250, 300, 350, 400 consecutive nucleotides in length, the consecutive nucleotides being of any one of the nucleic acid sequences given in Table A1 of the Examples section, or of a nucleic acid encoding an orthologue or paralogue of any one of the amino acid sequences given in Table A1 of the Examples section. Most preferably the portion is a portion of the nucleic acid of SEQ ID NO: 1. Preferably, the portion encodes a fragment of an amino acid sequence comprising a MYB/SANT domain, which when used in the construction of a phylogenetic tree such as the one depicted in Figure 3, clusters with the group of FSM1-like polypeptides comprising the amino acid sequence represented by SEQ ID NO: 2 rather than with other MYB proteins and which comprises one or more of the motifs 1 to 6 as outlined above.

Concerning PIF3-like polypeptides, portions useful in the methods of the invention, encode a PIF3-like polypeptide as defined herein, and have substantially the same biological activity as the amino acid sequences given in Table A2 of the Examples section. Preferably, the portion is a portion of any one of the nucleic acids given in Table A2 of the Examples section, or is a portion of a nucleic acid encoding an orthologue or paralogue of any one of the amino acid sequences given in Table A2 of the Examples section. Preferably the portion is at least 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000, 2050, 2100, 2150, 2200 consecutive nucleotides in length, the consecutive nucleotides being of any one of the nucleic acid sequences given in Table A2 of the Examples section, or of a nucleic acid encoding an orthologue or paralogue of any one of the amino acid sequences given in Table A2 of the Examples section. Most preferably the portion is a portion of the nucleic acid of SEQ ID NO: 292. Preferably, the portion encodes a fragment of an amino acid sequence which comprises one or more of the motifs as defined above and which, when used in the construction of a phylogenetic tree, such as the one depicted in Figure 8, clusters with the group of PIF3-like polypeptides comprising the amino acid sequence represented by SEQ ID NO: 293 rather than with any other group of bHLH proteins.

Concerning UROD polypeptides, portions useful in the methods of the invention, encode a UROD polypeptide as defined herein, and have substantially the same biological activity as the amino acid sequences given in Table A3 of the Examples section. Preferably, the portion is a portion of any one of the nucleic acids given in Table A3 of the Examples section, or is a portion of a nucleic acid encoding an orthologue or paralogue of any one of the amino acid sequences given in Table A3 of the Examples section. Preferably the portion is at least 500, 550, 600, 650, 700, 750, 800, 850, 900, 950, 1000, 1050, 1100, 1150, 1200, 1250 consecutive nucleotides in length, the consecutive nucleotides being of any one of the nucleic acid sequences given in Table A3 of the Examples section, or of a nucleic acid encoding an orthologue or paralogue of any one of the amino acid sequences given in Table A3 of the Examples section. Most preferably the portion is a portion of the nucleic acid of SEQ ID NO: 367. Preferably, the portion encodes a fragment of an amino acid sequence which, when used in the construction of a phylogenetic tree, such as the one depicted in Figure 12 or 13, clusters with the group of UROD polypeptides comprising the amino acid sequence represented by SEQ ID NO: 368 rather than with any other group and may further comprise any one or more of Motifs 13 to 30.

Concerning AS-MTT polypeptides, portions useful in the methods of the invention, encode an AS-MTT polypeptide as defined herein, and have substantially the same biological activity as the amino acid sequences given in Table A4 of the Examples section.

Preferably, the portion is a portion of any one of the nucleic acids given in Table A4 of the Examples section, or is a portion of a nucleic acid encoding an orthologue or paralogue of any one of the amino acid sequences given in Table A4 of the Examples section. Preferably the portion is at least 80, 90, 100, 120, 150, 200, 250, 300, 400, 500, 550, 600, 650, 700, 750, 800, 850, 900, 950, 1000, 1050 consecutive nucleotides in length, the consecutive nucleotides being of any one of the nucleic acid sequences given in Table A4 of the Examples section, or of a nucleic acid encoding an orthologue or paralogue of any one of the amino acid sequences given in Table A4 of the Examples section. Most preferably the portion is a portion of the nucleic acid of SEQ ID NO: 536. Preferably, the portion encodes a fragment of an amino acid sequence which, when used in the construction of a phylogenetic tree such as the one depicted in Figure 16, clusters within Clade A, preferably with the polypeptide represented by SEQ ID NO: 537.

Concerning EXO-1 polypeptides, portions useful in the methods of the invention, encode an EXO-1 polypeptide as defined herein, and have substantially the same biological activity as the amino acid sequences given in Table A5 of the Examples section. Preferably, the portion is a portion of any one of the nucleic acids given in Table A5 of the Examples section, or is a portion of a nucleic acid encoding an orthologue or paralogue of any one of the amino acid sequences given in Table A5 of the Examples section. Preferably the portion is at least 500, 550, 600, 650, 700, 750, 800, 850, 900, 950, 1000, 1250, 1500, 1750, 2000, 2250, 2500, 2750, 3000, 3250, 3500, 3750, 4000, 4250, 4500, 4750, 5000 consecutive nucleotides in length, the consecutive nucleotides being of any one of the nucleic acid sequences given in Table A5 of the Examples section, or of a nucleic acid encoding an orthologue or paralogue of any one of the amino acid sequences given in Table A5 of the Examples section. Most preferably the portion is a portion of the nucleic acid of SEQ ID NO: 762. Preferably, the portion encodes a fragment of an amino acid sequence which, when used in the construction of a phylogenetic tree, such as the one depicted in Figure 19, clusters with the group of EXO-1 polypeptides comprising the amino acid sequence represented by SEQ ID NO: 763 rather than with any other group and/or comprises motifs 40 to 42 and/or has nuclease activity and/or has at least 20% sequence identity to SEQ ID NO: 763.

Concerning YiAP2 polypeptides, portions useful in the methods of the invention, encode a YiAP2 polypeptide as defined herein, and have substantially the same biological activity as the amino acid sequences given in Table A6 of the Examples section. Preferably, the portion is a portion of any one of the nucleic acids given in Table A6 of the Examples section, or is a portion of a nucleic acid encoding an orthologue or paralogue of any one of the amino acid sequences given in Table A of the Examples section. Preferably the portion is at least 50,100, 200, 300, 400, 500, 550, 600, 650, 700, 750, 800, 850, 900, 950, 1000, 1050, 1100, 1150, 1200 consecutive nucleotides in length, the consecutive nucleotides being of any one of the nucleic acid sequences given in Table A6 of the Examples section, or of a nucleic acid encoding an orthologue or paralogue of any one of the amino acid sequences given in Table A6 of the Examples section. Most preferably the portion is a portion of the nucleic acid of SEQ ID NO: 835. Preferably, the portion encodes a fragment of an amino acid sequence comprising at least Two AP2 domains and at least one of motifs I to VI as described above.

Another nucleic acid variant useful in the methods of the invention is a nucleic acid capable of hybridising, under reduced stringency conditions, preferably under stringent conditions, with a nucleic acid encoding a FSM1-like polypeptide, or a PIF3 polypeptide, or a UROD polypeptide, or an AS-MTT polypeptide, or an EXO-1 polypeptide, or a YiAP2 polypeptide, as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table A1 to A6 of the Examples section, or comprising introducing and expressing in a plant a nucleic acid capable of hybridising to a nucleic acid encoding an orthologue, paralogue or homologue of any of the nucleic acid sequences given in Table A1 to A6 of the Examples section.

Concerning FSM1-like polypeptides, hybridising sequences useful in the methods of the invention encode a FSM1-like polypeptide as defined herein, having substantially the same biological activity as the amino acid sequences given in Table A1 of the Examples section. Preferably, the hybridising sequence is capable of hybridising to the complement of any one of the nucleic acids given in Table A1 of the Examples section, or to a portion of any of these sequences, a portion being as defined above, or the hybridising sequence is capable of hybridising to the complement of a nucleic acid encoding an orthologue or paralogue of any one of the amino acid sequences given in Table A1 of the Examples section. Most preferably, the hybridising sequence is capable of hybridising to the complement of a nucleic acid as represented by SEQ ID NO: 1 or to a portion thereof.

Preferably, the hybridising sequence encodes a polypeptide comprising a MYB/SANT domain and with an amino acid sequence which, when used in the construction of a phylogenetic tree such as the one depicted in Figure 3, clusters with the group of FSM1-like polypeptides comprising the amino acid sequence represented by SEQ ID NO: 2 rather than with other MYB proteins and which comprises one or more of the motifs 1 to 6 as outlined above.

Concerning PIF3-like polypeptides, hybridising sequences useful in the methods of the invention encode a PIF3-like polypeptide as defined herein, having substantially the same biological activity as the amino acid sequences given in Table A2 of the Examples section. Preferably, the hybridising sequence is capable of hybridising to the complement of any one of the nucleic acids given in Table A2 of the Examples section, or to a portion of any of these sequences, a portion being as defined above, or the hybridising sequence is capable of hybridising to the complement of a nucleic acid encoding an orthologue or paralogue of any one of the amino acid sequences given in Table A2 of the Examples section. Most preferably, the hybridising sequence is capable of hybridising to the complement of a nucleic acid as represented by SEQ ID NO: 292 or to a portion thereof.

Preferably, the hybridising sequence encodes a polypeptide with an amino acid sequence which comprises one or more of the motifs as defined above and which, when used in the construction of a phylogenetic tree, such as the one depicted in Figure 8, clusters with the group of PIF3-like polypeptides comprising the amino acid sequence represented by SEQ ID NO: 293 rather than with any other group of bHLH proteins.

Concerning UROD polypeptides, hybridising sequences useful in the methods of the invention encode a UROD polypeptide as defined herein, having substantially the same activity as the amino acid sequences given in Table A3 of the Examples section. Preferably, the hybridising sequence is capable of hybridising to the complement of any one of the nucleic acids given in Table A3 of the Examples section, or to a portion of any of these sequences, a portion being as defined above, or the hybridising sequence is capable of hybridising to the complement of a nucleic acid encoding an orthologue or paralogue of any one of the amino acid sequences given in Table A3 of the Examples section. Most preferably, the hybridising sequence is capable of hybridising to the complement of a nucleic acid as represented by SEQ ID NO: 367 or to a portion thereof.

Preferably, the hybridising sequence encodes a polypeptide with an amino acid sequence which, when full-length and used in the construction of a phylogenetic tree, such as the one depicted in Figure 12 or 13, clusters with the group of UROD polypeptides comprising the amino acid sequence represented by SEQ ID NO: 368 rather than with any other group and comprises any one or more of Motifs 13 to 30.

Concerning AS-MTT polypeptides, hybridising sequences useful in the methods of the invention encode an AS-MTT polypeptide as defined herein, having substantially the same biological activity as the amino acid sequences given in Table A4 of the Examples section. Preferably, the hybridising sequence is capable of hybridising to the complement of any one of the nucleic acids given in Table A4 of the Examples section, or to a portion of any of these sequences, a portion being as defined above, or the hybridising sequence is capable of hybridising to the complement of a nucleic acid encoding an orthologue or paralogue of any one of the amino acid sequences given in Table A4 of the Examples section. Most preferably, the hybridising sequence is capable of hybridising to the complement of a nucleic acid as represented by SEQ ID NO: 536 or to a portion thereof.

Preferably, the hybridising sequence encodes a polypeptide with an amino acid sequence which, when full-length and used in the construction of a phylogenetic tree, such as the one depicted in Figure 16, clusters within Clade A, preferably with the polypeptide represented by SEQ ID NO: 537.

Concerning EXO-1 polypeptides, hybridising sequences useful in the methods of the invention encode an EXO-1 polypeptide as defined herein, having substantially the same biological activity as the amino acid sequences given in Table A5 of the Examples section. Preferably, the hybridising sequence is capable of hybridising to the complement of any one of the nucleic acids given in Table A5 of the Examples section, or to a portion of any of these sequences, a portion being as defined above, or the hybridising sequence is capable of hybridising to the complement of a nucleic acid encoding an orthologue or paralogue of any one of the amino acid sequences given in Table A5 of the Examples section. Most preferably, the hybridising sequence is capable of hybridising to the complement of a nucleic acid as represented by SEQ ID NO: 762 or to a portion thereof.

Preferably, the hybridising sequence encodes a polypeptide with an amino acid sequence which, when full-length and used in the construction of a phylogenetic tree, such as the one depicted in Figure 19, clusters with the group of EXO-1 polypeptides comprising the amino acid sequence represented by SEQ ID NO: 763 rather than with any other group group an/or comprises motifs 40 to 42 and/or has nuclease activity and/or has at least 20% sequence identity to SEQ ID NO: 762.

Hybridising sequences useful in the methods of the invention encode a YiAP2 polypeptide as defined herein, having substantially the same biological activity as the amino acid sequences given in Table A6 of the Examples section. Preferably, the hybridising sequence is capable of hybridising to the complement of any one of the nucleic acids given in Table A6 of the Examples section, or to a portion of any of these sequences, a portion being as defined above, or the hybridising sequence is capable of hybridising to the complement of a nucleic acid encoding an orthologue or paralogue of any one of the amino acid sequences given in Table A6 of the Examples section. Most preferably, the hybridising sequence is capable of hybridising to the complement of a nucleic acid as represented by SEQ ID NO: 835 or to a portion thereof.

Preferably, the hybridising sequence encodes a polypeptide with an amino acid sequence comprising at least two AP2 domains and at least one of motifs 43 to 48 as described above.

Another nucleic acid variant useful in the methods of the invention is a splice variant encoding a FSM1-like polypeptide, or a PIF3 polypeptide, or a UROD polypeptide, or an AS-MTT polypeptide, or an EXO-1 polypeptide, or a YiAP2 polypeptide, as defined hereinabove, a splice variant being as defined herein.

According to the present invention, there is provided a method for enhancing yield-related traits in plants, comprising introducing and expressing in a plant a splice variant of any one of the nucleic acid sequences given in Table A1 to A6 of the Examples section, or a splice variant of a nucleic acid encoding an orthologue, paralogue or homologue of any of the amino acid sequences given in Table A1 to A6 of the Examples section.

Concerning FSM1-like polypeptides, preferred splice variants are splice variants of a nucleic acid represented by SEQ ID NO: 1, or a splice variant of a nucleic acid encoding an orthologue or paralogue of SEQ ID NO: 2. Preferably, the amino acid sequence encoded by the splice variant comprises a MYB/SANT domain and has a sequence which, when used in the construction of a phylogenetic tree such as the one depicted in Figure 3, clusters with the group of FSM1-like polypeptides comprising the amino acid sequence represented by SEQ ID NO: 2 rather than with other MYB proteins, and which comprises one or more of the motifs 1 to 6 as outlined above.

Concerning PIF3-like polypeptides, preferred splice variants are splice variants of a nucleic acid represented by SEQ ID NO: 292, or a splice variant of a nucleic acid encoding an orthologue or paralogue of SEQ ID NO: 293. Preferably, the amino acid sequence encoded by the splice variant comprises one or more of the motifs as defined above and, when used in the construction of a phylogenetic tree such as the one depicted in Figure 8, clusters with the group of PIF3-like polypeptides comprising the amino acid sequence represented by SEQ ID NO: 293 rather than with any other group of bHLH proteins..

Concerning UROD polypeptides, preferred splice variants are splice variants of a nucleic acid represented by SEQ ID NO: 367, or a splice variant of a nucleic acid encoding an orthologue or paralogue of SEQ ID NO: 368. Preferably, the amino acid sequence encoded by the splice variant, when used in the construction of a phylogenetic tree, such as the one depicted in Figure 12, clusters with the group of UROD polypeptides comprising the amino acid sequence represented by SEQ ID NO: 368 rather than with any other group and comprises any one or more of Motifs 13 to 30.

Concerning AS-MTT polypeptides, preferred splice variants are splice variants of a nucleic acid represented by SEQ ID NO: 536, or a splice variant of a nucleic acid encoding an orthologue or paralogue of SEQ ID NO: 537. Preferably, the amino acid sequence encoded by the splice variant, when used in the construction of a phylogenetic tree such as the one depicted in Figure 16, clusters within Clade A, preferably with the polypeptide represented by SEQ ID NO: 537.

Concerning EXO-1 polypeptides, preferred splice variants are splice variants of a nucleic acid represented by SEQ ID NO: 762, or a splice variant of a nucleic acid encoding an orthologue or paralogue of SEQ ID NO: 763. Preferably, the amino acid sequence encoded by the splice variant, when used in the construction of a phylogenetic tree, such as the one depicted in Figure 19, clusters with the group of EXO-1 polypeptides nucleases comprising the amino acid sequence represented by SEQ ID NO: 763 rather than with any other group and/or comprises motifs 40 to 42 and/or has nuclease activity and/or has at least 20% sequence identity to SEQ ID NO: 763.

Concerning YiAP2 polypeptides, preferred splice variants are splice variants of a nucleic acid represented by SEQ ID NO: 835, or a splice variant of a nucleic acid encoding an orthologue or paralogue of SEQ ID NO: 836. Preferably, the amino acid sequence encoded by the splice variant comprises at least two AP2 domains and at least one of motifs 43 to 48 as described above.

Another nucleic acid variant useful in performing the methods of the invention is an allelic variant of a nucleic acid encoding a FSM1-like polypeptide, or a PIF3 polypeptide, or a UROD polypeptide, or an AS-MTT polypeptide, or an EXO-1 polypeptide, or a YiAP2 polypeptide, as defined hereinabove, an allelic variant being as defined herein.

According to the present invention, there is provided a method for enhancing yield-related traits in plants, comprising introducing and expressing in a plant an allelic variant of any one of the nucleic acids given in Table A1 to A6 of the Examples section, or comprising introducing and expressing in a plant an allelic variant of a nucleic acid encoding an orthologue, paralogue or homologue of any of the amino acid sequences given in Table A1 to A6 of the Examples section.

Concerning FSM1-like polypeptides, the polypeptides encoded by allelic variants useful in the methods of the present invention have substantially the same biological activity as the FSM1-like polypeptide of SEQ ID NO: 2 and any of the amino acids depicted in Table A1 of the Examples section. Allelic variants exist in nature, and encompassed within the methods of the present invention is the use of these natural alleles. Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant comprises a MYB/SANT domain and has a sequence which, when used in the construction of a phylogenetic tree such as the one depicted in Figure 3, clusters with the group of FSM1-like polypeptides comprising the amino acid sequence represented by SEQ ID NO: 2 rather than with other MYB proteins and which comprises one or more of the motifs 1 to 6 as outlined above.

Concerning PIF3-like polypeptides, the polypeptides encoded by allelic variants useful in the methods of the present invention have substantially the same biological activity as the PIF3-like polypeptide of SEQ ID NO: 293 and any of the amino acids depicted in Table A2 of the Examples section. Allelic variants exist in nature, and encompassed within the methods of the present invention is the use of these natural alleles. Preferably, the allelic variant is an allelic variant of SEQ ID NO: 292 or an allelic variant of a nucleic acid encoding an orthologue or paralogue of SEQ ID NO: 293. Preferably, the amino acid sequence encoded by the allelic variant comprises one or more of the motifs as defined above and, when used in the construction of a phylogenetic tree such as the one depicted in Figure 8, clusters with the group of PIF3-like polypeptides comprising the amino acid sequence represented by SEQ ID NO: 293 rather than with any other group of bHLH proteins.

Concerning UROD polypeptides, the polypeptides encoded by allelic variants useful in the methods of the present invention have substantially the same biological activity as the UROD polypeptide of SEQ ID NO: 368 and any of the amino acids depicted in Table A3 of the Examples section. Allelic variants exist in nature, and encompassed within the methods of the present invention is the use of these natural alleles. Preferably, the allelic variant is an allelic variant of SEQ ID NO: 367 or an allelic variant of a nucleic acid encoding an orthologue or paralogue of SEQ ID NO: 368. Preferably, the amino acid sequence encoded by the allelic variant, when used in the construction of a phylogenetic tree, such as the one depicted in Figure 12 or 13, clusters with the UROD polypeptides comprising the amino acid sequence represented by SEQ ID NO: 368 rather than with any other group and comprises any one or more of Motifs 13 to 30.

Concerning AS-MTT polypeptides, the polypeptides encoded by allelic variants useful in the methods of the present invention have substantially the same biological activity as the AS-MTT polypeptide of SEQ ID NO: 537 and any of the amino acids depicted in Table A4 of the Examples section. Allelic variants exist in nature, and encompassed within the methods of the present invention is the use of these natural alleles. Preferably, the allelic variant is an allelic variant of SEQ ID NO: 536 or an allelic variant of a nucleic acid encoding an orthologue or paralogue of SEQ ID NO: 537. Preferably, the amino acid sequence encoded by the allelic variant, when used in the construction of a phylogenetic tree, such as the one depicted in Figure 16, clusters within Clade A, preferably with the polypeptide represented by SEQ ID NO: 537.

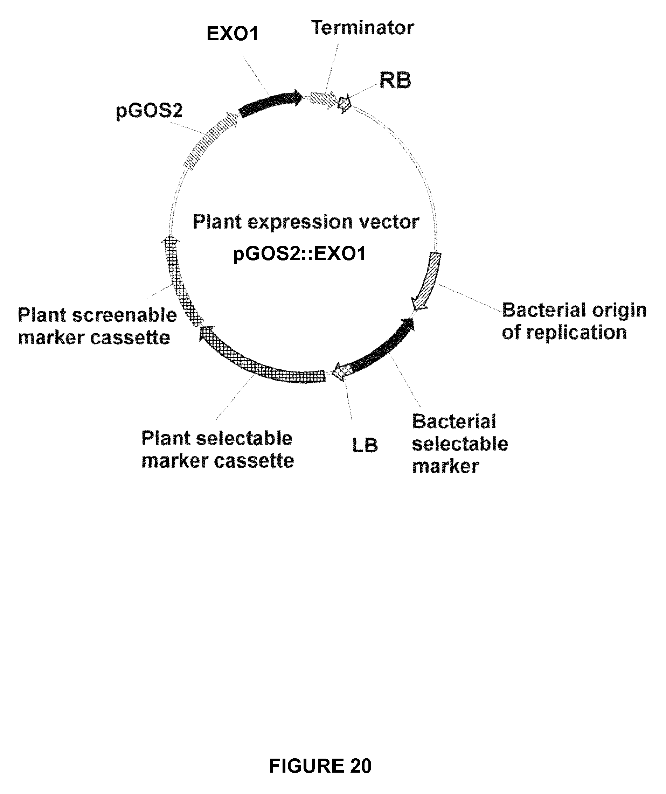

Concerning EXO-1 polypeptides, the polypeptides encoded by allelic variants useful in the methods of the present invention have substantially the same biological activity as the EXO-1 polypeptide of SEQ ID NO: 763 and any of the amino acids depicted in Table A5 of the Examples section. Allelic variants exist in nature, and encompassed within the methods of the present invention is the use of these natural alleles. Preferably, the allelic variant is an allelic variant of SEQ ID NO: 762 or an allelic variant of a nucleic acid encoding an orthologue or paralogue of SEQ ID NO: 763. Preferably, the amino acid sequence encoded by the allelic variant, when used in the construction of a phylogenetic tree, such as the one depicted in Figure 20, clusters with the EXO-1 polypeptides nucleases comprising the amino acid sequence represented by SEQ ID NO: 763 rather than with any other group and/or comprises motifs 40 to 42 and/or has nuclease activity and/or has at least 20% sequence identity to SEQ ID NO: 763.

Concerning YiAP2 polypeptides, the polypeptides encoded by allelic variants useful in the methods of the present invention have substantially the same biological activity as the YiAP2 polypeptide of SEQ ID NO: 836 and any of the amino acids depicted in Table A6 of the Examples section. Allelic variants exist in nature, and encompassed within the methods of the present invention is the use of these natural alleles. Preferably, the allelic variant is an allelic variant of SEQ ID NO: 835 or an allelic variant of a nucleic acid encoding an orthologue or paralogue of SEQ ID NO: 836. Preferably, the amino acid sequence encoded by the allelic variant comprises at least two AP2 domains and at least one of motifs 43 to 48 as described above.

Gene shuffling or directed evolution may also be used to generate variants of nucleic acids encoding FSM1-like polypeptides, or PIF3-like polypeptides, or UROD polypeptides, or AS-MTT polypeptides, or EXO-1 polypeptides, or YiAP2 polypeptides, as defined above; the term "gene shuffling" being as defined herein.

According to the present invention, there is provided a method for enhancing yield-related traits in plants, comprising introducing and expressing in a plant a variant of any one of the nucleic acid sequences given in Table A1 to A6 of the Examples section, or comprising introducing and expressing in a plant a variant of a nucleic acid encoding an orthologue, paralogue or homologue of any of the amino acid sequences given in Table A1 to A6 of the Examples section, which variant nucleic acid is obtained by gene shuffling.

Concerning FSM1-like polypeptides, preferably, the amino acid sequence encoded by the variant nucleic acid obtained by gene shuffling comprises a MYB/SANT domain and has a sequence which, when used in the construction of a phylogenetic tree such as the one depicted in Figure 3, clusters with the group of FSM1-like polypeptides comprising the amino acid sequence represented by SEQ ID NO: 2 rather than with other MYB proteins, and which comprises one or more of the motifs 1 to 6 as outlined above.

Concerning PIF3-like polypeptides, preferably, the amino acid sequence encoded by the variant nucleic acid obtained by gene shuffling comprises one or more of the motifs as defined above and, when used in the construction of a phylogenetic tree, such as the one depicted in Figure 8, clusters with the group of PIF3-like polypeptides comprising the amino acid sequence represented by SEQ ID NO: 293 rather than with any other group of bHLH proteins.

Concerning UROD polypeptides, preferably, the amino acid sequence encoded by the variant nucleic acid obtained by gene shuffling, when used in the construction of a phylogenetic tree such as the one depicted in Figure 12 or 13, clusters with the group of UROD polypeptides comprising the amino acid sequence represented by SEQ ID NO: 368 rather than with any other group and comprises any one or more of Motifs 13 to 30. Concerning AS-MTT polypeptides, preferably, the amino acid sequence encoded by the variant nucleic acid obtained by gene shuffling, when used in the construction of a phylogenetic tree such as the one depicted in Figure 16, clusters within Clade A, preferably with the polypeptide represented by SEQ ID NO: 537.

Concerning EXO-1 polypeptides, preferably, the amino acid sequence encoded by the variant nucleic acid obtained by gene shuffling, when used in the construction of a phylogenetic tree such as the one depicted in Figure 19, clusters with the group of EXO-1 polypeptides nucleases comprising the amino acid sequence represented by SEQ ID NO: 763 rather than with any other group and/or comprises motifs 40 to 42 and/or has nuclease activity and/or has at least 20% sequence identity to SEQ ID NO: 763.

Concerning YiAP2 polypeptides, preferably, the amino acid sequence encoded by the variant nucleic acid obtained by gene shuffling comprises at least two AP2 domains and at least one of motifs 43 to 48 as described above.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding FSM1-like polypeptides may be derived from any natural or artificial source. The nucleic acid may be modified from its native form in composition and/or genomic environment through deliberate human manipulation. Preferably the FSM1-like polypeptide-encoding nucleic acid is from a plant, further preferably from a dicotyledonous plant, more preferably from the family Solanaceae, most preferably the nucleic acid is from *Solanum lycopersicum.*

Nucleic acids encoding PIF3-like polypeptides may be derived from any natural or artificial source. The nucleic acid may be modified from its native form in composition and/or genomic environment through deliberate human manipulation. Preferably the PIF3-like polypeptide-encoding nucleic acid is from a plant, further preferably from a monocotyledonous plant, more preferably from the family Poaceae, most preferably the nucleic acid is from *Oryza sativa.*

Nucleic acids encoding UROD polypeptides may be derived from any natural or artificial source. The nucleic acid may be modified from its native form in composition and/or genomic environment through deliberate human manipulation. Preferably the UROD polypeptide-encoding nucleic acid is from a plant, further preferably from the family Salicaceae, most preferably the nucleic acid is from the genus *Populus,* most preferably from the species *P. trichocarpa.*

Nucleic acids encoding AS-MTT polypeptides may be derived from any natural or artificial source. The nucleic acid may be modified from its native form in composition and/or genomic environment through deliberate human manipulation. Preferably the AS-MTT polypeptide-encoding nucleic acid is from a plant, further preferably from a dicotyledonous plant, more preferably from the genus *Populus,* most preferably the nucleic acid is from *Populus trichocarpa.*

Nucleic acids encoding EXO-1 polypeptides may be derived from any natural or artificial source. The nucleic acid may be modified from its native form in composition and/or genomic environment through deliberate human manipulation. Preferably the EXO-1 polypeptide-encoding nucleic acid is from a plant, further preferably from a dicotyledonous plant, more preferably from the family Salicaceae, most preferably the nucleic acid is from *Populus trichocarpa.*

Nucleic acids encoding YiAP2 polypeptides may be derived from any natural or artificial source. The nucleic acid may be modified from its native form in composition and/or genomic environment through deliberate human manipulation. Preferably the YiAP2 polypeptide-encoding nucleic acid is from a plant, further preferably from a dicotyledonous plant, more preferably from the leguminous family, most preferably the nucleic acid is from *Medicago truncatula.*

Performance of the methods of the invention gives plants having enhanced yield-related traits. In particular performance of the methods of the invention gives plants having increased early vigour and/or increased yield, especially increased seed yield relative to control plants. The terms "yield" and "seed yield" are described in more detail in the "definitions" section herein.

Reference herein to enhanced yield-related traits is taken to mean an increase early vigour and/or in biomass (weight) of one or more parts of a plant, which may include aboveground (harvestable) parts and/or (harvestable) parts below ground. In particular, such harvestable parts are vegetative parts of the plant, but seeds are also included in said harvestable parts. Performance of the methods of the invention results in plants having increased biomass and seed yield relative to the plant biomass and seed yield of control plants.

The present invention provides a method for increasing yield-related traits, especially seed yield of plants and/or biomass (harvestable parts below ground), relative to control plants, which method comprises modulating expression in a plant of a nucleic acid encoding a FSM1-like polypeptide as defined herein.

The present invention also provides a method for increasing yield-related traits, especially early vigour and/or seed yield of plants, relative to control plants, which method comprises modulating expression in a plant of a nucleic acid encoding a PIF3-like polypeptide as defined herein.

The present invention also provides a method for increasing yield, especially seed yield of plants, relative to control plants, which method comprises modulating expression in a plant of a nucleic acid encoding a UROD polypeptide as defined herein.

The present invention also provides a method for increasing (yield-related traits - yield), especially seed yield of plants, relative to control plants, which method comprises modulating expression in a plant of a nucleic acid encoding an AS-MTT polypeptide as defined herein.

The present invention also provides a method for increasing yield-related traits, relative to control plants, which method comprises modulating expression in a plant of a nucleic acid encoding an EXO-1 polypeptide as defined herein.

The present invention also provides a method for enhancing yield-related traits, especially seed yield of plants, relative to control plants, which method comprises modulating expression in a plant of a nucleic acid encoding a YiAP2 polypeptide as defined herein.

Since the transgenic plants according to the present invention have increased yield related traits, it is likely that these plants exhibit an increased growth rate (during at least part of their life cycle), relative to the growth rate of control plants at a corresponding stage in their life cycle.

According to a preferred feature of the present invention, performance of the methods of the invention gives plants having an increased growth rate relative to control plants. Therefore, according to the present invention, there is provided a method for increasing the growth rate of plants, which method comprises modulating expression in a plant of a nucleic acid encoding a FSM1-like polypeptide, or a PIF3 polypeptide, or a UROD polypeptide, or an AS-MTT polypeptide, or an EXO-1 polypeptide, or a YiAP2 polypeptide, as defined herein.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid encoding a FSM1-like polypeptide, or a PIF3 polypeptide, or a UROD polypeptide, or an AS-MTT polypeptide, or an EXO-1 polypeptide, or a YiAP2 polypeptide.

In alternative, performance of the methods of the invention gives plants grown under drought-stress conditions or under mild drought conditions increased yield relative to control plants grown under comparable conditions. Therefore, according to the present invention, there is provided a method for increasing yield in plants grown under drought-stress conditions or under mild drought conditions, which method comprises modulating expression in a plant of a nucleic acid encoding an EXO-1 polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid encoding a FSM1-like polypeptide, or a PIF3 polypeptide, or a UROD polypeptide, or an AS-MTT polypeptide, or an EXO-1 polypeptide, or a YiAP2 polypeptide.

Performance of the methods of the invention gives plants grown under conditions of salt stress, increased yield relative to control plants grown under comparable conditions. Therefore, according to the present invention, there is provided a method for increasing yield in plants grown under conditions of salt stress, which method comprises modulating expression in a plant of a nucleic acid encoding a FSM1-like polypeptide, or a PIF3 polypeptide, or a UROD polypeptide, or an AS-MTT polypeptide, or an EXO-1 polypeptide, or a YiAP2 polypeptide.

The invention also provides genetic constructs and vectors to facilitate introduction and/or expression in plants of nucleic acids encoding FSM1-like polypeptides, or PIF3-like polypeptides, or UROD polypeptides, or AS-MTT polypeptides, or EXO-1 polypeptides, or YiAP2 polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a FSM1-like polypeptide, or a PIF3 polypeptide, or a UROD polypeptide, or an AS-MTT polypeptide, or an EXO-1 polypeptide, or a YiAP2 polypeptide, as defined above;
(b) one or more control sequences capable of driving expression of the nucleic acid sequence of (a); and optionally
(c) a transcription termination sequence.

Preferably, the nucleic acid encoding a FSM1-like polypeptide, or a PIF3 polypeptide, or a UROD polypeptide, or an AS-MTT polypeptide, or an EXO-1 polypeptide, or a YiAP2 polypeptide, is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence, but preferably the promoter is of plant origin. A constitutive promoter is particularly useful in the methods. Preferably the constitutive promoter is a ubiquitous constitutive promoter of medium strength. See the "Definitions" section herein for definitions of the various promoter types.

Concerning FSM1-like polypeptides, it should be clear that the applicability of the present invention is not restricted to the FSM1-like polypeptide-encoding nucleic acid represented by SEQ ID NO: 1, nor is the applicability of the invention restricted to expression of a FSM1-like polypeptide-encoding nucleic acid when driven by a constitutive promoter.

Preferably, the medium strength constitutive promoter is derived from a plant, such as a GOS2 promoter, more preferably it is the GOS2 promoter of rice. Furthermore preferably the constitutive promoter is represented by a nucleic acid sequence substantially similar to SEQ ID NO: 289, most preferably the constitutive promoter is as represented by SEQ ID NO: 289. See the "Definitions" section herein for further examples of constitutive promoters.

Optionally, one or more terminator sequences may be used in the construct introduced into a plant. Preferably, the construct comprises an expression cassette comprising a GOS2 promoter, substantially similar to SEQ ID NO: 289, and the nucleic acid encoding the FSM1-like polypeptide.

Concerning PIF3-like polypeptides, it should be clear that the applicability of the present invention is not restricted to the PIF3-like polypeptide-encoding nucleic acid represented by SEQ ID NO: 292, nor is the applicability of the invention restricted to expression of a PIF3-like polypeptide-encoding nucleic acid when driven by a constitutive promoter.

The constitutive promoter is preferably a medium strength promoter, more preferably selected from a plant, such as a GOS2 promoter, most preferably the promoter is the GOS2 promoter from rice. Further preferably the constitutive promoter is represented by a nucleic acid sequence substantially similar to SEQ ID NO: 366, most preferably the constitutive promoter is as represented by SEQ ID NO: 366. See the "Definitions" section herein for further examples of constitutive promoters.

Optionally, one or more terminator sequences may be used in the construct introduced into a plant. Preferably, the construct comprises an expression cassette comprising a GOS2 promoter, substantially similar to SEQ ID NO: 366, and the nucleic acid encoding the PIF3-like polypeptide.

Concerning UROD polypeptides, it should be clear that the applicability of the present invention is not restricted to the UROD polypeptide-encoding nucleic acid represented by SEQ ID NO: 367, nor is the applicability of the invention restricted to expression of a UROD polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 533, most preferably the constitutive promoter is as represented by SEQ ID NO: 533. See the "Definitions" section herein for further examples of constitutive promoters.

Optionally, one or more terminator sequences may be used in the construct introduced into a plant. Preferably, the construct comprises an expression cassette comprising a GOS2 promoter, substantially similar to SEQ ID NO: 533, and the nucleic acid encoding the UROD polypeptide.

Concerning AS-MTT polypeptides, it should be clear that the applicability of the present invention is not restricted to the AS-MTT polypeptide-encoding nucleic acid represented by SEQ ID NO: 536, nor is the applicability of the invention restricted to expression of an AS-MTT polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 659, most preferably the constitutive promoter is as represented by SEQ ID NO: 659. See the "Definitions" section herein for further examples of constitutive promoters.

Concerning EXO-1 polypeptides, it should be clear that the applicability of the present invention is not restricted to the EXO-1 polypeptide-encoding nucleic acid represented by SEQ ID NO: 762, nor is the applicability of the invention restricted to expression of an EXO-1 polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 831, most preferably the constitutive promoter is as represented by SEQ ID NO: 831. See the "Definitions" section herein for further examples of constitutive promoters.

According to another preferred feature of the invention, the nucleic acid encoding an EXO-1 polypeptide is operably linked to a tissue-specific promoter, more preferably a shoot-specific promoter. The shoot-specific promoter is preferably beta expansin promoter (WO 2004/070039), more preferably the beta expansin EXPB9 promoter from rice. Further preferably the EXPB9 promoter is represented by a nucleic acid sequence substantially similar to SEQ ID NO: 832, most preferably the promoter is as represented by SEQ ID NO: 832. Examples of other shoot-specific promoters which may also be used to perform the methods of the invention are shown in Table 2b in the "Definitions" section above.

Optionally, one or more terminator sequences may be used in the construct introduced into a plant. Preferably, the construct comprises an expression cassette comprising a GOS2 or EXPB9 promoter, substantially similar to SEQ ID NO: 831 or SEQ ID NO: 832, and the nucleic acid encoding the EXO-1 polypeptide.

Concerning YiAP2 polypeptides, it should be clear that the applicability of the present invention is not restricted to the YiAP2 polypeptide-encoding nucleic acid represented by SEQ ID NO: 835, nor is the applicability of the invention restricted to expression of a YiAP2 polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 889, most preferably the constitutive promoter is as represented by SEQ ID NO: 889. See the "Definitions" section herein for further examples of constitutive promoters.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding a FSM1-like polypeptide, or a PIF3 polypeptide, or a UROD polypeptide, or an AS-MTT polypeptide, or an EXO-1 polypeptide, or a YiAP2 polypeptide, is by introducing and expressing in a plant a nucleic acid encoding a FSM1-like polypeptide, or a PIF3 polypeptide, or a UROD polypeptide, or an AS-MTT polypeptide, or an EXO-1 polypeptide, or a YiAP2 polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The invention also provides a method for the production of transgenic plants having enhanced yield-related traits relative to control plants, comprising introduction and expression in a plant of any nucleic acid encoding a FSM1-like polypeptide, or a PIF3 polypeptide, or a UROD polypeptide, or an AS-MTT polypeptide, or an EXO-1 polypeptide, or a YiAP2 polypeptide, as defined hereinabove.

More specifically, the present invention provides a method for the production of transgenic plants having enhanced yield-related traits, particularly increased yield (seed yield and/or biomass of below ground plant parts), which method comprises:

(i) introducing and expressing in a plant or plant cell a nucleic acid encoding a FSM1-like polypeptide, or a PIF3 polypeptide, or a UROD polypeptide, or an AS-MTT polypeptide, or an EXO-1 polypeptide, or a YiAP2 polypeptide,; and
(ii) cultivating the plant cell under conditions promoting plant growth and development.

The nucleic acid of (i) may be any of the nucleic acids capable of encoding a a FSM1-like polypeptide, or a PIF3 polypeptide, or a UROD polypeptide, or an AS-MTT polypeptide, or an EXO-1 polypeptide, or a YiAP2 polypeptide, as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The present invention clearly extends to any plant cell or plant produced by any of the methods described herein, and to all plant parts and propagules thereof. The present invention encompasses plants or parts thereof (including seeds) obtainable by the methods according to the present invention. The plants or parts thereof comprise a nucleic acid transgene encoding a FSM1-like polypeptide, or a PIF3 polypeptide, or a UROD polypeptide, or an AS-MTT polypeptide, or an EXO-1 polypeptide, or a YiAP2 polypeptide, as defined above. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a FSM1-like polypeptide, or a PIF3 polypeptide, or a UROD polypeptide, or an AS-MTT polypeptide, or an EXO-1 polypeptide, or a YiAP2 polypeptide, as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, sugar beet, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid encoding a FSM1-like polypeptide. The invention furthermore relates to products derived, preferably directly derived, from a harvestable part of such a plant, such as dry pellets or powders, oil, fat and fatty acids, starch or proteins.

The present invention also encompasses use of nucleic acids encoding FSM1-like polypeptides as described herein and use of these FSM1-like polypeptides, or PIF3-like polypeptides, or UROD polypeptides, or AS-MTT polypeptides, or EXO-1 polypeptides, or YiAP2 polypeptides, in enhancing any of the aforementioned yield-related traits in plants. For example, nucleic acids encoding a FSM1-like polypeptide, or a PIF3 polypeptide, or a UROD polypeptide, or an AS-MTT polypeptide, or an EXO-1 polypeptide, or a YiAP2 polypeptide, described herein, or the FSM1-like polypeptides, or PIF3-like polypeptides, or UROD polypeptides, or AS-MTT polypeptides, or EXO-1 polypeptides, or YiAP2 polypeptides, themselves, may find use in breeding programmes in which a DNA marker is identified which may be genetically linked to a gene encoding a FSM1-like polypeptide, or a PIF3 polypeptide, or a UROD polypeptide, or an AS-MTT polypeptide, or an EXO-1 polypeptide, or a YiAP2 polypeptide. The nucleic acids/genes, or the FSM1-like polypeptides, or PIF3-like polypeptides, or UROD polypeptides, or AS-MTT polypeptides, or EXO-1 polypeptides, or YiAP2 polypeptides, themselves may be used to define a molecular marker. This DNA or protein marker may then be used in breeding programmes to select plants having enhanced yield-related traits as defined hereinabove in the methods of the invention. Furthermore, allelic variants of a nucleic acid/gene encoding a FSM1-like polypeptide, or a PIF3 polypeptide, or a UROD polypeptide, or an AS-MTT polypeptide, or an EXO-1 polypeptide, or a YiAP2 polypeptide, may find use in marker-assisted breeding programmes. Nucleic acids encoding a FSM1-like polypeptide, or a PIF3 polypeptide, or a UROD polypeptide, or an AS-MTT polypeptide, or an EXO-1 polypeptide, or a YiAP2 polypeptide, may also be used as probes for genetically and physically mapping the genes that they are a part of, and as markers for traits linked to those genes. Such information may be useful in plant breeding in order to develop lines with desired phenotypes.

**Items**

1. FSM1-like (Fruit Sant/Myb) polypeptides

1. A method for enhancing yield-related traits in plants relative to control plants, comprising modulating expression in a plant of a nucleic acid encoding a FSM1-like polypeptide, wherein said FSM1-like polypeptide comprises a SANT domain (SMART accession SM0017).

2. Method according to item 1, wherein said FSM1-like polypeptide comprises one or more of the following motifs:

(i) Motif 1: W[TS][PA]K[QE]NK[LA]FE[RK]ALAVYD[KR][DE]TPDRW[HSQ]N[VI]A [RK]A (SEQ ID NO: 283),
(ii) Motif 2: GGK[ST][AV][ED]EV[KR]RHYE[IL]L (SEQ ID NO: 284),
(iii) Motif 3: D[VL][KF][HF]I[ED][SN]G[RM]VPFP[NK]Y (SEQ ID NO: 285)

3. Method according to item 1 or 2, wherein said modulated expression is effected by introducing and expressing in a plant a nucleic acid encoding a FSM1-like polypeptide.

4. Method according to any one of items 1 to 3, wherein said nucleic acid encoding a FSM1-like polypeptide encodes any one of the proteins listed in Table A1 or is a portion of such a nucleic acid, or a nucleic acid capable of hybridising with such a nucleic acid.

5. Method according to any one of items 1 to 4, wherein said nucleic acid sequence encodes an orthologue or paralogue of any of the proteins given in Table A1.

6. Method according to any preceding item, wherein said enhanced yield-related traits comprise increased yield, preferably increased biomass and/or increased seed yield relative to control plants.

7. Method according to any one of items 1 to 6, wherein said enhanced yield-related traits are obtained under non-stress conditions.

8. Method according to any one of items 3 to 7, wherein said nucleic acid is operably linked to a constitutive promoter, preferably to a GOS2 promoter, most preferably to a GOS2 promoter from rice.

9. Method according to any one of items 1 to 8, wherein said nucleic acid encoding a FSM1-like polypeptide is of plant origin, preferably from a dicotyledonous plant, further preferably from the family Solanaceae, more preferably from the genus Lycopersicon, most preferably from *Solanum lycopersicum.*

10. Plant or part thereof, including seeds, obtainable by a method according to any one of items 1 to 9, wherein said plant or part thereof comprises a recombinant nucleic acid encoding a FSM1-like polypeptide.

11. Construct comprising:

(i) nucleic acid encoding a FSM1-like polypeptide as defined in items 1 or 2;
(ii) one or more control sequences capable of driving expression of the nucleic acid sequence of (a); and optionally
(iii) a transcription termination sequence.

12. Construct according to item 11, wherein one of said control sequences is a constitutive promoter, preferably a GOS2 promoter, most preferably a GOS2 promoter from rice.

13. Use of a construct according to item 11 or 12 in a method for making plants having increased yield, particularly increased biomass and/or increased seed yield relative to control plants.

14. Plant, plant part or plant cell transformed with a construct according to item 11 or 12.

15. Method for the production of a transgenic plant having increased yield, particularly increased biomass and/or increased seed yield relative to control plants, comprising:

(i) introducing and expressing in a plant a nucleic acid encoding a FSM1-like polypeptide as defined in item 1 or 2; and
(ii) cultivating the plant cell under conditions promoting plant growth and development.

16. Transgenic plant having increased yield, particularly increased biomass and/or increased seed yield, relative to control plants, resulting from modulated expression of a nucleic acid encoding a FSM1-like polypeptide as defined in item 1 or 2, or a transgenic plant cell derived from said transgenic plant.

17. Transgenic plant according to item 10, 14 or 16, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

18. Harvestable parts of a plant according to item 17, wherein said harvestable parts are preferably shoot biomass and/or seeds.

19. Products derived from a plant according to item 17 and/or from harvestable parts of a plant according to item 18.

20. Use of a nucleic acid encoding a FSM1-like polypeptide in increasing yield, particularly in increasing seed yield and/or shoot biomass in plants, relative to control plants.

## 2. PIF3-like (Phytochrome Interacting Factor 3) polypeptides

1. A method for enhancing yield-related traits in plants relative to control plants, comprising modulating expression in a plant of a nucleic acid encoding a PIF3-like polypeptide, wherein said PIF3-like polypeptide comprises a Helix-Loop-Helix domain (PF00010).

2. Method according to item 1, wherein said PIF3-like polypeptide comprises one or more of motifs 7 to 12 (SEQ ID NO: 358 to SEQ ID NO: 363).

3. Method according to item 1 or 2, wherein said modulated expression is effected by introducing and expressing in a plant a nucleic acid encoding a PIF3-like polypeptide.

4. Method according to any one of items 1 to 3, wherein said nucleic acid encoding a PIF3-like polypeptide encodes any one of the proteins listed in Table A2 or is a portion of such a nucleic acid, or a nucleic acid capable of hybridising with such a nucleic acid.

5. Method according to any one of items 1 to 4, wherein said nucleic acid sequence encodes an orthologue or paralogue of any of the proteins given in Table A2.

6. Method according to any preceding item, wherein said enhanced yield-related traits comprise increased early vigour and/or increased seed yield relative to control plants.

7. Method according to any one of items 1 to 6, wherein said enhanced yield-related traits are obtained under conditions of drought stress.

8. Method according to any one of items 3 to 7, wherein said nucleic acid is operably linked to a constitutive promoter, preferably to a GOS2 promoter, most preferably to a GOS2 promoter from rice.

9. Method according to any one of items 1 to 8, wherein said nucleic acid encoding a PIF3-like polypeptide is of plant origin, preferably from a dicotyledonous plant, further preferably from the family Poaceae, more preferably from the genus Oryza, most preferably from Oryza sativa.

10. Plant or part thereof, including seeds, obtainable by a method according to any one of items 1 to 10, wherein said plant or part thereof comprises a recombinant nucleic acid encoding a PIF3-like polypeptide.

11. Construct comprising:

(i) nucleic acid encoding a PIF3-like polypeptide as defined in items 1 or 2;
(ii) one or more control sequences capable of driving expression of the nucleic acid sequence of (a); and optionally
(iii) a transcription termination sequence.

12. Construct according to item 12, wherein one of said control sequences is a constitutive promoter, preferably a GOS2 promoter, most preferably a GOS2 promoter from rice.

13. Use of a construct according to item 12 or 13 in a method for making plants having increased yield, particularly increased biomass and/or increased seed yield relative to control plants.

14. Plant, plant part or plant cell transformed with a construct according to item 12 or 13.

15. Method for the production of a transgenic plant having increased yield, particularly increased biomass and/or increased seed yield relative to control plants, comprising:

(i) introducing and expressing in a plant a nucleic acid encoding a PIF3-like polypeptide as defined in item 1 or 2; and
(ii) cultivating the plant cell under conditions promoting plant growth and development.

16. Transgenic plant having increased yield, particularly increased biomass and/or increased seed yield, relative to control plants, resulting from modulated expression of a nucleic acid encoding a PIF3-like polypeptide as defined in item 1 or 2, or a transgenic plant cell derived from said transgenic plant.

17. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

18. Harvestable parts of a plant according to item 18, wherein said harvestable parts are preferably shoot biomass and/or seeds.

19. Products derived from a plant according to item 18 and/or from harvestable parts of a plant according to item 19.

20. Use of a nucleic acid encoding a PIF3-like polypeptide in increasing yield, particularly in increasing seed yield and/or shoot biomass in plants, relative to control plants.

21. An isolated nucleic acid molecule comprising any one of the following features selected from:

(i) a nucleic acid represented by SEQ ID NO: 356;
(ii) the complement of a nucleic acid represented by SEQ ID NO: 356;
(iii) a nucleic acid encoding a PIF3-like polypeptide having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 357, and additionally or alternatively comprising one or more motifs having in increasing order of preference less than 5, less than 4, less than 3, less than 2, or less than 1 substitutions (sequence mismatches) compared to any one or more of the motifs given in SEQ ID NO: 358 to SEQ ID NO:

363, and further preferably conferring enhanced yield-related traits relative to control plants.

(iv) a nucleic acid molecule which hybridizes with a nucleic acid molecule of (i) to (iii) under high stringency hybridization conditions and preferably confers enhanced yield-related traits relative to control plants.

22. An isolated polypeptide selected from:

(i) an amino acid sequence represented by SEQ ID NO: 357;
(ii) an amino acid sequence having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 357, and additionally or alternatively comprising one or more motifs having in increasing order of preference less than 5, less than 4, less than 3, less than 2, or less than 1 substitutions (sequence mismatches) compared to any one or more of the motifs given in SEQ ID NO: 358 to SEQ ID NO: 363, and further preferably conferring enhanced yield-related traits relative to control plants;
(iii) derivatives of any of the amino acid sequences given in (i) or (ii) above.

3. UROD (Uroporphyrinogen III decarboxylase) polypeptide

1. A method for enhancing yield-related traits in plants relative to control plants, comprising modulating expression in a plant of a nucleic acid encoding a polypeptide having uroporphyrinogen III decarboxylase activity (UROD).

2. Method according to item 1, wherein said UROD polypeptide comprises any one or more of Motifs 13 to 30.

3. Method according to item 1 or 2, wherein said modulated expression is effected by introducing and expressing in a plant a nucleic acid encoding a UROD polypeptide.

4. Method according to any one of items 1 to 3, wherein said nucleic acid encoding a UROD polypeptide encodes any one of the proteins listed in Table A3 or is a portion of such a nucleic acid, or a nucleic acid capable of hybridising with such a nucleic acid.

5. Method according to any one of items 1 to 4, wherein said nucleic acid sequence encodes an orthologue or paralogue of any of the proteins given in Table A3.

6. Method according to any preceding item, wherein said enhanced yield-related traits comprise increased seed yield relative to control plants.

7. Method according to any one of items 1 to 6, wherein said enhanced yield-related traits are obtained under non-stress conditions.

8. Method according to any one of items 1 to 6, wherein said enhanced yield-related traits are obtained under conditions of drought stress.

9. Method according to any one of items 3 to 8, wherein said nucleic acid is operably linked to a constitutive promoter, preferably to a GOS2 promoter, most preferably to a GOS2 promoter from rice.

10. Method according to any one of items 1 to 9, wherein said nucleic acid encoding a UROD polypeptide is of plant origin, family Salicaceae, more preferably from the genus *Populus,* most preferably from *Populus trichocarpa.*

11. Plant or part thereof, including seeds, obtainable by a method according to any one of items 1 to 10, wherein said plant or part thereof comprises a recombinant nucleic acid encoding a UROD polypeptide.

12. Construct comprising:

(i) nucleic acid encoding a UROD polypeptide as defined in items 1 or 2;
(ii) one or more control sequences capable of driving expression of the nucleic acid sequence of (a); and optionally

(iii) a transcription termination sequence.

13. Construct according to item 12, wherein one of said control sequences is a constitutive promoter, preferably a GOS2 promoter, most preferably a GOS2 promoter from rice.

14. Use of a construct according to item 12 or 13 in a method for making plants having increased yield, particularly increased seed yield relative to control plants.

15. Plant, plant part or plant cell transformed with a construct according to item 12 or 13.

16. Method for the production of a transgenic plant having increased yield, particularly increased biomass and/or increased seed yield relative to control plants, comprising:

(i) introducing and expressing in a plant a nucleic acid encoding a UROD polypeptide as defined in item 1 or 2; and
(ii) cultivating the plant cell under conditions promoting plant growth and development.

17. Transgenic plant having increased yield, particularly seed yield, relative to control plants, resulting from modulated expression of a nucleic acid encoding a UROD polypeptide as defined in item 1 or 2, or a transgenic plant cell derived from said transgenic plant.

18. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

19. Harvestable parts of a plant according to item 18, wherein said harvestable parts are preferably shoot biomass and/or seeds.

20. Products derived from a plant according to item 18 and/or from harvestable parts of a plant according to item 19.

21. Use of a nucleic acid encoding a UROD polypeptide in increasing yield, particularly in increasing seed yield and/or shoot biomass in plants, relative to control plants.

4. AS-MTT (Abiotic Stress Membrane Tethered Transcription factor) polypeptides

1. A method for enhancing yield-related traits in plants relative to control plants, comprising modulating expression in a plant of a nucleic acid encoding an AS-MTT polypeptide, wherein said AS-MTT polypeptide comprises a DUF1664 domain.

2. Method according to item 1, wherein said AS-MTT polypeptide comprises one or more of the following motifs:

(i) Motif 31: G[WL][SK][FL]SD[LV]M[YF][VA]T[KR]R[NS][ML][AS][ND]AV[SA][SN][VL][TS]K[QH]L[ED][QN]VS[ESD][AS]LAA[TA]K[RK]HL[TS]QR (SEQ ID NO: 648),
(ii) Motif 32: AA[AT][VL]G[AV][VLM]GY[GC]YMWWK (SEQ ID NO: 649),
(iii) Motif 33: GAG[LY]TG[ST][IV][LV][LA][KR][NE]G[KR]L (SEQ ID NO: 650)

3. Method according to item 1 or 2, wherein said modulated expression is effected by introducing and expressing in a plant a nucleic acid encoding an AS-MTT polypeptide.

4. Method according to any one of items 1 to 3, wherein said nucleic acid encoding an AS-MTT polypeptide encodes any one of the proteins listed in Table A4 or is a portion of such a nucleic acid, or a nucleic acid capable of hybridising with such a nucleic acid.

5. Method according to any one of items 1 to 4, wherein said nucleic acid sequence encodes an orthologue or paralogue of any of the proteins given in Table A4.

6. Method according to any preceding item, wherein said enhanced yield-related traits comprise increased increased seed yield relative to control plants.

7. Method according to any one of items 1 to 6, wherein said enhanced yield-related traits are obtained under non-stress conditions.

8. Method according to any one of items 1 to 6, wherein said enhanced yield-related traits are obtained under conditions of drought stress, salt stress or nitrogen deficiency.

9. Method according to any one of items 3 to 8, wherein said nucleic acid is operably linked to a constitutive promoter, preferably to a GOS2 promoter, most preferably to a GOS2 promoter from rice.

10. Method according to any one of items 1 to 9, wherein said nucleic acid encoding an AS-MTT polypeptide is of plant origin, preferably from a dicotyledonous plant, further preferably from the genus *Populus,* most preferably from *Populus trichocarpa.*

11. Plant or part thereof, including seeds, obtainable by a method according to any one of items 1 to 10, wherein said plant or part thereof comprises a recombinant nucleic acid encoding an AS-MTT polypeptide.

12. Construct comprising:

(i) nucleic acid encoding an AS-MTT polypeptide as defined in items 1 or 2;
(ii) one or more control sequences capable of driving expression of the nucleic acid sequence of (a); and optionally
(iii) a transcription termination sequence.

13. Construct according to item 12, wherein one of said control sequences is a constitutive promoter, preferably a GOS2 promoter, most preferably a GOS2 promoter from rice.

14. Use of a construct according to item 12 or 13 in a method for making plants having increased yield, particularly increased biomass and/or increased seed yield relative to control plants.

15. Plant, plant part or plant cell transformed with a construct according to item 12 or 13.

16. Method for the production of a transgenic plant having increased yield, particularly increased biomass and/or increased seed yield relative to control plants, comprising:

(i) introducing and expressing in a plant a nucleic acid encoding an AS-MTT polypeptide as defined in item 1 or 2; and
(ii) cultivating the plant cell under conditions promoting plant growth and development.

17. Transgenic plant having increased yield, particularly increased biomass and/or increased seed yield, relative to control plants, resulting from modulated expression of a nucleic acid encoding an AS-MTT polypeptide as defined in item 1 or 2, or a transgenic plant cell derived from said transgenic plant.

18. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

19. Harvestable parts of a plant according to item 18, wherein said harvestable parts are preferably shoot biomass and/or seeds.

20. Products derived from a plant according to item 18 and/or from harvestable parts of a plant according to item 19.

21. Use of a nucleic acid encoding an AS-MTT polypeptide in increasing yield, particularly in increasing seed yield and/or shoot biomass in plants, relative to control plants.

5. EXO-1 polypeptide

1. A method for enhancing yield-related traits in plants relative to control plants, comprising modulating expression in a plant of a nucleic acid encoding an EXO-1 polypeptide, wherein said EXO-1 polypeptide comprises at least one of the following domains: XPG_N with accession number PF00752, XPG_1 with accession number PF00867 or EXONUCLEASE 1 domain with accession number PTHR11081:SF8.

2. Method, according to item 1, wherein said EXO-1 polypeptide comprises one or more of the following motifs:

**Motif 40:** G[QKC][RT]VA[VI]D[TA]YSWLH[KR][GA]A[YL]SC[SA]RELC[KEL]GLPT (SEQ ID NO: 664),
**Motif 41:** Y[CF]M[HK]RVN[LM]L[RL]H[YH][GK][VI]KP[IV][LV]VFDGGRLPMK[AS][DE][QTE]ENKR[AR]R[SK]RKENL[EA]RA[KR]E[HL][ELW] (SEQ ID NO: 665),
**Motif 42:** V[DQA]A[VI]ITEDSDL[IL][AP][FY]GC[PK]R[IV][IF]FK[ML]D[KR][FYN]GQG (SEQ ID NO: 666)

3. Method, according to item 1 or 2, wherein said modulated expression is effected by introducing and expressing in a plant a nucleic acid encoding an EXO-1 polypeptide.

4. Method, according to any one of items 1 to 3, wherein said nucleic acid encoding an EXO-1 polypeptide encodes any one of the proteins listed in Table A5 or is a portion of such a nucleic acid, or a nucleic acid capable of hybridising with such a nucleic acid.

5. Method, according to any one of items 1 to 4, wherein said nucleic acid sequence encodes an orthologue or paralogue of any of the proteins given in Table A5.

6. Method, according to any preceding item, wherein said enhanced yield-related traits comprise increased (yield - early vigour), preferably increased biomass and/or increased seed yield relative to control plants.

7. Method, according to any one of items 1 to 6, wherein said enhanced yield-related traits are obtained under non-stress conditions.

8. Method, according to any one of items 1 to 6, wherein said enhanced yield-related traits are obtained under conditions of drought stress, salt stress or nitrogen deficiency.

9. Method, according to any one of items 3 to 8, wherein said nucleic acid is operably linked to a constitutive promoter, preferably to a GOS2 promoter, most preferably to a GOS2 promoter from rice.

10. Method, according to any one of items 3 to 8, wherein said nucleic acid is operably linked to a shoot-specific promoter, preferably to a beta expansin promoter, most preferably to an EXPB9 promoter from rice

11. Method, according to any one of items 1 to 10, wherein said nucleic acid encoding an EXO-1 polypeptide is of plant origin, preferably from a dicotyledonous plant, further preferably from the family *Salicaceae,* more preferably from the genus *Populus,* most preferably from *Populus trichocarpa.*

12. Plant or part thereof, including seeds, obtainable by a method according to any one of items 1 to 11, wherein said plant or part thereof comprises a recombinant nucleic acid encoding an EXO-1 polypeptide.

13. Construct comprising:

(i) nucleic acid encoding an EXO-1 polypeptide as defined in items 1 or 2;
(ii) one or more control sequences capable of driving expression of the nucleic acid sequence of (a); and optionally
(iii) a transcription termination sequence.

14. Construct according to item 13, wherein one of said control sequences is a constitutive promoter, preferably a GOS2 promoter, most preferably a GOS2 promoter from rice.

15. Construct, according to item 13, wherein one of said control sequences is a shoot-specific promoter, preferably a beta expansin promoter, most preferably an EXPB9 promoter from rice

16. Use of a construct, according to any of the items 13 to 15, in a method for making plants having increased yield, particularly increased biomass and/or increased seed yield relative to control plants.

17. Plant, plant part or plant cell transformed with a construct according to any of the items 13 to 15.

18. Method for the production of a transgenic plant having increased yield, particularly increased biomass and/or increased seed yield relative to control plants, comprising:

(i) introducing and expressing in a plant a nucleic acid encoding an EXO-1 polypeptide as defined in item 1 or 2; and
(ii) cultivating the plant cell under conditions promoting plant growth and development.

19. Transgenic plant having increased yield, particularly increased biomass and/or increased seed yield, relative to control plants, resulting from modulated expression of a nucleic acid encoding an EXO-1 polypeptide as defined in item 1 or 2, or a transgenic plant cell derived from said transgenic plant.

20. Transgenic plant, according to item 12, 17 or 19, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

21. Harvestable parts of a plant according to item 20, wherein said harvestable parts are preferably shoot biomass and/or seeds.

22. Products derived from a plant, according to item 20 and/or from harvestable parts of a plant according to item 21.

23. Use of a nucleic acid encoding an EXO-1 polypeptide in increasing yield, particularly in increasing seed yield and/or shoot biomass in plants, relative to control plants.

## 6. YiAP2 polypeptides

1. A method for enhancing yield-related traits in plants relative to control plants, comprising modulating expression in a plant of a nucleic acid encoding a YiAP2 polypeptide or a portion thereof.

2. Method according to item 1, wherein said YiAP2 polypeptide comprises at least two AP2 domains and one or more motifs having at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to any one of the following motifs:

(i) Motif 43 as represented by SEQ ID NO: 890;
(ii) Motif 44 as represented by SEQ ID NO: 891;
(iii) Motif 45 as represented by SEQ ID NO: 892;
(iv) Motif 46 as represented by SEQ ID NO: 893;
(v) Motif 47 as represented by SEQ ID NO: 894;
(vi) Motif 48 as represented by SEQ ID NO: 895.

3. Method according to item 1 or 2, wherein said modulated expression is effected by introducing and expressing in a plant a nucleic acid encoding a YiAP2 polypeptide.

4. Method according to any one of items 1 to 3, wherein said nucleic acid encoding a YiAP2 polypeptide encodes any one of the proteins listed in Table A6 or is a portion of such a nucleic acid, or a nucleic acid capable of hybridising with such a nucleic acid.

5. Method according to any one of items 1 to 4, wherein said nucleic acid sequence encodes an orthologue or paralogue of any of the proteins given in Table A6.

6. Method according to any preceding item, wherein said enhanced yield-related traits comprise increased yield preferably increased seed yield relative to control plants.

7. Method according to any one of items 1 to 6, wherein said enhanced yield-related traits are obtained under non-stress conditions.

8. Method according to any one of items 1 to 6, wherein said enhanced yield-related traits are obtained under conditions of drought stress, salt stress or nitrogen deficiency.

9. Method according to any one of items 3 to 8, wherein said nucleic acid is operably linked to a constitutive promoter, preferably to a GOS2 promoter, most preferably to a GOS2 promoter from rice.

10. Method according to any one of items 1 to 9, wherein said nucleic acid encoding a YiAP2 polypeptide is of plant origin, preferably from a dicotyledonous plant, more preferably from the genus *Medicago,* most preferably from *Medicago truncatula.*

11. Plant or part thereof, including seeds, obtainable by a method according to any one of items 1 to 10, wherein said plant or part thereof comprises a recombinant nucleic acid encoding a YiAP2 polypeptide.

12. Construct comprising:

(i) nucleic acid encoding a YiAP2 polypeptide as defined in items 1 or 2;
(ii) one or more control sequences capable of driving expression of the nucleic acid sequence of (a); and optionally
(iii) a transcription termination sequence.

13. Construct according to item 12, wherein one of said control sequences is a constitutive promoter, preferably a GOS2 promoter, most preferably a GOS2 promoter from rice.

14. Use of a construct according to item 12 or 13 in a method for making plants having increased yield, particularly increased biomass and/or increased seed yield relative to control plants.

15. Plant, plant part or plant cell transformed with a construct according to item 12 or 13.

16. Method for the production of a transgenic plant having increased yield, particularly increased biomass and/or increased seed yield relative to control plants, comprising:

(i) introducing and expressing in a plant a nucleic acid encoding a YiAP2 polypeptide as defined in item 1 or 2; and
(ii) cultivating the plant cell under conditions promoting plant growth and development.

17. Transgenic plant having increased yield, particularly increased biomass and/or increased seed yield, relative to control plants, resulting from modulated expression of a nucleic acid encoding a YiAP2 polypeptide as defined in item 1 or 2, or a transgenic plant cell derived from said transgenic plant.

18. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

19. Harvestable parts of a plant according to item 18, wherein said harvestable parts are preferably shoot biomass and/or seeds.

20. Products derived from a plant according to item 18 and/or from harvestable parts of a plant according to item 19.

21. Use of a nucleic acid encoding a YiAP2 polypeptide in increasing yield, particularly in increasing seed yield and/or shoot biomass in plants, relative to control plants.

**Description of figures**

The present invention will now be described with reference to the following figures in which:

**Figure 1** represents the domain structure of SEQ ID NO: 2 with the conserved motifs underlined and numbered. The MYB/SANT domain (PF00249) is indicated in italics and stretches from Ser11 to Leu60.

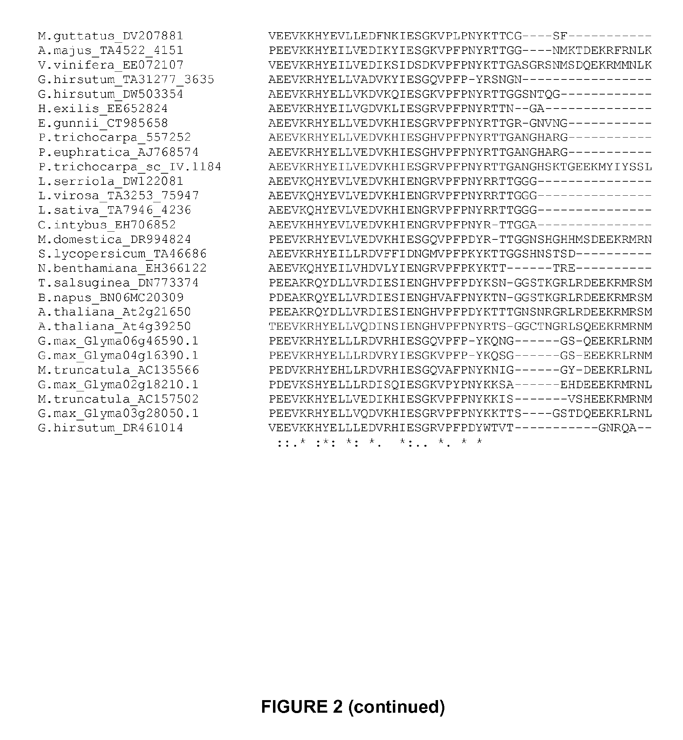

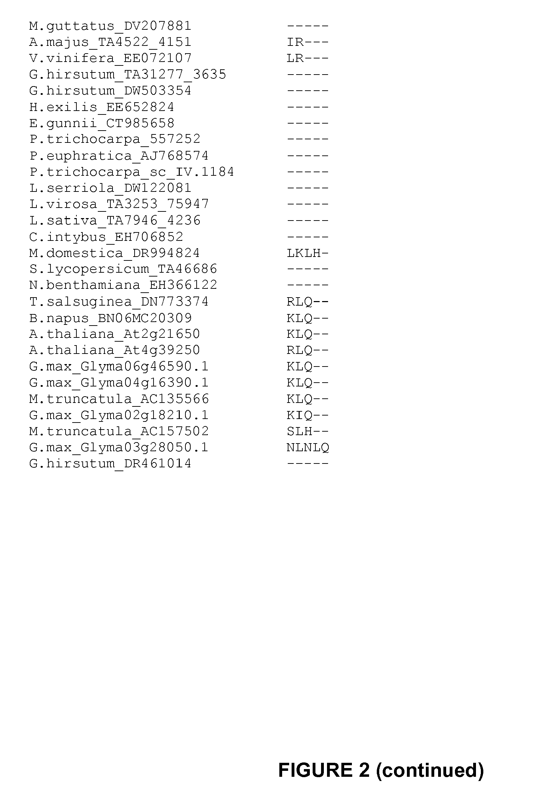

**Figure 2** represents a multiple alignment of various FSM1-like polypeptides. The asterisks indicate identical amino acids among the various protein sequences, colons represent highly conserved amino acid substitutions, and the dots represent less conserved amino acid substitution; on other positions there is no sequence conservation. These alignments can be used for defining further motifs, when using conserved amino acids.

**Figure 3** shows phylogenetic tree of FSM1-like polypeptides, constructed as described in Example 2. The SANT/MYB clade is in blue and comprises all the FSM1-like proteins given in the sequence listing, the other clades represent other MYB proteins.

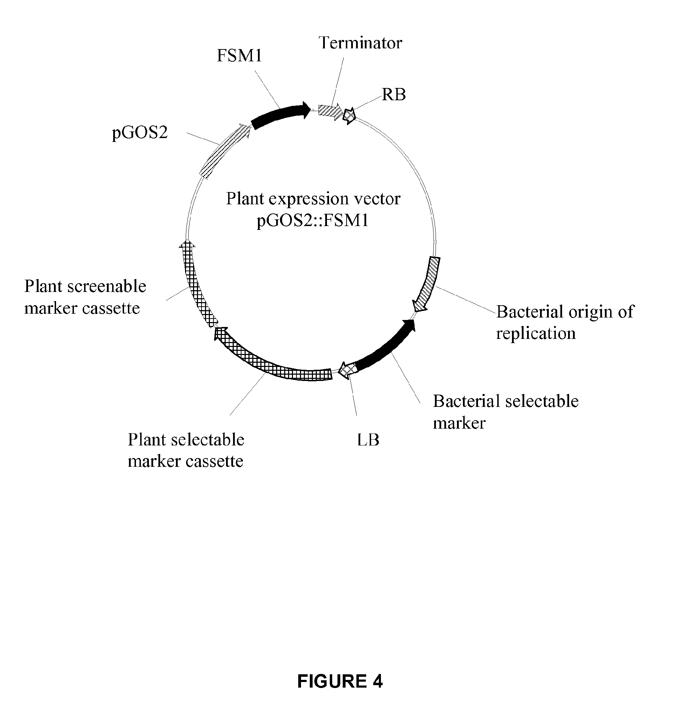

**Figure 4** represents the binary vector used for increased expression in Oryza sativa of a FSM1-like-encoding nucleic acid under the control of a rice GOS2 promoter (pGOS2)

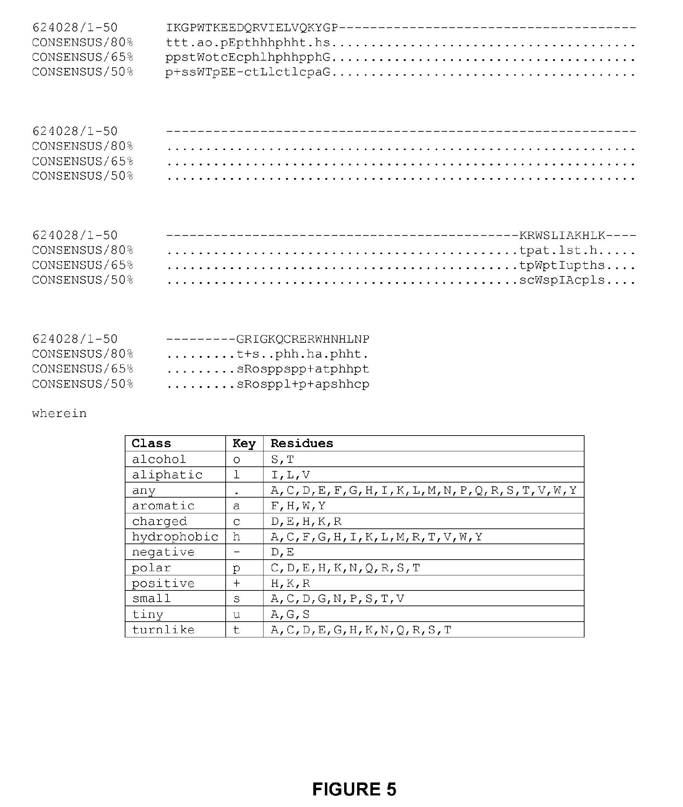

**Figure 5** represents the consensus sequence of the SANT domain as present in the SMART database. The different symbols in the motifs are clarified in the table where for each class of amino acids the respective residues are given with the one-letter code.

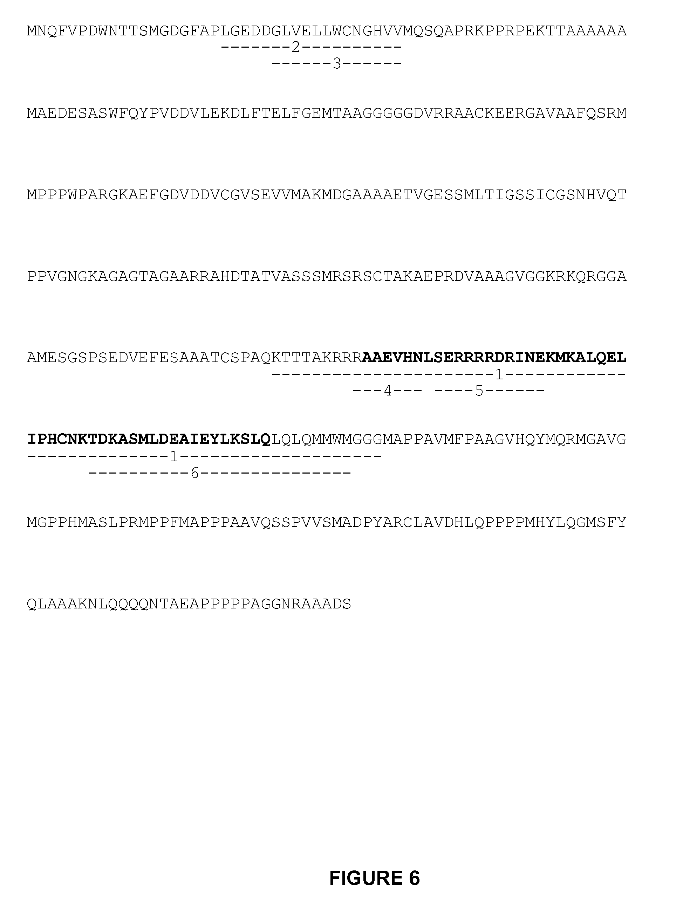

**Figure 6** represents the domain structure of SEQ ID NO: 293 with the HLH domain (PF00010) in bold and the conserved motifs underlined and numbered.

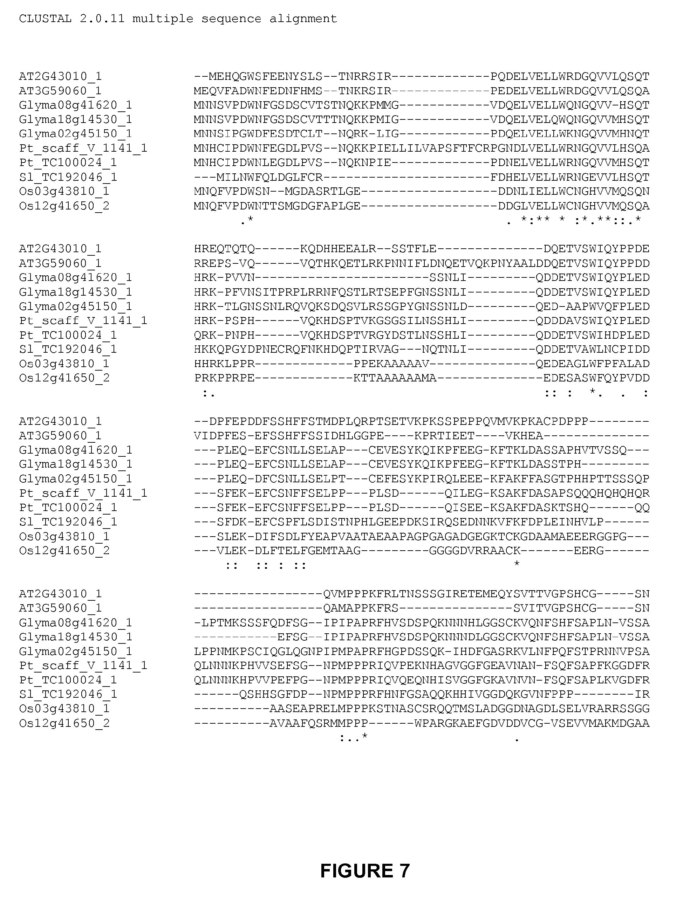

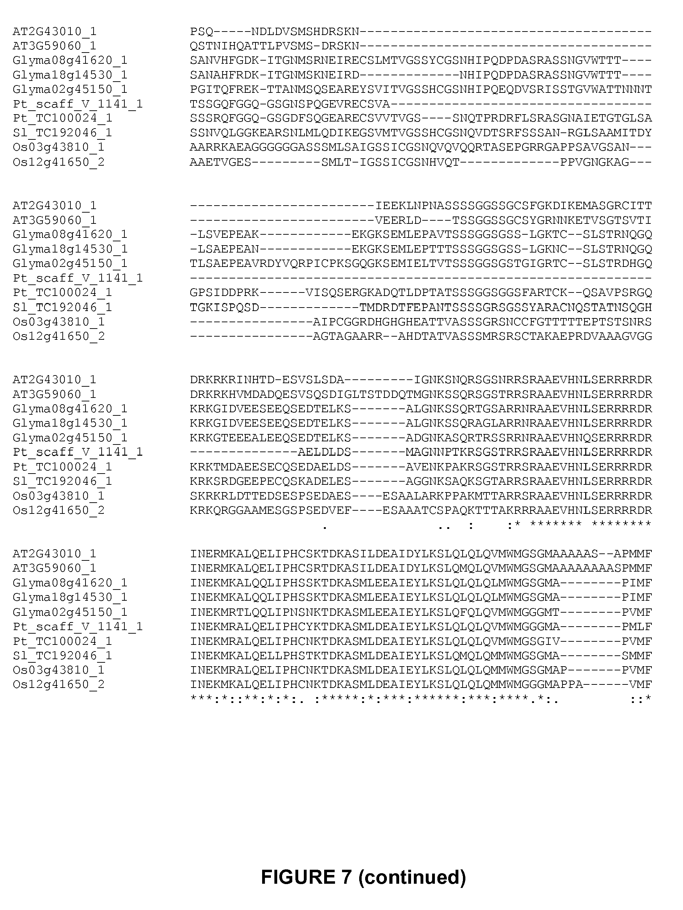

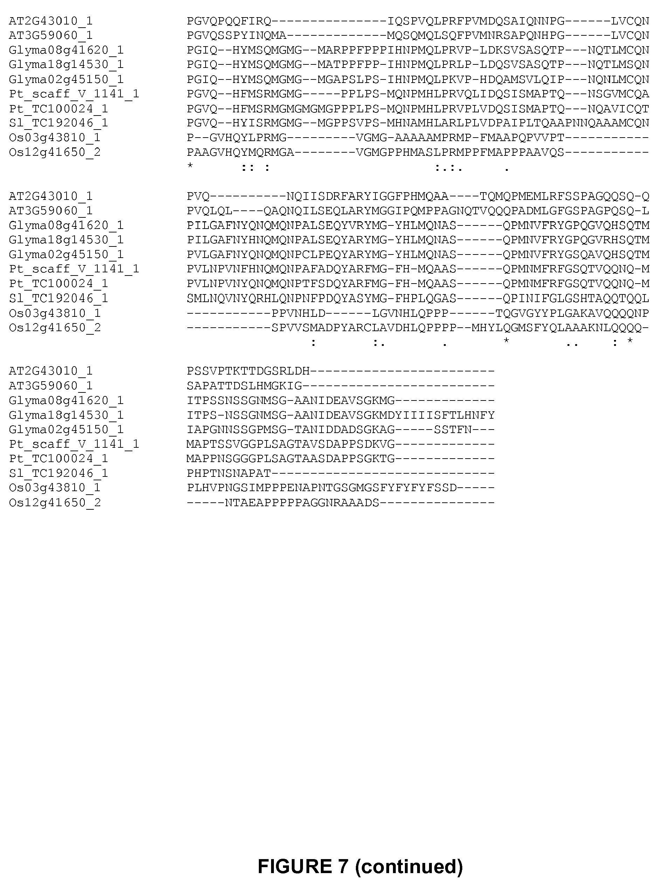

**Figure 7** represents a multiple alignment of various PIF3-like polypeptides. The asterisks indicate identical amino acids among the various protein sequences, colons represent highly conserved amino acid substitutions, and the dots represent less conserved amino acid substitution; on other positions there is no sequence conservation. These alignments can be used for defining further motifs, when using conserved amino acids.

**Figure 8** shows phylogenetic tree of PIF3-like polypeptides.

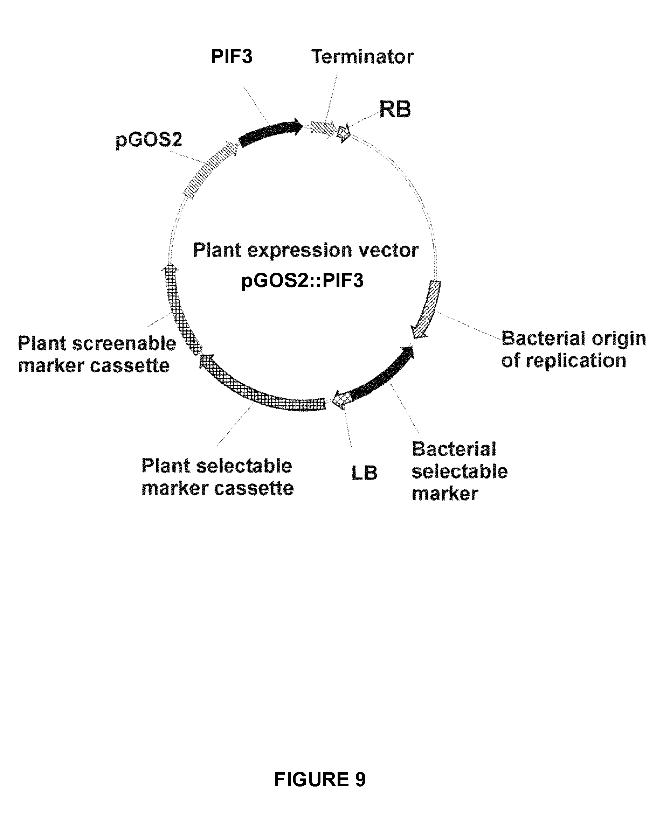

**Figure 9** represents the binary vector used for increased expression in Oryza sativa of a PIF3-like-encoding nucleic acid under the control of a rice GOS2 promoter (pGOS2)

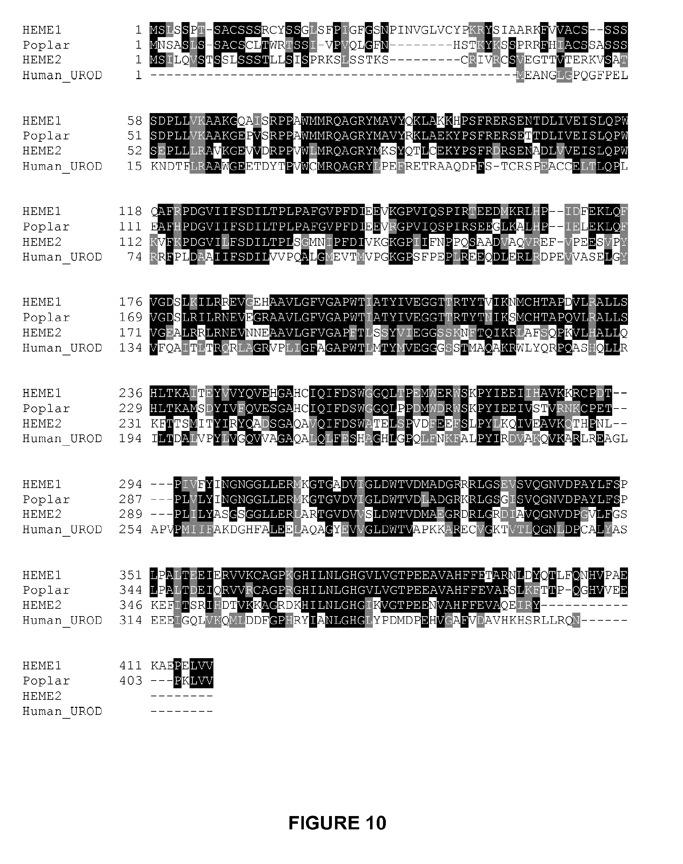

**Figure 10** is an alignment of the polypeptide sequence of SEQ ID NO: 368 with the *Arabidopsis thaliana* HEME1 and 2 sequences (At3g14930 and At2g40490), and human UROD proteins using ClustalW program. SEQ ID NO: 368 shares 75% amino acid identity with the *Arabidopsis* HEME1 sequence and 48% amino acid identity with the *Arabidopsis* HEME2 sequence. The alignment shows highly conserved residues even with the human sequence.

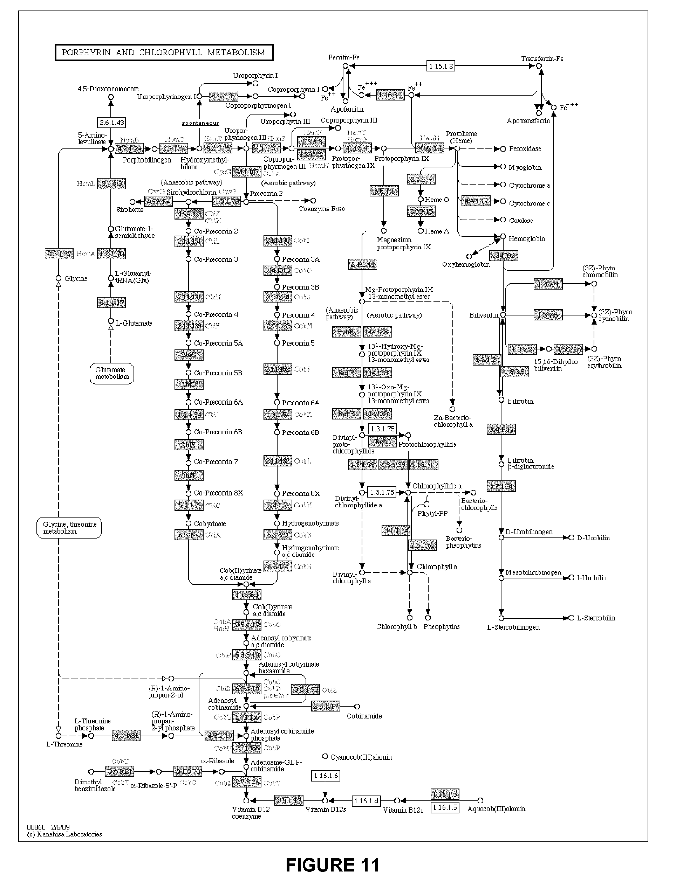

**Figure 11** shows the porphyrin and chlorophyll pathway and the involvement of UROD as a precursor in chlorophyll biosysnthesis.

**Figure 12** shows a phylogenetic tree of UROD polypeptides. The proteins were aligned using MAFT (Katoh and Toh (2008). Briefings in Bioinformatics 9:286-298). A neighbour-joining tree was calculated using QuickTree1.1 (Houwe et al. (2002). Bioinformatics 18(11):1546-7). A dendrogram was drawn using Dendroscope2.0.1 (Hudson et al. (2007).

Bioinformatics 8(1):460). At e=1e-59, both Arabidopsis URO genes (HEME1 and HEME2) were recovered. The tree was generated using representative members of each cluster.

Two groups were clearly identified according to their species of origin: the Non-Photosynthetic URO-D (1) and the Photosynthetic URO-D comprising the HEM1 cluster (2),

the HEM2 cluster (3), the CYANO cluster (4) and others (5). P. trichocarpa URO-D of SEQ ID NO: 368 is indicated as P.trichocarpa_URO_D_Populus trichocarpa_SPT.

**Figure 13** shows a further phylogenetic tree of UROD polypeptides. The proteins were aligned using MAFT (Katoh and Toh (2008). Briefings in Bioinformatics 9:286-298.). A neighbour-joining tree was calculated using QuickTree1.1 (Houwe et al. (2002).

Bioinformatics 18(11):1546-7). A circular dendrogram was drawn using Dendroscope2.0.1 (Hudson et al. (2007). Bioinformatics 8(1):460). At e=1 e-59, both Arabidopsis URO genes (HEME1 and HEME2) were recovered. The tree was generated using representative members of each cluster. Two groups were clearly identified according to their species of origin: the Non-Photosynthetic URO-D (1) and the Photosynthetic URO-D comprising the HEM1 cluster (2), the HEM2 cluster (3), the CYANO cluster (4) and others (5). P. trichocarpa URO-D of SEQ ID NO: 368 is indicated as P.trichocarpa_URO_D_Populus trichocarpa_SPT. SPT=Streptophyta, CHL=chlorphyta, STR=Stramenopile, BAC=Cyanobacteria.

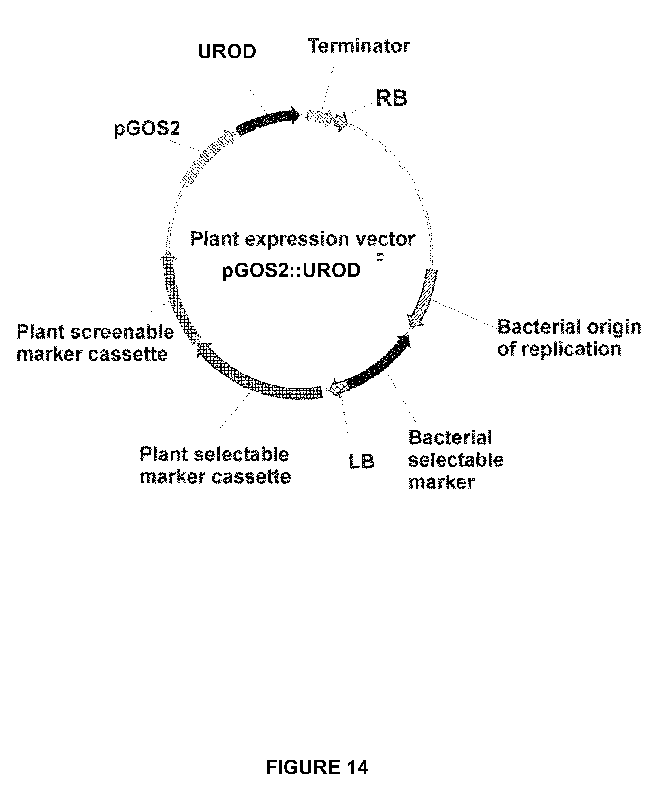

**Figure 14** represents the binary vector used for increased expression in Oryza sativa of a UROD-encoding nucleic acid under the control of a rice GOS2 promoter (pGOS2).

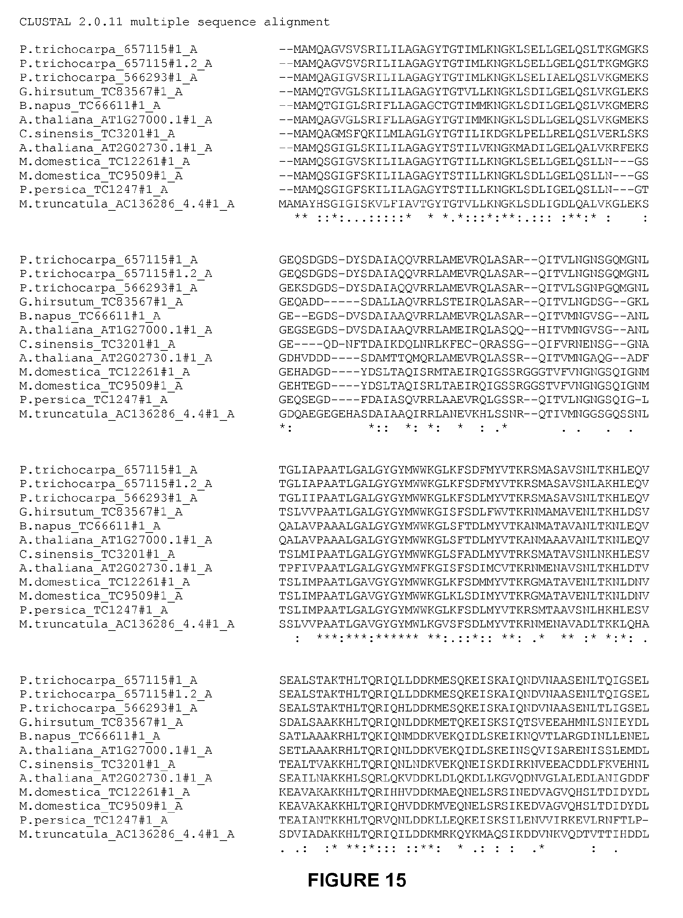

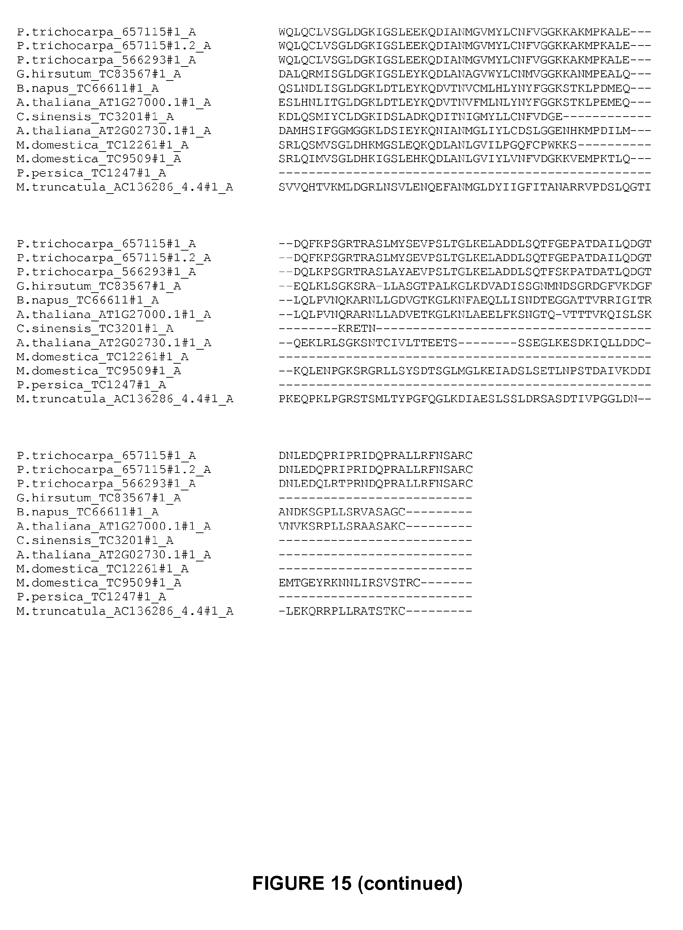

**Figure 15** represents a multiple alignment of various AS-MTT polypeptides. The asterisks indicate identical amino acids among the various protein sequences, colons represent highly conserved amino acid substitutions, and the dots represent less conserved amino acid substitution; on other positions there is no sequence conservation. These alignments can be used for defining further motifs, when using conserved amino acids.

**Figure 16** shows phylogenetic tree of AS-MTT polypeptides. Clades A and B are indicated.

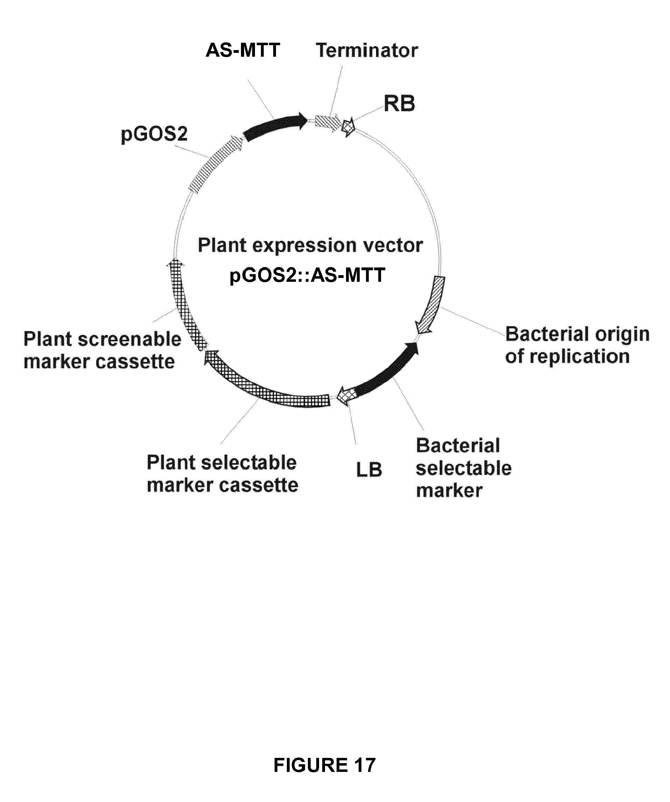

**Figure 17** represents the binary vector used for increased expression in Oryza sativa of an AS-MTT-encoding nucleic acid under the control of a rice GOS2 promoter (pGOS2).

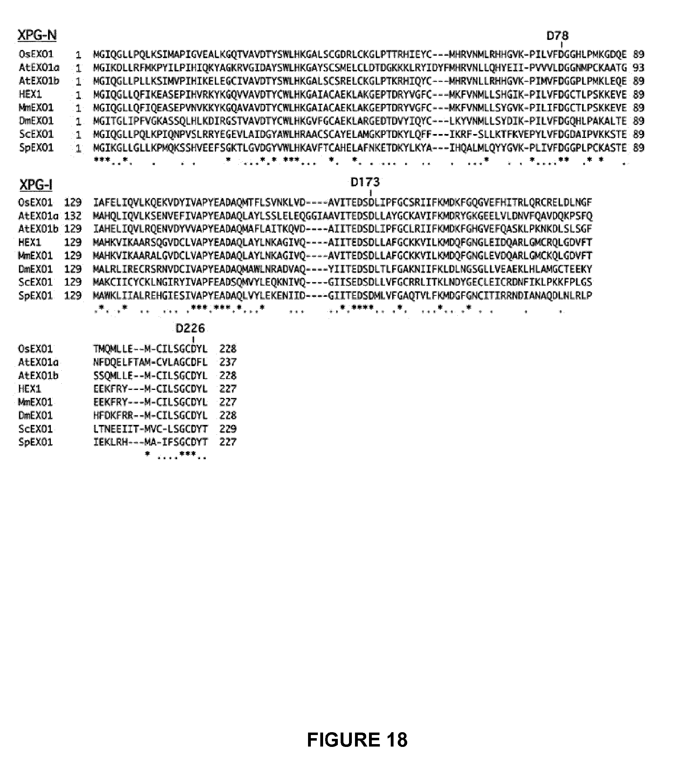

**Figure 18** represents the alignment of XPG-N and XPG-I domains between EXO1 from *Oryza sativa* (OsEXO1) and other EXO1 proteins. The accession numbers for each protein sequence; OsEXO1 (BAD60834), AtEXO1a (At1G18090), AtEXO1b (At1g29630), HEX1 (AAC32259), MmEXO1 (NP_036142), DmEXO1 (CAA61431), ScEXO1 (NP_014676), SpEXO1 (NP_596050).

**Figure 19** shows phylogenetic tree of EXO-1 polypeptides, where EXO-1 from P. *trichocarpa* (scaff_XI.493_III) is marked with a shadow in the clade of Class III. See the sequence listing for species abbreviations.

**Figure 20** represents the binary vector used for increased expression in *Oryza sativa* of an EXO-1-encoding nucleic acid under the control of a rice GOS2 promoter (pGOS2).

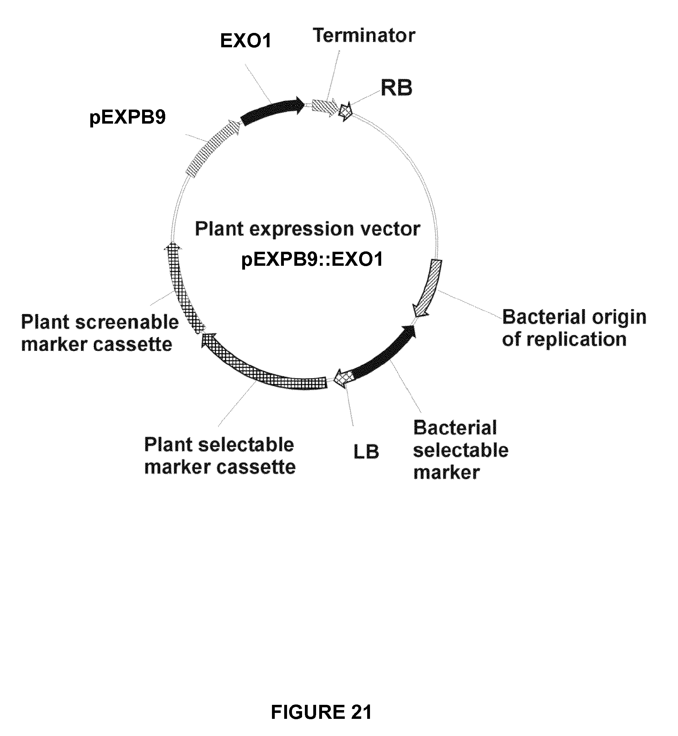

**Figure 21** represents the binary vector used for increased expression in *Oryza sativa* of an EXO-1-encoding nucleic acid under the control of a rice beta expansin promoter (EXPB9).

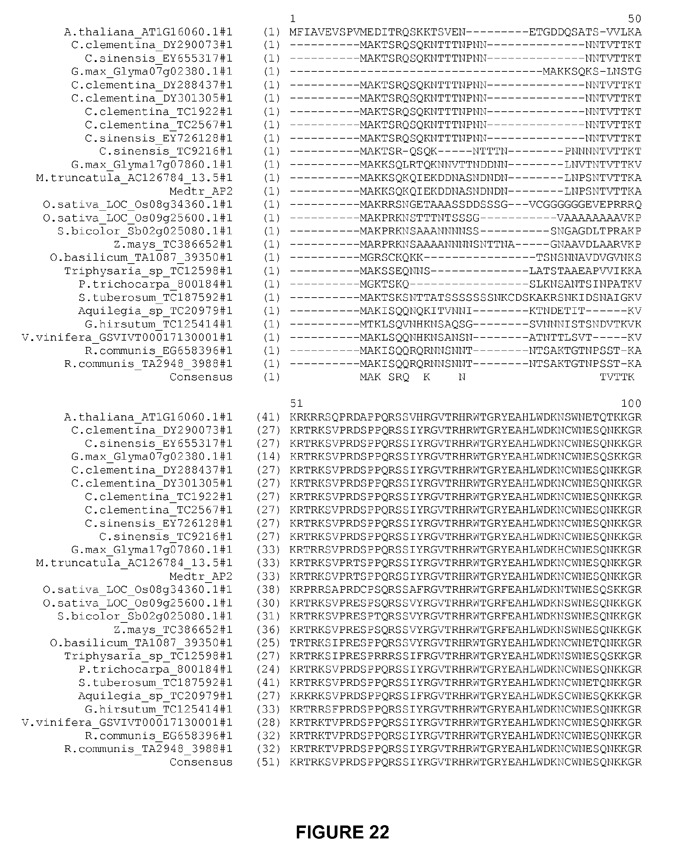

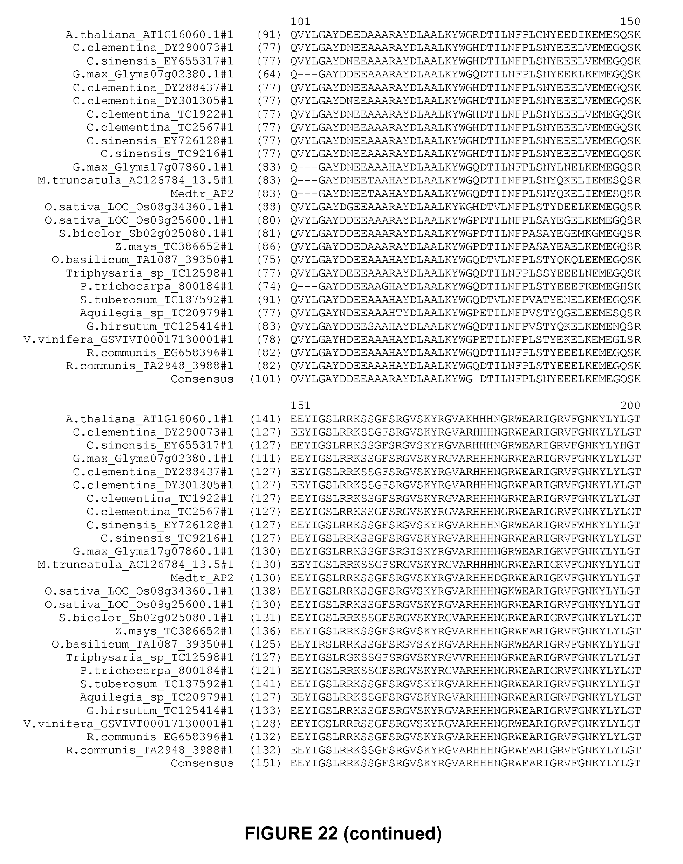

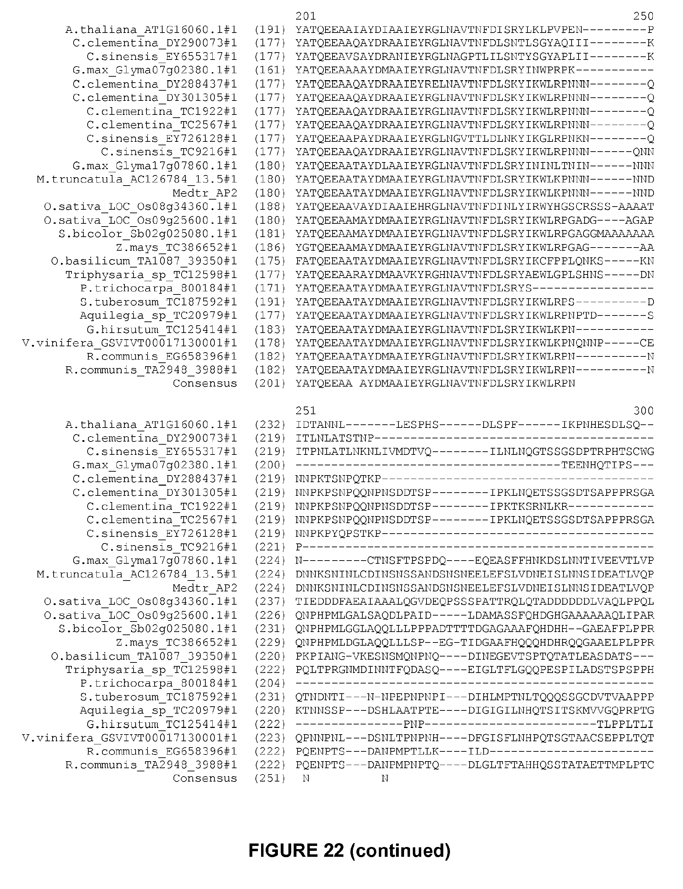

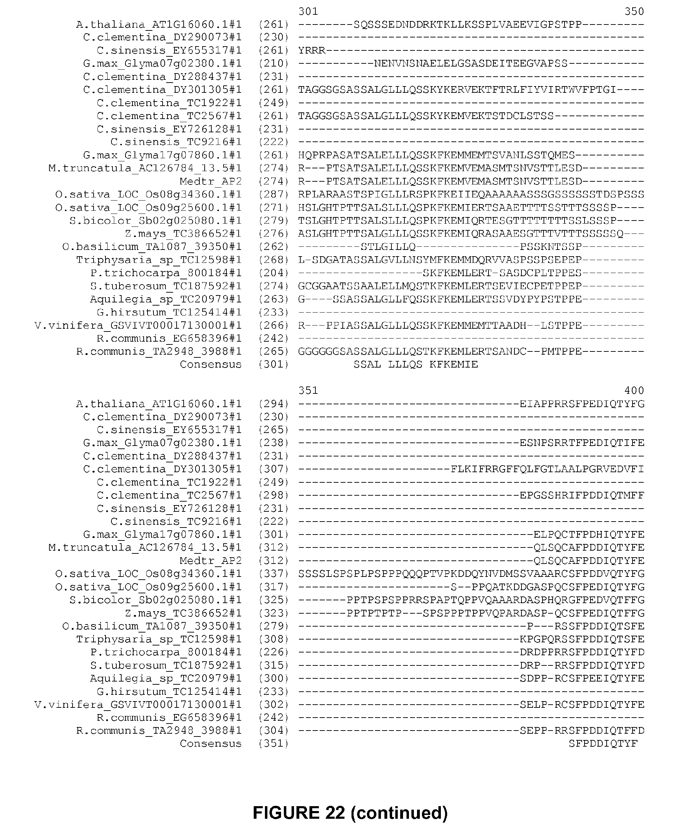

**Figure 22** represents a multiple alignment of various YiAP2 polypeptides. Highly conserved amino acids are indicated in the consensus sequences. Empty spaces in the consensus sequence represent any amino acid. These alignments can be used for defining further motifs, when using conserved amino acids.

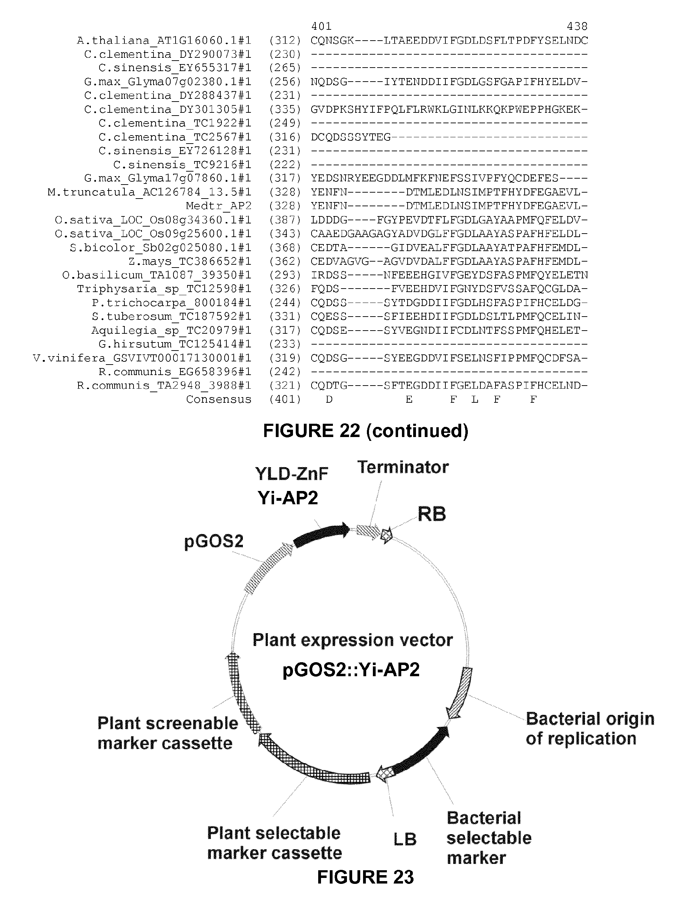

**Figure 23** represents the binary vector used for increased expression in Oryza sativa of a YiAP2-encoding nucleic acid under the control of a rice GOS2 promoter (pGOS2).

**Examples**

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

*Example 1: Identification of sequences related to the nucleic acid sequence used in the methods of the invention*

1. FSM1-like (Fruit Sant/Myb) polypeptides

Sequences (full length cDNA, ESTs or genomic) related to SEQ ID NO: 1 and SEQ ID NO: 2 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 1 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A1 provides a list of nucleic acid sequences related to SEQ ID NO: 1 and SEQ ID NO: 2.

**Table A1:** Examples of FSM1-like nucleic acids and polypeptides:

| Name | Nucleic acid SEQ ID NO: | Polypeptide SEQ ID NO: |
|---|---|---|
| S.lycopersicum_TA46686_4081#1 | 1 | 2 |
| A.majus_AJ793240#1 | 3 | 4 |
| A.majus_TA4522_4151#1 | 5 | 6 |
| A.thaliana_AT1G19510.1#1 | 7 | 8 |
| A.thaliana_AT1G75250.2#1 | 9 | 10 |

(continued)

| Name | Nucleic acid SEQ ID NO: | Polypeptide SEQ ID NO: |
|---|---|---|
| A.thaliana_AT2G21650.1#1 | 11 | 12 |
| At4g36570_I-box-binding-like _NA | 13 | 14 |
| A.thaliana_AT4G39250.1#1 | 15 | 16 |
| B.napus_BN06MC20309_46582753@20242#1 | 17 | 18 |
| B.napus_EE472702#1 | 19 | 20 |
| B.napus_EL589700#1 | 21 | 22 |
| B.rapa_L38243#1 | 23 | 24 |
| C.annuum_BM060888#1 | 25 | 26 |
| C.clementina_CX295458#1 | 27 | 28 |
| C.intybus_EH706852#1 | 29 | 30 |
| C.maculosa_EH726540#1 | 31 | 32 |
| C.sinensis_TA19011_2711#1 | 33 | 34 |
| E.grandis_CD669972#1 | 35 | 36 |
| E.gunnii_CT980385#1 | 37 | 38 |
| E.gunnii_CT985658#1 | 39 | 40 |
| E.tereticornis_CD668687#1 | 41 | 42 |
| F.arundinacea_DT700402#1 | 43 | 44 |
| F.vesca_EX665737#1 | 45 | 46 |
| G.hirsutum_DR461014#1 | 47 | 48 |
| G.hirsutum_DW476156#1 | 49 | 50 |
| G.hirsutum_DW497247#1 | 51 | 52 |
| G.hirsutum_DW503354#1 | 53 | 54 |
| G.hirsutum_TA26706_3635#1 | 55 | 56 |
| G.hirsutum_TA31277_3635#1 | 57 | 58 |
| G.hirsutum_TA36120_3635#1 | 59 | 60 |
| G.hirsutum_TA37973_3635#1 | 61 | 62 |
| G.hirsutum_TA43581_3635#1 | 63 | 64 |
| G.max_CA852793#1 | 65 | 66 |
| G.max_Glyma01g38250.1#1 | 67 | 68 |
| G.max_Glyma02g06230.1#1 | 69 | 70 |
| G.max_Glyma02g06240.1#1 | 71 | 72 |
| G.max_Glyma02g18210.1#1 | 73 | 74 |
| G.max_Glyma02g41670.1#1 | 75 | 76 |
| G.max_Glyma03g28050.1#1 | 77 | 78 |
| G.max_Glyma04g16390.1#1 | 79 | 80 |
| G.max_Glyma06g03490.1#1 | 81 | 82 |
| G.max_Glyma06g46590.1#1 | 83 | 84 |
| G.max_Glyma12g04680.1#1 | 85 | 86 |

(continued)

| Name | Nucleic acid SEQ ID NO: | Polypeptide SEQ ID NO: |
|------|-------------------------|------------------------|
| G.max_Glyma14g08090.3#1 | 87 | 88 |
| G.max_Glyma16g25250.1#1 | 89 | 90 |
| G.max_Glyma18g04120.1#1 | 91 | 92 |
| H.argophyllus_EE611072#1 | 93 | 94 |
| H.exilis_EE652824#1 | 95 | 96 |
| H.vulgare_BQ762901#1 | 97 | 98 |
| H.vulgare_CK125210#1 | 99 | 100 |
| I.batatas_EE882249#1 | 101 | 102 |
| I.nil_CJ754125#1 | 103 | 104 |
| L.perennis_DW074785#1 | 105 | 106 |
| L.sativa_TA7946_4236#1 | 107 | 108 |
| L.serriola_DW122081#1 | 109 | 110 |
| L.virosa_TA3253_75947#1 | 111 | 112 |
| M.acuminata_ES435134#1 | 113 | 114 |
| M.domestica_DR994824#1 | 115 | 116 |
| M.domestica_TA45680_3750#1 | 117 | 118 |
| M.esculenta_CK645030#1 | 119 | 120 |
| M.guttatus_DV207881#1 | 121 | 122 |
| M.truncatula_AC135566_10.5#1 | 123 | 124 |
| M.truncatula_AC157502_5.5#1 | 125 | 126 |
| M.truncatula_AC162787_11.4#1 | 127 | 128 |
| M.truncatula_AC162787_14.4#1 | 129 | 130 |
| M.truncatula_CT033768_12.4#1 | 131 | 132 |
| M.truncatula_CX518743#1 | 133 | 134 |
| N.benthamiana_EH366122#1 | 135 | 136 |
| 0.minuta_TA1082_63629#1 | 137 | 138 |
| O.sativa_LOC_Os01g44370.1#1 | 139 | 140 |
| O.sativa_LOC_Os01g44390.1#1 | 141 | 142 |
| O.sativa_LOC_Os01g47370.1#1 | 143 | 144 |
| O.sativa_LOC_Os02g47744.1#1 | 145 | 146 |
| O.sativa_LOC_Os05g49240.1#1 | 147 | 148 |
| O.sativa_LOC_Os05g50350.1#1 | 149 | 150 |
| O.sativa_LOC_Os06g28630.1#1 | 151 | 152 |
| O.sativa_LOC_Os07g26150.1#1 | 153 | 154 |
| O.sativa_LOC_Os12g33950.1#1 | 155 | 156 |
| O.sativa_Os05g0579600#1 | 157 | 158 |
| P.americana_CO997831#1 | 159 | 160 |
| P.americana_DT578382#1 | 161 | 162 |

(continued)

| Name | Nucleic acid SEQ ID NO: | Polypeptide SEQ ID NO: |
|---|---|---|
| P.coccineus_CA897021#1 | 163 | 164 |
| P.euphratica_AJ768009#1 | 165 | 166 |
| P.euphratica_AJ768184#1 | 167 | 168 |
| P.euphratica_AJ768574#1 | 169 | 170 |
| P.glauca_DR559298#1 | 171 | 172 |
| P.pinaster_BX682614#1 | 173 | 174 |
| P.taeda_TA26731_3352#1 | 175 | 176 |
| P.tremula_BU823356#1 | 177 | 178 |
| P.tremula_TA8294_113636#1 | 179 | 180 |
| P.trichocarpa_553339#1 | 181 | 182 |
| P.trichocarpa_557252#1 | 183 | 184 |
| P.trichocarpa_scaff_57.171#1 | 185 | 186 |
| P.trichocarpa_scaff_IV.1184#1 | 187 | 188 |
| P.trichocarpa_scaff_IX.506#1 | 189 | 190 |
| P.trichocarpa_scaff_V.1348#1 | 191 | 192 |
| P.trichocarpa_TA23239_3694#1 | 193 | 194 |
| S.bicolor_Sb01g040910.1#1 | 195 | 196 |
| S.bicolor_Sb03g028950.1#1 | 197 | 198 |
| S.bicolor_Sb03g028960.1#1 | 199 | 200 |
| S.bicolor_Sb03g030330.1#1 | 201 | 202 |
| S.bicolor_Sb04g030510.1#1 | 203 | 204 |
| S.bicolor_Sb09g028790.1#1 | 205 | 206 |
| S.bicolor_Sb09g029560.1#1 | 207 | 208 |
| S.henryi_TA107_13258#1 | 209 | 210 |
| S.indicum_BU668323#1 | 211 | 212 |
| S.officinarum_CA111191#1 | 213 | 214 |
| S.officinarum_CA283940# | 215 | 216 |
| S.officinarum_TA43900_4547#1 | 217 | 218 |
| S.officinarum_TA47846_4547#1 | 219 | 220 |
| S.tuberosum_BI433702#1 | 221 | 222 |
| S.tuberosum_CV500497#1 | 223 | 224 |
| S.tuberosum_CV504810#1 | 225 | 226 |
| T.aestivum_BE427243#1 | 227 | 228 |
| T.aestivum_BM259034#1 | 229 | 230 |
| T.aestivum_CA623533#1 | 231 | 232 |
| T.aestivum_CV775992#1 | 233 | 234 |
| T.aestivum_TA110455_4565#1 | 235 | 236 |
| T.aestivum_TA95415_4565#1 | 237 | 238 |

(continued)

| Name | Nucleic acid SEQ ID NO: | Polypeptide SEQ ID NO: |
|---|---|---|
| T.erecta_SIN-31b-CS_SCR20-B14.b1--------@5804#1 | 239 | 240 |
| T.pratense_BB920325#1 | 241 | 242 |
| T.salsuginea_DN773374#1 | 243 | 244 |
| V.vinifera_CA812415#1 | 245 | 246 |
| V.vinifera_CB345061#1 | 247 | 248 |
| V.vinifera_CB972578#1 | 249 | 250 |
| V.vinifera_EE072107#1 | 251 | 252 |
| V.vinifera_GSVIVT00014602001#1 | 253 | 254 |
| V.vinifera_GSVIVT00028247001#1 | 255 | 256 |
| V.vinifera_GSVIVT00036374001#1 | 257 | 258 |
| Z.mays_c58977309gm030403@3901#1 | 259 | 260 |
| Z.mays_c68506706gm030403@11442#1 | 261 | 262 |
| Z.mays_DR787399#1 | 263 | 264 |
| Z.mays_s58275634gm030403@34722#1 | 265 | 266 |
| Z.mays_TA203683_4577#1 | 267 | 268 |
| Z.mays_TA206461_4577#1 | 269 | 270 |
| Z.mays_TA208256_4577#1 | 271 | 272 |
| Z.mays_TA210755_4577#1 | 273 | 274 |
| Z.mays_TA219702_4577#1 | 275 | 276 |
| Z.mays_ZM07MSbpsHQ_57388861.r01@38768#1 | 277 | 278 |
| Z.officinale_DY348802#1 | 279 | 280 |
| Z.officinale_DY369792#1 | 281 | 282 |

Sequences have been tentatively assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, such as by the Joint Genome Institute. Furthermore, access to proprietary databases, has allowed the identification of novel nucleic acid and polypeptide sequences.

2. PIF3-like (Phytochrome Interacting Factor 3) polypeptides

Sequences (full length cDNA, ESTs or genomic) related to SEQ ID NO: 292 and SEQ ID NO: 293 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 292 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A2 provides a list of nucleic acid sequences related to SEQ ID NO: 292 and SEQ ID NO: 293.

**Table A2:** Examples of PIF3-like nucleic acids and polypeptides:

| Name | Nucleic acid SEQ ID NO: | Polypeptide SEQ ID NO: |
|---|---|---|
| O_sativa_LOC_Os12g41650_2 (SEQ ID NO: 292) | 292 | 293 |
| A_thaliana_AT2G20180_2_1 | 294 | 295 |
| A_thaliana_AT2G43010_1_1 | 296 | 297 |
| A_thaliana_AT3G59060_1_1 | 298 | 299 |
| G_max_Glyma02g45150_1_1 | 300 | 301 |
| G_max_Glyma03g32740_1_1 | 302 | 303 |
| G_max_Glyma08g41620_1_1 | 304 | 305 |
| G_max_Glyma10g04890_1_1 | 306 | 307 |
| G_max_Glyma10g28290_1_1 | 308 | 309 |
| G_max_Glyma13g19250_1_1 | 310 | 311 |
| G_max_Glyma18g14530_1_1 | 312 | 313 |
| M_truncatula_AC161749_19_5_1 | 314 | 315 |
| M_truncatula_TC130203_1 | 316 | 317 |
| O_sativa_LOC_Os03g43810_1_1 | 318 | 319 |
| O_sativa_LOC_Os03g56950_2 | 320 | 321 |
| O_sativa_LOC_Os03g56950_5_1 | 322 | 323 |
| O_sativa_LOC_Os03g56950_6_1 | 324 | 325 |
| O_sativa_LOC_Os03g56950_7_1 | 326 | 327 |
| O_sativa_LOC_Os07g05010_1_1 | 328 | 329 |
| A_thaliana_AT1G09530_1_1 | 330 | 331 |
| O_sativa_Os03g0782500_1 | 332 | 333 |
| O_sativa_Os07g0143200_1 | 334 | 335 |
| P_trichocarpa_581594_1 | 336 | 337 |
| P_trichocarpa_756351_1 | 338 | 339 |
| P_trichocarpa_scaff_131_21_1 | 340 | 341 |
| P_trichocarpa_scaff_II_2575_ 1 | 342 | 343 |
| P_trichocarpa_scaff_V_1141_1 | 344 | 345 |
| P_trichocarpa_scaff_XIII_13_1 | 346 | 347 |
| P_trichocarpa_TC100024_1 | 348 | 349 |
| S_lycopersicum_TC192046_1 | 350 | 351 |
| Oryza Sativa TMCDS_14571 | 352 | 353 |
| Populus trichocarpa TMCDS_30166 | 354 | 355 |
| Z_mays_ZM07MC05422_62035389_5410_1 | 356 | 357 |

Sequences have been tentatively assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, such as by the Joint Genome Institute. Furthermore, access to proprietary databases, has allowed the identification of novel nucleic acid and polypeptide sequences.

3. UROD (Uroporphyrinogen III decarboxylase) polypeptide

Sequences (full length cDNA, ESTs or genomic) related to SEQ ID NO: 367 and SEQ ID NO: 368 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 367 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A3 provides a list of nucleic acid sequences related to SEQ ID NO: 367 and SEQ ID NO: 368.

**Table A3:** Examples of UROD nucleic acids and polypeptides:

| Name | Nucleic acid SEQ ID NO: | Polypeptide SEQ ID NO: |
|---|---|---|
| P.trichocarpa | 367 | 368 |
| A.anophagefferens_30614#1 | 369 | 370 |
| A.thaliana_AT2G40490.1#1 | 371 | 372 |
| A.thaliana_AT3G14930.1#1 | 373 | 374 |
| A.thaliana_AT3G14930.2#1 | 375 | 376 |
| Aquilegia_sp_TC24226#1 | 377 | 378 |
| Aquilegia_sp_TC24505#1 | 379 | 380 |
| B.napus_TC68105#1 | 381 | 382 |
| B.napus_TC76241#1 | 383 | 384 |
| B.napus_TC77092#1 | 385 | 386 |
| C.reinhardtii_132194#1 | 387 | 388 |
| C.reinhardtii_186446#1 | 389 | 390 |
| C.reinhardtii_195818#1 | 391 | 392 |
| C.sinensis_TC316#1 | 393 | 394 |
| C.vulgaris_25726#1 | 395 | 396 |
| C.vulgaris_61627#1 | 397 | 398 |
| C.vulgaris_69032#1 | 399 | 400 |
| Chlorella_32323#1 | 401 | 402 |
| Chlorella_48884#1 | 403 | 404 |
| E.esula_TC8568#1 | 405 | 406 |
| E.huxleyi_361296#1 | 407 | 408 |
| E.huxleyi_465530#1 | 409 | 410 |
| G.hirsutum_TC101219#1 | 411 | 412 |

(continued)

| Name | Nucleic acid SEQ ID NO: | Polypeptide SEQ ID NO: |
|---|---|---|
| G.max_Glyma11g35910.1#1 | 413 | 414 |
| G.max_Glyma12g35880.2#1 | 415 | 416 |
| G.max_Glyma13g34500.2#1 | 417 | 418 |
| G.max_Glyma18g02490.1#1 | 419 | 420 |
| H.vulgare_TC175532#1 | 421 | 422 |
| L.serriola_TC1532#1 | 423 | 424 |
| L.virosa_TA4736_75947#1 | 425 | 426 |
| M.domestica_TC16580#1 | 427 | 428 |
| M.truncatula_AC119409_9.4#1 | 429 | 430 |
| M.truncatula_AC149472_12.4#1 | 431 | 432 |
| N.tabacum_TC14277#1 | 433 | 434 |
| 0.basilicum_TA2234_39350#1 | 435 | 436 |
| O.glaberrima_Og013395.01#1 | 437 | 438 |
| O.lucimarinus_17656#1 | 439 | 440 |
| O.lucimarinus_18446#1 | 441 | 442 |
| O.lucimarinus_28976#1 | 443 | 444 |
| O.RCC809_32990#1 | 445 | 446 |
| O.RCC809_53774#1 | 447 | 448 |
| O.sativa_LOC_Os01g43390.1#1 | 449 | 450 |
| O.sativa_LOC_Os03g21900.1#1 | 451 | 452 |
| O.taurii_13162#1 | 453 | 454 |
| O.taurii_14772#1 | 455 | 456 |
| O.taurii_9156#1 | 457 | 458 |
| P.patens_112020#1 | 459 | 460 |
| P.patens_112870#1 | 461 | 462 |
| P.patens_188035#1 | 463 | 464 |
| P.persica_TC4822#1 | 465 | 466 |
| P.taeda_TA15428_3352#1 | 467 | 468 |
| P.tremuloides_826111#1 | 469 | 470 |
| P.tricornutum_19188#1 | 471 | 472 |
| P.tricornutum_20757#1 | 473 | 474 |
| R.communis_TA2993_3988#1 | 475 | 476 |
| S.bicolor_Sb01g036030.1#1 | 477 | 478 |
| S.bicolor_Sb03g028330.1#1 | 479 | 480 |
| S.lycopersicum_TC195523#1 | 481 | 482 |
| S.lycopersicum_TC209939#1 | 483 | 484 |
| S.moellendorffii_231982#1 | 485 | 486 |
| S.moellendorffii_438672#1 | 487 | 488 |

(continued)

| Name | Nucleic acid SEQ ID NO: | Polypeptide SEQ ID NO: |
|---|---|---|
| S.officinarum_TC104808#1 | 489 | 490 |
| S.tuberosum_TC166562#1 | 491 | 492 |
| T.aestivum_TC347283#1 | 493 | 494 |
| T.pseudonana_3974#1 | 495 | 496 |
| Triphysaria_sp_TC2645#1 | 497 | 498 |
| V.carteri_103889#1 | 499 | 500 |
| V.carteri_74033#1 | 501 | 502 |
| V.carteri_83541#1 | 503 | 504 |
| V.vinifera_GSVIVT00027816001#1 | 505 | 506 |
| Z.mays_EB164247#1 | 507 | 508 |
| Z.mays_TC386418#1 | 509 | 510 |
| Z.mays_TC407629#1 | 511 | 512 |
| Z.mays_ZM07MC20070_BFb0115I19@20019#1 | 513 | 514 |

Sequences have been tentatively assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, such as by the Joint Genome Institute. Furthermore, access to proprietary databases, has allowed the identification of novel nucleic acid and polypeptide sequences.

4. AS-MTT (Abiotic Stress Membrane Tethered Transcription factor) polypeptides

Sequences (full length cDNA, ESTs or genomic) related SEQ ID NO: 536 and SEQ ID NO: 537 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 536 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A4 provides a list of nucleic acid sequences related to the nucleic acid sequence used in the methods of the present invention.

**Table A4:** Examples of AS-MTT nucleic acids and polypeptides:

| Gene name in class | Nucleic acid SEQ ID NO: | Polypeptide SEQ ID NO: |
|---|---|---|
| P.trichocarpa_657115#1_A | 536 | 537 |
| P.trichocarpa_657115#1.2_A | 538 | 539 |
| P.persica_TC1247#1_A | 540 | 541 |
| C.sinensis_TC3201#1_A | 542 | 543 |

(continued)

| Gene name in class | Nucleic acid SEQ ID NO: | Polypeptide SEQ ID NO: |
|---|---|---|
| M.domestica_TC12261#1_A | 544 | 545 |
| A.thaliana_AT2G02730.1#1_A | 546 | 547 |
| G.hirsutum_TC83567#1_A | 548 | 549 |
| B.napus_TC66611#1_A | 550 | 551 |
| A.thaliana_AT1G27000.1#1_A | 552 | 553 |
| M.domestica_TC9509#1_A | 554 | 555 |
| M.truncatula_AC136286_4.4#1_A | 556 | 557 |
| P.trichocarpa_566293#1_A | 558 | 559 |
| S.officinarum_TC86637#1_B | 560 | 561 |
| P.americana_TA1256_3435#1_B | 562 | 563 |
| C.maculosa_TA1398_215693#1_B | 564 | 565 |
| O.sativa_LOC_Os01g08990.1#1_B | 566 | 567 |
| 0.sativa_LOC_Os05g08980.1#1_B | 568 | 569 |
| T.officinale_TA751_50225#1_B | 570 | 571 |
| F.arundinacea_TC6829#1_B | 572 | 573 |
| S.bicolor_Sb03g003440.1#1_B | 574 | 575 |
| I.nil_TC6724#1_B | 576 | 577 |
| S.tuberosum_TC170010#1_B | 578 | 579 |
| Aquilegia_sp_TC25212#1_B | 580 | 581 |
| V.vinifera_GSVIVT00029000001#1_B | 582 | 583 |
| H.annuus_TC32349#1_B | 584 | 585 |
| A.comosus_DT336453#1_bZIP17-like | 586 | 587 |
| Triphysaria_sp_TC1899#1_bZIP17-like | 588 | 589 |
| M.truncatula_CU024880_148.4#1_bZIP17-like | 590 | 591 |
| L.sativa_TC16705#1_bZIP17-like | 592 | 593 |
| P.taeda_TA8248_3352#1_bZIP17-like | 594 | 595 |
| H.paradoxus_TA2663_73304#1_bZIP17-like | 596 | 597 |
| E.esula_TC4805#1_bZIP17-like | 598 | 599 |
| I.nil_TC113#1_bZIP17-like | 600 | 601 |
| A.thaliana_AT1G04960.1#1_bZIP17-like | 602 | 603 |
| O.sativa_LOC_Os09g30478.1#1_bZIP17-like | 604 | 605 |
| Aquilegia_sp_TC26001#1_bZIP17-like | 606 | 607 |
| H.vulgare_TC157185#1_bZIP17-like | 608 | 609 |
| P.trichocarpa_645036#1_bZIP17-like | 610 | 611 |
| T.aestivum_TC297361#1_bZIP17-like | 612 | 613 |
| S.tuberosum_TC182348#1_bZIP17-like | 614 | 615 |
| E.esula_TC6178#1_Unk | 616 | 617 |
| L.sativa_TC18726#1_Unk | 618 | 619 |

(continued)

| Gene name in class | Nucleic acid SEQ ID NO: | Polypeptide SEQ ID NO: |
|---|---|---|
| P.patens_136490#1_Unk | 620 | 621 |
| V.vinifera_GSVIVT00002774001#1_Unk | 622 | 623 |
| C.solstitialis_TA4216_347529#1_Unk | 624 | 625 |
| S.moellendorffii_271950#1_Unk | 626 | 627 |
| A.thaliana_AT1G24267.1#1_Unk | 628 | 629 |
| G.hirsutum_TC128629#1_Unk | 630 | 631 |
| A.thaliana_AT1G24265.2#1_Unk | 632 | 633 |
| G.max_Glyma08g29150.1#1_Unk | 634 | 635 |
| S.bicolor_Sb03g009200.1#1_Unk | 636 | 637 |
| O.sativa_LOC_Os01g02180.1#1_Unk | 638 | 639 |
| Aquilegia_sp_TC28043#1_Unk | 640 | 641 |
| S.tuberosum_TC184255#1_Unk | 642 | 643 |
| P.trichocarpa_554754#1_Unk | 644 | 645 |
| Triphysaria_sp_TC917#1_Unk | 646 | 647 |

5. EXO-1 polypeptide

Sequences (full length cDNA, ESTs or genomic) related to SEQ ID NO: 762 and SEQ ID NO: 763 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 762 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A5 provides a list of nucleic acid sequences related to SEQ ID NO: 762 and SEQ ID NO: 763.

Table A5: Examples of EXO-1 polypeptides:

| Name | Nucleic acid SEQ ID NO: | Polypeptide SEQ ID NO: |
|---|---|---|
| A.thaliana_AT3G28030.1_I | 660 | 661 |
| D.melanogaster_mus201-PB_I | 662 | 663 |
| G.max_Glyma01g16370.1_ | 664 | 665 |
| H.sapiens_NM_000123.2_ | 666 | 667 |
| O.sativa_Os03g0205400_ | 668 | 669 |
| P.trichocarpa_scaff_XVII.231_I | 670 | 671 |
| S.bicolor_Sb01g043560.1_ | 672 | 673 |
| V.vinifera_GSVIVT00020778001_ | 674 | 675 |
| A.anophagefferens_19112_ | 676 | 677 |

(continued)

| Name | Nucleic acid SEQ ID NO: | Polypeptide SEQ ID NO: |
|---|---|---|
| A.anophagefferens_53007_ | 678 | 679 |
| Aquilegia_sp_TC27969_ | 680 | 681 |
| Aquilegia_sp_TC29285_ | 682 | 683 |
| B.distachyon_TA2015_15368_ | 684 | 685 |
| B.napus_TC86178_ | 686 | 687 |
| C.reinhardtii_30834_ | 688 | 689 |
| C.vulgaris_45023_ | 690 | 691 |
| Chlorella_37697_ | 692 | 693 |
| D.melanogaster_Fen1-PA_ | 694 | 695 |
| G.max_Glyma10g28200.1_ | 696 | 697 |
| G.max_Glyma20g22190.2_ | 698 | 699 |
| H.sapiens_NM_004111.4_ | 700 | 701 |
| H.vulgare_TC165804_ | 702 | 703 |
| M.truncatula_AC148406_42.5_II | 704 | 705 |
| O.lucimarinus_42373_ | 706 | 707 |
| O.RCC809_87153_ | 708 | 709 |
| O.sativa_LOC_Os03g61820.1_II | 710 | 711 |
| O.sativa_Os4005g0540100_II | 712 | 713 |
| 0.taurii_28688_ | 714 | 715 |
| P.patens_207454_ | 716 | 717 |
| S.bicolor_Sb09g026950.1_ | 718 | 719 |
| S.cerevisiae_YKL113C_ | 720 | 721 |
| S.lycopersicum_TC192754_ | 722 | 723 |
| S.moellendorffii_111465_ | 724 | 725 |
| S.tuberosum_TC179198_ | 726 | 727 |
| T.aestivum_class_II | 728 | 729 |
| T.pseudonana_269347_ | 730 | 731 |
| V.carteri_75802_ | 732 | 733 |
| Z.mays_TC425293_ | 734 | 735 |
| A.thaliana_AT1G18090.1_III | 736 | 737 |
| A.thaliana_AT1G29630.2_III | 738 | 739 |
| C.vulgaris_30308_ | 740 | 741 |
| Chlorella_24009_ | 742 | 743 |
| D.melanogaster_tos-PA_III | 744 | 745 |
| G.max_Glyma05g29660.1_ | 746 | 747 |
| G.max_Glyma07g11320.1_ | 748 | 749 |
| H.sapiens_NM_130398.2_ | 750 | 751 |
| M.truncatula_AC153460_23.4_ | 752 | 753 |

(continued)

| Name | Nucleic acid SEQ ID NO: | Polypeptide SEQ ID NO: |
|---|---|---|
| 0.lucimarinus_13915_ | 754 | 755 |
| O.sativa_LOC_Os01g56940.1_III | 756 | 757 |
| P.patens_114516_ | 758 | 759 |
| P.patens_194153_ | 760 | 761 |
| P.trichocarpa_scaff_XI.493_III | 762 | 763 |
| P.trichocarpa_scaff_XV.367_ | 764 | 765 |
| S.cerevisiae_YDR263C_ | 766 | 767 |
| S.cerevisiae_YOR033C_ | 768 | 769 |
| S.moellendorffii_175620_ | 770 | 771 |
| V.carteri_61592_ | 772 | 773 |
| S.cerevisiae_YGR258C_ | 774 | 775 |
| A.thaliana_AT3G48900.2_IV | 776 | 777 |
| O.sativa_LOC_Os08g01130.1_IVa | 778 | 779 |
| P.trichocarpa_scaff_XV.1202_ | 780 | 781 |
| S.bicolor_Sb07g000270.1_ | 782 | 783 |
| V.vinifera_GSVIVT00009204001_ | 784 | 785 |
| A.thaliana_AT1G01880.1_ | 786 | 787 |
| O.sativa_LOC_Os09g35000.1_IVb | 788 | 789 |
| P.trichocarpa_scaff_XIV.232_ | 790 | 791 |
| S.bicolor_Sb02g030290.1_ | 792 | 793 |
| V.vinifera_GSVIVT00026338001_ | 794 | 795 |
| D.melanogaster_Gen-PA_V | 796 | 797 |
| H.sapiens_NM_182625.2_ | 798 | 799 |
| A.thaliana_AT1G74390.1_ | 800 | 801 |
| A.thaliana_AT4G39810.1_ | 802 | 803 |
| A.thaliana_AT5G07710.1_ | 804 | 805 |
| A.thaliana_AT5G61390.1_ | 806 | 807 |
| G.max_Glyma05g00890.1_ | 808 | 809 |
| G.max_Glyma17g11030.1_ | 810 | 811 |
| O.sativa_LOC_Os05g01200.1_ | 812 | 813 |
| O.sativa_LOC_Os10g26720.1_ | 814 | 815 |
| O.sativa_LOC_Os10g26730.1_ | 816 | 817 |
| O.sativa_LOC_Os10g29090.1_ | 818 | 819 |
| P.trichocarpa_scaff_129.36_ | 820 | 821 |
| P.trichocarpa_scaff_V.182_ | 822 | 823 |
| P.trichocarpa_scaff_VII.573_ | 824 | 825 |
| S.cerevisiae_YER041W_ | 826 | 827 |

Sequences have been tentatively assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, such as by the Joint Genome Institute. Furthermore, access to proprietary databases, has allowed the identification of novel nucleic acid and polypeptide sequences.

6. YiAP2 polypeptides

Sequences (full length cDNA, ESTs or genomic) related to SEQ ID NO: 835 and SEQ ID NO: 836 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 835 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A6 provides a list of nucleic acid sequences related to SEQ ID NO: 835 and SEQ ID NO: 836.

Table A6: Examples of YiAP2 polypeptides:

| Name | Nucleic acid SEQ ID NO: | Polypeptide SEQ ID NO: |
|---|---|---|
| Medtr_AP2 | 835 | 836 |
| A.thaliana_AT1G16060.1#1 | 837 | 838 |
| Aquilegia_sp_TC20979#1 | 839 | 840 |
| C.clementina_DY288437#1 | 841 | 842 |
| C.clementina_DY290073#1 | 843 | 844 |
| C.clementina_DY301305#1 | 845 | 846 |
| C.clementina_TC1922#1 | 847 | 848 |
| C.clementina_TC2567#1 | 849 | 850 |
| C.sinensis_EY655317#1 | 851 | 852 |
| C.sinensis_EY726128#1 | 853 | 854 |
| C.sinensis_TC9216#1 | 855 | 856 |
| G.hirsutum_TC125414#1 | 857 | 858 |
| G.max_Glyma07g02380.1#1 | 859 | 860 |
| G.max_Glyma17g07860.1#1 | 861 | 862 |
| M.truncatula_AC126784_13.5#1 | 863 | 864 |
| 0.basilicum_TA1087_39350#1 | 865 | 866 |
| O.sativa_LOC_Os08g34360.1#1 | 867 | 868 |
| O.sativa_LOC_Os09g25600.1#1 | 869 | 870 |
| P.trichocarpa_800184#1 | 871 | 872 |
| R.communis_EG658396#1 | 873 | 874 |
| R.communis_TA2948_3988#1 | 875 | 876 |

(continued)

| Name | Nucleic acid SEQ ID NO: | Polypeptide SEQ ID NO: |
|---|---|---|
| S.bicolor_Sb02g025080.1#1 | 877 | 878 |
| S.tuberosum_TC187592#1 | 879 | 880 |
| Triphysaria_sp_TC12598#1 | 881 | 882 |
| V.vinifera_GSVIVT00017130001#1 | 883 | 884 |
| Z.mays_TC386652#1 | 885 | 886 |

Sequences have been tentatively assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, such as by the Joint Genome Institute. Furthermore, access to proprietary databases, has allowed the identification of novel nucleic acid and polypeptide sequences.

*Example 2: Alignment of sequences related to the polypeptide sequences used in the methods of the invention*

1. FSM1-like (Fruit Sant/Myb) polypeptides

Alignment of a number of FSM1-like polypeptide sequences was performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing was done to further optimise the alignment. The FSM1-like polypeptides are aligned in Figure 2.

A phylogenetic tree of FSM1-like polypeptides (Figure 3) was constructed as follows: the alignment was generated using MAFFT (Katoh and Toh (2008) Briefings in Bioinformatics 9:286-298). A neighbour-joining tree was calculated using QuickTree (Howe et al. (2002), Bioinformatics 18(11): 1546-7), 100 bootstrap repetitions. The circular cladogram was drawn using Dendroscope (Huson et al. (2007), BMC Bioinformatics 8(1):460). Confidence for 100 bootstrap repetitions is indicated for major branching.

2. PIF3-like (Phytochrome Interacting Factor 3) polypeptides

Alignment of polypeptide sequences was performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing was done to further optimise the alignment. Minor manual editing was done to further optimise the alignment. The PIF3-like polypeptides are aligned in Figure 7.

A phylogenetic tree of PIF3-like polypeptides was constructed using MAFFT (Katoh and Toh (2008) Briefings in Bioinformatics 9:286-298) (Figure 8). A neighbour-joining tree was calculated using QuickTree (Howe et al. (2002), Bioinformatics 18(11): 1546-7), 100 bootstrap repetitions. The slanted cladogram was drawn using Dendroscope (Huson et al. (2007), BMC Bioinformatics 8(1):460). Confidence for 100 bootstrap repetitions is indicated for the major branches.

3. UROD (Uroporphyrinogen III decarboxylase) polypeptide

Alignment of polypeptide sequences was performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet (Blosum 62 for polypeptide alignment) gap opening penalty 10, gap extension penalty: 0.2). Minor manual editing was done to further optimise the alignment. An alignment of UROD polypeptides is shown in Figure 11.

A phylogenetic tree of UROD polypeptides (Figure 12 and 13) was constructed as described under the Description of Figures.

4. AS-MTT (Abiotic Stress Membrane Tethered Transcription factor) polypeptides

Alignment of polypeptide sequences was performed using the CLUSTAL 2.0.11 algorithm of progressive alignment (Thompson et al. (1997) Nucleic Acids Res 25:4876-4882; Chenna et al. (2003). Nucleic Acids Res 31:3497-3500) with standard setting (slow alignment, gap opening penalty 10, gap extension penalty: 0.2). Minor manual editing was done to further optimise the alignment. The AS-MTT polypeptides are aligned in Figure 16.

To perform phylogenetic analysis the polypeptides of Table A4 were aligned using MAFT (Katoh and Toh (2008). Briefings in Bioinformatics 9:286-298.). A neighbour-joining tree was calculated using QuickTree1.1 (Howe et al. (2002). Bioinformatics 18(11):1546-7). A cladogram was drawn using Dendroscope2.0.1 (Hudson et al. (2007). Bioinformatics 8(1):460). The polypeptide represented by SE QID NO: 2 clustered with the polypeptides of clade A. Polypeptides in clade B represent closer evolutionary related SEQ ID NO: 537 homologues.

Polypeptides falling within clade A are the following: P.trichocarpa_566293#1_A, P.trichocarpa_657115#1_A, P.trichocarpa_657115#1.2_A, P.persica_TC1247#1_A, M.domestica_TC12261#1_A, G.hirsutum_TC83567#1_A, A.thaliana_AT2G02730.1#1_A, A.thaliana_AT1G27000.1#1_A, M.domestica_TC9509#1_A, B.napus_TC66611#1_A, C.sinensis_TC3201#1_A, M.truncatula_AC136286_4.4#1_A.

Polypeptides falling within clade B are the following: S.officinarum_TC86637#1_B, P.americana_TA1256_3435#1_B, C.maculosa_TA1398_215693#1_B, O.sativa_LOC_Os01g08990.1 #1_B, O.sativa_LOC_Os05g08980.1#1_B, T.officinale_TA751_50225#1_B, F.arundinacea_TC6829#1_B, S.bicolor_Sb03g003440.1#1_B, I.nil_TC6724#1_B, S.tuberosum_TC170010#1_B, Aquilegia_sp_TC25212#1_B, V.vinifera_GSVIVT00029000001#1_B, H.annuus_TC32349#1_B.

5. EXO-1 polypeptide

The alignment was generated using MAFFT (Katoh and Toh (2008) Briefings in Bioinformatics 9:286-298). A phylogenetic tree of EXO-1 polypeptides (Figure 19) was constructed using a neighbour-joining tree, which was calculated using Quick-Tree (Howe et al. (2002), Bioinformatics 18(11): 1546-7), 100 bootstrap repetitions. The circular phylogram was drawn using Dendroscope (Huson et al. (2007), BMC Bioinformatics 8(1):460). Confidence for 100 bootstrap repetitions is indicated for major branching. Major branching position is indicated by circles.

6. YiAP2 polypeptides

A multiple alignment of YiAP2 polypeptides (Figure 22) was performed using default values (gap open penalty of 10, for the gap extension penalty of 0,1 and the selected weight matrix is Blosum 62) with the align algorithm as provided in the AlignX programme from the Vector NTI (Invitrogen), which is based on the ClustalW algorithm of progressive alignment (Thompson et al. (1997) Nucleic Acids Res 25:4876-4882; Chenna et al. (2003). Nucleic Acids Res 31:3497-3500).

*Example 3: Calculation of global percentage identity between polypeptide sequences useful in performing the methods of the invention*

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were: Scoring matrix: Blosum62, First Gap: 12, Extending Gap: 2.

1. FSM1-like (Fruit Sant/Myb) polypeptides

Results of the analysis are shown in Table B1 for the global similarity and identity over the full length of a number of FSM1-like polypeptide sequences. The sequence identity (in %) between the FSM1-like polypeptide sequences useful in performing the methods of the invention is generally 50% or higher compared to SEQ ID NO: 2, but can be as low as 19% within the group of FSM1-like polypeptides given in the sequence listing.

**Table B1:** MatGAT results for global similarity and identity over the full length of the polypeptide sequences.

| | 1 | 2 | 3 | 4 | 5 | 6 | 7 | 8 | 9 | 10 | 11 | 12 | 13 |
|---|---|---|---|---|---|---|---|---|---|---|---|---|---|
| 1. M.guttatus_DV207881 | | 59.4 | 55.7 | 59.0 | 61.2 | 57.4 | 52.9 | 57.8 | 60.5 | 63.9 | 62.8 | 62.7 | 63.9 |
| 2. A.majus_TA4522_4151 | 71.0 | | 54.2 | 58.3 | 58.1 | 69.3 | 68.6 | 59.8 | 58.3 | 61.3 | 63.9 | 61.2 | 61.9 |
| 3. S.lycopersicum_TA46686 | 70.5 | 73.1 | | 79.5 | 63.6 | 62.4 | 60.8 | 63.6 | 67.0 | 64.8 | 68.2 | 67.4 | 68.2 |
| 4. N.benthamiana_EH366122 | 74.7 | 73.1 | 86.4 | | 69.0 | 57.4 | 59.8 | 73.2 | 69.8 | 74.1 | 72.9 | 78.3 | 78.0 |
| 5. G.hirsutum_DR461014 | 77.1 | 72.0 | 72.7 | 80.2 | | 56.4 | 61.8 | 72.9 | 72.1 | 72.8 | 75.3 | 70.6 | 71.8 |
| 6. V.vinifera_EE072107 | 67.3 | 77.2 | 69.3 | 65.3 | 71.3 | | 77.5 | 63.4 | 65.3 | 59.4 | 68.3 | 63.4 | 64.4 |
| 7. M.domestica_DR994824 | 66.7 | 82.4 | 71.6 | 66.7 | 70.6 | 88.2 | | 68.6 | 69.9 | 61.8 | 72.8 | 69.6 | 71.6 |
| 8. G.hirsutum_TA31277_3635 | 72.3 | 73.1 | 76.1 | 84.0 | 87.7 | 70.3 | 73.5 | | 80.2 | 79.3 | 80.2 | 80.7 | 81.7 |
| 9. G.hirsutum_DW503354 | 76.7 | 74.2 | 78.4 | 79.1 | 84.9 | 74.3 | 77.5 | 86.0 | | 73.3 | 83.7 | 77.9 | 79.1 |
| 10. H.exilis_EE652824 | 77.1 | 71.0 | 73.9 | 80.2 | 80.2 | 66.3 | 66.7 | 86.3 | 81.4 | | 76.5 | 79.5 | 81.7 |
| 11. E.gunnii_CT985658 | 77.6 | 78.5 | 79.5 | 81.2 | 84.7 | 77.2 | 79.4 | 85.9 | 90.7 | 80.0 | | 84.9 | 84.9 |
| 12. L.sativa_TA7946_4236 | 75.9 | 78.5 | 77.3 | 84.3 | 86.7 | 74.3 | 75.5 | 91.6 | 88.4 | 84.3 | 91.8 | | 96.4 |
| 13. C.intybus_EH706852 | 75.9 | 77.4 | 77.3 | 84.1 | 87.8 | 75.2 | 76.5 | 91.5 | 89.5 | 85.4 | 90.6 | 96.4 | |
| 14. P.trichocarpa_sc_IV.1184 | 64.3 | 80.6 | 73.5 | 73.5 | 74.5 | 82.2 | 84.3 | 77.6 | 80.6 | 71.4 | 79.6 | 80.6 | 80.6 |
| 15. P.trichocarpa_557252 | 70.1 | 76.3 | 78.4 | 79.3 | 82.8 | 76.2 | 76.5 | 85.1 | 89.7 | 80.5 | 89.7 | 90.8 | 88.5 |
| 16. P.euphratica_AJ768574 | 71.3 | 77.4 | 79.5 | 80.5 | 83.9 | 77.2 | 77.5 | 86.2 | 90.8 | 79.3 | 90.8 | 92.0 | 89.7 |
| 17. M.truncatula_AC135566 | 65.9 | 82.8 | 73.6 | 72.5 | 71.4 | 72.3 | 75.5 | 71.4 | 72.5 | 67.0 | 71.4 | 72.5 | 72.5 |
| 18. G.max_Glyma06g46590.1 | 65.2 | 83.9 | 72.8 | 70.7 | 76.1 | 76.2 | 80.4 | 77.2 | 77.2 | 67.4 | 73.9 | 78.3 | 78.3 |
| 19. G.max_Glyma04g16390.1 | 66.3 | 83.9 | 75.0 | 70.7 | 75.0 | 78.2 | 82.4 | 77.2 | 78.3 | 69.6 | 75.0 | 79.3 | 79.3 |
| 20. G.max_Glyma02g18210.1 | 65.6 | 81.7 | 69.9 | 69.9 | 71.0 | 76.2 | 78.4 | 72.0 | 75.3 | 65.6 | 72.0 | 75.3 | 74.2 |
| 21. M.truncatula_AC157502 | 64.1 | 80.6 | 71.7 | 70.7 | 78.3 | 80.2 | 81.4 | 76.1 | 77.2 | 70.7 | 76.1 | 79.3 | 78.3 |
| 22. G.max_Glyma03g28050.1 | 64.9 | 80.4 | 69.1 | 68.0 | 70.1 | 81.2 | 80.4 | 71.1 | 74.2 | 70.1 | 76.3 | 77.3 | 76.3 |
| 23. A.thaliana_At4g39250 | 63.0 | 77.0 | 68.0 | 65.0 | 67.0 | 84.2 | 85.3 | 69.0 | 72.0 | 65.0 | 73.0 | 73.0 | 72.0 |
| 24. T.salsuginea_DN773374 | 58.0 | 76.0 | 65.0 | 67.0 | 66.0 | 81.2 | 84.3 | 69.0 | 73.0 | 64.0 | 70.0 | 69.0 | 71.0 |
| 25. B.napus_BN06MC20309 | 56.0 | 73.0 | 62.0 | 64.0 | 63.0 | 79.2 | 81.4 | 66.0 | 70.0 | 62.0 | 68.0 | 68.0 | 68.0 |
| 26. A.thaliana_At2g21650 | 57.4 | 78.2 | 65.3 | 66.3 | 66.3 | 82.2 | 84.3 | 68.3 | 72.3 | 64.4 | 69.3 | 70.3 | 69.3 |

|  | 14 | 15 | 16 | 17 | 18 | 19 | 20 | 21 | 22 | 23 | 24 | 25 | 26 |
|---|---|---|---|---|---|---|---|---|---|---|---|---|---|
| 1. M.guttatus_DV207881 | 52.0 | 56.3 | 57.5 | 54.7 | 52.6 | 53.7 | 51.6 | 55.3 | 51.5 | 49.0 | 43.0 | 43.0 | 44.6 |
| 2. A.majus_TA4522_4151 | 66.7 | 62.9 | 63.9 | 64.9 | 64.2 | 65.3 | 66.0 | 69.1 | 64.9 | 57.0 | 56.4 | 55.4 | 58.8 |
| 3. S.lycopersicum_TA46686 | 60.2 | 62.5 | 63.6 | 60.0 | 62.1 | 62.1 | 52.6 | 56.4 | 58.6 | 56.0 | 52.0 | 50.0 | 54.5 |
| 4. N.benthamiana_EH366122 | 64.3 | 67.8 | 69.0 | 56.4 | 55.9 | 57.0 | 53.2 | 58.1 | 60.2 | 55.0 | 53.0 | 51.0 | 56.4 |
| 5. G.hirsutum_DR461014 | 60.2 | 69.0 | 70.1 | 60.4 | 65.6 | 64.5 | 58.1 | 64.1 | 59.8 | 55.0 | 54.0 | 50.0 | 55.4 |
| 6. V.vinifera_EE072107 | 71.3 | 68.3 | 69.3 | 59.8 | 63.7 | 64.7 | 62.4 | 69.3 | 66.3 | 68.3 | 64.4 | 63.4 | 68.6 |
| 7. M.domestica_DR994824 | 74.5 | 70.6 | 71.6 | 65.0 | 71.8 | 69.9 | 61.8 | 69.6 | 69.2 | 70.6 | 66.7 | 63.7 | 70.6 |
| 8. G.hirsutum_TA31277_3635 | 68.4 | 79.3 | 80.5 | 58.9 | 69.1 | 68.1 | 57.9 | 64.9 | 62.6 | 61.0 | 60.0 | 54.0 | 59.4 |
| 9. G.hirsutum_DW503354 | 68.4 | 78.2 | 79.3 | 55.8 | 65.3 | 66.3 | 57.9 | 63.8 | 65.7 | 63.4 | 57.4 | 55.4 | 57.4 |
| 10. H.exilis_EE652824 | 67.3 | 74.7 | 73.6 | 58.2 | 55.9 | 57.0 | 51.6 | 58.7 | 61.9 | 55.0 | 52.0 | 49.0 | 54.5 |
| 11. E.gunnii_CT985658 | 73.5 | 83.9 | 85.1 | 60.6 | 65.6 | 64.5 | 58.5 | 67.7 | 68.4 | 65.3 | 56.4 | 55.4 | 60.4 |
| 12. L.sativa_TA7946_4236 | 71.4 | 79.5 | 80.7 | 58.9 | 62.1 | 61.1 | 55.8 | 64.9 | 64.6 | 62.4 | 55.0 | 53.5 | 59.4 |
| 13. C.intybus_EH706852 | 72.4 | 79.3 | 80.5 | 60.0 | 63.2 | 62.1 | 55.8 | 63.8 | 64.6 | 63.0 | 56.0 | 54.0 | 59.4 |
| 14. P.trichocarpa_sc_IV.1184 |  | 80.6 | 81.6 | 58.6 | 59.6 | 58.6 | 57.1 | 65.3 | 63.6 | 64.0 | 59.0 | 56.0 | 63.4 |
| 15. P.trichocarpa_557252 | 85.7 |  | 98.9 | 57.9 | 64.9 | 64.9 | 57.9 | 68.1 | 65.7 | 63.0 | 62.0 | 59.0 | 65.3 |
| 16. P.euphratica_AJ768574 | 86.7 | 98.9 |  | 58.9 | 66.0 | 64.9 | 58.9 | 67.0 | 64.6 | 64.0 | 61.0 | 58.0 | 64.4 |
| 17. M.truncatula_AC135566 | 74.5 | 71.4 | 72.5 |  | 80.6 | 79.6 | 72.3 | 72.0 | 73.5 | 58.4 | 60.4 | 60.4 | 59.8 |
| 18. G.max_Glyma06g46590.1 | 75.5 | 76.1 | 77.2 | 88.0 |  | 92.4 | 74.5 | 76.3 | 76.5 | 67.3 | 66.3 | 62.4 | 64.7 |
| 19. G.max_Glyma04g16390.1 | 76.5 | 78.3 | 78.3 | 90.2 | 96.7 |  | 77.7 | 77.4 | 75.5 | 67.3 | 66.3 | 62.4 | 65.7 |
| 20. G.max_Glyma02g18210.1 | 75.5 | 73.1 | 74.2 | 86.0 | 89.2 | 89.2 |  | 76.6 | 72.2 | 61.0 | 59.0 | 60.0 | 61.4 |
| 21. M.truncatula_AC157502 | 80.6 | 80.4 | 79.3 | 83.7 | 88.0 | 89.1 | 86.0 |  | 79.4 | 66.0 | 66.0 | 64.0 | 65.3 |
| 22. G.max_Glyma03g28050.1 | 79.6 | 77.3 | 76.3 | 82.5 | 87.6 | 89.7 | 85.6 | 90.7 |  | 65.7 | 62.7 | 61.8 | 64.1 |
| 23. A.thaliana_At4g39250 | 78.0 | 74.0 | 75.0 | 75.0 | 81.0 | 81.0 | 81.0 | 77.0 | 80.0 |  | 75.0 | 72.0 | 73.3 |
| 24. T.salsuginea_DN773374 | 77.0 | 74.0 | 73.0 | 77.0 | 80.0 | 82.0 | 78.0 | 79.0 | 81.0 | 87.0 |  | 88.0 | 89.1 |
| 25. B.napus_BN06MC20309 | 74.0 | 71.0 | 70.0 | 75.0 | 77.0 | 79.0 | 75.0 | 76.0 | 78.0 | 84.0 | 97.0 |  | 83.2 |
| 26. A.thaliana_At2g21650 | 76.2 | 74.3 | 73.3 | 73.3 | 77.2 | 80.2 | 78.2 | 78.2 | 80.2 | 84.2 | 94.1 | 91.1 |  |

## 2. PIF3-like (Phytochrome Interacting Factor 3) polypeptides

Results of the MatGat analysis are shown in Table B2 for the global similarity and identity over the full length of the polypeptide sequences. The sequence identity (in %) between the PIF3-like polypeptide sequences useful in performing the methods of the invention can be as low as 28.6 %, and does not go higher than 47.2 % when compared to SEQ ID NO: 293 (sequence 7, Os12g41650), which shows that outside of the conserved regions, there is little sequence conservation.

**Table B2:** MatGAT results for global similarity and identity over the full length of the polypeptide sequences.

|  | 1 | 2 | 3 | 4 | 5 | 6 | 7 | 8 | 9 | 10 |
|---|---|---|---|---|---|---|---|---|---|---|
| 1. AT2G43010_1 |  | 56.9 | 33.7 | 35.9 | 34.8 | 30.4 | 32.4 | 38.6 | 38.3 | 33.6 |
| 2. AT3G59060_1 | 72.9 |  | 31.6 | 36.1 | 34.5 | 28.9 | 31.2 | 38.0 | 36.5 | 34.0 |
| 3. Glyma02g45150_1 | 47.2 | 46.1 |  | 64.2 | 62.1 | 31.9 | 28.6 | 40.1 | 50.5 | 42.4 |
| 4. Glyma08g41620_1 | 51.4 | 49.2 | 73.1 |  | 82.9 | 34.5 | 33.0 | 43.1 | 53.2 | 44.4 |
| 5. Glyma18g14530_1 | 50.2 | 47.7 | 72.6 | 89.4 |  | 33.1 | 30.3 | 43.6 | 50.4 | 42.0 |
| 6. Os03g43810_1 | 44.5 | 42.7 | 43.1 | 48.4 | 46.3 |  | 47.2 | 28.9 | 34.7 | 32.4 |
| 7. Os12g41650_2 | 48.8 | 47.9 | 42.9 | 45.1 | 41.9 | 56.7 |  | 32.0 | 33.8 | 34.9 |

(continued)

| | 1 | 2 | 3 | 4 | 5 | 6 | 7 | 8 | 9 | 10 |
|---|---|---|---|---|---|---|---|---|---|---|
| 8. Pt_scaff_V_1141_1 | 53.6 | 56.9 | 52.0 | 58.2 | 57.7 | 44.1 | 49.1 | | 65.0 | 38.7 |
| 9. Pt_TC100024_1 | 51.1 | 52.1 | 63.5 | 66.6 | 66.0 | 46.6 | 45.8 | 71.1 | | 48.7 |
| 10. SI_TC192046_1 | 49.7 | 49.5 | 56.8 | 62.8 | 59.6 | 44.8 | 47.1 | 56.0 | 64.0 | |

## 3. UROD (Uroporphyrinogen III decarboxylase) polypeptide

Results of the software analysis are shown in Table B3 for the global similarity and identity over the full length of the polypeptide sequences. The sequence identity (in %) between the UROD polypeptide sequences useful in performing the methods of the invention can be as low as 24 % (but is generally higher than 30%) compared to SEQ ID NO: 368.

Table B3: MatGAT results for global similarity and identity over the full length of the polypeptide sequences.

| | 1 | 2 | 3 | 4 | 5 | 6 | 7 |
|---|---|---|---|---|---|---|---|
| 1. H.vulgare_TC175532#1_SPT_HEM1 | | 88.5 | 86 | 87 | 65.4 | 66.7 | 66 |
| 2. S.bicolor_Sb03g028330.1#1_SPT_HEM1 | | | 94.6 | 88.9 | 66.8 | 67.6 | 68.4 |
| 3. Z.mays_ZM07MC20070_BFb0115I19@20019#1_SPT_HEM1 | | | | 86.9 | 65.4 | 65.3 | 67 |
| 4. O.sativa_LOC_Os01g43390.1#1_SPT_HEM1 | | | | | 66.1 | 65.8 | 68 |
| 5. A.thaliana_AT3G14930.1#1_SPT_HEM1 | | | | | | 89.8 | 71.5 |
| 6. B.napus_TC76241#1_SPT_HEM1 | | | | | | | 73 |
| 7. M.truncatula_AC119409_9.4#1_SPT_HEM1 | | | | | | | |
| 8. G.max_Glyma12g35880.2#1_SPT_HEM1 | | | | | | | |
| 9. G.max_Glyma13g34500.2#1_SPT_HEM1 | | | | | | | |
| 10. P.trichocarpa_URO-D_Poplus_trichocarpa_SPT_HEM1 | | | | | | | |
| 11. V.vinifera_GSVIVT00027816001#1_SPT_HEM1 | | | | | | | |
| 12. Aquilegia_sp_TC24505#1_SPT_HEM1 | | | | | | | |
| 13. S.lycopersicum_TC195523#1_SPT_HEM1 | | | | | | | |
| 14. C.reinhardtii_186446#1_CHL_HEM1 | | | | | | | |
| 15. C.vulgaris_61627#1_CHL_HEM2 | | | | | | | |
| 16. Chlorella_32323#1_CHL_HEM2 | | | | | | | |
| 17. O.lucimarinus_17656#1_CHL_HEM2 | | | | | | | |
| 18. O.taurii_13162#1_CHL_HEM2 | | | | | | | |
| 19. A.thaliana_AT2G40490.1#1_SPT_HEM2 | | | | | | | |
| 20. B.napus_TC68105#1_SPT_HEM2 | | | | | | | |
| 21. C.sinensis_TC316#1_SPT_HEM2 | | | | | | | |

| | | | | | | | |
|---|---|---|---|---|---|---|---|
| 22. G.hirsutum_TC101219#1_SPT_HEM2 | | | | | | | |
| 23. G.max_Glyma18g02490.1#1_SPT_HEM2 | | | | | | | |
| 24. M.truncatula_AC149472_12.4#1_SPT_HEM2 | | | | | | | |
| 25. M.domestica_TC16580#1_SPT_HEM2 | | | | | | | |
| 26. N.tabacum_TC14277#1_SPT_HEM2 | | | | | | | |
| 27. S.lycopersicum_TC209939#1_SPT_HEM2 | | | | | | | |
| 28. O.basilicum_TA2234_39350#1_SPT_HEM2 | | | | | | | |
| 29. O.sativa_LOC_Os03g21900.1#1_SPT_HEM2 | | | | | | | |
| 30. S.bicolor_Sb01g036030.1#1_SPT_HEM2 | | | | | | | |
| 31. P.taeda_TA15428_3352#1_SPT_HEM2 | | | | | | | |
| 32. P.patens_188035#1_SPT_HEM2 | | | | | | | |
| 33. S.moellendorffii_438672#1_SPT_HEM2 | | | | | | | |
| 34. Cyanothece_sp._YP_001804380.1_Cyanothece_sp._BAC_Cyano | | | | | | | |
| 35. Synechocystis_sp._NP_442753.1_Synechocystis_sp._BAC_Cyano | | | | | | | |
| 36. Cyanothece_sp._YP_002373141.1_Cyanothece_sp._BAC_Cyano | | | | | | | |
| 37. Cyanothece_sp._YP_002377951.1_BAC_Cyano | | | | | | | |
| 38. M.aeruginosa_YP_001658143.1_Microcystis_aeruginosa_BAC_Cyano | | | | | | | |
| 39. Synechococcus_sp._YP_001734083.1_Synechococcus_sp._BAC_Cyano | | | | | | | |
| 40. T.erythraeum_YP_721451.1_Trichodesmium_erythraeum_BAC_Cyano | | | | | | | |
| 41. P.marinus_YP_001014466.1_Prochlorococcus_marinus_BAC_Cyano | | | | | | | |
| 42. P.marinus_YP_291214.1_Prochlorococcus_marinus_BAC_Cyano | | | | | | | |
| 43. Synechococcus_sp._YP_376928.1_Synechococcus_sp._BAC_Cyano | | | | | | | |
| 44. C.reinhardtii_195818#1_CHL_Others | | | | | | | |

| | | | | | | | |
|---|---|---|---|---|---|---|---|
| 45. C.vulgaris_25726#1_CHL_Others | | | | | | | |
| 46. O.lucimarinus_28976#1_CHL_Others | | | | | | | |
| 47. O.RCC809_53774#1_CHL_Others | | | | | | | |
| 48. O.taurii_14772#1_CHL_Others | | | | | | | |
| 49. P.patens_112870#1_SPT_Others | | | | | | | |
| 50. A.anophagefferens_30614#1_STR_Others | | | | | | | |
| 51. P.tricornutum_20757#1_STR_Others | | | | | | | |
| 52. T.pseudonana_3974#1_STR_Others | | | | | | | |
| 53. A.fumigatus_XP_750255.1_Aspergillus_fumigatus_FUNGI_Non-P | | | | | | | |
| 54. A.terreus_XP_001211750.1_Aspergillus_terreus_FUNGI_Non-P | | | | | | | |
| 55. G.zeae_XP_381537.1_Gibberella_zeae_FUNGI_Non-P | | | | | | | |
| 56. A.gossypii_NP_986457.1_AGL210Cp_Ashbya_gossypii_FUNGI_Non-P | | | | | | | |
| 57. C.glabrata_XP_447163.1_Candida_glabrata_FUNGI_Non-P | | | | | | | |
| 58. C.albicans_EAK98116.1_Candida_albicans_FUNGI_Non-P | | | | | | | |
| 59. D.hansenii_XP_460636.1_Debaryomyces_hansenii_FUNGI_Non-P | | | | | | | |
| 60. P.stipitis_XP_001384951.1_Pichia_stipitis_FUNGI_Non-P | | | | | | | |
| 61. S.pombe_NP_588085.2_Schizosaccharomyces_pombe_FUNGI_Non-P | | | | | | | |
| 62. D.melanogasterNP_610501.1_Drosophila_melanogaster_INSECT_Non-P | | | | | | | |
| 63. D.simulans_XP_002080776.1_GD10665_Drosophila_simulans_INSECT_Non-P | | | | | | | |
| 64. Drosophila_yakuba_XP_002089810.1_Drosophila_yakuba_INSECT_Non-P | | | | | | | |
| 65. D.pseudoobscura_XP_001361569.2_Drosophila_pseudoobscura_pseudoobscura_INSECT_Non-P | | | | | | | |
| 66. T.castaneum_XP_972457.1_similar_to_Tribolium_castaneum_INSECT_Non-P | | | | | | | |

67. H.sapiens_NP_000365.3_Homo_sapiens_HUMAN_Non-P

68. D.discoideum_XP_629682.1_Dictyostelium_discoideum_PROTOZOA_Non-P

69. P.berghei_CAH96991.1_putative_Plasmodium_berghei_PROTOZOA_Non-P

70. P.chabaudi_CAH75805.1_putative_Plasmodium_chabaudi_chabaudi_PROTOZOA_Non-P

71. P.falciparum_XP_966063.1_Plasmodium_falciparum_PROTOZOA_Non-P

72. Plasmodium_vivax_XP_001616097.1_Plasmodium_vivax_PROTOZOA_Non-P

| 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 |
|---|---|---|---|---|---|---|---|---|---|---|---|---|---|---|---|---|---|---|---|---|---|---|---|---|
| 66.6 | 55.6 | 66.7 | 67 | 66.4 | 64.2 | 55.1 | 46 | 47.1 | 44.3 | 45 | 48.8 | 47.8 | 47 | 50.5 | 49.3 | 47.8 | 50.4 | 48.3 | 49.2 | 47.8 | 47.7 | 48.4 | 48.3 | 47.4 |
| 68.3 | 56.9 | 68 | 67.7 | 67.6 | 64.9 | 54.3 | 46.2 | 47.6 | 44.2 | 44.9 | 48.2 | 49.3 | 47.2 | 49.6 | 49.6 | 49.3 | 50.5 | 49.3 | 49.4 | 48.2 | 49.5 | 49.4 | 48 | 47.9 |
| 67.4 | 56.5 | 68 | 67.2 | 67.4 | 63 | 54.3 | 46.6 | 48 | 44.4 | 45.1 | 48.2 | 49.5 | 46.9 | 49.8 | 49 | 48.5 | 49.8 | 48.5 | 49.5 | 48.3 | 49.1 | 48.7 | 48 | 47.7 |
| 66.6 | 56 | 67.2 | 66.3 | 69.1 | 65.9 | 56.3 | 46.4 | 47.3 | 45.7 | 46.4 | 49.4 | 50 | 48.1 | 50.6 | 49.3 | 48.9 | 49.9 | 49.3 | 50.7 | 48.9 | 48.9 | 48.9 | 49.5 | 47.2 |
| 74.5 | 59.1 | 75 | 72.9 | 72.1 | 71.1 | 53.6 | 44.4 | 45.8 | 42.7 | 43.4 | 47.1 | 47 | 48.7 | 47.5 | 46.3 | 46.1 | 47.3 | 48.4 | 49.2 | 47.3 | 46.8 | 46.2 | 46.5 | 48.7 |
| 74.7 | 59.2 | 75.5 | 71.9 | 72.8 | 72.2 | 54 | 44.3 | 45 | 43.3 | 43.1 | 48 | 48.1 | 48.8 | 47.8 | 47.2 | 45.9 | 47.8 | 47.8 | 48.6 | 47.4 | 47.4 | 46.9 | 46.4 | 49.5 |
| 85.2 | 63.7 | 76.4 | 74.2 | 74.6 | 74.5 | 54.3 | 46.8 | 43.9 | 42.2 | 42.6 | 47.7 | 48.8 | 47.8 | 48.4 | 50 | 49.2 | 48.9 | 49.1 | 50.1 | 48.8 | 48 | 47.7 | 48.2 | 47.9 |
|  | 71.9 | 79.4 | 78.2 | 75.4 | 72.5 | 54 | 46.6 | 44.4 | 42 | 42.7 | 46.3 | 48.1 | 47.9 | 48.4 | 48.3 | 47.8 | 46.9 | 48.3 | 47.8 | 48.8 | 47.3 | 47.5 | 47.8 | 47.4 |
|  |  | 62 | 63.8 | 58.1 | 55.6 | 42.8 | 40.1 | 34.7 | 38.6 | 39.7 | 36.3 | 37.3 | 37.6 | 37 | 38 | 37.5 | 36.7 | 37.6 | 38.1 | 37.5 | 37.4 | 36.6 | 37.1 | 35.1 |
|  |  |  | 82.2 | 76.1 | 75.4 | 52.6 | 46.8 | 43.8 | 42.4 | 42.2 | 47.7 | 48.3 | 47.3 | 47.6 | 47.8 | 48.1 | 46.4 | 47.5 | 46.6 | 49.8 | 47.4 | 48.2 | 47.7 | 46.7 |
|  |  |  |  | 77.3 | 73.2 | 52.8 | 46.3 | 43.9 | 42.3 | 42.3 | 47.7 | 47.8 | 46.6 | 46.6 | 47.4 | 47.2 | 47.3 | 48.6 | 48 | 48.6 | 46.6 | 47.3 | 46.6 | 44.6 |
|  |  |  |  |  | 75.8 | 54.7 | 46.1 | 44.7 | 43.5 | 44.4 | 48.1 | 49 | 48.6 | 48.8 | 47.6 | 48.6 | 48.3 | 50.5 | 50 | 48.1 | 46.7 | 47.1 | 48.4 | 48.2 |
|  |  |  |  |  |  | 52.9 | 44.2 | 44.1 | 41.6 | 41.8 | 46.2 | 46.3 | 46.3 | 46.3 | 46.6 | 46.6 | 45.6 | 47.2 | 48.2 | 48 | 45.9 | 45.8 | 46.1 | 46.5 |
|  |  |  |  |  |  |  | 50.1 | 50.6 | 48.1 | 49.6 | 50.2 | 50.8 | 52.4 | 51.9 | 52.4 | 51 | 52.5 | 51.8 | 52.5 | 52.7 | 53.1 | 51.7 | 51.1 | 50.6 |
|  |  |  |  |  |  |  |  | 61.4 | 59.8 | 60.6 | 58.8 | 59.3 | 59.3 | 59.4 | 59.4 | 57.5 | 57.8 | 58.3 | 59.5 | 59.9 | 58.2 | 57.6 | 59.8 | 59.3 |
|  |  |  |  |  |  |  |  |  | 52.5 | 53 | 55.4 | 54.9 | 55.6 | 56.3 | 55.1 | 55 | 54.9 | 55.1 | 56.3 | 54.3 | 57.6 | 56.1 | 54.5 | 58.4 |
|  |  |  |  |  |  |  |  |  |  | 92.3 | 55.3 | 56 | 55.4 | 55.3 | 53.5 | 52.7 | 54 | 54 | 53.6 | 54.8 | 53.8 | 53.3 | 55.2 | 54.5 |
|  |  |  |  |  |  |  |  |  |  |  | 54.3 | 55 | 55.2 | 55.3 | 54.3 | 53.5 | 54.8 | 54.5 | 54.4 | 55.6 | 54.9 | 54.3 | 54.7 | 54.3 |
|  |  |  |  |  |  |  |  |  |  |  |  | 93.7 | 78.2 | 78.7 | 78.5 | 76 | 78.5 | 77.2 | 77.9 | 76.6 | 74.4 | 74.9 | 71.9 | 66.4 |
|  |  |  |  |  |  |  |  |  |  |  |  |  | 78.4 | 79.5 | 80.1 | 77.2 | 79 | 78.8 | 79.5 | 77 | 74.5 | 74.9 | 72.3 | 65.7 |
|  |  |  |  |  |  |  |  |  |  |  |  |  |  | 84.8 | 83.5 | 79.9 | 82.3 | 81.1 | 81.7 | 81.7 | 77.5 | 74.4 | 74.4 | 65.7 |
|  |  |  |  |  |  |  |  |  |  |  |  |  |  |  | 84.4 | 81.2 | 82.9 | 82.6 | 84.4 | 82.7 | 75.7 | 76.2 | 73.3 | 66.7 |
|  |  |  |  |  |  |  |  |  |  |  |  |  |  |  |  | 87.9 | 82.6 | 81.6 | 82.5 | 81.1 | 77 | 75.6 | 71.9 | 65.2 |
|  |  |  |  |  |  |  |  |  |  |  |  |  |  |  |  |  | 80.6 | 80.2 | 82.3 | 82.8 | 75.2 | 73.6 | 71.1 | 62.8 |
|  |  |  |  |  |  |  |  |  |  |  |  |  |  |  |  |  |  | 80.7 | 81.3 | 81.3 | 77.8 | 76.7 | 73.9 | 66.2 |
|  |  |  |  |  |  |  |  |  |  |  |  |  |  |  |  |  |  |  | 90.8 | 83.9 | 76.5 | 75.4 | 73.4 | 66 |
|  |  |  |  |  |  |  |  |  |  |  |  |  |  |  |  |  |  |  |  | 84 | 76.8 | 74.9 | 73.1 | 66.7 |
|  |  |  |  |  |  |  |  |  |  |  |  |  |  |  |  |  |  |  |  |  | 76.1 | 74.7 | 73.7 | 65.9 |
|  |  |  |  |  |  |  |  |  |  |  |  |  |  |  |  |  |  |  |  |  |  | 91.6 | 73 | 64.7 |
|  |  |  |  |  |  |  |  |  |  |  |  |  |  |  |  |  |  |  |  |  |  |  | 72.4 | 65.2 |
|  |  |  |  |  |  |  |  |  |  |  |  |  |  |  |  |  |  |  |  |  |  |  |  | 65.2 |

| 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 |
|---|---|---|---|---|---|---|---|---|---|---|---|---|---|---|---|---|---|---|---|---|---|---|---|---|
| 46.4 | 45.8 | 44.3 | 46.8 | 46.8 | 45.8 | 45.5 | 48.3 | 46 | 45.5 | 45.8 | 42.4 | 44.2 | 41.9 | 43.1 | 41.4 | 40.2 | 39.9 | 37.4 | 36.1 | 29 | 30 | 29.4 | 28.6 | 27.9 |
| 46.6 | 45.9 | 43.7 | 45.9 | 47.4 | 45.9 | 45.4 | 47.9 | 46.4 | 45.9 | 47.2 | 42 | 43.3 | 41.4 | 43.6 | 41.1 | 41.1 | 39 | 36.9 | 36.1 | 29.7 | 31.8 | 30.3 | 28.3 | 28.1 |
| 46.1 | 46.6 | 43.9 | 46.4 | 47.1 | 46.4 | 44.6 | 48.1 | 46.4 | 45.9 | 46.4 | 42.2 | 43.6 | 41.5 | 42.8 | 41.4 | 41.2 | 39.8 | 36.7 | 36.6 | 30 | 31 | 31.1 | 28.3 | 27.8 |
| 47.1 | 47.1 | 44.2 | 46.9 | 47.4 | 45.9 | 45.7 | 48.1 | 46.7 | 46.2 | 47.1 | 42.8 | 42.5 | 42.1 | 43.8 | 42.6 | 40.6 | 40 | 37.8 | 35.9 | 30.3 | 31.9 | 32.6 | 29.4 | 28.4 |
| 45.6 | 46.3 | 44.9 | 46.3 | 46.8 | 45.6 | 45.1 | 45.1 | 44.6 | 44.2 | 43.9 | 41.9 | 43.2 | 40.2 | 41.7 | 39.8 | 40 | 40 | 34.5 | 33.6 | 27.9 | 28.7 | 27.3 | 26.7 | 26 |
| 46.1 | 46.2 | 45 | 45.5 | 46.9 | 46.2 | 45.7 | 45.2 | 44.7 | 44.3 | 45 | 40.6 | 43 | 41.1 | 41.3 | 40.1 | 39.9 | 40 | 36.5 | 34.9 | 27.8 | 29.9 | 28 | 27.7 | 26.3 |

| | | | | | | | | | | | | | | | | | | | | | | | | |
|---|---|---|---|---|---|---|---|---|---|---|---|---|---|---|---|---|---|---|---|---|---|---|---|---|
| 47.2 | 46.1 | 45.1 | 46.1 | 47.5 | 45.8 | 46.8 | 48 | 47.2 | 47.4 | 48.8 | 43.2 | 41.6 | 41.1 | 40.8 | 40.6 | 40.6 | 40.3 | 36.9 | 36.5 | 28.3 | 28.5 | 27.5 | 26.7 | 26.2 |
| 47.7 | 46.3 | 46.1 | 46.3 | 46.8 | 45.4 | 46.8 | 47.3 | 46.8 | 46.8 | 48 | 44.8 | 42.7 | 40.5 | 40.8 | 40.4 | 40.6 | 40 | 35.9 | 35.6 | 28.1 | 29.6 | 28.3 | 27.5 | 25.6 |
| 36.4 | 41.2 | 40.4 | 41.2 | 41.9 | 40.7 | 40.9 | 43.1 | 42.3 | 42.5 | 43.4 | 35.8 | 35.6 | 31.7 | 34 | 32.2 | 33.6 | 31.9 | 25.1 | 26.7 | 25.6 | 23.8 | 25.8 | 23.8 | 23.2 |
| 44.7 | 45.6 | 44.6 | 45.3 | 46.1 | 45.1 | 45.8 | 47.1 | 46.3 | 46.3 | 47.1 | 43 | 43.1 | 41.7 | 40.6 | 40.6 | 40.8 | 40.2 | 33.3 | 33.3 | 27.3 | 29.3 | 27.7 | 26.7 | 25.2 |
| 45.5 | 47 | 44.8 | 45.8 | 47.5 | 44.8 | 46 | 48 | 46.8 | 46.8 | 47 | 42.2 | 42.5 | 42.3 | 41.2 | 41.7 | 40.5 | 39.3 | 33.8 | 35 | 29.2 | 29.3 | 28.6 | 27.3 | 27.3 |
| 47.3 | 45.9 | 44.7 | 44.9 | 46.4 | 44.2 | 45.4 | 45.9 | 46.4 | 45.9 | 46.4 | 41.6 | 42.2 | 41.9 | 41.4 | 41.4 | 39.7 | 39.3 | 34.2 | 35.9 | 27.9 | 28.8 | 28.3 | 26.8 | 26.8 |
| 45.8 | 44.2 | 44.2 | 44.9 | 45.1 | 42.8 | 44.7 | 44.9 | 45.6 | 45.1 | 46.6 | 40.6 | 39.8 | 40.4 | 39.8 | 41 | 38.5 | 39 | 35.1 | 36 | 28.6 | 29 | 27.4 | 25.6 | 25.3 |
| 51.4 | 49.4 | 49.1 | 49.6 | 50.6 | 48.1 | 49.9 | 47.8 | 47.3 | 47.3 | 49.1 | 41.8 | 43.6 | 45.9 | 44.7 | 43.7 | 41.1 | 41.2 | 39.2 | 39.2 | 32.6 | 34.6 | 33 | 31.9 | 29.5 |
| 61.5 | 59.9 | 58.7 | 58 | 58.4 | 57 | 58.4 | 56.6 | 55 | 55 | 57.7 | 39.3 | 41.3 | 41.4 | 43.4 | 42.1 | 40.9 | 41.7 | 36.1 | 35.1 | 31.8 | 30.7 | 32.3 | 30.8 | 30 |
| 56.8 | 50.4 | 49.5 | 47.1 | 50.8 | 50.8 | 50.3 | 47.9 | 49.6 | 49.4 | 50.4 | 41.7 | 43.4 | 43.2 | 44.8 | 41.6 | 41.2 | 42.1 | 38.2 | 38.4 | 31.1 | 31.4 | 30.1 | 30 | 29.6 |
| 55 | 54.8 | 55.7 | 53.7 | 56.4 | 55.4 | 55.1 | 54.2 | 52.6 | 52.3 | 55.1 | 41 | 41.3 | 43.9 | 46.6 | 43.7 | 39.2 | 41.3 | 35 | 36.1 | 33.2 | 30.8 | 31.1 | 32.9 | 32.7 |
| 54.5 | 54.2 | 55.1 | 53.4 | 55 | 53.7 | 54 | 53.1 | 51.4 | 51.1 | 53.1 | 41.5 | 41.8 | 42.2 | 45.2 | 42.4 | 39.7 | 41 | 35.6 | 35.9 | 33.4 | 31.8 | 31.9 | 34.2 | 34.3 |
| 67.8 | 54.3 | 52.3 | 52.3 | 52.6 | 52.5 | 53 | 53.3 | 52 | 51.8 | 54 | 41.7 | 41.7 | 43 | 43.2 | 43.8 | 42.2 | 41.7 | 37.5 | 38.6 | 32.6 | 33.7 | 31.4 | 32.6 | 31.2 |
| 68.6 | 54.9 | 52.9 | 52.9 | 53 | 52.7 | 53.7 | 53.9 | 52.9 | 52.6 | 54.7 | 41.6 | 41.9 | 43.9 | 43.3 | 43.1 | 42.6 | 41.3 | 37 | 39 | 33.1 | 33.4 | 31.9 | 33.1 | 31.7 |
| 68.7 | 56.4 | 54.9 | 53.8 | 55 | 54.4 | 57 | 53.1 | 53.3 | 53.3 | 55.1 | 41.1 | 42.9 | 45.1 | 45.3 | 43.5 | 43.9 | 41.8 | 36.6 | 37.9 | 32.8 | 34 | 30.8 | 32.8 | 32.8 |
| 68 | 56 | 55 | 52.9 | 53.8 | 53.7 | 56 | 53.2 | 53.4 | 53.2 | 56 | 41.5 | 42.4 | 43.7 | 45.5 | 43.4 | 42.8 | 43 | 38.4 | 39.4 | 32.7 | 33.8 | 32 | 33.3 | 33 |
| 66.9 | 55.5 | 55 | 53.4 | 54.1 | 52.7 | 55.8 | 52.9 | 54.2 | 54.2 | 55.5 | 42.5 | 42.1 | 43.3 | 44.4 | 44 | 43.3 | 41 | 36.6 | 39.9 | 33.2 | 33.5 | 32.3 | 34 | 33.3 |
| 66.5 | 54.7 | 54.2 | 53.2 | 53.3 | 52.2 | 54.5 | 50.4 | 53.7 | 53.9 | 54.2 | 42.5 | 42.9 | 43.1 | 44 | 45.1 | 42.6 | 41 | 37 | 39.9 | 33.7 | 34.8 | 32.6 | 33.6 | 33.3 |
| 67.2 | 54.2 | 52.5 | 51.5 | 52.9 | 52.3 | 52.5 | 51.5 | 52 | 52.2 | 53.2 | 42.8 | 43.3 | 44.1 | 44.7 | 43.8 | 43.6 | 40.5 | 37 | 40.4 | 33.2 | 34 | 32 | 33.5 | 33.3 |
| 66.2 | 53.7 | 53.7 | 53.7 | 52.8 | 51.9 | 53.9 | 52.4 | 53.7 | 53.4 | 54.7 | 41.8 | 42.6 | 43.9 | 45.3 | 43.9 | 41.7 | 41.5 | 38 | 40 | 34.1 | 35.8 | 31.4 | 33.2 | 33.4 |
| 66.9 | 55.1 | 54.4 | 53.3 | 53.7 | 52.6 | 54.9 | 52.5 | 55.3 | 55.1 | 55.6 | 41.6 | 41.4 | 42.7 | 43.4 | 42.7 | 42.7 | 41.1 | 38 | 38.9 | 34.7 | 34.4 | 31.9 | 33.4 | 33.4 |
| 68.3 | 54.5 | 54.5 | 53.7 | 54.6 | 54.2 | 55.3 | 53.4 | 54.5 | 55 | 55.5 | 42.2 | 41.7 | 43.9 | 45.7 | 44.7 | 43.1 | 41.6 | 37.2 | 40.1 | 35.4 | 35.6 | 32 | 33 | 33.5 |

| | | | | | | | | | | | | | | | | | | | | | | | | |
|---|---|---|---|---|---|---|---|---|---|---|---|---|---|---|---|---|---|---|---|---|---|---|---|---|
| 66.3 | 54.5 | 52.8 | 52.5 | 53.9 | 53.1 | 53.8 | 52.8 | 54 | 54 | 53.5 | 40.4 | 41.3 | 42.7 | 44.9 | 42.5 | 43.2 | 41.4 | 37 | 40.1 | 31.4 | 32.5 | 31.8 | 31.8 | 33 |
| 66.8 | 54.3 | 52.8 | 53 | 53.9 | 51.5 | 53.5 | 54.3 | 52.3 | 52.3 | 53 | 42 | 42.3 | 41.5 | 43.9 | 41 | 41.7 | 42 | 38.2 | 40 | 32.1 | 33.1 | 31.5 | 31.7 | 32.2 |
| 69.8 | 54.9 | 53.9 | 52.4 | 54.2 | 53.9 | 55.7 | 54.4 | 53.4 | 53.4 | 54.4 | 42.5 | 42.4 | 44.6 | 46.5 | 44.2 | 42.1 | 43.3 | 38.4 | 41.4 | 33.1 | 34.2 | 32.2 | 30.9 | 32.2 |
| 69.6 | 56.8 | 55.2 | 53.5 | 55.5 | 53.8 | 54.5 | 51.6 | 50.8 | 50.6 | 52.5 | 41 | 43 | 44.4 | 43.6 | 43.4 | 39.7 | 44.8 | 39 | 39 | 30.8 | 31.6 | 30.3 | 31.8 | 30.2 |
|  | 59.4 | 56.7 | 57.9 | 55.5 | 55.9 | 57 | 54.6 | 53.1 | 53.1 | 54.6 | 43.8 | 43.2 | 45.8 | 47 | 46.2 | 43.2 | 44.8 | 38.4 | 41.8 | 32.9 | 34 | 31.8 | 32.8 | 31.8 |
|  |  | 86.4 | 86.2 | 83.6 | 80.5 | 82.2 | 74 | 69.2 | 69.2 | 69.2 | 46.5 | 47.8 | 48.7 | 51.3 | 47.7 | 48.7 | 44.1 | 40.4 | 43.2 | 33 | 31.3 | 33.4 | 33 | 32.8 |
|  |  |  | 81.6 | 82.7 | 82.9 | 83.4 | 72.9 | 68.6 | 68.6 | 68.9 | 47 | 47.3 | 47.5 | 50.9 | 46.2 | 45.6 | 45 | 38.9 | 42.8 | 34.2 | 32.2 | 33.3 | 31.4 | 33.1 |
|  |  |  |  | 80.8 | 78.5 | 76.3 | 72.6 | 66.7 | 66.7 | 65 | 46.8 | 47.3 | 47.2 | 48.7 | 44.7 | 47.7 | 42.5 | 39.2 | 42.3 | 32.2 | 30.6 | 31.5 | 31.6 | 30.9 |
|  |  |  |  |  | 87 | 79.9 | 72 | 66.7 | 66.7 | 67.2 | 44 | 46.6 | 46.2 | 49.2 | 42.9 | 46.1 | 44.1 | 39.3 | 41.4 | 32.2 | 31.2 | 32.7 | 32.3 | 32.1 |
|  |  |  |  |  |  | 80 | 70.6 | 65.5 | 65.8 | 66.7 | 44.5 | 46.9 | 45.5 | 48.4 | 42.9 | 45.9 | 45.4 | 39.8 | 42.8 | 33.1 | 30.7 | 32.7 | 31.4 | 32.6 |
|  |  |  |  |  |  |  | 71.5 | 68.1 | 68.1 | 68.9 | 47.3 | 46.9 | 46.7 | 48.7 | 46.2 | 45.9 | 46.7 | 41.2 | 43.3 | 32.8 | 31.2 | 33.2 | 33.3 | 32.9 |
|  |  |  |  |  |  |  |  | 63.3 | 63.3 | 65.8 | 45.5 | 44.4 | 44.9 | 46.8 | 42.9 | 44.8 | 45.2 | 38.8 | 42.3 | 33.8 | 30.3 | 30.5 | 31.4 | 32.2 |
|  |  |  |  |  |  |  |  |  | 98.6 | 81.8 | 41.5 | 44 | 42.7 | 43.7 | 41.7 | 43.1 | 44.8 | 35.1 | 38.3 | 35.9 | 32.3 | 33.2 | 31.9 | 34.1 |
|  |  |  |  |  |  |  |  |  |  | 81.8 | 41.5 | 43.7 | 42.7 | 43.1 | 41.7 | 43.1 | 44.6 | 34.9 | 38.1 | 35.4 | 32.1 | 32.9 | 31.4 | 33.8 |
|  |  |  |  |  |  |  |  |  |  |  | 44.3 | 44.4 | 44.2 | 45.2 | 41.9 | 43.8 | 45.8 | 35.6 | 38.6 | 34.6 | 31.8 | 33.9 | 32.7 | 32.5 |
|  |  |  |  |  |  |  |  |  |  |  |  | 58 | 55.8 | 55.9 | 57.6 | 57.9 | 42.4 | 42.6 | 45.1 | 31.6 | 32 | 30.1 | 30 | 29.2 |
|  |  |  |  |  |  |  |  |  |  |  |  |  | 56.2 | 58.1 | 54.9 | 57.3 | 40.1 | 38.9 | 43.7 | 29.7 | 29.3 | 27 | 30.4 | 28.1 |
|  |  |  |  |  |  |  |  |  |  |  |  |  |  | 84.1 | 82.7 | 58.9 | 39.8 | 45.5 | 47.2 | 30.5 | 31.3 | 28.9 | 29.8 | 30.8 |
|  |  |  |  |  |  |  |  |  |  |  |  |  |  |  | 79.5 | 60.9 | 42.3 | 42 | 48 | 32.1 | 32.3 | 31.5 | 32.5 | 34 |
|  |  |  |  |  |  |  |  |  |  |  |  |  |  |  |  | 58.7 | 40 | 43.5 | 47.9 | 30.5 | 33.4 | 29.7 | 30.5 | 31.6 |
|  |  |  |  |  |  |  |  |  |  |  |  |  |  |  |  |  | 36.2 | 41.8 | 43.9 | 30.4 | 31.4 | 29.8 | 31.2 | 31.7 |
|  |  |  |  |  |  |  |  |  |  |  |  |  |  |  |  |  |  | 38.2 | 40.3 | 29.7 | 29.9 | 29.8 | 30.6 | 30.1 |

| | | | | | |
|---|---|---|---|---|---|
| 67.8 | 26 | 28.2 | 26.6 | 26 | 26.2 |
| | 28.6 | 29.3 | 27.6 | 27.2 | 28.9 |
| | | 81.6 | 72.7 | 62.1 | 61.6 |
| | | | 67.6 | 58.2 | 56.9 |
| | | | | 60.6 | 61 |
| | | | | | 80.7 |

| 58 | 59 | 60 | 61 | 62 | 63 | 64 | 65 | 66 | 67 | 68 | 69 | 70 | 71 | 72 |
|---|---|---|---|---|---|---|---|---|---|---|---|---|---|---|
| 29 | 27.9 | 28.4 | 30.1 | 31.8 | 31.8 | 31.8 | 32 | 31.4 | 28.3 | 30.7 | 22.6 | 23.4 | 23.8 | 24.7 |
| 28.9 | 27.8 | 28.5 | 30.5 | 32.7 | 32.7 | 32.7 | 32.2 | 31.9 | 28.5 | 29.9 | 23.2 | 24.1 | 23.6 | 26 |
| 28 | 27.8 | 28.8 | 30.6 | 33.3 | 33.3 | 33.3 | 33 | 31.3 | 28.3 | 29.7 | 22.8 | 23.8 | 24.1 | 25.3 |
| 29.8 | 28.9 | 29.6 | 31.9 | 32.4 | 32.4 | 32.4 | 32.1 | 31.3 | 29.1 | 30 | 23.2 | 23.5 | 23.6 | 25.1 |
| 27.3 | 25.3 | 25.8 | 29.5 | 29.5 | 29.5 | 29.5 | 30.2 | 28.5 | 28.2 | 28.7 | 24.2 | 22.6 | 24.5 | 23.6 |
| 27.7 | 25.8 | 26.3 | 30.5 | 29.8 | 29.6 | 29.6 | 30.3 | 28.8 | 28.3 | 29.7 | 24.3 | 23.6 | 24.9 | 23.1 |
| 26.6 | 26.4 | 26.2 | 28.9 | 30.8 | 30.5 | 30.5 | 30.5 | 30 | 28.7 | 30.4 | 24.1 | 24.6 | 24 | 25.8 |
| 27.1 | 25.8 | 26.3 | 30.6 | 33.3 | 33 | 33.3 | 33 | 31.6 | 28.8 | 29.5 | 23.5 | 24.8 | 23.8 | 26.1 |
| 23.7 | 22.5 | 22.8 | 28.4 | 30 | 30 | 30 | 29.8 | 28.4 | 25.9 | 26.9 | 18.7 | 20.3 | 19 | 19.9 |
| 28.2 | 26.4 | 27.1 | 30.3 | 32 | 31.7 | 32 | 31.5 | 28.8 | 28.3 | 29.7 | 23.6 | 24.6 | 23.9 | 23.8 |
| 26.5 | 26.8 | 26.8 | 29.5 | 32.4 | 32.2 | 32.4 | 32.4 | 28.5 | 29.2 | 28.6 | 22.7 | 23.6 | 24.5 | 26.7 |
| 26.2 | 26.3 | 27 | 30.8 | 32 | 31.7 | 31.7 | 31.5 | 27.9 | 27.1 | 28.8 | 23.6 | 22.8 | 23.6 | 24.4 |
| 26.2 | 25.6 | 25.4 | 29.2 | 30.8 | 30.5 | 30.5 | 30.3 | 27.7 | 26.5 | 27.6 | 24.5 | 23.4 | 23.8 | 25.3 |
| 29.9 | 30 | 30.7 | 32.8 | 32.8 | 32.8 | 32.8 | 33 | 30.9 | 30.7 | 32.4 | 23.5 | 24.9 | 23.9 | 25.6 |
| 32.1 | 30 | 28.9 | 31.7 | 32.2 | 32.2 | 32.2 | 32.5 | 31.2 | 29.5 | 31.2 | 23.2 | 24.2 | 24.9 | 26 |
| 31.2 | 29.7 | 30 | 31.8 | 33.9 | 33.9 | 33.7 | 33.9 | 28.2 | 30.6 | 29.9 | 25.7 | 25.4 | 24.5 | 27.3 |
| 32.8 | 34.1 | 33 | 35.9 | 33.8 | 33.8 | 33.5 | 34.1 | 30.9 | 30.7 | 34 | 25 | 26.1 | 25.9 | 24.6 |
| 33.9 | 34.3 | 34.1 | 36 | 35.8 | 35.8 | 35.5 | 35.8 | 33.1 | 32.3 | 34 | 25.5 | 26.9 | 25.6 | 24.2 |
| 32.2 | 32.8 | 31.9 | 33.9 | 30.8 | 30.8 | 30.8 | 30.6 | 31.8 | 29.4 | 31.6 | 27.4 | 28.9 | 26.3 | 27.7 |
| 32.7 | 32.8 | 31.4 | 34.4 | 31.3 | 31.3 | 31.3 | 31.1 | 32 | 29.9 | 32.6 | 27.6 | 28.4 | 26.3 | 25.8 |

| | | | | | | | | | | | | | | |
|---|---|---|---|---|---|---|---|---|---|---|---|---|---|---|
| 31.4 | 31.8 | 31.3 | 34 | 32.7 | 32.7 | 32.7 | 32.7 | 32.7 | 31.3 | 34.5 | 24.1 | 25.7 | 25.4 | 27.7 |
| 32.5 | 32.7 | 32.4 | 33.4 | 32.6 | 32.6 | 32.6 | 32.3 | 32.3 | 28.9 | 32.9 | 26.2 | 26.3 | 27.3 | 25.9 |
| 32.3 | 33.9 | 33.2 | 33.8 | 31.6 | 31.6 | 31.6 | 31.9 | 33.1 | 29.9 | 33.2 | 28.1 | 26.7 | 29 | 25.9 |
| 31.6 | 33.3 | 32.5 | 33.8 | 31.5 | 31.5 | 31.5 | 31.2 | 33.2 | 30 | 32.5 | 25.8 | 26.1 | 27.6 | 25.7 |
| 32.8 | 32.9 | 32.7 | 33.5 | 31.2 | 31.2 | 31.2 | 31.4 | 32.1 | 29.5 | 31.4 | 26.5 | 26 | 25.9 | 27 |
| 32.4 | 33.5 | 32.8 | 33.4 | 32.6 | 32.3 | 32.3 | 31.6 | 31.7 | 31.3 | 34.1 | 26.7 | 27.3 | 26.9 | 26.8 |
| 32.7 | 33.5 | 32.8 | 34.2 | 32.2 | 32 | 32 | 31.7 | 32 | 30.8 | 33.3 | 27.4 | 28.7 | 26.9 | 26.2 |
| 33.5 | 33.1 | 32.2 | 35.3 | 33.1 | 33.1 | 33.1 | 32.3 | 31.3 | 31.4 | 33.4 | 26.2 | 27.1 | 27.6 | 26.3 |
| 34 | 33.2 | 32.2 | 33.7 | 31.4 | 31.4 | 31.4 | 31.1 | 32.1 | 30.6 | 32.7 | 25.5 | 26.1 | 26.4 | 28.4 |
| 32.9 | 32.4 | 31.1 | 32.4 | 30.6 | 30.6 | 30.6 | 30.3 | 31.8 | 30.3 | 32.3 | 25.3 | 27.3 | 26.8 | 27.1 |
| 32.9 | 32.8 | 32.1 | 35.1 | 31.8 | 31.8 | 31.8 | 31.8 | 30.7 | 29.1 | 31.6 | 26.9 | 27.3 | 27.4 | 27.8 |
| 29.8 | 29.8 | 29.3 | 31.4 | 30.8 | 30.5 | 30.5 | 30 | 29.8 | 30.6 | 32 | 26.7 | 26.3 | 24.6 | 28.5 |
| 32.6 | 33.4 | 32.7 | 36 | 33.6 | 33.6 | 33.6 | 33.3 | 33.1 | 31.1 | 32.4 | 25.3 | 25.8 | 25.2 | 27.5 |
| 34.3 | 32.2 | 32 | 38.4 | 34.1 | 33.8 | 34.1 | 34.3 | 31.8 | 34.2 | 33.1 | 23.8 | 25.8 | 25.1 | 25.6 |
| 34 | 32.7 | 32.4 | 37.7 | 33.6 | 33.3 | 33.6 | 33.6 | 33 | 33.4 | 34.7 | 23.7 | 25.8 | 23.9 | 23.6 |
| 32.5 | 31.8 | 31.8 | 35.9 | 33.2 | 33 | 33.2 | 33.5 | 32.3 | 33.9 | 34.7 | 25.9 | 27.4 | 25.4 | 26.4 |
| 33.3 | 31.3 | 32.5 | 37.4 | 33.1 | 32.8 | 33.1 | 33.9 | 32.7 | 33.4 | 34.2 | 23.4 | 24.8 | 24.6 | 26.1 |
| 33.9 | 32.6 | 32.6 | 36.6 | 33.6 | 33.3 | 33.6 | 34.4 | 33 | 32.9 | 34.2 | 24.5 | 25.5 | 24.9 | 25.6 |
| 34.5 | 32.7 | 33.7 | 38.7 | 34.2 | 33.9 | 34.2 | 34.4 | 33.2 | 32.3 | 35 | 23.3 | 25.6 | 24.9 | 25.3 |
| 32.2 | 31.4 | 32 | 36.4 | 32.7 | 32.4 | 32.7 | 32.4 | 32.3 | 32.2 | 33.1 | 26.3 | 26.8 | 26.5 | 25.4 |
| 34.1 | 32.5 | 33.3 | 37.9 | 34.3 | 34.3 | 34.3 | 34.1 | 32.3 | 32.2 | 32.1 | 26.8 | 27 | 27.7 | 25.5 |
| 33.8 | 32 | 32.8 | 37.9 | 34.1 | 34.1 | 34.1 | 33.8 | 32.3 | 32 | 31.8 | 26.8 | 27.3 | 27.5 | 25.8 |

| | | | | | | | | | | | | | | |
|---|---|---|---|---|---|---|---|---|---|---|---|---|---|---|
| 31.9 | 31.8 | 31.3 | 37.2 | 35.4 | 35.2 | 35.4 | 35.7 | 32.8 | 31.2 | 31.4 | 26 | 27.2 | 27.2 | 26.5 |
| 30.1 | 31.2 | 30 | 32 | 33.6 | 33.6 | 33.6 | 33.3 | 31.4 | 34.3 | 33.3 | 23.9 | 25.4 | 23.3 | 25.7 |
| 29.1 | 29.2 | 28.3 | 29.3 | 29.9 | 29.9 | 29.9 | 29.9 | 29.6 | 32.5 | 30.4 | 22.8 | 23.8 | 23.1 | 25.9 |
| 30.4 | 30.8 | 31 | 33.3 | 34 | 34 | 34 | 33.3 | 29.3 | 34.3 | 33.5 | 24 | 24.8 | 26.4 | 27.2 |
| 32.6 | 33.2 | 33 | 35.4 | 35.3 | 35.3 | 35.3 | 34 | 30.1 | 35.6 | 34.8 | 22.7 | 23.4 | 24.1 | 25.6 |
| 30.4 | 31.8 | 32.3 | 33.4 | 33.8 | 33.8 | 33.8 | 33.3 | 30.3 | 34.6 | 32 | 22.1 | 24.3 | 23.9 | 25.9 |
| 30.8 | 31.4 | 30.9 | 34.3 | 34.4 | 34.4 | 34.4 | 34.2 | 31.8 | 35.7 | 33.8 | 23.9 | 24.6 | 23.1 | 26.8 |
| 30.6 | 29.2 | 30 | 30 | 30.8 | 30.8 | 30.8 | 31.5 | 31.3 | 31.1 | 30.8 | 27.1 | 25.6 | 25.7 | 24.4 |
| 24 | 25.1 | 24.4 | 27.9 | 28.7 | 28.7 | 28.9 | 28.9 | 28.6 | 27.7 | 27.6 | 22.5 | 21.7 | 21.2 | 22.7 |
| 26.2 | 26.9 | 27.1 | 31.1 | 30.1 | 30.1 | 30.1 | 30.6 | 28.4 | 30 | 27.8 | 22.7 | 22.7 | 24.3 | 22.6 |
| 59.7 | 60.1 | 62.3 | 55.6 | 47 | 47 | 47 | 46.4 | 47.4 | 48.4 | 45.9 | 27.8 | 29.9 | 28.4 | 26.9 |
| 56.6 | 56.5 | 58.3 | 51.1 | 44.3 | 44.3 | 44.3 | 43.8 | 45.5 | 47.3 | 44.4 | 26.7 | 28.7 | 27.5 | 29.2 |
| 58 | 57.5 | 59.4 | 55.2 | 47.3 | 47.3 | 47.3 | 46.7 | 46.7 | 48 | 43.6 | 27.6 | 29.9 | 28.3 | 27.3 |
| 68.3 | 69.8 | 71.2 | 55.7 | 45.2 | 45.2 | 45.2 | 44.7 | 46.2 | 50.1 | 46.5 | 26 | 28.7 | 25.3 | 29.2 |
| 72 | 71.6 | 73 | 59.6 | 46.6 | 46.6 | 46.6 | 46.3 | 47.1 | 50.1 | 48.1 | 28.4 | 30 | 28 | 29.3 |
| | 78.8 | 82.4 | 56.7 | 47.1 | 47.1 | 47.1 | 47.1 | 43.8 | 49.3 | 45.8 | 29.9 | 31.8 | 29.9 | 29.8 |
| | | 88.1 | 53.9 | 47 | 47 | 47 | 46.2 | 43.4 | 48.3 | 46.8 | 29.9 | 32.9 | 29.9 | 29.5 |
| | | | 55.5 | 47.3 | 47.3 | 47.3 | 47.3 | 42.3 | 48.3 | 47.1 | 28.7 | 31.4 | 29.1 | 30 |
| | | | | 52.2 | 52.2 | 52.2 | 51.6 | 51.2 | 51.1 | 47.7 | 27.4 | 29.3 | 27.4 | 27.8 |
| | | | | | 99.7 | 99.2 | 94.1 | 59.5 | 52 | 51.8 | 28.4 | 30.7 | 29.2 | 29.5 |
| | | | | | | 99.2 | 94.4 | 59.5 | 52 | 51.8 | 28.4 | 30.7 | 29.2 | 29.5 |
| | | | | | | | 94.1 | 60.1 | 52 | 51.5 | 28.2 | 30.5 | 29.2 | 29.3 |
| | | | | | | | | 59.5 | 52 | 50.9 | 28.4 | 31.9 | 29 | 28.8 |
| | | | | | | | | | 52.4 | 47.3 | 26.8 | 29.6 | 29.5 | 28.1 |
| | | | | | | | | | | 55.6 | 25 | 28.3 | 26.4 | 32 |
| | | | | | | | | | | | 28.5 | 31.1 | 29.7 | 30.5 |
| | | | | | | | | | | | | 84 | 62 | 52 |
| | | | | | | | | | | | | | 60.2 | 52.5 |
| | | | | | | | | | | | | | | 53.7 |

A MATGAT table for local alignment of a specific domain, or data on % identity/similarity between specific domains may also be included.

4. AS-MTT (Abiotic Stress Membrane Tethered Transcription factor) polypeptides

Results of the MATGAT analysis are shown in Table B4

**Table B4:**

|  | 1 | 2 | 3 | 4 | 5 | 6 | 7 | 12 | 13 | 14 |
|---|---|---|---|---|---|---|---|---|---|---|
| 1. A.comosus_DT336453#1 |  | 72 | 74 | 86 | 86 | 70 | 70 | 67 | 65 | 65 |
| 2. S.tuberosum_TC170010#1 |  |  | 84 | 67 | 67 | 72 | 72 | 81 | 74 | 77 |
| 3. I.nil_TC6724#1 |  |  |  | 70 | 70 | 74 | 74 | 84 | 74 | 77 |
| 4. T.aestivum_TC297361#1 |  |  |  |  | 100 | 72 | 70 | 63 | 63 | 63 |
| 5. H.vulgare_TC157185#1 |  |  |  |  |  | 72 | 70 | 63 | 63 | 63 |
| 6. S.officinarum_TC86637#1 |  |  |  |  |  |  | 98 | 72 | 67 | 70 |
| 7. O.sativa_LOC_Os05g08980.1#1 |  |  |  |  |  |  |  | 72 | 67 | 70 |
| 12. H.annuus_TC32349#1 |  |  |  |  |  |  |  |  | 77 | 79 |
| 13. P.trichocarpa_657115#1 |  |  |  |  |  |  |  |  |  | 98 |

5. EXO-1 polypeptide

Results of the software analysis are shown in Table B5 for the global similarity over the full length of the polypeptide sequences. The sequence similarity (in %) between the EXO-1 polypeptide sequences useful in performing the methods of the invention can be as low as 20 % compared to SEQ ID NO: 763.

EP 2 949 661 A1

**Table B5:** MatGAT results for global similarity and identity over the full length of the polypeptide sequences.

| | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| 39. A.thaliana_AT1G18090.1_III | 30,3 | 47,5 | 23,4 | 30,6 | 23,5 | 24,8 | 25,3 | 24,6 | 28,3 | 51,4 | 22,5 | 22,1 | 27,9 | 25,8 |
| 40. A.thaliana_AT1G29630.2_III | 41,8 | 27,5 | 28 | 44,1 | 22 | 42,5 | 27,3 | 26,9 | 53,4 | 28 | 18,8 | 23,9 | 29,7 | 22,3 |
| 41. C.vulgaris_30308_ | 35,6 | 24,3 | 17,6 | 29,2 | 47 | 21,5 | 50,4 | 47,6 | 23 | 25,5 | 26,8 | 19,3 | 41,9 | 56,3 |
| 42. Chlorella_24009_ | 32 | 24,5 | 16,6 | 26,6 | 47,1 | 19,9 | 45,2 | 41,6 | 22,1 | 25 | 27,9 | 18,5 | 39,9 | 51,9 |
| 43. D.melanogaster_tos-PA_III | 23,9 | 24,2 | 30,4 | 26,5 | 16,1 | 23,6 | 17,8 | 17,6 | 25,9 | 23,4 | 19,6 | 23,4 | 20,8 | 16,9 |
| 44. G.max_Glyma05g29660.1_ | | 29,7 | 22,1 | 54,9 | 30,4 | 34,4 | 40 | 39,2 | 42 | 31,3 | 25,2 | 22,8 | 43 | 32,2 |
| 45. G.max_Glyma07g11320.1_ | | | 22,3 | 31,7 | 23,1 | 24 | 25 | 25,7 | 27,8 | 56,4 | 22,6 | 23,2 | 29,7 | 25,4 |
| 46. H.sapiens_NM_130398.2_ | | | | 25,9 | 17,6 | 26,4 | 16,2 | 15,2 | 25,6 | 23,6 | 19,1 | 23,8 | 18,4 | 18,1 |
| 47. M.truncatula_AC153460_23.4_ | | | | | 25,7 | 38,1 | 34,1 | 33,3 | 47,7 | 30,1 | 21,4 | 26,3 | 35,6 | 27,6 |
| 48. O.lucimarinus_13915_ | | | | | | 19,7 | 47,3 | 42,7 | 20,9 | 23,4 | 26,7 | 18,2 | 39,3 | 43,5 |
| 49. O.sativa_LOC_Os01g56940.1_III | | | | | | | 23,7 | 23,7 | 43,9 | 23,1 | 17,5 | 24,3 | 25,5 | 20,1 |
| 50. P.patens_114516_ | | | | | | | | 76,5 | 27,2 | 25,9 | 23,7 | 17,4 | 48,9 | 43,6 |
| 51. P.patens_194153_ | | | | | | | | | 26,9 | 24,9 | 24 | 17,2 | 48,6 | 43 |
| 52. P.trichocarpa_scaff_XI.493_III | | | | | | | | | | 26,8 | 18,9 | 25,6 | 30 | 21,8 |
| 53. P.trichocarpa_scaff_XV.367_ | | | | | | | | | | | 24,1 | 23,2 | 28,5 | 25,4 |
| 54. S.cerevisiae_YDR263C_ | | | | | | | | | | | | 33 | 26,3 | 25,3 |
| 55. S.cerevisiae_YOR033C_ | | | | | | | | | | | | | 20,6 | 17,8 |
| 56. S.moellendorffii_175620_ | | | | | | | | | | | | | | 40,1 |
| 57. V.carteri_61592_ | | | | | | | | | | | | | | |

## 6. YiAP2 polypeptides

Results of the software analysis are shown in Table B6 for the global similarity and identity over the full length of the polypeptide sequences. The sequence identity (in %) between the YiAP2 polypeptide sequence of SEQ ID NO: 836 and other YiAP2 polypeptides of Table B6 are 66.9%, 77%, 58.4%, 57.9%, 67.1%, and 84%.

**Table B6:** MatGAT results for global similarity and identity over the full length of the polypeptide sequences.

| Polypeptide Nr. | Name | 1 | 2 | 3 | 4 | 5 | 6 | 7 | 8 | 9 | 10 |
|---|---|---|---|---|---|---|---|---|---|---|---|
| 1 | A.thaliana_AT1G16060.1#1 | | 57.7 | 51 | 49.9 | 50.7 | 60.7 | 52.1 | 51.5 | 51.2 | 58 |
| 2 | Aquilegia_sp_TC20979#1 | 74.1 | | 53.7 | 51.7 | 53.8 | 61.8 | 64.2 | 61.7 | 61.5 | 61.4 |
| 3 | C.clementina_DY288437#1 | 58.6 | 58.3 | | 77 | 77.5 | 61.7 | 54.1 | 53.8 | 53.5 | 55.8 |
| 4 | C.sinensis_EY655317#1 | 58 | 58.6 | 78.4 | | 63.3 | 59.3 | 53.7 | 51.5 | 51.3 | 54.7 |
| 5 | G.hirsutum_TC125414#1 | 58 | 60.9 | 86.6 | 72 | | 58.6 | 52.1 | 52.6 | 52.4 | 55.3 |
| 6 | G.max_Glyma07g02380.1#1 | 70.7 | 70.7 | 68.6 | 69.3 | 67.9 | | 60.4 | 58.4 | 58.1 | 63.1 |
| 7 | G.max_Glyma17g07860.1#1 | 66.9 | 79.1 | 58 | 60.3 | 58.3 | 68 | | 70.1 | 69.8 | 57.3 |
| 8 | M.truncatula_AC126784_15#1 | 66.9 | 77 | 58.4 | 57.9 | 57.9 | 67.1 | 84 | | 99.7 | 57.7 |
| 9 | Medtr_AP2 | 66.9 | 77 | 58.4 | 57.9 | 57.9 | 67.1 | 84 | 100 | | 57.4 |
| 10 | O.basilicum_TA1087_39350#1 | 71 | 74.1 | 62.5 | 63.1 | 62.2 | 73.8 | 71.1 | 69.7 | 69.7 | |
| 11 | O.sativa_LOC_Os08g34360.1#1 | 57.8 | 61.6 | 47.7 | 48 | 46.1 | 56.6 | 60.4 | 61.1 | 61.1 | 59.4 |
| 12 | O.sativa_LOC_Os09g25600.1#1 | 66.8 | 69.7 | 53.3 | 54.9 | 53 | 63.9 | 67 | 66.2 | 66.2 | 66.2 |
| 13 | P.trichocarpa_800184#1 | 65.8 | 71.3 | 72.7 | 72.7 | 73.1 | 81.5 | 69.1 | 65.4 | 65.4 | 72.6 |
| 14 | R.communis_EG658396#1 | 60.3 | 61.8 | 86.3 | 74.6 | 88 | 72.5 | 59.7 | 59.6 | 59.6 | 65.8 |
| 15 | S.bicolor_Sb02g025080.1#1 | 63.1 | 65.6 | 50.3 | 50.8 | 52 | 59 | 63.6 | 62.1 | 62.1 | 61.6 |
| 16 | S.tuberosum_TC187592#1 | 71 | 78.2 | 58 | 58.8 | 58.3 | 66.3 | 76.2 | 74.6 | 74.6 | 72.7 |
| 17 | Triphysaria_sp_TC12598#1 | 71 | 72.7 | 56.3 | 58.6 | 55.8 | 67.9 | 72.1 | 71.9 | 71.9 | 73.5 |
| 18 | V.vinifera_GSVIVT00017130001#1 | 73.1 | 85.1 | 59.7 | 59.7 | 60.6 | 68 | 81.1 | 78.1 | 78.1 | 75.1 |
| 19 | Z.mays_TC386652#1 | 63.1 | 66.9 | 50.3 | 51.8 | 52.3 | 59.3 | 63.6 | 64.4 | 64.4 | 62.9 |

| Polypeptide Nr. | Name | 11 | 12 | 13 | 14 | 15 | 16 | 17 | 18 | 19 |
|---|---|---|---|---|---|---|---|---|---|---|
| 1 | A.thaliana_AT1G16060.1#1 | 47.8 | 54.7 | 57.6 | 53.3 | 48.7 | 59.1 | 56.8 | 57 | 51.7 |
| 2 | Aquilegia_sp_TC20979#1 | 49.9 | 58.2 | 63.4 | 56.1 | 55.9 | 67.7 | 59.9 | 74.1 | 56.4 |
| 3 | C.clementina_DY288437#1 | 41.1 | 49.5 | 65.8 | 81.7 | 46.2 | 52.8 | 50.7 | 55.1 | 46.8 |
| 4 | C.sinensis_EY655317#1 | 40.8 | 48.4 | 64 | 67.7 | 45.7 | 51.1 | 50.4 | 54 | 46.3 |
| 5 | G.hirsutum_TC125414#1 | 40.6 | 47.8 | 64.8 | 80.2 | 47.2 | 54.3 | 48.5 | 56.9 | 47.1 |
| 6 | G.max_Glyma07g02380.1#1 | 49.2 | 55.9 | 73.4 | 63.6 | 51.8 | 59.9 | 58.8 | 61.5 | 52.5 |
| 7 | G.max_Glyma17g07860.1#1 | 48.5 | 56.3 | 59.2 | 54.1 | 51.5 | 60.4 | 56.8 | 68.3 | 53.5 |
| 8 | M.truncatula_AC126784_15#1 | 48 | 56.2 | 56.3 | 54 | 52.6 | 57.9 | 55.1 | 65.4 | 53.6 |
| 9 | Medtr_AP2 | 47.8 | 55.9 | 56 | 53.8 | 52.3 | 57.6 | 54.9 | 65.1 | 53.3 |
| 10 | O.basilicum_TA1087_39350#1 | 47.1 | 55.3 | 62.5 | 57.8 | 51.3 | 62.6 | 61 | 63.1 | 52.3 |
| 11 | O.sativa_LOC_Os08g34360.1#1 | | 57.5 | 46.5 | 41.8 | 56.2 | 51.8 | 50 | 49.9 | 57.2 |
| 12 | O.sativa_LOC_Os09g25600.1#1 | 69.5 | | 56.2 | 51.6 | 74.4 | 59.6 | 57.4 | 60 | 76.7 |
| 13 | P.trichocarpa_800184#1 | 55.4 | 62.5 | | 68.2 | 51.9 | 63.5 | 56.8 | 64.2 | 52.5 |
| 14 | R.communis_EG658396#1 | 50.1 | 55.4 | 75.3 | | 47.4 | 55.2 | 52.5 | 58.2 | 48 |
| 15 | S.bicolor_Sb02g025080.1#1 | 70.6 | 80.9 | 57.8 | 52.3 | | 55.7 | 53.6 | 55.8 | 81.1 |
| 16 | S.tuberosum_TC187592#1 | 62.5 | 70.2 | 69.1 | 60.5 | 67.1 | | 60.6 | 66.3 | 56.3 |
| 17 | Triphysaria_sp_TC12598#1 | 60.6 | 68.6 | 63.9 | 59.2 | 64.3 | 75.4 | | 63.6 | 55.6 |
| 18 | V.vinifera_GSVIVT00017130001#1 | 61.3 | 70.7 | 71.1 | 62.6 | 65.1 | 78.7 | 76.1 | | 58.4 |
| 19 | Z.mays_TC386652#1 | 72.6 | 82.3 | 59.6 | 51.8 | 87.4 | 69.2 | 66.4 | 68.2 | |

*Example 4: Identification of domains comprised in polypeptide sequences useful in performing the methods of the invention*

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

1. FSM1-like (Fruit Sant/Myb) polypeptides

The results of the InterPro scan of the polypeptide sequence as represented by SEQ ID NO: 2 are presented in Table C1.

**Table C1:** InterPro scan results (major accession numbers) of the polypeptide sequence as represented by SEQ ID NO: 2.

| Method | AccNumber | ShortName | Score | Location |
|---|---|---|---|---|
| INTERPRO | IPR001005 | SANT, DNA-binding | | |
| SMART | SM00717 | SANT | 7.5E-9 | [10-62]T |
| PROFILE | PS50090 | MYB_3 | 0.0 | [6-60]T |
| INTERPRO | IPR009057 | Homeodomain-like | | |
| SUPERFAMILY | SSF46689 | Homeodomain_like | 7.0E-13 | [13-67]T |
| INTERPRO | IPR014778 | Myb, DNA-binding | | |
| PFAM | PF00249 | Myb_DNA-binding | 4.8E-4 | [11-60]T |
| INTERPRO | IPR015609 | Molecular chaperone, heat shock protein, Hsp40, DnaJ | | |
| PANTHER | PTHR11821 | Hsp40/DnaJ_Rel | 9.0E-8 | [13-59]T |
| INTERPRO | nolPR | unintegrated | | |
| PANTHER | PTHR11821:SF29 | PTHR11821:SF29 | 9.0E-8 | [13-59]T |

2. PIF3-like (Phytochrome Interacting Factor 3) polypeptides

The results of the InterPro scan of the polypeptide sequence as represented by SEQ ID NO: 293 are presented in Table C2.

**Table C2:** InterPro scan results (major accession numbers) of the polypeptide sequence as represented by SEQ ID NO: 293.

| Database | Accession number | Accession name | Amino acid coordinates on SEQ ID NO 2 |
|---|---|---|---|
| InterPro | IPR001092 | Basic helix-loop-helix dimerisation region bHLH | |
| HMMPfam | PF00010 | HLH | T[270-319] 7.6e-17 |
| HMMSmart | SM00353 | no description | T[275-324] 4.1e-19 |
| ProfileScan | PS50888 | HLH | T[266-319] 16.780 |
| InterPro | IPR011598 | Helix-loop-helix DNA-binding | |
| Gene3D | G3DSA:4.10.280.10 | no description | T[266-324] 2.5e-08 |

(continued)

| Database | Accession number | Accession name | Amino acid coordinates on SEQ ID NO 2 |
|---|---|---|---|
| Superfamily | SSF47459 | HLH, helix-loop-helix DNA-binding domain | T[267-351] 3.2e-19 |
| InterPro | NULL | NULL | |
| HMMPanther | PTHR12565 | STEROL REGULATORY ELEMENT-BINDING PROTEIN | T[278-318] 2.2e-09 |
| HMMPanther | PTHR12565:SF7 | CENTROMERE-BINDING PROTEIN 1, CBP-1 | T[278-318] 2.2e-09 |
| InterPro | IPR001092 | Basic helix-loop-helix dimerisation region bHLH | |

3. UROD (Uroporphyrinogen III decarboxylase) polypeptide

The results of the InterPro scan of the polypeptide sequence as represented by SEQ ID NO: 368 are presented in Table C3.

**Table C3:** InterPro scan results (major accession numbers) of the polypeptide sequence as represented by SEQ ID NO: 368.

| Method | | Domain ID and name | Short Name | Location |
|---|---|---|---|---|
| Interpro | IPR000257 | Uroporphyrinogen decarboxylase | URO-D | 4.8e-146 [50-390]T |
| PFAM | PF01208 | Uroporphyrinogen decarboxylase | UROD_1 | NA [68-77]? |
| PROSITE | PS00907 | Uroporphyrinogen decarboxylase | UROD_2 | 8e-5[187-203]T |
| Interpro | IPR006361 | Uroporphyrinogen decarboxylase HemE | | |
| PANTHER | PTHR21091: SF2 | Uroporphyrinogen decarboxylase | | 1e-127 [65-390]T |
| TIGRFAMs | TIGR01464 | hemE: uroporphyrinogen decarboxylase | | 3.6e-187 [54-389]T |

4. AS-MTT (Abiotic Stress Membrane Tethered Transcription factor) polypeptides

The results of the InterPro scan of the polypeptide sequence as represented by SEQ ID NO: 537 are presented in Table C4.

**Table C4:** InterPro scan results (major accession numbers) of the polypeptide sequence as represented by SEQ ID NO: 537.

| Interpro ID | Domain ID | Domain name | Short Name | Location (Amino acid coordinates) |
|---|---|---|---|---|
| IPRO12458 | PF07889 | Protein of unknown function DUF1664 | DUF 1664 | 94-216 |
| Unintegrated | SIGNALP | Signal-peptide | SignalP | 1-23 |
| | TMHMM | TRANSMEMBRANE-REGIONS | | 5-25 and 95-115 |

5. EXO-1 polypeptide

The results of the InterPro scan of the polypeptide sequence as represented by SEQ ID NO: 763 are presented in Table C5.

**Table C5:** InterPro scan results (major accession numbers) of the polypeptide sequence as represented by SEQ ID NO: 763.

| Database | Accession number | Accession name | Amino acid coordinates on SEQ ID NO: 763 |
|---|---|---|---|
| HMMPfam | PF00752 | XPG_N | 1 - 99 |
| HMMPfam | PF00867 | XPG_I | 138-230 |
| HMMPanther | PTHR11081:SF8 | EXONUCLEASE 1 | 17-333 |
| HMMPanther | PTHR11081 | XP-G/RAD2 DNA REPAIR ENDONUCLEASE FAMILY | 17-333 |
| superfamily | SSF88723 | PIN domain-like | 2-226 |
| ScanRegExp | PS00841 | XPG_1 | 71 - 85 |
| FPrintScan | PR00853 | XPGRADSUPER | 24-38 |
| FPrintScan | PR00853 | XPGRADSUPER | 73-92 |
| FPrintScan | PR00853 | XPGRADSUPER | 137-154 |
| FPrintScan | PR00853 | XPGRADSUPER | 158 178 |
| FPrintScan | PR00853 | XPGRADSUPER | 216 231 |
| HMMSmart | SM00485 | XPGN | 1 99 |
| HMMSmart | SM00484 | XPGI | 138 208 |
| HMMSmart | SM00279 | HhH2 | 213 246 |
| Gene3D | G3DSA:3.40.50.1010 | no description | 16 195 |

6. YiAP2 polypeptides

The results of the InterPro scan of the polypeptide sequence as represented by SEQ ID NO: 836 are presented in Table C6.

Table C6: InterPro scan results (major accession numbers) of the polypeptide sequence as represented by SEQ ID NO: 836.

| InterPro | Database | Accession nr | Name | Amino Acid Coordinates |
|---|---|---|---|---|
| **IPR001471** | PRINTS | PR00367 | ETHRSPELEMNT | [51-62] and [189-209] |
| Domain | GENE3D | G3DSA:3.30.730.10 | no description | [49-116] and [148-210] |
| Pathogenesis-related transcriptional factor/ERF, DNA-binding | PFAM | PF00847 | AP2 | [49-106] and [148-200] |
| | SMART | SM00380 | AP2 | [50-119] and [149-213] |
| | PROFILE | PS51032 | AP2_ERF | [50-113] and [149-207] |

*Example 5: Topology prediction of the polypeptide sequences useful in performing the methods of the invention*

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

## 1. FSM1-like (Fruit Sant/Myb) polypeptides

The results of TargetP 1.1 analysis of the polypeptide sequence as represented by SEQ ID NO: 2 are presented Table D1. The "plant" organism group has been selected, no cutoffs defined, and the predicted length of the transit peptide requested. The subcellular localization of the polypeptide sequence as represented by SEQ ID NO: 2 may be the cytoplasm or nucleus, no transit peptide is predicted.

Table D1: TargetP 1.1 analysis of the polypeptide sequence as represented by SEQ ID NO: 2. Abbreviations: Len, Length; cTP, Chloroplastic transit peptide; mTP, Mitochondrial transit peptide, SP, Secretory pathway signal peptide, other, Other subcellular targeting, Loc, Predicted Location; RC, Reliability class; TPlen, Predicted transit peptide length.

| Name | Len | cTP | mTP | SP | other | Loc | RC | TPlen |
|---|---|---|---|---|---|---|---|---|
| SEQ ID NO: 2 | 88 | 0.505 | 0.093 | 0.089 | 0.548 | - | 5 | - |
| cutoff | | 0.000 | 0.000 | 0.000 | 0.000 | | | |

When using the protein localisation prediction algorithm (PLOC, Park and Kanehisa, Bioinformatics, 19, 1656-1663, 2003), SEQ ID NO: 2 is predicted to have a nuclear localisation, which is in line with its postulated function as transcription factor.

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)

## 2. PIF3-like (Phytochrome Interacting Factor 3) polypeptides

The results of TargetP 1.1 analysis of the polypeptide sequence as represented by SEQ ID NO: 293 are presented Table D2. The "plant" organism group has been selected, no cutoffs defined, and the predicted length of the transit peptide requested. The subcellular localization of the polypeptide sequence as represented by SEQ ID NO: 293 may be the cytoplasm or nucleus, no transit peptide is predicted.

**Table D2:** TargetP 1.1 analysis of the polypeptide sequence as represented by SEQ ID NO: 293. Abbreviations: Len, Length; cTP, Chloroplastic transit peptide; mTP, Mitochondrial transit peptide, SP, Secretory pathway signal peptide, other, Other subcellular targeting, Loc, Predicted Location; RC, Reliability class; TPlen, Predicted transit peptide length.

| Name | Len | cTP | mTP | SP | other | Loc | RC | TPlen |
|---|---|---|---|---|---|---|---|---|
| OsPIF3 | 445 | 0.173 | 0.067 | 0.046 | 0.897 | _ | 2 | - |

(continued)

| Name | Len | cTP | mTP | SP | other | Loc | RC | TPlen |
|------|-----|-----|-----|-----|-------|-----|-----|-------|
| cutoff | | 0.000 | 0.000 | 0.000 | 0.000 | | | |

When analysed with PLOC (Park and Kanehisa, Bioinformatics, 19, 1656-1663, 2003), a nuclear localisation is predicted, which is in line with the experimental data (Fairchild et al., 2000).
Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)

3. UROD (Uroporphyrinogen III decarboxylase) polypeptide

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

4. AS-MTT (Abiotic Stress Membrane Tethered Transcription factor) polypeptides

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

5. EXO-1 polypeptide

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

6. YiAP2 polypeptides

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

*Example 6: Assay related to the polypeptide sequences useful in performing the methods of the invention*

1. FSM1-like (Fruit Sant/Myb) polypeptides

DNA binding assays are well known in the art, including PCR-assisted DNA binding site selection and a DNA binding gel-shift assay; for a general reference, see Current Protocols in Molecular Biology, Volumes 1 and 2, Ausubel et al. (1994), Current Protocols.

A DNA binding assay for MYB7, as an example for MYB proteins in general, is described in Li and Parish (Plant J. 8, 963-972, 1995). Briefly, the MYB7 coding sequence is cloned in frame with the T7 gene 10 leader sequence and expressed in E. coli. The proteins are purified and analysed in a mobility retardation assay, using 32P-labeled c-myb binding site (MBS) and the binding site of the maize P gene product (PBS). In this way, it was shown that MYB7 from Arabidopsis thaliana did bind to the MBS site though not with high affinity, and had a binding preference for the PBS site.

2. PIF3-like (Phytochrome Interacting Factor 3) polypeptides

In Arabidopsis, PIF3 forms a complex with HFR1. Fairchild et al. (2000) describe a yeast two-hybrid binding assay as well as immunoprecipitations for demonstrating this interaction in vitro. The yeast two-hybrid assay was carried out according to standard procedures, for the immunoprecipitations, either mixtures of PIF3, GAD (galactose activation domain) and the HFR1-GAD fusion, or mixtures of HFR, GAD and the PIF3-GAD fusion were used (Fairchild et al., 2000).

Following a pulse of red light, phyA, phyB, phyC, and phyD interact in vivo with the PHYTOCHROME INTERACTING FACTOR3 basic helix-loop-helix transcription factor. These interactions were demonstrated using a yeast two-hybrid binding assay as well as immunoprecipitations (Clack et al., Plant Cell. 2009 Mar;21(3):786-99) Al-Sady et al. (Molecular Cell. 2006 Aug 4;23(3):439-46) provide evidence that photoactivation of phy induces rapid in vivo phosphorylation of PIF3 preceding degradation. Both phyA and phyB redundantly induce this PIF3 phosphorylation, as well as nuclear speckle formation and degradation, by direct interaction with PIF3 via separate binding sites.

3. UROD (Uroporphyrinogen III decarboxylase) polypeptide

Porphyrin activity is measured using chromatographic and spectral methods, such as HPLC and TLC as described in Jacobs and Jacobs, 1993 (Plant Physiol. 101(4): 1181-1187) and Magnetic Circular Dichroism as described in Ivanetich et al., 1984 (Clin Chem. 30(3): 391-4).

4. EXO-1 polypeptide

Functional assays were performed according to Furukawa et al., 2008 - Plant Mol Biol (2008) 66:519-531. Enzymatic assays were performed according to Furukawa et al., 2003 - Plant Molecular Biology 51: 59-70, 2003.

*Example 7: Cloning of the nucleic acid sequence used in the methods of the invention*

1. FSM1-like (Fruit Sant/Myb) polypeptides

The nucleic acid sequence was amplified by PCR using as template a custom-made *Solanum lycopersicum* seedlings cDNA library (in pCMV Sport 6.0; Invitrogen, Paisley, UK). PCR was performed using Hifi Taq DNA polymerase in standard conditions, using 200 ng of template in a 50 μl PCR mix. The primers used were prm10562 (SEQ ID NO: 290; sense, start codon in bold): 5'-ggggacaagtttgtacaaaaaagcaggcttaaaca**atg**agctcgatgtcgtctca-3' and prm10563 (SEQ ID NO: 291; reverse, complementary): 5'-ggggaccactttgtacaagaaagctggg taagtgttaggttttgctcctttt-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pFSM1-like. Plasmid pDONR201 was purchased from Invitrogen, as part of the Gateway® technology.

The entry clone comprising SEQ ID NO: 1 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 289) for constitutive specific expression was located upstream of this Gateway cassette.

After the LR recombination step, the resulting expression vector pGOS2::FSM1-like (Figure 4) was transformed into *Agrobacterium* strain LBA4044 according to methods well known in the art.

2. PIF3-like (Phytochrome Interacting Factor 3) polypeptides

The nucleic acid sequence was amplified by PCR using as template a custom-made *Oryza sativa* seedlings cDNA library (in pCMV Sport 6.0; Invitrogen, Paisley, UK). PCR was performed using Hifi Taq DNA polymerase in standard conditions, using 200 ng of template in a 50 µl PCR mix. The primers used were prm12326 (SEQ ID NO: 364; sense, start codon in bold): 5'-ggggacaagtttgtacaaaaaagcaggcttaaaca**atg**aaccagttcgtccctg-3' and prm12327 (SEQ ID NO: 365; reverse, complementary): 5'-ggggaccactttgtacaagaaagctgggtcagaa tgtcgtcaggagtcag-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pPIF3-like. Plasmid pDONR201 was purchased from Invitrogen, as part of the Gateway® technology.

The entry clone comprising SEQ ID NO: 292 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 366) for constitutive specific expression was located upstream of this Gateway cassette.

After the LR recombination step, the resulting expression vector pGOS2::PIF3-like (Figure 9) was transformed into *Agrobacterium* strain LBA4044 according to methods well known in the art.

3. UROD (Uroporphyrinogen III decarboxylase) polypeptide

The nucleic acid sequence was amplified by PCR using a *Populus trichocarpa* cDNA library (in pCMV Sport 6.0; Invitrogen, Paisley, UK). PCR was performed using Hifi Taq DNA polymerase in standard conditions, using 200 ng of template in a 50 µl PCR mix. The primers used were prm12179 (SEQ ID NO: 534; sense, start codon in bold): 5'-ggggacaag tttgtacaaaaaagcaggcttaaacaatgaactctgcttctcttagc -3' and prm12180 (SEQ ID NO: 535; reverse, complementary): 5'-ggggaccactttgtacaagaaagctgggtgacaccactccttaaactacc-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pUROD. Plasmid pDONR201 was purchased from Invitrogen, as part of the Gateway® technology.

The entry clone comprising SEQ ID NO: 367 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 533) for constitutive specific expression was located upstream of this Gateway cassette.

After the LR recombination step, the resulting expression vector pGOS2::UROD (Figure 14) was transformed into *Agrobacterium* strain LBA4044 according to methods well known in the art.

4. AS-MTT (Abiotic Stress Membrane Tethered Transcription factor) polypeptides

The nucleic acid sequence used in the methods of the invention was amplified by PCR using as template a custom-made *Populus trichocarpa v1.1*seedlings cDNA library (in pCMV Sport 6.0; Invitrogen, Paisley, UK). PCR was performed using Hifi Taq DNA polymerase in standard conditions, using 200 ng of template in a 50 µl PCR mix. The primers used were as represented by SEQ ID NO: 657 (sense) and SEQ ID NO: 658 (reverse, complementary) which include the AttB sites for Gateway recombination. The amplified PCR fragment was purified also using standard methods.

The first step of the Gateway procedure, the BP reaction, was then performed, during which the PCR fragment recombined in vivo with the pDONR201 plasmid to produce, according to the Gateway terminology, an "entry clone", pAS_MTT. Plasmid pDONR201 was purchased from Invitrogen, as part of the Gateway® technology.

The entry clone comprising SEQ ID NO: 536 was then used in an LR reaction with a destination vector used for *Oryza sativa* transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 659) for constitutive specific expression was located upstream of this Gateway cassette.

After the LR recombination step, the resulting expression vector pGOS2::AS-MTT (Figure 17) was transformed into *Agrobacterium* strain LBA4044 according to methods well known in the art.

5. EXO-1 polypeptide

The nucleic acid sequence was amplified by PCR using as template a custom-made *Populus trichocarpa* seedlings cDNA library (in pCMV Sport 6.0; Invitrogen, Paisley, UK). PCR was performed using Hifi Taq DNA polymerase in standard conditions, using 200 ng of template in a 50 μl PCR mix.

The primers used were prm12748 (SEQ ID NO: 833; sense, start codon in bold): 5'-gggga caagtttgtacaaaaaagcaggcttaaaca**atg**ggaatacaagggcttta-3' and prm12749 (SEQ ID NO: 834; reverse, complementary): 5'-ggggaccactttgtacaagaaagctgggtgtttccaaaagtcagcgtct-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pEXO-1. Plasmid pDONR201 was purchased from Invitrogen, as part of the Gateway® technology.

The entry clone comprising SEQ ID NO: 762 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone.

A rice GOS2 promoter (SEQ ID NO: 831) for constitutive specific expression was located upstream of this Gateway cassette. After the LR recombination step, the resulting expression vector pGOS2::EXO-1 (Figure 20) was transformed into *Agrobacterium* strain LBA4044 according to methods well known in the art.

A rice beta expansin EXPB9 promoter (SEQ ID NO: 832) for shoot specific expression was located upstream of this Gateway cassette. After the LR recombination step, the resulting expression vector p EXPB9::EXO-1 (Figure 21) was transformed into *Agrobacterium* strain LBA4044 according to methods well known in the art.

6. YiAP2 polypeptides

The nucleic acid sequence was amplified by PCR using as template a custom-made *Medicago truncatula* seedlings cDNA library (in pCMV Sport 6.0; Invitrogen, Paisley, UK). PCR was performed using Hifi Taq DNA polymerase in standard conditions, using 200 ng of template in a 50 μl PCR mix. The primers used were: 5'-ggggacaagtttgtacaaaaa agcaggcttaaaca**atg**gcgaaaaaatcgca-3' (SEQ ID NO: 887; sense, start) and 5'-ggggaccactt tgtacaagaaagctgggtccatattctcttttgaattgagc-3' (SEQ ID NO: 888; reverse, complementary) which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pYiAP2. Plasmid pDONR201 was purchased from Invitrogen, as part of the Gateway® technology.

The entry clone comprising SEQ ID NO: 835 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 890) for constitutive specific expression was located upstream of this Gateway cassette.

After the LR recombination step, the resulting expression vector pGOS2::YiAP2 (Figure 23) was transformed into *Agrobacterium* strain LBA4044 according to methods well known in the art.

*Example 8: Plant transformation*

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% HgCl$_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD$_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

**Example 9: Transformation of other crops**

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseedlcanola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 $\mu$m acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 $\mu$g/ml cefotaxime. The seeds are then transferred to SH-medium with 50$\mu$g/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 $\mu$g/ml MgCL2, and with 50 to 100 $\mu$g/ml cefotaxime and 400-500 $\mu$g/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

*Example 10: Phenotypic evaluation procedure*

10.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds were grown in potting soil under normal conditions until they approached the heading stage. They were then transferred to a "dry" section where irrigation was withheld. Humidity probes were inserted in randomly chosen pots to monitor the soil water content (SWC). When SWC went below certain thresholds, the plants were automatically re-watered continuously until a normal level was reached again. The plants were then re-transferred again to normal conditions. The rest of the cultivation (plant maturation, seed harvest) was the same as for plants not grown under abiotic stress conditions. Growth and yield parameters were recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

10.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Where two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

10.3 Parameters measured

*Biomass-related parameter measurement*

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant (RootMax)), or as the maximum biomass of roots with a thickness above a certain threshold observed during the lifespan of a plant (RootThickMax); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

*Examples 11: Results of the phenotypic evaluation of the transgenic plants*

1. FSM1-like (Fruit Sant/Myb) polypeptides

The results of the evaluation of transgenic rice plants expressing SEQ ID NO: 1 under non-stress conditions are presented below. T1 plants exhibited increased seed yield and increased root biomass, and these observations were confirmed in a second evaluation with T2 plants, Tables E1 and E2 list the parameters for which the overall increase was at least 5%.

**Table E1:** Data summary for transgenic rice plants of the T1 generation; for each parameter the overall percent increase is shown and for each parameter the p-value is <0.05.

| Parameter | Overall |
|-----------|---------|
| Fillrate | 19.9 |
| RootThickMax | 6.5 |

**Table E2:** Data summary for transgenic rice plants of the T2 generation; for each parameter the overall percent increase is shown and for each parameter the p-value is <0.05.

| Parameter | Overall |
|-----------|---------|
| RootMax | 7.5 |
| Fillrate | 12.5 |

Furthermore, T1 plants had increased total seed weight (+17.1% overall increase, p<0.05), increased Harvest Index (+16.5% overall increase, p<0.05), increased number of filled seeds (+17.9% overall increase, p<0.05) and increased number of flowers per panicle (+10.6% overall increase, p<0.05). In addition, one of the tested lines in both T1 and T2 generation showed increased early vigour.

2. PIF3-like (Phytochrome Interacting Factor 3) polypeptides (drought stress)

Transgenic rice plants expressing SEQ ID NO: 293 under control of the rice GOS2 promoter and grown under drought stress showed increased Thousand Kernel Weight in both T1 and T2 generation, the overall increase in T2 was 6.6% with a p-value <0.05. The plants also had increased early vigour, with an overall increase of 27.7% in T2 (p-value <0.05)

3. UROD (Uroporphyrinogen III decarboxylase) polypeptide

a. Results under normal growth conditions

As shown in the tables below, transgenic rice expressing the UROD gene of SEQ ID NO: 367 under the control of the constitutive GOS2 promoter, and grown under normal conditions as described hereinabove, showed improved yield-related traits compared to control plants (corresponding nullizygotes).

Positive tendencies were observed in a first evaluation in some lines for the following parameters: aboveground area/biomass, emergence vigour, early flowering, total weight of seeds, seed fill rate, Thousand Kernel Weight, number of filled seeds, number of flowers per panicle, plant height.

The parameterFor each parameter, the percentage overall is shown if it reaches p<0.05 and above the 5% threshold.

b. Results under drought conditions

As shown in the tables below, transgenic rice expressing the UROD gene of SEQ ID NO: 367 under the control of the constitutive GOS2 promoter, and grown under drought conditions as described hereinabove, showed improved yield-related traits compared to control plants (corresponding nullizygotes). For each parameter, the percentage overall is shown if it reaches p<0.05 and above the 5% threshold.

In a first evaluation the following parameters showed p<0.05 and were above the 5% threshold: total weight of seeds, seed fill rate, harvest index.

1 st Evaluation

**Table E3:**

| Parameter | Overall |
|-----------|---------|
| Total weight seeds | 21.1 |
| Seed filling rate | 33.7 |
| Harvest index | 25.0 |

In a second evaluation the parameters shown in the table below gave p<0.05 and were above the 5% threshold.

Although there was a decrease in the number of first panicles, there was an increase in the number of flowers per panicle.

2nd Evaluation

**Table E4:**

| Parameter | Overall |
|---|---|
| Aboveground area | 6.6 |
| Emergence Vigour | 11.0 |
| Number of panicles | -14.0 |
| Number of flowers per panicle | 12.8 |

4. AS-MTT (Abiotic Stress Membrane Tethered Transcription factor) polypeptides

The results of the evaluation of transgenic rice plants in the T1 generation and expressing a nucleic acid comprising the longest Open Reading Frame in SEQ ID NO: 536 under the drought screen growth conditions as specified above are presented below. See previous Examples for details on the generations of the transgenic plants.

The results of the evaluation of transgenic rice plants under non-stress conditions are presented below. An increase of at least 5 % was observed for root biomass (RootMax), root to shoot index (RootShlnd), total weight of seeds (totalwgseeds), seed filling rate (fillrate), harvest index (harvestindex), thousand kernel weight (TKW), number of filled seeds (nrfilledseed), Gravity center (GravityYMax), Thick roots (RootThickMax).

Gravity center (GravityYMax) is a measurement that correlate with increased in seed size and weight and a change in the shape of the canopy of the plant, such that the plant height and/or the leaf angle are altered in such a way that gravity center of the canopy is higher. RootThickMax is a measure of the proportion of thick roots compared to the thin roots in the root system of a plant. The proportion of thick roots is typically used as a measure for the biomass of roots above a given thickness threshold. It is typically used as a measure for estimating soil penetration, plant stability, nutrient uptake, water uptake, and tolerance to biotic and abiotic stresses.

**Table E5:**

| Parameter | Overall |
|---|---|
| RootMax | 6.9 |
| RootShlnd | 7.6 |
| totalwgseeds | 27.1 |
| fillrate | 24.5 |
| harvestindex | 32.1 |
| TKW | 5.6 |
| nrfilledseed | 21.6 |
| GravityYMax | 6.2 |
| RootThickMax | 11.7 |

5. EXO-1 polypeptide

The results of the evaluation of transgenic rice plants in the T1 generation and expressing a nucleic acid comprising the longest Open Reading Frame in SEQ ID NO: 762 under non-stress conditions are presented below. See previous Examples for details on the generations of the transgenic plants.

The results of the evaluation of transgenic rice plants under non-stress conditions are presented below (Table E6). An increase of at least 5 % was observed for aboveground biomass (AreaMax), total root biomass (RootMax), root-shoot index (RootShlnd), yield per plant (totalwgseeds), fill rate (fillrate), harvest index (harvestindex), greenness of a plant before flowering (GNbfFlow), height of the plant (HeightMax; GravityYMax) and of 3,3% for thousand kernel weight (TKW).

**Table E6:** Data summary for transgenic rice plants; for each parameter, the overall percent increase relative to the wild type plant is shown. For each parameter the p-value is <0.05.

| Paramete r | Overall |
|---|---|
| AreaMax | 10.2 |
| RootMax | 12.5 |
| RootShlnd | 6.2 |
| totalwgsee ds | 19.7 |
| fillrate | 6.3 |
| harvestind ex | 12.1 |
| TKW | 3.3 |
| GNbFlow | 6.6 |
| HeightMax | 5.4 |
| GravityYM ax | 10.6 |

6. YiAP2 polypeptides

The results of the evaluation of transgenic rice plants in the T1 generation and expressing a nucleic acid comprising the longest Open Reading Frame in SEQ ID NO: 835 under the control of the GOS2 promoter and grown under non-stress conditions are presented below. See previous Examples for details on the generations of the transgenic plants. An increase of at least 5 % was observed for aboveground biomass (AreaMax), emergence vigour (early vigour), total seed yield (totalwgseeds), number of filled seeds (nrfilledseed), fill rate (fillrate), number of flowers per panicle, harvest index (harvestindex).

**Table E7:** Data summary for transgenic rice plants; for each parameter (yield trait), the overall percent increase is shown for the evaluation of plants of the T generation), for each parameter the p-value is <0.05.

**Table E7:**

| Parameter | Overall increase |
|---|---|
| totalwgseeds | 26.5 |
| fillrate | 13.8 |
| harvestindex | 23.3 |
| nrfilledseed | 24.0 |

**Claims**

1. A method for enhancing yield-related traits in plants relative to control plants, comprising modulating expression in a plant of a nucleic acid encoding an AS-MTT polypeptide, wherein said AS-MTT polypeptide comprises a DUF1664 domain.

2. Method according to claim 1, wherein said AS-MTT polypeptide comprises one or more of the following motifs:

   (i)  Motif 31:  G[WL][SK][FL]SD[LV]M[YF][VA]T[KR]R[NS][ML][AS][ND]AV[SA][SN][VL][TS]K[QH]L[ED][QN]VS[ESD][AS]LAA[TA]K[RK]HL[TS]QR (SEQ ID NO: 648),
   (ii) Motif 32: AA[AT][VL]G[AV][VLM]GY[GC]YMWWK (SEQ ID NO: 649),
   (iii) Motif 33: GAG[LY]TG[ST][IV][LV][LA][KR][NE]G[KR]L (SEQ ID NO: 650).

3. Method according to claim 1 or 2, wherein said modulated expression is effected by introducing and expressing in a plant a nucleic acid encoding an AS-MTT polypeptide.

**4.** Method according to any one of claims 1 to 3, wherein said nucleic acid encoding an AS-MTT polypeptide encodes any one of the proteins listed in Table A4 or is a portion of such a nucleic acid, or a nucleic acid capable of hybridising with such a nucleic acid.

**5.** Method according to any one of claims 1 to 4, wherein said nucleic acid sequence encodes an orthologue or paralogue of any of the proteins given in Table A4.

**6.** Method according to any one of claims 1 to 5, wherein said enhanced yield-related traits comprise increased biomass and/or increased seed yield relative to control plants.

**7.** Method according to any one of claims 1 to 6, wherein said enhanced yield-related traits are obtained under non-stress conditions.

**8.** Method according to any one of claims 3 to 7, wherein said nucleic acid is operably linked to a constitutive promoter, preferably to a GOS2 promoter, most preferably to a GOS2 promoter from rice.

**9.** Method according to any one of claims 1 to 8, wherein said nucleic acid encoding an AS-MTT polypeptide is of plant origin, preferably from a dicotyledonous plant, further preferably from the genus *Populus,* most preferably from *Populus trichocarpa.*

**10.** Plant or part thereof, including seeds, obtainable by a method according to any one of claims 1 to 9, wherein said plant or part thereof comprises a recombinant nucleic acid encoding an AS-MTT polypeptide.

**11.** Construct comprising:

(i) nucleic acid encoding a polypeptide as defined in claims 1 or 2;
(ii) one or more control sequences capable of driving expression of the nucleic acid sequence of (a); and optionally
(iii) a transcription termination sequence.

**12.** Construct according to claim 11, wherein one of said control sequences is a constitutive promoter, preferably a GOS2 promoter, most preferably a GOS2 promoter from rice.

**13.** Use of a construct according to claim 11 or 12 in a method for making plants having increased yield, particularly increased biomass and/or increased seed yield relative to control plants.

**14.** Plant, plant part or plant cell transformed with a construct according to claim 11 or 12.

**15.** Method for the production of a transgenic plant having increased yield, particularly increased biomass and/or increased seed yield relative to control plants, comprising:

(i) introducing and expressing in a plant a nucleic acid encoding a polypeptide as defined in claim 1 or 2; and
(ii) cultivating the plant cell under conditions promoting plant growth and development.

**16.** Transgenic plant having increased yield, particularly increased biomass and/or increased seed yield, relative to control plants, resulting from modulated expression of a nucleic acid encoding a polypeptide as defined in claim 1 or 2, or a transgenic plant cell derived from said transgenic plant.

**17.** Transgenic plant according to claim 10, 14 or 16, or a transgenic plant cell derived thereof, wherein said plant is a crop plant such as sugar beet, or a monocot plant such as sugarcane, or a cereal, such as rice, maize, wheat, barley, millet, rye, triticale, sorghum, emmer, spelt, secale, einkorn, teff, milo and oats.

**18.** Harvestable parts of a plant according to claim 17, wherein said harvestable parts are preferably shoot biomass and/or seeds.

**19.** Products derived from a plant according to claim 17 and/or from harvestable parts of a plant according to claim 17.

**20.** Use of a nucleic acid encoding a polypeptide as defined in claim 1 or 2 in increasing yield, particularly in increasing seed yield and/or shoot biomass in plants, relative to control plants.

MSSMSSQHGSS*GSWTAKQNKAFEKALAVYDKETRDRWSNVAKAVGGKTAEEVKRHYEILLRDVFFID*
           1               2         3
----------------4----------------- ------5------ --

NGMVPFPKYKTTGGSHNSTSD
  3
-6-----

# FIGURE 1

CLUSTAL 2.0.11 multiple sequence alignment

```
M.guttatus_DV207881          MASSSAN-RDSGRKWTAKENKDFEEALAEFDKDTPERWSNVARAVG-TRT
A.majus_TA4522_4151          MAST----RGSGRPWSAKENKAFERALAVYDKDTPDRWANVARAVE-GRT
V.vinifera_EE072107          MASSSMSSRSSGSSWTAKQNKAFEEALAVYDKDTPDRWYNVARAVG-GKT
G.hirsutum_TA31277_3635      MASSSMSSRGSGS-WTAKQNKDFERALAVYDKDTPDRWYNVAKAVG-GKT
G.hirsutum_DW503354          MASSSMSSQGSSS-WTAKQNKDFEKVLAVYDKDTPDRWYNVAKAIG-GKT
H.exilis_EE652824            MSS-----RGSGS-WTVKQNKDFEEALAVFDKDTPDRWRNVAKAVG-GKT
E.gunnii_CT985658            MASSSMS-RGSSS-WTAKQNKAFEEALAVYDKDTPDRWHNVAKAVG-GKT
P.trichocarpa_557252         MASSSMSSRGSGS-WTVQQNKAFERALAVYDRDTPDRWYNVARAVG-GKT
P.euphratica_AJ768574        MASSSMSSRGSGS-WTAQQNKAFERALAVYDKDTPDRWYNVARAVG-GKT
P.trichocarpa_sc_IV.1184     MASSSVSSRGSGS-WTAQQNKAFERALAVYDRDTPDRWHNVARAVG-GKT
L.serriola_DW122081          MASSSMSSRGSGS-WTAKQNKAFEKALAVFDKDTPDRWHNVAKAVG-GKT
L.virosa_TA3253_75947        MASSSMSSRGSGS-WTAKQNKAFEKALAVFDKDTPDRWHNVAKAVG-GKT
L.sativa_TA7946_4236         MASSSMSSRGSGS-WTAKQNKAFEKALAVFDKDTPDRWHNVAKAVG-GKT
C.intybus_EH706852           MASSSMSSRGSGS-WTAKQNKAFEKALAVFDKDTPDRWHNVAKAVG-GKT
M.domestica_DR994824         MASSSMSSRGSGSSWTAKQNKAFEKALAVYDKDTADRWYNVAKAVG-GKT
S.lycopersicum_TA46686       MSSMS-SQHGSSGSWTAKQNKAFEKALAVYDKETRDRWSNVAKAVG-GKT
N.benthamiana_EH366122       MSSMS-SQHGS-GSWTAKQNKAFEKALAVYDKTCDRWSNVAKAVG-GKT
T.salsuginea_DN773374        MASNTMSSYGSGS-WTVKQNKAFERALATYDQDTPDRWYNVARAVG-GKT
B.napus_BN06MC20309          MASNSMSAYGSGS-WTVKQSKAFESALATYDQDSPDRWYNVARAVG-GTT
A.thaliana_At2g21650         MASGSMSSYGSGS-WTVKQNKAFERALAVYDQDTPDRWHNVARAVG-GKT
A.thaliana_At4g39250         MASSSMSSQSSGS-WTAKQNKAFEQALATYDQDTPNRWQNVAKVVG-GKT
G.max_Glyma06g46590.1        MASSSMSSTGS---WSAKDNKAFERALAVYDKDTPDRWYNVAKAVAVGKT
G.max_Glyma04g16390.1        MASSSMSSTGS---WSTKDNKAFERALAVYDKDTPDRWYNVAKAVGGGKT
M.truncatula_AC135566        MAS--MSASGS---WSAKDNKAFERALAVFDKDTPDRWSNVAQAVGGGKT
G.max_Glyma02g18210.1        MASSSMSATGS---WSAKDNKAFERALAVYDKDTPDRWKDVARAVG-GKT
M.truncatula_AC157502        MASSSMSASGS---WSVKENKAFERALAVYDKDTPDRWYNVAHAVG-GKT
G.max_Glyma03g28050.1        MASSSISASGS---WSVKDNKAFEKALAVYDKDTPDRWYNVAHAVG-GKT
G.hirsutum_DR461014          MSSNSMSAS-----WTAKQNKDFERALAVYDKDTPDRWYNVAKAVG-GKT
                             *:*          *:.::.* ** .** :*::: :** :**:..    *
```

# FIGURE 2

```
M.guttatus_DV207881        VEEVKKHYEVLLEDFNKIESGKVPLPNYKTTCG----SF-----------
A.majus_TA4522_4151        PEEVKKHYEILVEDIKYIESGKVPFPNYRTTGG----NMKTDEKRFRNLK
V.vinifera_EE072107        VEEVKRHYEILVEDIKSIDSDKVPFPNYKTTGASGRSNMSDQEKRMMNLK
G.hirsutum_TA31277_3635    AEEVKRHYELLVADVKYIESGQVPFP-YRSNGN-----------------
G.hirsutum_DW503354        AEEVKRHYELLVKDVKQIESGKVPFPNYRTTGGSNTQG------------
H.exilis_EE652824          AEEVKRHYEILVGDVKLIESGRVPFPNYRTTN--GA--------------
E.gunnii_CT985658          AEEVKRHYELLVEDVKHIESGRVPFPNYRTTGR-GNVNG-----------
P.trichocarpa_557252       AEEVKRHYELLVEDVKHIESGHVPFPNYRTTGANGHARG-----------
P.euphratica_AJ768574      AEEVKRHYELLVEDVKHIESGHVPFPNYRTTGANGHARG-----------
P.trichocarpa_sc_IV.1184   AEEVKRHYEILVEDVKHIESGRVPFPNYRTTGANGHSKTGEEKMYIYSSL
L.serriola_DW122081        AEEVKQHYEVLVEDVKHIENGRVPFPNYRRTTGGG---------------
L.virosa_TA3253_75947      AEEVKQHYEVLVEDVKHIENGRVPFPNYRRTTGGG---------------
L.sativa_TA7946_4236       AEEVKQHYEVLVEDVKHIENGRVPFPNYRRTTGGG---------------
C.intybus_EH706852         AEEVKHHYEVLVEDVKHIENGRVPFPNYR-TTGGA---------------
M.domestica_DR994824       PEEVKRHYEVLVEDVKHIESGQVPFPDYR-TTGGNSHGHHMSDEEKRMRN
S.lycopersicum_TA46686     AEEVKRHYEILLRDVFFIDNGMVPFPKYKTTGGSHNSTSD----------
N.benthamiana_EH366122     AEEVKQHYEILVHDVLYIENGRVPFPKYKTT------TRE----------
T.salsuginea_DN773374      PEEAKRQYDLLVRDIESIENGHVPFPDYKSN-GGSTKGRLRDEEKRMRSM
B.napus_BN06MC20309        PDEAKRQYELLVRDIESIENGHVAFPNYKTN-GGSTKGRLRDEEKRMRSM
A.thaliana_At2g21650       PEEAKRQYDLLVRDIESIENGHVPFPDYKTTTGNSNRGRLRDEEKRMRSM
A.thaliana_At4g39250       TEEVKRHYELLVQDINSIENGHVPFPNYRTS-GGCTNGRLSQEEKRMRNM
G.max_Glyma06g46590.1      PEEVKRHYELLLRDVRHIESGQVPFP-YKQNG------GS-QEEKRLRNM
G.max_Glyma04g16390.1      PEEVKRHYELLLRDVRYIESGKVPFP-YKQSG------GS-EEEKRLRNM
M.truncatula_AC135566      PEDVKRHYEHLLRDVRHIESGQVAFPNYKNIG------GY-DEEKRLRNL
G.max_Glyma02g18210.1      PDEVKSHYELLRDISQIESGKVPYPNYKKSA------EHDEEKRMRNL
M.truncatula_AC157502      PEEVKKHYELLVEDIKHIESGKVPFPNYKKIS-------VSHEEKRMRNM
G.max_Glyma03g28050.1      PEEVKRHYELLVQDVKHIESGRVPFPNYKKTTS----GSTDQEEKRLRNL
G.hirsutum_DR461014        VEEVKKHYELLLEDVRHIESGRVPFPDYWTVT----------GNRQA--
                            ::.*  :*:  *:  *.    *:.. *.  *  *
```

**FIGURE 2 (continued)**

```
M.guttatus_DV207881              -----
A.majus_TA4522_4151              IR---
V.vinifera_EE072107              LR---
G.hirsutum_TA31277_3635          -----
G.hirsutum_DW503354              -----
H.exilis_EE652824                -----
E.gunnii_CT985658                -----
P.trichocarpa_557252             -----
P.euphratica_AJ768574            -----
P.trichocarpa_sc_IV.1184         -----
L.serriola_DW122081              -----
L.virosa_TA3253_75947            -----
L.sativa_TA7946_4236             -----
C.intybus_EH706852               -----
M.domestica_DR994824             LKLH-
S.lycopersicum_TA46686           -----
N.benthamiana_EH366122           -----
T.salsuginea_DN773374            RLQ--
B.napus_BN06MC20309              KLQ--
A.thaliana_At2g21650             KLQ--
A.thaliana_At4g39250             RLQ--
G.max_Glyma06g46590.1            KLQ--
G.max_Glyma04g16390.1            KLQ--
M.truncatula_AC135566            KLQ--
G.max_Glyma02g18210.1            KIQ--
M.truncatula_AC157502            SLH--
G.max_Glyma03g28050.1            NLNLQ
G.hirsutum_DR461014              -----
```

# FIGURE 2 (continued)

**FIGURE 3**

FSM1

Terminator

RB

pGOS2

Plant expression vector
pGOS2::FSM1

Plant screenable
marker cassette

Bacterial origin of
replication

Plant selectable
marker cassette

LB

Bacterial selectable
marker

**FIGURE 4**

```
624028/1-50      IKGPWTKEEDQRVIELVQKYGP------------------------------------
CONSENSUS/80%    ttt.ao.pEpthhhphht.hs........................................
CONSENSUS/65%    ppstWotcEcphlhphhpphG........................................
CONSENSUS/50%    p+ssWTpEE-ctLlctlcpaG........................................


624028/1-50      ------------------------------------------------------------
CONSENSUS/80%    ............................................................
CONSENSUS/65%    ............................................................
CONSENSUS/50%    ............................................................


624028/1-50      --------------------------------------------KRWSLIAKHLK----
CONSENSUS/80%    ............................................tpat.lst.h......
CONSENSUS/65%    ............................................tpWptIupths....
CONSENSUS/50%    ............................................scWspIAcpls....


624028/1-50      ---------GRIGKQCRERWHNHLNP
CONSENSUS/80%    .........t+s..phh.ha.phht.
CONSENSUS/65%    .........sRosppspp+atphhpt
CONSENSUS/50%    .........sRosppl+p+apshhcp
```

wherein

| Class | Key | Residues |
|---|---|---|
| alcohol | o | S,T |
| aliphatic | l | I,L,V |
| any | . | A,C,D,E,F,G,H,I,K,L,M,N,P,Q,R,S,T,V,W,Y |
| aromatic | a | F,H,W,Y |
| charged | c | D,E,H,K,R |
| hydrophobic | h | A,C,F,G,H,I,K,L,M,R,T,V,W,Y |
| negative | - | D,E |
| polar | p | C,D,E,H,K,N,Q,R,S,T |
| positive | + | H,K,R |
| small | s | A,C,D,G,N,P,S,T,V |
| tiny | u | A,G,S |
| turnlike | t | A,C,D,E,G,H,K,N,Q,R,S,T |

# FIGURE 5

```
MNQFVPDWNTTSMGDGFAPLGEDDGLVELLWCNGHVVMQSQAPRKPPRPEKTTAAAAAA
                -------2----------
                ------3------


MAEDESASWFQYPVDDVLEKDLFTELFGEMTAAGGGGGDVRRAACKEERGAVAAFQSRM


MPPPWPARGKAEFGDVDDVCGVSEVVMAKMDGAAAAETVGESSMLTIGSSICGSNHVQT


PPVGNGKAGAGTAGAARRAHDTATVASSSMRSRSCTAKAEPRDVAAAGVGGKRKQRGGA


AMESGSPSEDVEFESAAATCSPAQKTTTAKRRRAAEVHNLSERRRRDRINEKMKALQEL
                                 ---------------------1------------
                                    ---4--- ----5------


IPHCNKTDKASMLDEAIEYLKSLQLQLQMMWMGGGMAPPAVMFPAAGVHQYMQRMGAVG
--------------1--------------------
        ----------6---------------


MGPPHMASLPRMPPFMAPPPAAVQSSPVVSMADPYARCLAVDHLQPPPPMHYLQGMSFY


QLAAAKNLQQQQNTAEAPPPPPAGGNRAAADS
```

# FIGURE 6

CLUSTAL 2.0.11 multiple sequence alignment


```
AT2G43010_1       --MEHQGWSFEENYSLS--TNRRSIR------------PQDELVELLWRDGQVVLQSQT
AT3G59060_1       MEQVFADWNFEDNFHMS--TNKRSIR------------PEDELVELLWRDGQVVLQSQA
Glyma08g41620_1   MNNSVPDWNFGSDSCVTSTNQKKPMMG-----------VDQELVELLWQNGQVV-HSQT
Glyma18g14530_1   MNNSVPDWNFGSDSCVTTTNQKKPMIG-----------VDQELVELQWQNGQVVMHSQT
Glyma02g45150_1   MNNSIPGWDFESDTCLT--NQRK-LIG-----------PDQELVELLWKNGQVVMHNQT
Pt_scaff_V_1141_1 MNHCIPDWNFEGDLPVS--NQKKPIELLILVAPSFTFCRPGNDLVELLWRNGQVVLHSQT
Pt_TC100024_1     MNHCIPDWNLEGDLPVS--NQKNPIE------------PDNELVELLWRNGQVVMHSQT
Sl_TC192046_1     ---MILNWFQLDGLFCR-------------------FDHELVELLWRNGEVVLHSQT
Os03g43810_1      MNQFVPDWSN--MGDASRTLGE-------------DDNLIELLWCNGHVVMQSQN
Os12g41650_2      MNQFVPDWNTTSMGDGFAPLGE-------------DDGLVELLWCNGHVVMQSQA
                          .*                              . *:** * :*.**::.*


AT2G43010_1       HREQTQTQ------KQDHHEEALR--SSTFLE-------------DQETVSWIQYPPDE
AT3G59060_1       RREPS-VQ------VQTHKQETLRKPNNIFLDNQETVQKPNYAALDDQETVSWIQYPPDD
Glyma08g41620_1   HRK-PVVN---------------------SSNLI---------QDDETVSWIQYPLED
Glyma18g14530_1   HRK-PFVNSITPRPLRRNFQSTLRTSEPFGNSSNLI---------QDDETVSWIQYPLED
Glyma02g45150_1   HRK-TLGNSSNLRQVQKSDQSVLRSSGPYGNSSNLD---------QED-AAPWVQFPLED
Pt_scaff_V_1141_1 HRK-PSPH------VQKHDSPTVKGSGSILNSSHLI---------QDDAVSWIQYPLED
Pt_TC100024_1     QRK-PNPH------VQKHDSPTVRGYDSTLNSSHLI---------QDDETVSWIHDPLED
Sl_TC192046_1     HKKQPGYDPNECRQFNKHDQPTIRVAG---NQTNLI--------QDDETVAWLNCPIDD
Os03g43810_1      HHRKLPPR-------------PPEKAAAAAV-------------QEDEAGLWFPFALAD
Os12g41650_2      PRKPPRPE-------------KTTAAAAAMA-------------EDESASWFQYPVDD
                  :.                                      :: :  *.   . :


AT2G43010_1       --DPFEPDDFSSHFFSTMDPLQRPTSETVKPKSSPEPPQVMVKPKACPDPPP--------
AT3G59060_1       VIDPFES-EFSSHFFSSIDHLGGPE----KPRTIEET----VKHEA--------------
Glyma08g41620_1   ---PLEQ-EFCSNLLSELAP---CEVESYKQIKPFEEG-KFTKLDASSAPHVTVSSQ---
Glyma18g14530_1   ---PLEQ-EFCSNLLSELAP---CEVESYKQIKPFEEG-KFTKLDASSTPH---------
Glyma02g45150_1   ---PLEQ-DFCSNLLSELPT---CEFESYKPIRQLEEE-KFAKFFASGTPHHPTTSSSQP
Pt_scaff_V_1141_1 ---SFEK-EFCSNFFSELPP---PLSD------QILEG-KSAKFDASAPSQQQHQHQHQR
Pt_TC100024_1     ---SFEK-EFCSNFFSELPP---PLSD------QISEE-KSAKFDASKTSHQ------QQ
Sl_TC192046_1     ---SFDK-EFCSPFLSDISTNPHLGEEPDKSIRQSEDNNKVFKFDPLEINHVLP------
Os03g43810_1      ---SLEK-DIFSDLFYEAPVAATAEAAPAGPGAGADGEGKTCKGDAAMAEEEERGGPG---
Os12g41650_2      ---VLEK-DLFTELFGEMTAAG---------GGGGDVRRAACK-------EERG------
                     ::   :: : ::                                *


AT2G43010_1       ----------------QVMPPPKFRLTNSSSGIRETEMEQYSVTTVGPSHCG-----SN
AT3G59060_1       ----------------QAMAPPKFRS--------------SVITVGPSHCG-----SN
Glyma08g41620_1   -LPTMKSSSFQDFSG--IPIPAPRFHVSDSPQKNNNHLGGSCKVQNFSHFSAPLN-VSSA
Glyma18g14530_1   ----------EFSG--IPIPAPRFHVSDSPQKNNNDLGGSCKVQNFSHFSAPLN-VSSA
Glyma02g45150_1   LPPNMKPSCIQGLQGNPIPMPAPRFHGPDSSQK-IHDFGASRKVLNFPQFSTPRNNVPSA
Pt_scaff_V_1141_1 QLNNNKPHVVSEFSG--NPMPPPRIQVPEKNHAGVGGFGEAVNAN-FSQFSAPFKGGDFR
Pt_TC100024_1     QLNNNKHPVVPEFPG--NPMPPPRIQVQEQNHISVGGFGKAVNVN-FSQFSAPLKVGDFR
Sl_TC192046_1     ------QSHHSGFDP--NPMPPPRFHNFGSAQQKHHIVGGDQKGVNFPPP--------IR
Os03g43810_1      ----------AASEAPRELMPPPKSTNASCSRQQTMSLADGGDNAGDLSELVRARRSSGG
Os12g41650_2      ----------AVAAFQSRMMPPP------WPARGKAEFGDVDDVCG-VSEVVMAKMDGAA
                                  :..*                       .
```


**FIGURE 7**

```
AT2G43010_1       PSQ-----NDLDVSMSHDRSKN-------------------------------------
AT3G59060_1       QSTNIHQATTLPVSMS-DRSKN-------------------------------------
Glyma08g41620_1   SANVHFGDK-ITGNMSRNEIRECSLMTVGSSYCGSNHIPQDPDASRASSNGVWTTT----
Glyma18g14530_1   SANAHFRDK-ITGNMSKNEIRD-------------NHIPQDPDASRASSNGVWTTT----
Glyma02g45150_1   PGITQFREK-TTANMSQSEAREYSVITVGSSHCGSNHIPQEQDVSRISSTGVWATTNNNT
Pt_scaff_V_1141_1 TSSGQFGGQ-GSGNSPQGEVRECSVA--------------------------------
Pt_TC100024_1     SSSRQFGGQ-GSGDFSQGEARECSVVTVGS----SNQTPRDRFLSRASGNAIETGTGLSA
Sl_TC192046_1     SSNVQLGGKEARSNLMLQDIKEGSVMTVGSSHCGSNQVDTSRFSSSAN-RGLSAAMITDY
Os03g43810_1      AARRKAEAGGGGGGGASSSMLSAIGSSICGSNQVQVQQRTASEPGRRGAPPSAVGSAN---
Os12g41650_2      AAETVGES---------SMLT-IGSSICGSNHVQT------------PPVGNGKAG---


AT2G43010_1       -----------------------IEEKLNPNASSSSGGSSGCSFGKDIKEMASGRCITT
AT3G59060_1       -----------------------VEERLD----TSSGGSSGCSYGRNNKETVSGTSVTI
Glyma08g41620_1   -LSVEPEAK-----------EKGKSEMLEPAVTSSSGGSGSS-LGKTC--SLSTRNQGQ
Glyma18g14530_1   -LSAEPEAN-----------EKGKSEMLEPTTTSSSGGSGSS-LGKNC--SLSTRNQGQ
Glyma02g45150_1   TLSAEPEAVRDYVQRPICPKSGQGKSEMIELTVTSSSGGSGSTGIGRTC--SLSTRDHGQ
Pt_scaff_V_1141_1 ----------------------------------------------------------
Pt_TC100024_1     GPSIDDPRK------VISQSERGKADQTLDPTATSSSGGSGGSFARTCK--QSAVPSRGQ
Sl_TC192046_1     TGKISPQSD------------TMDRDTFEPANTSSSSGRSGSSYARACNQSTATNSQGH
Os03g43810_1      --------------AIPCGGRDHGHGHEATTVASSSGRSNCCFGTTTTTTEPTSTSNRS
Os12g41650_2      --------------AGTAGAARR--AHDTATVASSSMRSRSCTAKAEPRDVAAAGVGG


AT2G43010_1       DRKRKRINHTD-ESVSLSDA---------IGNKSNQRSGSNRRSRAAEVHNLSERRRRDR
AT3G59060_1       DRKRKHVMDADQESVSQSDIGLTSTDDQTMGNKSSQRSGSTRRSRAAEVHNLSERRRRDR
Glyma08g41620_1   KRKGIDVEESEEQSEDTELKS-------ALGNKSSQRTGSARRNRAAEVHNLSERRRRDR
Glyma18g14530_1   KRKGIDVEESEEQSEDTELKS-------ALGNKSSQRAGLARRNRAAEVHNLSERRRRDR
Glyma02g45150_1   KRKGTEEEALEEQSEDTELKS-------ADGNKASQRTRSSRRNRAAEVHNQSERRRRDR
Pt_scaff_V_1141_1 -------------AELDLDS-------MAGNNPTKRSGSTRRSRAAEVHNLSERRRRDR
Pt_TC100024_1     KRKTMDAEESECQSEDAELDS------AVENKPAKRSGSTRRSRAAEVHNLSERRRRDR
Sl_TC192046_1     KRKSRDGEEPECQSKADELES-------AGGNKSAQKSGTARRSRAAEVHNLSERRRRDR
Os03g43810_1      SKRKRLDTTEDSESPSEDAES----ESAALARKPPAKMTTARRSRAAEVHNLSERRRRDR
Os12g41650_2      KRKQRGGAAMESGSPSEDVEF----ESAAATCSPAQKTTTAKRRRAAEVHNLSERRRRDR
                                   .          ..  :      :*  ******  *******


AT2G43010_1       INERMKALQELIPHCSKTDKASILDEAIDYLKSLQLQLQVMWMGSGMAAAAAS--APMMF
AT3G59060_1       INERMKALQELIPHCSRTDKASILDEAIDYLKSLQMQLQVMWMGSGMAAAAAAAASPMMF
Glyma08g41620_1   INEKMKALQQLIPHSSKTDKASMLEEAIEYLKSLQLQLQLMWMGSGMA--------PIMF
Glyma18g14530_1   INEKMKALQQLIPHSSKTDKASMLEEAIEYLKSLQLQLQLMWMGSGMA--------PIMF
Glyma02g45150_1   INEKMRTLQQLIPNSNKTDKASMLEEAIEYLKSLQFQLQVMWMGGGMT--------PVMF
Pt_scaff_V_1141_1 INEKMRALQELIPHCYKTDKASMLDEAIEYLKSLQLQLQVMWMGGGMA--------PMLF
Pt_TC100024_1     INEKMRALQELIPHCNKTDKASMLDEAIEYLKSLQLQLQVMWMGSGIV--------PVMF
Sl_TC192046_1     INEKMKALQELLPHSTKTDKASMLDEAIEYLKSLQMQLQMMWMGSGMA--------SMMF
Os03g43810_1      INEKMRALQELIPHCNKTDKASMLDEAIEYLKSLQLQLQMMWMGSGMAP-------PVMF
Os12g41650_2      INEKMKALQELIPHCNKTDKASMLDEAIEYLKSLQLQLQMMWMGGGMAPPA------VMF
                  ***:*::**:*:*:. :*****:*:***:******:***:****.*:.       ::*
```

**FIGURE 7 (continued)**

```
AT2G43010_1        PGVQPQQFIRQ---------------IQSPVQLPRFPVMDQSAIQNNPG------LVCQN
AT3G59060_1        PGVQSSPYINQMA-------------MQSQMQLSQFPVMNRSAPQNHPG------LVCQN
Glyma08g41620_1    PGIQ--HYMSQMGMG--MARPPFPPPIHNPMQLPRVP-LDKSVSASQTP---NQTLMCQN
Glyma18g14530_1    PGIQ--HYMSQMGMG--MATPPFPP-IHNPMQLPRLP-LDQSVSASQTP---NQTLMSQN
Glyma02g45150_1    PGIQ--HYMSQMGMG--MGAPSLPS-IHNPMQLPKVP-HDQAMSVLQIP---NQNLMCQN
Pt_scaff_V_1141_1  PGVQ--HFMSRMGMG-----PPLPS-MQNPMHLPRVQLIDQSISMAPTQ---NSGVMCQA
Pt_TC100024_1      PGVQ--HFMSRMGMGMGMGPPPLPS-MQNPMHLPRVPLVDQSISMAPTQ---NQAVICQT
Sl_TC192046_1      PGVQ--HYISRMGMG--MGPPSVPS-MHNAMHLARLPLVDPAIPLTQAAPNNQAAAMCQN
Os03g43810_1       P--GVHQYLPRMG---------VGMG-AAAAAMPRMP-FMAAPQPVVPT-----------
Os12g41650_2       PAAGVHQYMQRMGA--------VGMGPPHMASLPRMPPFMAPPPAAVQS-----------
                   *         ::  :                          :.:..        .


AT2G43010_1        PVQ----------NQIISDRFARYIGGFPHMQAA----TQMQPMEMLRFSSPAGQQSQ-Q
AT3G59060_1        PVQLQL----QAQNQILSEQLARYMGGIPQMPPAGNQTVQQQPADMLGFGSPAGPQSQ-L
Glyma08g41620_1    PILGAFNYQNQMQNPALSEQYVRYMG-YHLMQNAS------QPMNVFRYGPQGVQHSQTM
Glyma18g14530_1    PILGAFNYHNQMQNPALSEQYARYMG-YHLMQNAS------QPMNVFRYGPQGVRHSQTM
Glyma02g45150_1    PVLGAFNYQNQMQNPCLPEQYARYMG-YHLMQNAS------QPMNVFRYGSQAVQHSQTM
Pt_scaff_V_1141_1  PVLNPVNFHNQMQNPAFADQYARFMG-FH-MQAAS------QPMNMFRFGSQTVQQNQ-M
Pt_TC100024_1      PVLNPVNYQNQMQNPTFSDQYARFMG-FH-MQAAS------QPMNMFRFGSQTVQQNQ-M
Sl_TC192046_1      SMLNQVNYQRHLQNPNFPDQYASYMG-FHPLQGAS------QPINIFGLGSHTAQQTQQL
Os03g43810_1       -----------PPVNHLD------LGVNHLQPPP------TQGVGYYPLGAKAVQQQQNP
Os12g41650_2       -----------SPVVSMADPYARCLAVDHLQPPPP--MHYLQGMSFYQLAAAKNLQQQQ-
                            :       :.     .      *      ..   : *


AT2G43010_1        PSSVPTKTTDGSRLDH----------------------
AT3G59060_1        SAPATTDSLHMGKIG-----------------------
Glyma08g41620_1    ITPSSNSSGNMSG-AANIDEAVSGKMG-------------
Glyma18g14530_1    ITPS-NSSGNMSG-AANIDEAVSGKMDYIIIISFTLHNFY
Glyma02g45150_1    IAPGNNSSGPMSG-TANIDDADSGKAG-----SSTFN---
Pt_scaff_V_1141_1  MAPTSSVGGPLSAGTAVSDAPPSDKVG-------------
Pt_TC100024_1      MAPPNSGGGPLSAGTAASDAPPSGKTG-------------
Sl_TC192046_1      PHPTNSNAPAT---------------------------
Os03g43810_1       PLHVPNGSIMPPPENAPNTGSGMGSFYFYFYFSSD-----
Os12g41650_2       -----NTAEAPPPPPAGGNRAAADS---------------
```

**FIGURE 7 (continued)**

FIGURE 8

**FIGURE 9**

```
HEME1         1 MSLSSPT-SACSSSRCYSSGLSFPIGFGSNPINVGLVCYPKRYSIAARKFVVACS--SSS
Poplar        1 MNSASLS-SACSCLTWRTSSI-VPVQLGFN-------HSTKYKSSPRRFHIACSSASSS
HEME2         1 MSILQVSTSSLSSSTLLSISPRKSLSSTKS---------CRIVRCSVEGTTVTERKVSAT
Human_UROD    1 -------------------------------------------MEANGLGPQGFPEL

HEME1        58 SDPLLVKAAKCQAISRPPAWMMRQAGRYMAVYQKLAKKHPSFRERSENTDLIVEISLQPW
Poplar       51 SDPLLVKAAKGEPVSRPPAWMMRQAGRYMAVYRKLAEKYPSFRERSETTDLIVEISLQPW
HEME2        52 SEPLLLRAVKGEVVDRPPVWLMRQAGRYMKSYQTLCEKYPSFRDRSENADLVVEISLQPW
Human_UROD   15 KNDTFLRAAWGEETDYTPVWCMRQAGRYLPEERETRAAQDFFS-TCRSPEACCELTLQPL

HEME1       118 QAFRPDGVIIFSDILTPLPAFGVPFDIEEVKGPVIQSPIRTEEDMKRLHP--IDFEKLQF
Poplar      111 EAFHPDGVIIFSDILTPLPAFGVPFDIEEVRGPVIQSPIRSEEGLKALHP--IELEKLQF
HEME2       112 KVFKPDGVILFSDILTPLSGMNIPFDIVKGKGPIIFNPPQSAADVAQVREF-VPEESVPY
Human_UROD   74 RRFPLDAAIIFSDILVVPQALGMEVTMVPGKGPSFPEPLREEQDLERLRDPEVVASELGY

HEME1       176 VGDSLKILRREVGEHAAVLGFVGAPWTIATYIVEGGTTRTYTVIKNMCHTAPDVLRALLS
Poplar      169 VGDSLRILRNEVEGRAAVLGFVGAPWTIATYIVEGGTTRTYTNIKSMCHTAPQVLRALLS
HEME2       171 VGEALRRLRNEVNNEAAVLGFVGAPFTLSSYVIEGGSSKNFTQIKRLAFSQPKVLHALIQ
Human_UROD  134 VFQAITLTRGRLAGRVPLIGFAGAPWTLMTYMVEGGGSSTMAQAKRWLYQRPQASHQLLR

HEME1       236 HLTKAITEYVVYQVEHGAHCIQIFDSWGGQLIPEMWERWSKPYIEEIIHAVKKRCPDT--
Poplar      229 HLTKAMSDYIVFQVESGAHCIQIFDSWGGQLPPDMWDRWSKPYIEEIVSTVRNKCPET--
HEME2       231 KFTTSMITYIRYQADSGAQAVQIFDSWATELSPVDFEEFSLPYLKQIVEAVKQTHPNL--
Human_UROD  194 ILTDALVPYLVGQVVAGAQALQLFESHAGHLGPQLFNKFALPYIRDVAKQVKARLREAGL

HEME1       294 ---PIVFYINGNGGLLERMKGTGADVIGLDWTVDMADGRRRLGSEVSVQGNVDPAYLFSP
Poplar      287 ---PLVLYINGNGGLLERMKGTGVDVIGLDWTVDLADGRKRLGSGISVQGNVDPAYLFSP
HEME2       289 ---PLILYASGSGGLLERLARTGVDVVSLDWTVDMAEGRDRLGRDIAVQGNVDPGVLFGS
Human_UROD  254 APVPMIIFAKDGHFALEELAQAGYEVVGLDWTVAPKKARECVGKTVTLQGNLDPCALYAS

HEME1       351 LPALTEEIERVVKCAGPKGHILNLGHGVLVGTPEEAVAHFFETARNLDYQTLFQNHVPAE
Poplar      344 LPALTDEIQRVVECAGPRGHILNLGHGVLVGTPEEAVAHFFEVARSLKFTTP-QGHVVEE
HEME2       346 KEFITSRIHDTVKKAGRDKHILNLGHGIKVGTPEENVAHFFEVAQEIRY-----------
Human_UROD  314 EEEIGQLVKQMLDDFGPHRYIANLGHGLYPDMDPEHVGAFVDAVHKHSRLLRQN------

HEME1       411 KAEPELVV
Poplar      403 ---PKLVV
HEME2           --------
Human_UROD      --------
```

**FIGURE 10**

**FIGURE 11**

FIGURE 12

**FIGURE 13**

FIGURE 14

CLUSTAL 2.0.11 multiple sequence alignment

```
P.trichocarpa_657115#1_A          --MAMQAGVSVSRILILAGAGYTGTIMLKNGKLSELLGELQSLTKGMGKS
P.trichocarpa_657115#1.2_A        --MAMQAGVSVSRILILAGAGYTGTIMLKNGKLSELLGELQSLTKGMGKS
P.trichocarpa_566293#1_A          --MAMQAGIGVSRILILAGAGYTGTIMLKNGKLSELIAELQSLVKGMEKS
G.hirsutum_TC83567#1_A            --MAMQTGVGLSKILILAGAGYTGTVLLKNGKLSDILGELQSLVKGLEKS
B.napus_TC66611#1_A               --MAMQTGIGLSRIFLLAGAGCTGTIMMKNGKLSDILGELQSLVKGMERS
A.thaliana_AT1G27000.1#1_A        --MAMQAGVGLSRIFLLAGAGYTGTIMMKNGKLSDLLGELQSLVKGMEKS
C.sinensis_TC3201#1_A             --MAMQAGMSFQKILMLAGLGYTGTILIKDGKLPELLRELQSLVERLSKS
A.thaliana_AT2G02730.1#1_A        --MAMQSGIGLSKILILAGAGYTSTILVKNGKMADILGELQALVKRFEKS
M.domestica_TC12261#1_A           --MAMQSGIGVSKILILAGAGYTGTILLKNGKLSELLGELQSLLN---GS
M.domestica_TC9509#1_A            --MAMQSGIGFSKILILAGAGYTSTILLKNGKLSDLLGELQSLLN---GS
P.persica_TC1247#1_A              --MAMQSGIGFSKILILAGAGYTSTILLKNGKLSDLIGELQSLLN---GT
M.truncatula_AC136286_4.4#1_A     MAMAYHSGIGISKVLFIAVTGYTGTVLLKNGKLSDLIGDLQALVKGLEKS
                                    **  ::*:...:::::*   *  *.*:::*:**:.::: :**:*  :      :


P.trichocarpa_657115#1_A          GEQSDGDS-DYSDAIAQQVRRLAMEVRQLASAR--QITVLNGNSGQMGNL
P.trichocarpa_657115#1.2_A        GEQSDGDS-DYSDAIAQQVRRLAMEVRQLASAR--QITVLNGNSGQMGNL
P.trichocarpa_566293#1_A          GEKSDGDS-DYSDAIAQQVRRLAMEVRQLASAR--QITVLSGNPGQMGNL
G.hirsutum_TC83567#1_A            GEQADD-----SDALLAQVRRLSTEIRQLASAR--QITVLNGDSG--GKL
B.napus_TC66611#1_A               GE--EGDS-DVSDAIAAQVRRLAMEVRQLASAR--QITVMNGVSG--ANL
A.thaliana_AT1G27000.1#1_A        GEGSEGDS-DVSDAIAAQVRRLAMEIRQLASQQ--HITVMNGVSG--ANL
C.sinensis_TC3201#1_A             GE----QD-NFTDAIKDQLNRLKFEC-QRASSG--QIFVRNENSG--GNA
A.thaliana_AT2G02730.1#1_A        GDHVDDD----SDAMTTQMQRLAMEVRQLASSR--QITVMNGAQG--ADF
M.domestica_TC12261#1_A           GEHADGD----YDSLTAQISRMTAEIRQIGSSRGGGTVFVNGNGSQIGNM
M.domestica_TC9509#1_A            GEHTEGD----YDSLTAQISRLTAEIRQIGSSRGGSTVFVNGNGSQIGNM
P.persica_TC1247#1_A              GEQSEGD----FDAIASQVRRLAAEVRQLGSSR--QITVLNGNGSQIG-L
M.truncatula_AC136286_4.4#1_A     GDQAEGEGEHASDAIAAQIRRLANEVKHLSSNR--QTIVMNGGSGQSSNL
                                   *:          *::  *: *:  *   : .*        .   .   .


P.trichocarpa_657115#1_A          TGLIAPAATLGALGYGYMWWKGLKFSDFMYVTKRSMASAVSNLTKHLEQV
P.trichocarpa_657115#1.2_A        TGLIAPAATLGALGYGYMWWKGLKFSDFMYVTKRSMASAVSNLAKHLEQV
P.trichocarpa_566293#1_A          TGLIIPAATLGALGYGYMWWKGLKFSDLMYVTKRSMASAVSNLTKHLEQV
G.hirsutum_TC83567#1_A            TSLVVPAATLGALGYGYMWWKGISFSDLFWVTKRNMAMAVENLTKHLDSV
B.napus_TC66611#1_A               QALAVPAAALGALGYGYMWWKGLSFTDLMYVTKANMATAVANLTKNLEQV
A.thaliana_AT1G27000.1#1_A        QALAVPAAALGALGYGYMWWKGLSFTDLMYVTKANMAAAVANLTKNLEQV
C.sinensis_TC3201#1_A             TSLMIPAATLGALGYGYMWWKGLSFADLMYVTRKSMATAVSNLNKHLESV
A.thaliana_AT2G02730.1#1_A        TPFIVPAATLGALGYGYMWFKGISFSDIMCVTKRNMENAVSNLTKHLDTV
M.domestica_TC12261#1_A           TSLIMPAATLGAVGYGYMWWKGLKFSDMMYVTKRGMATAVENLTKNLDNV
M.domestica_TC9509#1_A            TSLIMPAATLGAVGYGYMWWKGLKLSDIMYVTKRGMATAVENLTKNLDNV
P.persica_TC1247#1_A              TSLIMPAATLGALGYGYMWWKGLKFSDLMYVTKRSMTAAVSNLHKHLESV
M.truncatula_AC136286_4.4#1_A     SSLVVPAATLGAVGYGYMWLKGVSFSDLMYVTKRNMENAVADLTKKLQHA
                                   :  ***:***:****** **:.::*:: **: .*   ** :* *:*: .


P.trichocarpa_657115#1_A          SEALSTAKTHLTQRIQLLDDKMESQKEISKAIQNDVNAASENLTQIGSEL
P.trichocarpa_657115#1.2_A        SEALSTAKTHLTQRIQLLDDKMESQKEISKAIQNDVNAASENLTQIGSEL
P.trichocarpa_566293#1_A          SEALSTAKTHLTQRIQHLDDKMESQKEISKAIQNDVNAASENLTLIGSEL
G.hirsutum_TC83567#1_A            SDALSAAKKHLTQRIQNLDDKMETQKEISKSIQTSVEEAHMNLSNIEYDL
B.napus_TC66611#1_A               SATLAAAKRHLTQKIQNMDDKVEKQIDLSKEIKNQVTLARGDINLLENEL
A.thaliana_AT1G27000.1#1_A        SETLAAAKRHLTQRIQNLDDKVEKQIDLSKEINSQVISARENISSLEMDL
C.sinensis_TC3201#1_A             TEALTVAKKHLTQRIQNLNDKVEKQNEISKDIRKNVEEACDDLFKVEHNL
A.thaliana_AT2G02730.1#1_A        SEAILNAKKHLSQRLQKVDDKLDLQKDLLKGVQDNVGLALEDLANIGDDF
M.domestica_TC12261#1_A           KEAVAKAKKHLTQRIHHVDDKMAEQNELSRSINEDVAGVQHSLTDIDYDL
M.domestica_TC9509#1_A            KEAVAKAKKHLTQRIQHVDDKMVEQNELSRSIKEDVAGVQHSLTDIDYDL
P.persica_TC1247#1_A              TEAIANTKKHLTQRVQNLDDKLLEQKEISKSILENVVIRKEVLRNFTLP-
M.truncatula_AC136286_4.4#1_A     SDVIADAKKHLTQRIQILDDKMRKQYKMAQSIKDDVNKVQDTVTTIHDDL
                                   . .:  :*  ** **:*::: ::**:  *  .: :  :  .*     :  .
```

**FIGURE 15**

```
P.trichocarpa_657115#1_A        WQLQCLVSGLDGKIGSLEEKQDIANMGVMYLCNFVGGKKAKMPKALE---
P.trichocarpa_657115#1.2_A      WQLQCLVSGLDGKIGSLEEKQDIANMGVMYLCNFVGGKKAKMPKALE---
P.trichocarpa_566293#1_A        WQLQCLVSGLDGKIGSLEEKQDIANMGVMYLCNFVGGKKAKMPKALE---
G.hirsutum_TC83567#1_A          DALQRMISGLDGKIGSLEYKQDLANAGVWYLCNMVGGKKANMPEALQ---
B.napus_TC66611#1_A             QSLNDLISGLDGKLDTLEYKQDVTNVCMLHLYNYFGGKSTKLPDMEQ---
A.thaliana_AT1G27000.1#1_A      ESLHNLITGLDGKLDTLEYKQDVTNVFMLNLYNYFGGKSTKLPEMEQ---
C.sinensis_TC3201#1_A           KDLQSMIYCLDGKIDSLADKQDITNIGMYLLCNFVDGE------------
A.thaliana_AT2G02730.1#1_A      DAMHSIFGGMGGKLDSIEYKQNIANMGLIYLCDSLGGENHKMPDILM---
M.domestica_TC12261#1_A         SRLQSMVSGLDHKMGSLEQKQDLANLGVILPGQFCPWKKS----------
M.domestica_TC9509#1_A          SRLQIMVSGLDHKIGSLEHKQDLANLGVIYLVNFVDGKKVEMPKTLQ---
P.persica_TC1247#1_A            --------------------------------------------------
M.truncatula_AC136286_4.4#1_A   SVVQHTVKMLDGRLNSVLENQEFANMGLDYIIGFITANARRVPDSLQGTI


P.trichocarpa_657115#1_A        --DQFKPSGRTRASLMYSEVPSLTGLKELADDLSQTFGEPATDAILQDGT
P.trichocarpa_657115#1.2_A      --DQFKPSGRTRASLMYSEVPSLTGLKELADDLSQTFGEPATDAILQDGT
P.trichocarpa_566293#1_A        --DQLKPSGRTRASLAYAEVPSLTGLKELADDLSQTFSKPATDATLQDGT
G.hirsutum_TC83567#1_A          --EQLKLSGKSRA-LLASGTPALKGLKDVADISSGNMNDSGRDGFVKDGF
B.napus_TC66611#1_A             --LQLPVNQKARNLLGDVGTKGLKNFAEQLLISNDTEGGATTVRRIGITR
A.thaliana_AT1G27000.1#1_A      --LQLPVNQRARNLLADVETKGLKNLAEELFKSNGTQ-VTTTVKQISLSK
C.sinensis_TC3201#1_A           ---------KRETN------------------------------------
A.thaliana_AT2G02730.1#1_A      --QEKLRLSGKSNTCIVLTTEETS--------SSEGLKESDKIQLLDDC-
M.domestica_TC12261#1_A         --------------------------------------------------
M.domestica_TC9509#1_A          --KQLENPGKSRGRLLSYSDTSGLMGLKEIADSLSETLNPSTDAIVKDDI
P.persica_TC1247#1_A            --------------------------------------------------
M.truncatula_AC136286_4.4#1_A   PKEQPKLPGRSTSMLTYPGFQGLKDIAESLSSLDRSASDTIVPGGLDN--


P.trichocarpa_657115#1_A        DNLEDQPRIPRIDQPRALLRFNSARC
P.trichocarpa_657115#1.2_A      DNLEDQPRIPRIDQPRALLRFNSARC
P.trichocarpa_566293#1_A        DNLEDQLRTPRNDQPRALLRFNSARC
G.hirsutum_TC83567#1_A          -------------------------
B.napus_TC66611#1_A             ANDKSGPLLSRVASAGC---------
A.thaliana_AT1G27000.1#1_A      VNVKSRPLLSRAASAKC---------
C.sinensis_TC3201#1_A           -------------------------
A.thaliana_AT2G02730.1#1_A      -------------------------
M.domestica_TC12261#1_A         -------------------------
M.domestica_TC9509#1_A          EMTGEYRKNNLIRSVSTRC-------
P.persica_TC1247#1_A            -------------------------
M.truncatula_AC136286_4.4#1_A   -LEKQRRPLLRATSTKC---------
```

# FIGURE 15 (continued)

FIGURE 16

**FIGURE 17**

## XPG-N

```
                                                                                    D78
                                                                                     |
OsEXO1   1  MGIQGLLPQLKSIMAPIGVEALKGQTVAVDTYSWLHKGALSCGDRLCKGLPTTRHIEYC---MHRVNMLRHHGVK-PILVFDGGHLPMKGDQE 89
AtEXO1a  1  MGIKDLLRFMKPYILPIHIQKYAGKRVGIDAYSWLHKGAYSCSMELCLDTDGKKKLRYIDYFMHRVNLLQHYEII-PVVVLDGGNMPCKAATG 93
AtEXO1b  1  MGIQGLLPLLKSIMVPIHIKELEGCIVAVDTYSWLHKGALSCSRELCKGLPTKRHIQYC---MHRVNLLRHHGVK-PIMVFDGGPLPMKLEQE 89
HEX1     1  MGIQGLLQFIKEASEPIHVRKYKGQVVAVDTYCWLHKGAIACAEKLAKGEPTDRYVGFC---MKFVNMLLSHGIK-PILVFDGCTLPSKKEVE 89
MmEXO1   1  MGIQGLLQFIQEASEPVNVKKYKGQAVAVDTYCWLHKGAIACAEKLAKGEPTDRYVGFC---MKFVNMLLSYGVK-PILIFDGCTLPSKKEVE 89
DmEXO1   1  MGITGLIPFVGKASSQLHLKDIRGSTVAVDTYCWLHKGVFGCAEKLARGEDTDVYIQYC---LKYVNMLLSYDIK-PILVFDGQHLPAKALTE 89
ScEXO1   1  MGIQGLLPQLKPIQNPVSLRRYEGEVLAIDGYAWLHRAACSCAYELAMGKPTDKYLQFF---IKRF-SLLKTFKVEPYLVFDGDAIPVKKSTE 89
SpEXO1   1  MGIKGLLGLLKPMQKSSHVEEFSGKTLGVDGYVWLHKAVFTCAHELAFNKETDKYLKYA---IHQALMLQYYGVK-PLIVFDGGPLPCKASTE 89
            ***..*.    .    . .     *  ...*.* ***...  *.   *. . ...  ..    .  ...*   . . *....** .* *   .
```

## XPG-I

```
                                                      D173
                                                       |
OsEXO1   129  IAFELIQVLKQEKVDYIVAPYEADAQMTFLSVNKLVD----AVITEDSDLIPFGCSRIIFKMDKFGQGVEFHITRLQRCRELDLNGF
AtEXO1a  132  MAHQLIQVLKSENVEFIVAPYEADAQLAYLSSLELEQGGIAAVITEDSDLLAYGCKAVIFKMDRYGKGEELVLDNVFQAVDQKPSFQ
AtEXO1b  129  IAHELIQVLRQENVDYVVAPYEADAQMAFLAITKQVD----AIITEDSDLIPFGCLRIIFKMDKFGHGVEFQASKLPKNKDLSLSGF
HEX1     129  MAHKVIKAARSQGVDCLVAPYEADAQLAYLNKAGIVQ----AIITEDSDLLAFGCKKVILKMDQFGNGLEIDQARLGMCRQLGDVFT
MmEXO1   129  MAHKVIKAARALGVDCLVAPYEADAQLAYLNKAGIVQ----AVITEDSDLLAFGCKKVILKMDQFGNGLEVDQARLGMCKQLGDVFT
DmEXO1   129  MALRLIRECRSRNVDCIVAPYEADAQMAWLNRADVAQ----YIITEDSDLTLFGAKNIIFKLDLNGSGLLVEAEKLHLAMGCTEEKY
ScEXO1   129  MAKCIICYCKLNGIRYIVAPFEADSQMVYLEQKNIVQ----GIISEDSDLLVFGCRRLITKLNDYGECLEICRDNFIKLPKKFPLGS
SpEXO1   129  MAWKLIIALREHGIESIVAPYEADAQLVYLEKENIID----GIITEDSDMLVFGAQTVLFKMDGFGNCITIRRNDIANAQDLNLRLP
              .*. .*   ..   ... .***.***.*...*      ...   ..*.****.. .*.  ...*... *  . .       .  .  .
```

```
              D226
               |
OsEXO1   TMQMLLE--M-CILSGCDYL  228
AtEXO1a  NFDQELFTAM-CVLAGCDFL  237
AtEXO1b  SSQMLLE--M-CILSGCDYL  228
HEX1     EEKFRY---M-CILSGCDYL  227
MmEXO1   EEKFRY---M-CILSGCDYL  227
DmEXO1   HFDKFRR--M-CILSGCDYL  228
ScEXO1   LTNEEIIT-MVC-LSGCDYT  229
SpEXO1   IEKLRH---MA-IFSGCDYT  227
            *  ....***..
```

# FIGURE 18

**FIGURE 19**

**FIGURE 20**

FIGURE 21

```
                                        1                                              50
         A.thaliana_AT1G16060.1#1   (1) MFIAVEVSPVMEDITRQSKKTSVEN---------ETGDDQSATS-VVLKA
         C.clementina_DY290073#1    (1) ----------MAKTSRQSQKNTTTNPNN--------------NNTVTTKT
           C.sinensis_EY655317#1    (1) ----------MAKTSRQSQKNTTTNPNN--------------NNTVTTKT
            G.max_Glyma07g02380.1#1 (1) ----------------------------------MAKKSQKS-LNSTG
         C.clementina_DY288437#1    (1) ----------MAKTSRQSQKNTTTNPNN--------------NNTVTTKT
         C.clementina_DY301305#1    (1) ----------MAKTSRQSQKNTTTNPNN--------------NNTVTTKT
           C.clementina_TC1922#1    (1) ----------MAKTSRQSQKNTTTNPNN--------------NNTVTTKT
           C.clementina_TC2567#1    (1) ----------MAKTSRQSQKNTTTNPNN--------------NNTVTTKT
            C.sinensis_EY726128#1    (1) ----------MAKTSRQSQKNTTTNPNN--------------NNTVTTKT
             C.sinensis_TC9216#1    (1) ----------MAKTSR-QSQK-----NTTTN--------PNNNNTVTTKT
          G.max_Glyma17g07860.1#1   (1) ----------MAKKSQLRTQKNNVTTNDDNN--------LNVTNTVTTKV
      M.truncatula_AC126784_13.5#1  (1) ----------MAKKSQKQIEKDDNASNDNDN--------LNPSNTVTTKA
                        Medtr_AP2   (1) ----------MAKKSQKQIEKDDNASNDNDN--------LNPSNTVTTKA
      O.sativa_LOC_Os08g34360.1#1   (1) ----------MAKRRSNGETAAASSDDSSSG---VCGGGGGGEVEPRRRQ
      O.sativa_LOC_Os09g25600.1#1   (1) ----------MAKPRKNSTTTNTSSSG-----------VAAAAAAAVKP
         S.bicolor_Sb02g025080.1#1  (1) ----------MAKPRKNSAAANNNNSS---------SNGAGDLTPRAKP
               Z.mays_TC386652#1    (1) ----------MARPRKNSAAAANNNNSNTTNA-----GNAAVDLAARVKP
       O.basilicum_TA1087_39350#1   (1) ----------MGRSCKQKK---------------TSNSNNAVDVGVNKS
         Triphysaria_sp_TC12598#1   (1) ----------MAKSSEQNNS--------------LATSTAAEAPVVIKKA
          P.trichocarpa_800184#1    (1) ----------MGKTSKQ----------------SLKNSANTSINPATKV
           S.tuberosum_TC187592#1   (1) ----------MAKTSKSNTTATSSSSSSSNKCDSKAKRSNKIDSNAIGKV
          Aquilegia_sp_TC20979#1    (1) ----------MAKISQQNQKITVNNI--------KTNDETIT------KV
            G.hirsutum_TC125414#1   (1) ----------MTKLSQVNHKNSAQSG--------SVNNNISTSNDVTKVK
    V.vinifera_GSVIVT00017130001#1  (1) ----------MAKLSQQNHKNSANSN--------ATNTTLSVT-----KV
            R.communis_EG658396#1   (1) ----------MAKISQQRQRNNSNNT--------NTSAKTGTNPSST-KA
          R.communis_TA2948_3988#1  (1) ----------MAKISQQRQRNNSNNT--------NTSAKTGTNPSST-KA
                       Consensus    (1)          MAK SRQ  K     N               TVTTK


                                        51                                             100
         A.thaliana_AT1G16060.1#1   (41) KRKRRSQPRDAPPQRSSVHRGVTRHRWTGRYEAHLWDKNSWNETQTKKGR
         C.clementina_DY290073#1    (27) KRTRKSVPRDSPPQRSSIYRGVTRHRWTGRYEAHLWDKNCWNESQNKKGR
           C.sinensis_EY655317#1    (27) KRTRKSVPRDSPPQRSSIYRGVTRHRWTGRYEAHLWDKNCWNESQNKKGR
            G.max_Glyma07g02380.1#1 (14) KRTRKSVPRDSPPQRSSIYRGVTRHRWTGRYEAHLWDKNCWNESQSKKGR
         C.clementina_DY288437#1    (27) KRTRKSVPRDSPPQRSSIYRGVTRHRWTGRYEAHLWDKNCWNESQNKKGR
         C.clementina_DY301305#1    (27) KRTRKSVPRDSPPQRSSIYRGVTRHRWTGRYEAHLWDKNCWNESQNKKGR
           C.clementina_TC1922#1    (27) KRTRKSVPRDSPPQRSSIYRGVTRHRWTGRYEAHLWDKNCWNESQNKKGR
           C.clementina_TC2567#1    (27) KRTRKSVPRDSPPQRSSIYRGVTRHRWTGRYEAHLWDKNCWNESQNKKGR
            C.sinensis_EY726128#1    (27) KRTRKSVPRDSPPQRSSIYRGVTRHRWTGRYEAHLWDKNCWNESQNKKGR
             C.sinensis_TC9216#1    (27) KRTRKSVPRDSPPQRSSIYRGVTRHRWTGRYEAHLWDKNCWNESQNKKGR
          G.max_Glyma17g07860.1#1   (33) KRTRRSVPRDSPPQRSSIYRGVTRHRWTGRYEAHLWDKHCWNESQNKKGR
      M.truncatula_AC126784_13.5#1  (33) KRTRKSVPRTSPPQRSSIYRGVTRHRWTGRYEAHLWDKNCWNESQNKKGR
                        Medtr_AP2   (33) KRTRKSVPRTSPPQRSSIYRGVTRHRWTGRYEAHLWDKNCWNESQNKKGR
      O.sativa_LOC_Os08g34360.1#1   (38) KRPRRSAPRDCPSQRSSAFRGVTRHRWTGRFEAHLWDKNTWNESQSKKGR
      O.sativa_LOC_Os09g25600.1#1   (30) KRTRKSVPRESPSQRSSVYRGVTRHRWTGRFEAHLWDKNSWNESQNKKGK
         S.bicolor_Sb02g025080.1#1  (31) KRTRKSVPRESPTQRSSVYRGVTRHRWTGRFEAHLWDKNSWNESQNKKGK
               Z.mays_TC386652#1    (36) KRTRKSVPRESPSQRSSVYRGVTRHRWTGRFEAHLWDKNSWNESQNKKGK
       O.basilicum_TA1087_39350#1   (25) TRTRKSIPRESPPQRSSVYRGVTRHRWTGRYEAHLWDKNCWNETQNKKGR
         Triphysaria_sp_TC12598#1   (27) KRTRKSIPRESPRRRSSIFRGVTRHRWTGRYEAHLWDKNSWNESQSKKGR
          P.trichocarpa_800184#1    (24) KRTRKSVPRDSPPQRSSIYRGVTRHRWTGRYEAHLWDKNCWNESQNKKGR
           S.tuberosum_TC187592#1   (41) KRTRKSVPRDSPPQRSSIYRGVTRHRWTGRYEAHLWDKNCWNETQNKKGR
          Aquilegia_sp_TC20979#1    (27) KRKRKSVPRDSPPQRSSIFRGVTRHRWTGRYEAHLWDKSCWNESQKKGR
            G.hirsutum_TC125414#1   (33) KRTRRSFPRDSPPQRSSIYRGVTRHRWTGRYEAHLWDKNCWNESQNKKGR
    V.vinifera_GSVIVT00017130001#1  (28) KRTRKSVPRDSPPQRSSIYRGVTRHRWTGRYEAHLWDKNCWNESQNKKGR
            R.communis_EG658396#1   (32) KRTRKTVPRDSPPQRSSIYRGVTRHRWTGRYEAHLWDKNCWNESQNKKGR
          R.communis_TA2948_3988#1  (32) KRTRKTVPRDSPPQRSSIYRGVTRHRWTGRYEAHLWDKNCWNESQNKKGR
                       Consensus    (51) KRTRKSVPRDSPPQRSSIYRGVTRHRWTGRYEAHLWDKNCWNESQNKKGR
```

**FIGURE 22**

```
                                      101                                              150
         A.thaliana_AT1G16060.1#1  (91)  QVYLGAYDEEDAAARAYDLAALKYWGRDTILNFPLCNYEEDIKEMESQSK
          C.clementina_DY290073#1  (77)  QVYLGAYDNEEAAARAYDLAALKYWGHDTILNFPLSNYEEELVEMEGQSK
            C.sinensis_EY655317#1  (77)  QVYLGAYDNEEAAARAYDLAALKYWGHDTILNFPLSNYEEELVEMEGQSK
           G.max_Glyma07g02380.1#1  (64)  Q---GAYDDEEAAARAYDLAALKYWGQDTILNFPLSNYEEKLKEMEGQSK
          C.clementina_DY288437#1  (77)  QVYLGAYDNEEAAARAYDLAALKYWGHDTILNFPLSNYEEELVEMEGQSK
          C.clementina_DY301305#1  (77)  QVYLGAYDNEEAAARAYDLAALKYWGHDTILNFPLSNYEEELVEMEGQSK
           C.clementina_TC1922#1  (77)  QVYLGAYDNEEAAARAYDLAALKYWGHDTILNFPLSNYEEELVEMEGQSK
           C.clementina_TC2567#1  (77)  QVYLGAYDNEEAAARAYDLAALKYWGHDTILNFPLSNYEEELVEMEGQSK
            C.sinensis_EY726128#1  (77)  QVYLGAYDNEEAAARAYDLAALKYWGHDTILNFPLSNYEEELVEMEGQSK
             C.sinensis_TC9216#1  (77)  QVYLGAYDNEEAAARAYDLAALKYWGHDTILNFPLSNYEEELVEMEGQSK
          G.max_Glyma17g07860.1#1  (83)  Q---GAYDNEEAAAHAYDLAALKYWGQDTILNFPLSNYLNELKEMEGQSR
    M.truncatula_AC126784_13.5#1  (83)  Q---GAYDNEETAAHAYDLAALKYWGQDTIINFPLSNYQKELIEMESQSR
                        Medtr_AP2  (83)  Q---GAYDNEETAAHAYDLAALKYWGQDTIINFPLSNYQKELIEMESQSR
       O.sativa_LOC_Os08g34360.1#1  (88)  QVYLGAYDGEEAAARAYDLAALKYWGHDTVLNFPLSTYDEELKEMEGQSR
       O.sativa_LOC_Os09g25600.1#1  (80)  QVYLGAYDDEEAAARAYDLAALKYWGPDTILNFPLSAYEGELKEMEGQSR
        S.bicolor_Sb02g025080.1#1  (81)  QVYLGAYDDEEAAARAYDLAALKYWGPDTILNFPASAYEGEMKGMEGQSR
              Z.mays_TC386652#1  (86)  QVYLGAYDDEDAAARAYDLAALKYWGPDTILNFPASAYEAELKEMEGQSR
         O.basilicum_TA1087_39350#1  (75)  QVYLGAYDDEEAAAHAYDLAALKYWGQDTVLNFPLSTYQKQLEEMEGQSK
          Triphysaria_sp_TC12598#1  (77)  QVYLGAYDEEEAAARAYDLAALKYWGQDTILNFPLSSYEEELNEMEGQSK
            P.trichocarpa_800184#1  (74)  Q---GAYDDEEAAGHAYDLAALKYWGQDTILNFPLSTYEEEFKEMEGHSK
             S.tuberosum_TC187592#1  (91)  QVYLGAYDDEEAAAHAYDLAALKYWGQDTVLNFPVATYENELKEMEGQSK
            Aquilegia_sp_TC20979#1  (77)  QVYLGAYNDEEAAAHTYDLAALKYWGPETILNFPVSTYQGELEEMESQSR
             G.hirsutum_TC125414#1  (83)  QVYLGAYDDEESAAHAYDLAALKYWGQDTILNFPVSTYQKELKEMENQSR
     V.vinifera_GSVIVT00017130001#1  (78)  QVYLGAYHDEEAAAHAYDLAALKYWGPETILNFPLSTYEKELKEMEGLSR
            R.communis_EG658396#1  (82)  QVYLGAYDDEEAAAHAYDLAALKYWGQDTILNFPLSTYEEELKEMEGQSK
           R.communis_TA2948_3988#1  (82)  QVYLGAYDDEEAAAHAYDLAALKYWGQDTILNFPLSTYEEELKEMEGQSK
                         Consensus (101)  QVYLGAYDDEEAAARAYDLAALKYWG DTILNFPLSNYEEELKEMEGQSK


                                      151                                              200
         A.thaliana_AT1G16060.1#1 (141)  EEYIGSLRRKSSGFSRGVSKYRGVAKHHHNGRWEARIGRVFGNKYLYLGT
          C.clementina_DY290073#1 (127)  EEYIGSLRRKSSGFSRGVSKYRGVARHHHNGRWEARIGRVFGNKYLYLGT
            C.sinensis_EY655317#1 (127)  EEYIGSLRRKSSGFSRGVSKYRGVARHHHNGRWEARIGRVFGNKYLYHGT
           G.max_Glyma07g02380.1#1 (111)  EEYIGSLRRKSSGFSRGVSKYRGVARHHHNGRWEARIGRVFGNKYLYLGT
          C.clementina_DY288437#1 (127)  EEYIGSLRRKSSGFSRGVSKYRGVARHHHNGRWEARIGRVFGNKYLYLGT
          C.clementina_DY301305#1 (127)  EEYIGSLRRKSSGFSRGVSKYRGVARHHHNGRWEARIGRVFGNKYLYLGT
           C.clementina_TC1922#1 (127)  EEYIGSLRRKSSGFSRGVSKYRGVARHHHNGRWEARIGRVFGNKYLYLGT
           C.clementina_TC2567#1 (127)  EEYIGSLRRKSSGFSRGVSKYRGVARHHHNGRWEARIGRVFGNKYLYLGT
            C.sinensis_EY726128#1 (127)  EEYIGSLRRKSSGFSRGVSKYRGVARHHHNGRWEARIGRVFWHKYLYLGT
             C.sinensis_TC9216#1 (127)  EEYIGSLRRKSSGFSRGVSKYRGVARHHHNGRWEARIGRVFGNKYLYLGT
          G.max_Glyma17g07860.1#1 (130)  EEYIGSLRRKSSGFSRGISKYRGVARHHHNGRWEARIGKVFGNKYLYLGT
    M.truncatula_AC126784_13.5#1 (130)  EEYIGSLRRKSSGFSRGVSKYRGVARHHHNGRWEARIGKVFGNKYLYLGT
                        Medtr_AP2 (130)  EEYIGSLRRKSSGFSRGVSKYRGVARHHHDGRWEARIGKVFGNKYLYLGT
       O.sativa_LOC_Os08g34360.1#1 (138)  EEYIGSLRRKSSGFSRGVSKYRGVARHHHNGKWEARIGRVFGNKYLYLGT
       O.sativa_LOC_Os09g25600.1#1 (130)  EEYIGSLRRKSSGFSRGVSKYRGVARHHHNGRWEARIGRVFGNKYLYLGT
        S.bicolor_Sb02g025080.1#1 (131)  EEYIGSLRRKSSGFSRGVSKYRGVARHHHNGRWEARIGRVFGNKYLYLGT
              Z.mays_TC386652#1 (136)  EEYIGSLRRKSSGFSRGVSKYRGVARHHHNGRWEARIGRVFGNKYLYLGT
         O.basilicum_TA1087_39350#1 (125)  EEYIRSLRRKSSGFSRGVSKYRGVARHHHNGRWEARIGRVFGNKYLYLGT
          Triphysaria_sp_TC12598#1 (127)  EEYIGSLRGKSSGFSRGVSKYRGVVRHHHNGRWEARIGRVFGNKYLYLGT
            P.trichocarpa_800184#1 (121)  EEYIGSLRRKSSGFSRGVSKYRGVARHHHNGRWEARIGRVFGNKYLYLGT
             S.tuberosum_TC187592#1 (141)  EEYIGSLRRKSSGFSRGVSKYRGVARHHHNGRWEARIGRVFGNKYLYLGT
            Aquilegia_sp_TC20979#1 (127)  EEYIGSLRRKSSGFSRGVSKYRGVARHHHNGRWEARIGRVFGNKYLYLGT
             G.hirsutum_TC125414#1 (133)  EEYIGSLRRKSSGFSRGVSKYRGVARHHHNGRWEARIGRVFGNKYLYLGT
     V.vinifera_GSVIVT00017130001#1 (128)  EEYIGSLRRKSSGFSRGVSKYRGVARHHHNGRWEARIGRVFGNKYLYLGT
            R.communis_EG658396#1 (132)  EEYIGSLRRKSSGFSRGVSKYRGVARHHHNGRWEARIGRVFGNKYLYLGT
           R.communis_TA2948_3988#1 (132)  EEYIGSLRRKSSGFSRGVSKYRGVARHHHNGRWEARIGRVFGNKYLYLGT
                         Consensus (151)  EEYIGSLRRKSSGFSRGVSKYRGVARHHHNGRWEARIGRVFGNKYLYLGT
```

**FIGURE 22 (continued)**

```
                                            201                                            250
          A.thaliana_AT1G16060.1#1   (191) YATQEEAAIAYDIAAIEYRGLNAVTNFDISRYLKLPVPEN---------P
          C.clementina_DY290073#1    (177) YATQEEAAQAYDRAAIEYRGLNAVTNFDLSNTLSGYAQIII--------K
            C.sinensis_EY655317#1    (177) YATQEEAVSAYDRANIEYRGLNAGPTLILSNTYSGYAPLII--------K
            G.max_Glyma07g02380.1#1  (161) YATQEEAAAAYDMAAIEYRGLNAVTNFDLSRYINWPRPK-----------
          C.clementina_DY288437#1    (177) YATQEEAAQAYDRAAIEYRELNAVTNFDLSKYIKWLRPNNN--------Q
          C.clementina_DY301305#1    (177) YATQEEAAQAYDRAAIEYRGLNAVTNFDLSKYIKWLRPNNN--------Q
            C.clementina_TC1922#1    (177) YATQEEAAQAYDRAAIEYRGLNAVTNFDLSKYIKWLRPNNN--------Q
            C.clementina_TC2567#1    (177) YATQEEAAQAYDRAAIEYRGLNAVTNFDLSKYIKWLRPNNN--------Q
            C.sinensis_EY726128#1    (177) YATQEEAAPAYDAAIEYRGLNGVTTLDLNKYIKGLRPNKN--------Q
            C.sinensis_TC9216#1      (177) YATQEEAAQAYDRAAIEYRGLNAVTNFDLSKYIKWLRPNNN------QNN
            G.max_Glyma17g07860.1#1  (180) YATQEEATAYDLAAIEYRGLNAVTNFDLSRYININLTNIN------NNN
          M.truncatula_AC126784_13.5#1 (180) YATQEEATAYDMAAIEYRGLNAVTNFDLSRYIKWLKPNNN------NND
                          Medtr_AP2    (180) YATQEEATAYDMAAIEYRGLNAVTNFDLSRYIKWLKPNNN------NND
          O.sativa_LOC_Os08g34360.1#1 (188) YATQEEAVAYDIAAIEHRGLNAVTNFDINLYIRWYHGSCRSSS-AAAAT
          O.sativa_LOC_Os09g25600.1#1 (180) YATQEEAMAYDMAAIEYRGLNAVTNFDLSRYIKWLRPGADG----AGAP
          S.bicolor_Sb02g025080.1#1   (181) YATQEEAMAYDMAAIEYRGLNAVTNFDLSRYIKWLRPGAGGMAAAAAAA
              Z.mays_TC386652#1      (186) YGTQEEAMAYDMAAIEYRGLNAVTNFDLSRYIKWLRPGAG-------AA
          O.basilicum_TA1087_39350#1  (175) FATQEEAATAYDMAAIEYRGLNAVTNFDLSRYIKCFPPLQNKS-----KN
          Triphysaria_sp_TC12598#1    (177) YATQEEAARAYDMAAVKYRGHNAVTNFDLSRYAEWLGPLSHNS-----DN
          P.trichocarpa_800184#1      (171) YATQEEAATAYDMAAIEYRGLNAVTNFDLSRYS----------------
          S.tuberosum_TC187592#1      (191) YATQEEAATAYDMAAIEYRGLNAVTNFDLSRYIKWLRPS---------D
          Aquilegia_sp_TC20979#1      (177) YATQEEAATAYDMAAIEYRGLNAVTNFDLSRYIKWLRPNPTD-------S
          G.hirsutum_TC125414#1       (183) YATQEEAATAYDMAAIEYRGLNAVTNFDLSRYIKWLKPN-----------
      V.vinifera_GSVIVT00017130001#1  (178) YATQEEAATAYDMAAIEYRGLNAVTNFDLSRYIKWLKPNQNNP-----CE
          R.communis_EG658396#1       (182) YATQEEAATAYDMAAIEYRGLNAVTNFDLSRYIKWLRPN----------N
          R.communis_TA2948_3988#1    (182) YATQEEAATAYDMAAIEYRGLNAVTNFDLSRYIKWLRPN----------N
                          Consensus    (201) YATQEEAA AYDMAAIEYRGLNAVTNFDLSRYIKWLRPN

                                            251                                            300
          A.thaliana_AT1G16060.1#1   (232) IDTANNL-------LESPHS------DLSPF------IKPNHESDLSQ--
          C.clementina_DY290073#1    (219) ITLNLATSTNP---------------------------------------
            C.sinensis_EY655317#1    (219) ITPNLATLNKNLIVMDTVQ--------ILNLNQGTSSGSDPTRPHTSCWG
            G.max_Glyma07g02380.1#1  (200) -----------------------------------TEENHQTIPS---
          C.clementina_DY288437#1    (219) NNPKTSNPQTKP--------------------------------------
          C.clementina_DY301305#1    (219) NNPKPSNPQQNPNSDDTSP--------IPKLNQETSSGSDTSAPPPRSGA
            C.clementina_TC1922#1    (219) NNPKPSNPQQNPNSDDTSP--------IPKTKSRNLKR-----------
            C.clementina_TC2567#1    (219) NNPKPSNPQQNPNSDDTSP--------IPKLNQETSSGSDTSAPPPRSGA
            C.sinensis_EY726128#1    (219) NNPKPYQPSTKP--------------------------------------
            C.sinensis_TC9216#1      (221) P------------------------------------------------
            G.max_Glyma17g07860.1#1  (224) N---------CTNSFTPSPDQ----EQEASFFHNKDSLNNTIVEEVTLVP
          M.truncatula_AC126784_13.5#1 (224) DNNKSNINLCDINSNSSANDSNSNEELEFSLVDNEISLNNSIDEATLVQP
                          Medtr_AP2    (224) DNNKSNINLCDINSNSSANDSNSNEELEFSLVDNEISLNNSIDEATLVQP
          O.sativa_LOC_Os08g34360.1#1 (237) TIEDDDFAEAIAAALQGVDEQPSSSPATTRQLQTADDDDDDLVAQLPPQL
          O.sativa_LOC_Os09g25600.1#1 (226) QNPHPMLGALSAQDLPAID-----LDAMASSFQHDGHGAAAAAAQLIPAR
          S.bicolor_Sb02g025080.1#1   (231) QNPHPMLGGLAQQLLLPPPADTTTTDGAGAAAFQHDHH--GAEAFPLPPR
              Z.mays_TC386652#1      (229) QNPHPMLDGLAQQLLLSP--EG-TIDGAAFHQQQHDHRQQGAAELPLPPR
          O.basilicum_TA1087_39350#1  (220) PKPIANG-VKESNSMQNPNQ----DINEGEVTSPTQTATLEASDATS---
          Triphysaria_sp_TC12598#1    (222) PQLTPRGNMDINNTFQDASQ----EIGLTFLGQQPESPILADSTSPSPPH
          P.trichocarpa_800184#1      (204) -------------------------------------------------
          S.tuberosum_TC187592#1      (231) QTNDNTI---N-NPEPNPNPI---DIHLMPTNLTQQQSSGCDVTVAAPPP
          Aquilegia_sp_TC20979#1      (220) KTNNSSP---DSHLAATPTE----DIGIGILNHQTSITSKMVVGQPRPTG
          G.hirsutum_TC125414#1       (222) --------------PNP-----------------------TLPPLTLI
      V.vinifera_GSVIVT00017130001#1  (223) QPNNPNL---DSNLTPNPNH----DFGISFLNHPQTSGTAACSEPPLTQT
          R.communis_EG658396#1       (222) PQENPTS---DANPMPTLLK----ILD----------------------
          R.communis_TA2948_3988#1    (222) PQENPTS---DANPMPNPTQ----DLGLTFTAHHQSSTATAETTMPLPTC
                          Consensus    (251) N              N
```

**FIGURE 22 (continued)**

```
                                         301                                                   350
         A.thaliana_AT1G16060.1#1  (261)  --------SQSSSEDNDDRKTKLLKSSPLVAEEVIGPSTPP---------
          C.clementina_DY290073#1  (230)  --------------------------------------------------
            C.sinensis_EY655317#1  (261)  YRRR----------------------------------------------
           G.max_Glyma07g02380.1#1 (210)  -----------NENVNSNAELELGSASDEITEEGVAPSS-----------
          C.clementina_DY288437#1  (231)  --------------------------------------------------
          C.clementina_DY301305#1  (261)  TAGGSGSASSALGLLLQSSKYKERVEKTFTRLFIYVIRTWVFPTGI----
           C.clementina_TC1922#1   (249)  --------------------------------------------------
           C.clementina_TC2567#1   (261)  TAGGSGSASSALGLLLQSSKYKEMVEKTSTDCLSTSS-------------
            C.sinensis_EY726128#1  (231)  --------------------------------------------------
             C.sinensis_TC9216#1   (222)  --------------------------------------------------
           G.max_Glyma17g07860.1#1 (261)  HQPRPASATSALELLLQSSKFKEMMEMTSVANLSSTQMES----------
      M.truncatula_AC126784_13.5#1 (274)  R---PTSATSALELLLQSSKFKEMVEMASMTSNVSTTLESD---------
                        Medtr_AP2   (274)  R---PTSATSALELLLQSSKFKEMVEMASMTSNVSTTLESD---------
       O.sativa_LOC_Os08g34360.1#1 (287)  RPLARAASTSPIGLLLRSPKFKEIIEQAAAAAASSSGSSSSSSTDSPSSS
       O.sativa_LOC_Os09g25600.1#1 (271)  HSLGHTPTTSALSLLLQSPKFKEMIERTSAAETTTTSSTTTSSSSP----
         S.bicolor_Sb02g025080.1#1 (279)  TSLGHTPTTSALSLLLQSPKFKEMIQRTESGTTTTTTTTSSLSSSP----
                   Z.mays_TC386652#1 (276)  ASLGHTPTTSALGLLLQSSKFKEMIQRASAAESGTTTVTTTSSSSSQ---
         O.basilicum_TA1087_39350#1 (262)  ---------STLGILLQ--------------PSSKNTSSP----------
          Triphysaria_sp_TC12598#1 (268)  L-SDGATASSALGVLLNSYMFKEMMDQRVVASPSSPSEPEP---------
           P.trichocarpa_800184#1  (204)  -----------------SKFKEMLERT-SASDCPLTPPES----------
           S.tuberosum_TC187592#1  (274)  GCGGAATSSAALELLMQSTKFKEMLERTSEVIECPETPPEP---------
           Aquilegia_sp_TC20979#1  (263)  G----SSASSALGLLFQSSKFKEMLERTSSVDYPYPSTPPE---------
             G.hirsutum_TC125414#1  (233)  --------------------------------------------------
     V.vinifera_GSVIVT00017130001#1 (266)  R---PPIASSALGLLLQSSKFKEMMEMTTAADH--LSTPPE---------
            R.communis_EG658396#1  (242)  --------------------------------------------------
          R.communis_TA2948_3988#1 (265)  GGGGGGGSASSALGLLLQSTKFKEMLERTSANDC--PMTPPE--------
                         Consensus  (301)            SSAL LLLQS KFKEMIE


                                         351                                                   400
         A.thaliana_AT1G16060.1#1  (294)  --------------------------------EIAPPRRSFPEDIQTYFG
          C.clementina_DY290073#1  (230)  --------------------------------------------------
            C.sinensis_EY655317#1  (265)  --------------------------------------------------
           G.max_Glyma07g02380.1#1 (238)  --------------------------------ESNPSRRTFPEDIQTIFE
          C.clementina_DY288437#1  (231)  --------------------------------------------------
          C.clementina_DY301305#1  (307)  --------------------FLKIFRRGFFQLFGTLAALPGRVEDVFI
           C.clementina_TC1922#1   (249)  --------------------------------------------------
           C.clementina_TC2567#1   (298)  --------------------------------EPGSSHRIFPDDIQTMFF
            C.sinensis_EY726128#1  (231)  --------------------------------------------------
             C.sinensis_TC9216#1   (222)  --------------------------------------------------
           G.max_Glyma17g07860.1#1 (301)  --------------------------------ELPQCTFPDHIQTYFE
      M.truncatula_AC126784_13.5#1 (312)  --------------------------------QLSQCAFPDDIQTYFE
                        Medtr_AP2   (312)  --------------------------------QLSQCAFPDDIQTYFE
       O.sativa_LOC_Os08g34360.1#1 (337)  SSSSLSPSPLPSPPPQQQPTVPKDDQYNVDMSSVAAARCSFPDDVQTYFG
       O.sativa_LOC_Os09g25600.1#1 (317)  --------------------S--PPQATKDDGASPQCSFPEDIQTYFG
         S.bicolor_Sb02g025080.1#1 (325)  -------PPTPSPSPPRRSPAPTQPPVQAAARDASPHQRGFPEDVQTFFG
                   Z.mays_TC386652#1 (323)  -------PPTPTPTP---SPSPPPTPPVQPARDASP-QCSFPDDTFFG
         O.basilicum_TA1087_39350#1 (279)  --------------------------------P---RSSFPDDIQTSFE
          Triphysaria_sp_TC12598#1 (308)  --------------------------------KPGPQRSSFPDDIQTSFE
           P.trichocarpa_800184#1  (226)  --------------------------------DRDPPRRSFPDDIQTYFD
           S.tuberosum_TC187592#1  (315)  --------------------------------DRP--RRSFPDDIQTYFD
           Aquilegia_sp_TC20979#1  (300)  --------------------------------SDPP-RCSFPEEIQTYFE
             G.hirsutum_TC125414#1  (233)  --------------------------------------------------
     V.vinifera_GSVIVT00017130001#1 (302)  --------------------------------SELP-RCSFPDDIQTYFE
            R.communis_EG658396#1  (242)  --------------------------------------------------
          R.communis_TA2948_3988#1 (304)  --------------------------------SEPP-RRSFPDDIQTFFD
                         Consensus  (351)                                  SFPDDIQTYF
```

# FIGURE 22 (continued)

```
                                             401                                         438
          A.thaliana_AT1G16060.1#1    (312)  CQNSGK----LTAEEDDVIFGDLDSFLTPDFYSELNDC
          C.clementina_DY290073#1     (230)  --------------------------------------
            C.sinensis_EY655317#1     (265)  --------------------------------------
          G.max_Glyma07g02380.1#1     (256)  NQDSG-----IYTENDDIIFGDLGSFGAPIFHYELDV-
          C.clementina_DY288437#1     (231)  --------------------------------------
          C.clementina_DY301305#1     (335)  GVDPKSHYIFPQLFLRWKLGINLKKQKPWEPPHGKEK-
            C.clementina_TC1922#1     (249)  --------------------------------------
            C.clementina_TC2567#1     (316)  DCQDSSSYTEG---------------------------
             C.sinensis_EY726128#1    (231)  --------------------------------------
              C.sinensis_TC9216#1     (222)  --------------------------------------
          G.max_Glyma17g07860.1#1     (317)  YEDSNRYEEGDDLMFKFNEFSSIVPFYQCDEFES----
    M.truncatula_AC126784_13.5#1      (328)  YENFN--------DTMLEDLNSIMPTFHYDFEGAEVL-
                       Medtr_AP2      (328)  YENFN--------DTMLEDLNSIMPTFHYDFEGAEVL-
      O.sativa_LOC_Os08g34360.1#1     (387)  LDDDG----FGYPEVDTFLFGDLGAYAAPMFQFELDV-
      O.sativa_LOC_Os09g25600.1#1     (343)  CAAEDGAAGAGYADVDGLFFGDLAAYASPAFHFELDL-
        S.bicolor_Sb02g025080.1#1     (368)  CEDTA------GIDVEALFFGDLAAYATPAFHFEMDL-
                  Z.mays_TC386652#1   (362)  CEDVAGVG--AGVDVDALFFGDLAAYASPAFHFEMDL-
        O.basilicum_TA1087_39350#1    (293)  IRDSS-----NFEEEHGIVFGEYDSFASPMFQYELETN
            Triphysaria_sp_TC12598#1  (326)  FQDS-------FVEEHDVIFGNYDSFVSSAFQCGLDA-
           P.trichocarpa_800184#1     (244)  CQDSS-----SYTDGDDIIFGDLHSFASPIFHCELDG-
           S.tuberosum_TC187592#1     (331)  CQESS-----SFIEEHDIIFGDLDSLTLPMFQCELIN-
           Aquilegia_sp_TC20979#1     (317)  CQDSE-----SYVEGNDIIFCDLNTFSSPMFQHELET-
            G.hirsutum_TC125414#1     (233)  --------------------------------------
V.vinifera_GSVIVT00017130001#1        (319)  CQDSG-----SYEEGDDVIFSELNSFIPPMFQCDFSA-
          R.communis_EG658396#1       (242)  --------------------------------------
          R.communis_TA2948_3988#1    (321)  CQDTG-----SFTEGDDIIFGELDAFASPIFHCELND-
                        Consensus     (401)  D         E     F L F    F
```

### FIGURE 22 (continued)

FIGURE 23

Europäisches
Patentamt

European
Patent Office

Office européen
des brevets

# EUROPEAN SEARCH REPORT

Application Number

EP 15 16 2086

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | H. GAO ET AL: "A membrane-tethered transcription factor defines a branch of the heat stress response in Arabidopsis thaliana", PROCEEDINGS OF THE NATIONAL ACADEMY OF SCIENCES, vol. 105, no. 42, 21 October 2008 (2008-10-21), pages 16398-16403, XP055206957, ISSN: 0027-8424, DOI: 10.1073/pnas.0808463105 * page 16401, last paragraph - page 16402, paragraph 1 * | 1,3,5, 8-12,14, 16-19 | INV. C07K14/415 C12N15/82 |
| A | WO 2007/099096 A1 (CROPDESIGN NV [BE]; FRANKARD VALERIE [BE]) 7 September 2007 (2007-09-07) | 1-20 | |
| A | WO 2009/000789 A1 (CROPDESIGN NV [BE]; BRUCE WESLEY [US]) 31 December 2008 (2008-12-31) | 1-20 | TECHNICAL FIELDS SEARCHED (IPC) |
| A | CHEN Y N ET AL: "Membrane-tethered transcription factors in Arabidopsis thaliana: novel regulators in stress response and development", CURRENT OPINION IN PLANT BIOLOGY, QUADRANT SUBSCRIPTION SERVICES, GB, vol. 11, no. 6, 1 December 2008 (2008-12-01), pages 695-701, XP025685974, ISSN: 1369-5266, DOI: 10.1016/J.PBI.2008.10.005 [retrieved on 2008-11-18] | 1-20 | C07K C12N |

The present search report has been drawn up for all claims

| Place of search | Date of completion of the search | Examiner |
|---|---|---|
| Munich | 10 August 2015 | Kania, Thomas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 15 16 2086

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2015

| Patent document cited in search report | | Publication date | Patent family member(s) | | Publication date |
|---|---|---|---|---|---|
| WO 2007099096 | A1 | 07-09-2007 | AR | 059650 A1 | 16-04-2008 |
| | | | CA | 2642964 A1 | 07-09-2007 |
| | | | EP | 1991683 A1 | 19-11-2008 |
| | | | US | 2009019606 A1 | 15-01-2009 |
| | | | WO | 2007099096 A1 | 07-09-2007 |
| WO 2009000789 | A1 | 31-12-2008 | AR | 067138 A1 | 30-09-2009 |
| | | | WO | 2009000789 A1 | 31-12-2008 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007144190 A
- US 5811238 A
- US 6395547 B
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A
- WO 0015815 A
- WO 9322443 A, Zarling

- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- EP 1198985 A1
- WO 2007093444 A
- US 5164310 A
- US 5159135 A

**Non-patent literature cited in the description**

- **WANG et al.** *Planta,* 2003, vol. 218, 1-14
- **BARG et al.** *Planta,* 2005, vol. 221, 197-211
- **CORLEY et al.** *Proc. Natl. Acad. Sci.,* 2005, vol. 102, 5068-5073
- **BAXTER et al.** *Plant J.,* 2007, vol. 52, 105-113
- **CLACK et al.** *Plant Cell,* March 2009, vol. 21 (3), 786-99
- **CASTILLON et al.** *Trands in Plant Science,* November 2007, vol. 12 (11), 514-521
- **MARTINS et al.** *The Journal of Biological Chemistry,* 23 November 2001, vol. 276 (47), 44108-44116
- **MOHANTY et al.** *Planta,* 2006, vol. 224, 692-699
- **MOCK ; GRIMM.** *Plant Physiol.,* 1997, vol. 113, 1101-1112
- **SEO et al.** *Trends Plant Sci.,* 2008, vol. 13 (10), 550-556
- **CHEN et al.** *Curr Opin Plant Biol.,* 2008, vol. 11 (6), 695-701
- **GIL-HUMANES et al.** *BMC Plant Biol.,* 2009, vol. 9 (1), 66
- **KIM et al.** *Mol Biol Evol.,* January 2006, vol. 23 (1), 107-20
- **TERPE.** *Appl. Microbiol. Biotechnol.,* 2003, vol. 60, 523-533
- **SCHULTZ et al.** *Proc. Natl. Acad. Sci. USA,* 1998, vol. 95, 5857-5864

- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2002, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** *BMC Bioinformatics,* 10 July 2003, vol. 4, 29
- **SMITH TF ; WATERMAN MS.** *J. Mol. Biol,* 1981, vol. 147 (1), 195-7
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press

- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **KOYAMA et al.** *J Biosci Bioeng.,* January 2005, vol. 99 (1), 38-42
- **MUDGE et al.** *Plant J.,* 2002, vol. 31, 341
- **XIAO et al.** *Plant Biol (Stuttg).,* July 2006, vol. 8 (4), 439-49
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* vol. 17 (6), 1139-1154
- **DOWNEY et al.** *J. Biol. Chem.,* 2000, vol. 275, 39420
- **W SONG.** *PhD Thesis, North Carolina State University,* 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270

- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **FUKAVAMA et al.** *Plant Physiol.,* November 2001, vol. 127 (3), 1136-46
- **KAUSCH et al.** *Plant Mol Biol.,* January 2001, vol. 45 (1), 1-15
- **LIN et al.** *DNA Seq,* August 2004, vol. 15 (4), 269-76
- **NOMURA et al.** *Plant Mol Biol,* September 2000, vol. 44 (1), 99-106
- **WAGNER ; KOHORN.** *Plant Cell,* 2001, vol. 13 (2), 303-318
- **TRIBBLE et al.** *J. Biol. Chem.,* 2000, vol. 275, 22255-22267
- **VELMURUGAN et al.** *J. Cell Biol.,* 2000, vol. 149, 553-566
- **BUCHMAN ; BERG.** *Mol. Cell biol.,* vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 2001, vol. 15, 6131-6148
- **INOUE et al.** *FEBS Lett,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants. Academic Press, 1993, vol. 1, 15-38
- **FELDMAN, KA ; MARKS MD.** *Mol Gen Genet,* 1987, vol. 208, 1-9
- **FELDMANN K.** Methods in Arabidopsis Research. Word Scientific, Singapore, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199

- **CLOUGH, SJ ; BENT AF.** *The Plant J,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol,* 2003, vol. 21, 20-28
- **HAYASHI et al.** *Science,* 1992, 1350-1353
- **REDEI GP ; KONCZ C.** Methods in Arabidopsis Research. Singapore, World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet,* 1991, vol. 7, 149-154
- **LAAN et al.** *Genome Res,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **BAILEY ; ELKAN.** Proceedings of the Second International Conference on Intelligent Systems for Molecular Biology. AAAI Press, 1994, 28-36
- **R.D. FINN ; J. TATE ; J. MISTRY ; P.C. COGGILL ; J.S. SAMMUT ; H.R. HOTZ ; G. CERIC ; K. FORSLUND ; S.R. EDDY ; E.L. SONNHAMMER.** *Nucleic Acids Research,* vol. 36, D281-D288
- **R.D. FINN ; J. TATE ; J. MISTRY ; P.C. COGGILL ; J.S. SAMMUT ; H.R. HOTZ ; G. CERIC ; K. FORSLUND ; S.R. EDDY ; E.L. SONNHAMMER.** *Nucleic Acids Research,* 2008, vol. 36, D281-D288
- **BAILEY et al.** *Nucleic Acids Research,* 2006, vol. 34, W369-W373
- **LETUNIC et al.** *Nucleic Acids Res,* 2006, vol. 34, D257-D260
- **OHME-TAKAGI ; SHINSHI.** *Plant Cell,* 1995, vol. 7, 173-182
- **LIU et al.** *FEBS Lett,* 2006, vol. 580 (5), 1303-8
- **YOH-SAKUMAA.** *Biochemical and Biophysical Research Communications,* 2002, vol. 290, 998-1009
- **MIDDLETON et al.** *Plant Cell,* 2007, vol. 19, 1221-1234
- **KIZIS ; PAGES.** *Plant J.,* 2002, vol. 30 (6), 679-89
- **JACOBS ; JACOBS.** *Plant Physiol.,* 1993, vol. 101 (4), 1181-1187
- **IVANETICH et al.** *Clin Chem.,* 1984, vol. 30 (3), 391-4
- **MARTINS et al.** *J Biol Chem.,* 2001, vol. 276 (47), 44108-44116
- Current Protocols in Molecular Biology
- **KATOH ; TOH.** *Briefings in Bioinformatics,* 2008, vol. 9, 286-298
- **HOUWE et al.** *Bioinformatics,* 2002, vol. 18 (11), 1546-7
- *Bioinformatics,* 2002, vol. 18 (11), 1546-7
- **HUDSON et al.** *Bioinformatics,* 2007, vol. 8 (1), 460
- **SAMBROOK.** Molecular Cloning: a laboratory manual. Cold Spring Harbor Laboratory Press, 2001
- Current Protocols in Molecular Biology. **AUSUBEL et al.** Current Protocols. 1994, vol. 1, 2
- **R.D.D. CROY.** Plant Molecular Biology Labfax. BIOS Scientific Publications Ltd, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500

- **HOWE et al.** *Bioinformatics,* 2002, vol. 18 (11), 1546-7
- **HUSON et al.** *BMC Bioinformatics,* 2007, vol. 8 (1), 460
- **CAMPANELLA JJ ; BITINCKA L ; SMALLEY J.** MatGAT: an application that generates similarity/identity matrices using protein or DNA sequences. *BMC Bioinformatics,* 2003, vol. 4, 29
- **PARK ; KANEHISA.** *Bioinformatics,* 2003, vol. 19, 1656-1663
- **PARK ; KANEHISA.** *Bioinformatics,* 2003, vol. 19, 1656-1663, Bioinformatics
- **AUSUBEL et al.** Current Protocols in Molecular Biology. Current Protocols, 1994, vol. 1, 2
- **LI ; PARISH.** *Plant J.,* 1995, vol. 8, 963-972
- **CLACK et al.** *Plant Cell.,* March 2009, vol. 21 (3), 786-99
- **AL-SADY et al.** *Molecular Cell,* 04 August 2006, vol. 23 (3), 439-46
- **FURUKAWA et al.** *Plant Mol Biol,* 2008, vol. 66, 519-531
- **FURUKAWA et al.** *Plant Molecular Biology,* 2003, vol. 51, 59-70
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158